# EUROPEAN PATENT SPECIFICATION

(11) **EP 3 162 896 B1**
(45) Date of publication and mention of the grant of the patent: **13.05.2020**
(21) Application number: 15814080.6
(22) Date of filing: 26.06.2015
(51) Int. Cl.: C12N 15/09, C12N 1/19, C12P 7/46, C12N 9/04, C12N 9/88, C12N 9/00

(54) **TRANSFORMANT, METHOD FOR PRODUCING SAME, AND METHOD FOR PRODUCING C4-DICARBOXYLIC ACID**
TRANSFORMANT, VERFAHREN ZUR HERSTELLUNG DAVON UND VERFAHREN ZUR HERSTELLUNG VON C4-DICARBONSÄURE
TRANSFORMANT, SON PROCÉDÉ DE PRODUCTION ET PROCÉDÉ DE PRODUCTION D'ACIDE CARBOXYLIQUE EN C4

(30) Priority: 30.06.2014 JP 2014135043
(43) Date of publication of application: 03.05.2017
(73) Proprietor: JMTC Enzyme Corporation, Tokyo 104-0061 (JP)
(72) Inventor: HARA Futoshi, Tokyo 100-8405 (JP); KIMURA Shuichiro, Tokyo 100-8405 (JP); KOTANI Tetsuya, Tokyo 100-8405 (JP); TANAKA Takayuki, Tokyo 100-8405 (JP)
(74) Representative: Lavoix
(86) International application number: PCT/JP2015/068589
(87) International publication number: WO 2016/002680

(56) References cited:
- EP-A1- 2 495 304
- WO-A1-2007/061590
- WO-A1-2008/144626
- WO-A1-2009/065780
- US-A1- 2013 302 866
- ZELLE R ET AL: "Malic acid production by Saccharomyces cerevisiae: engineering of pyruvate carboxylation, oxaloacetate reduction, and malate export", APPLIED AND ENVIRONMENTAL MICROBIOLOGY,, vol. 74, no. 9, 1 May 2008 (2008-05-01), pages 2766-2777, XP008095574, ISSN: 0099-2240, DOI: 10.1128/AEM.02591-71
- GUOQIANG XU ET AL: "Reconstruction of cytosolic fumaric acid biosynthetic pathways in Saccharomyces cerevisiae", MICROBIAL CELL FACTORIES,, vol. 11, no. 1, 15 February 2012 (2012-02-15), page 24, XP021125148, ISSN: 1475-2859, DOI: 10.1186/1475-2859-11-24
- R.E. SUBDEN ET AL: "Mutational analysis of malate pathways in Schizosaccharomyces pombe", FOOD RESEARCH INTERNATIONAL, vol. 31, no. 1, 1 January 1998 (1998-01-01), pages 37-42, XP055429325, AMSTERDAM, NL ISSN: 0963-9969, DOI: 10.1016/S0963-9969(98)00056-8
- DATABASE [Online] 14 May 2014 'RecName: Full=Phosphoenolpyruvate carboxykinase [ATP].', XP055379136 Retrieved from NCBI Database accession no. Q6FRR0
- LANGELANDSVIK A.S. ET AL.: 'Properties and primary structure of a thermostable L-malate dehydrogenase from Archaeoglobus fulgidus.' ARCH. MICROBIOL. vol. 168, no. 1, 1997, pages 59 - 67, XP055251157
- VILJOEN M. ET AL.: 'Transcriptional regulation of the Schizosaccharomyces pombe malic enzyme gene , mae2.' J. BIOL. CHEM. vol. 274, no. 15, 1999, ISSN 0021-9258 pages 9969 - 9975, XP055251156

### SEQUENCE LISTING

<110> ASAHI GLASS COMPANY, LIMITED
<120> TRANSFORMANT AND PROCESS FOR PRODUCTION THEREOF, AND PROCESS FOR
   PRODUCTION OF C4 DICARBOXYLIC ACID
<130> F20150192
<150> JP2014-135043
   <151> 2014-06-30
<160> 162
<210> 1
   <211> 1192
   <212> PRT
   <213> Aspergillus niger
<220>
   <223> AniPYC
<400> 1
   Met Ala Ala Pro Arg Gln Pro Glu Glu Ala Val Asp Asp Thr Glu Phe
                   5                   10                  15
   Ile Asp Asp His His Asp Gln His Arg Asp Ser Val His Thr Arg Leu
               20                  25                  30
   Arg Ala Asn Ser Ala Ile Met Gln Phe Gln Lys Ile Leu Val Ala Asn
           35                  40                  45
   Arg Gly Glu Ile Pro Ile Arg Ile Phe Arg Thr Ala His Glu Leu Ser
       50                  55                  60
   Leu Gln Thr Val Ala Val Tyr Ser His Glu Asp Arg Leu Ser Met His
   65                  70                  75                  80
   Arg Gln Lys Ala Asp Glu Ala Tyr Met Ile Gly Lys Arg Gly Gln Tyr
                   85                  90                  95
   Thr Pro Val Gly Ala Tyr Leu Ala Ile Asp Glu Ile Val Lys Ile Ala
               100                 105                 110
   Leu Glu His Gly Val His Leu Ile His Pro Gly Tyr Gly Phe Leu Ser
           115                 120                 125
   Glu Asn Ala Glu Phe Ala Arg Lys Val Glu Gln Ser Gly Met Val Phe
       130                 135                 140
   Val Gly Pro Thr Pro Gln Thr Ile Glu Ser Leu Gly Asp Lys Val Ser
   145                 150                 155                 160
   Ala Arg Gln Leu Ala Ile Arg Cys Asp Val Pro Val Val Pro Gly Thr
                   165                 170                 175
   Pro Gly Pro Val Glu Arg Tyr Glu Glu Val Lys Ala Phe Thr Asp Thr
               180                 185                 190
   Tyr Gly Phe Pro Ile Ile Ile Lys Ala Ala Phe Gly Gly Gly Gly Arg
           195                 200                 205
   Gly Met Arg Val Val Arg Asp Gln Ala Glu Leu Arg Asp Ser Phe Glu
       210                 215                 220
   Arg Ala Thr Ser Glu Ala Arg Ser Ala Phe Gly Asn Gly Thr Val Phe
   225                 230                 235                 240
   Val Glu Arg Phe Leu Asp Arg Pro Lys His Ile Glu Val Gln Leu Leu
                   245                 250                 255
   Gly Asp Asn His Gly Asn Val Val His Leu Phe Glu Arg Asp Cys Ser
               260                 265                 270
   Val Gln Arg Arg His Gln Lys Val Val Glu Ile Ala Pro Ala Lys Asp
           275                 280                 285
   Leu Pro Ala Asp Val Arg Asp Arg Ile Leu Ala Asp Ala Val Lys Leu
       290                 295                 300
   Ala Lys Ser Val Asn Tyr Arg Asn Ala Gly Thr Ala Glu Phe Leu Val
   305                 310                 315                 320
   Asp Gln Gln Asn Arg Tyr Tyr Phe Ile Glu Ile Asn Pro Arg Ile Gln
                       325                 330                 335
   Val Glu His Thr Ile Thr Glu Glu Ile Thr Gly Ile Asp Ile Val Ala
               340                 345                 350
   Ala Gln Ile Gln Ile Ala Ala Gly Ala Thr Leu Glu Gln Leu Gly Leu
           355                 360                 365
   Thr Gln Asp Arg Ile Ser Thr Arg Gly Phe Ala Ile Gln Cys Arg Ile
       370                 375                 380
   Thr Thr Glu Asp Pro Ser Lys Gly Phe Ser Pro Asp Thr Gly Lys Ile
   385                 390                 395                 400
   Glu Val Tyr Arg Ser Ala Gly Gly Asn Gly Val Arg Leu Asp Gly Gly
                   405                 410                 415
   Asn Gly Phe Ala Gly Ala Ile Ile Thr Pro His Tyr Asp Ser Met Leu
               420                 425                 430
   Val Lys Cys Thr Cys Arg Gly Ser Thr Tyr Glu Ile Ala Arg Arg Lys
           435                 440                 445
   Val Val Arg Ala Leu Val Glu Phe Arg Ile Arg Gly Val Lys Thr Asn
       450                 455                 460
   Ile Pro Phe Leu Thr Ser Leu Leu Ser His Pro Val Phe Val Asp Gly
   465                 470                 475                 480
   Thr Cys Trp Thr Thr Phe Ile Asp Asp Thr Pro Glu Leu Phe Ala Leu
                   485                 490                 495
   Val Gly Ser Gln Asn Arg Ala Gln Lys Leu Leu Ala Tyr Leu Gly Asp
               500                 505                 510
   Val Ala Val Asn Gly Ser Ser Ile Lys Gly Gln Ile Gly Glu Pro Lys
           515                 520                 525
   Leu Lys Gly Asp Ile Ile Lys Pro Val Leu His Asp Ala Ala Gly Lys
       530                 535                 540
   Pro Leu Asp Val Ser Val Pro Ala Thr Lys Gly Trp Lys Gln Ile Leu
   545                 550                 555                 560
   Asp Ser Glu Gly Pro Glu Ala Phe Ala Arg Ala Val Arg Ala Asn Lys
                   565                 570                 575
   Gly Cys Leu Ile Met Asp Thr Thr Trp Arg Asp Ala His Gln Ser Leu
               580                 585                 590
   Leu Ala Thr Arg Val Arg Thr Ile Asp Leu Leu Asn Ile Ala His Glu
           595                 600                 605
   Thr Ser His Ala Leu Ala Asn Ala Tyr Ser Leu Glu Cys Trp Gly Gly
       610                 615                 620
   Ala Thr Phe Asp Val Ala Met Arg Phe Leu Tyr Glu Asp Pro Trp Asp
   625                 630                 635                 640
   Arg Leu Arg Lys Leu Arg Lys Ala Val Pro Asn Ile Pro Phe Gln Met
                   645                 650                 655
   Leu Leu Arg Gly Ala Asn Gly Val Ala Tyr Ser Ser Leu Pro Asp Asn
               660                 665                 670
   Ala Ile Tyr His Phe Cys Lys Gln Ala Lys Lys Cys Gly Val Asp Ile
           675                 680                 685
   Phe Arg Val Phe Asp Ala Leu Asn Asp Val Asp Gln Leu Glu Val Gly
       690                 695                 700
   Ile Lys Ala Val His Ala Ala Glu Gly Val Val Glu Ala Thr Ile Cys
   705                 710                 715                 720
   Tyr Ser Gly Asp Met Leu Asn Pro Ser Lys Lys Tyr Asn Leu Pro Tyr
                   725                 730                 735
   Tyr Leu Asp Leu Val Asp Lys Val Val Gln Phe Lys Pro His Val Leu
               740                 745                 750
   Gly Ile Lys Asp Met Ala Gly Val Leu Lys Pro Gln Ala Ala Arg Leu
           755                 760                 765
   Leu Ile Gly Ser Ile Arg Glu Arg Tyr Pro Asp Leu Pro Ile His Val
       770                 775                 780
   His Thr His Asp Ser Ala Gly Thr Gly Val Ala Ser Met Ile Ala Cys
   785                 790                 795                 800
   Ala Gln Ala Gly Ala Asp Ala Val Asp Ala Ala Thr Asp Ser Leu Ser
                   805                 810                 815
   Gly Met Thr Ser Gln Pro Ser Ile Gly Ala Ile Leu Ala Ser Leu Glu
               820                 825                 830
   Gly Thr Glu His Asp Pro Gly Leu Asn Ser Ala Gln Val Arg Ala Leu
           835                 840                 845
   Asp Thr Tyr Trp Ala Gln Leu Arg Leu Leu Tyr Ser Pro Phe Glu Ala
       850                 855                 860
   Gly Leu Thr Gly Pro Asp Pro Glu Val Tyr Glu His Glu Ile Pro Gly
   865                 870                 875                 880
   Gly Gln Leu Thr Asn Leu Ile Phe Gln Ala Ser Gln Leu Gly Leu Gly
                   885                 890                 895
   Gln Gln Trp Ala Glu Thr Lys Lys Ala Tyr Glu Ser Ala Asn Asp Leu
               900                 905                 910
   Leu Gly Asp Val Val Lys Val Thr Pro Thr Ser Lys Val Val Gly Asp
           915                 920                 925
   Leu Ala Gln Phe Met Val Ser Asn Lys Leu Thr Ala Glu Asp Val Ile
       930                 935                 940
   Ala Arg Ala Gly Glu Leu Asp Phe Pro Gly Ser Val Leu Glu Phe Leu
   945                 950                 955                 960
   Glu Gly Leu Met Gly Gln Pro Tyr Gly Gly Phe Pro Glu Pro Leu Arg
                   965                 970                 975
   Ser Arg Ala Leu Arg Asp Arg Arg Lys Leu Asp Lys Arg Pro Gly Leu
               980                 985                 990
   Tyr Leu Glu Pro Leu Asp Leu Ala Lys Ile Lys Ser Gln Ile Arg Glu
           995                 1000                1005
   Asn Tyr Gly Ala Ala Thr Glu Tyr Asp Val Ala Ser Tyr Ala Met Tyr
      1010                 1015                1020
   Pro Lys Val Phe Glu Asp Tyr Lys Lys Phe Val Ala Lys Phe Gly Asp
   1025                1030                1035                1040
   Leu Ser Val Leu Pro Thr Arg Tyr Phe Leu Ala Lys Pro Glu Ile Gly
                   1045                1050                1055
   Glu Glu Phe His Val Glu Leu Glu Lys Gly Lys Val Leu Ile Leu Lys
               1060                1065                1070
   Leu Leu Ala Ile Gly Pro Leu Ser Glu Gln Thr Gly Gln Arg Glu Val
           1075                1080                1085
   Phe Tyr Glu Val Asn Gly Glu Val Arg Gln Val Ser Val Asp Asp Lys
       1090                1095                1100
   Lys Ala Ser Val Glu Asn Thr Ala Arg Pro Lys Ala Glu Leu Gly Asp
   1105                1110                1115                1120
   Ser Ser Gln Val Gly Ala Pro Met Ser Gly Val Val Val Glu Ile Arg
                   1125                1130                1135
   Val His Asp Gly Leu Glu Val Lys Lys Gly Asp Pro Ile Ala Val Leu
               1140                1145                1150
   Ser Ala Met Lys Met Glu Met Val Ile Ser Ala Pro His Ser Gly Lys
           1155                1160                1165
   Val Ser Ser Leu Leu Val Lys Glu Gly Asp Ser Val Asp Gly Gln Asp
       1170                1175                1180
   Leu Val Cys Lys Ile Val Lys Ala
   1185                1190
<210> 2
   <211> 1148
   <212> PRT
   <213> Brevibacillus brevis
<220>
   <223> BbrPYC
<400> 2
   Met Lys Lys Arg Lys Ile Asn Arg Leu Leu Val Ala Asn Arg Gly Glu
                   5                   10                  15
   Ile Ala Ile Arg Ile Phe Arg Ala Ala Thr Glu Leu Gly Ile Arg Thr
               20                  25                  30
   Val Ala Val Tyr Ser Glu Gln Asp Asn Val Ser Ile His Arg Phe Lys
           35                  40                  45
   Ala Asp Glu Ser Tyr Leu Val Gly Ala Gly Lys Gly Pro Ile Glu Ala
       50                  55                  60
   Tyr Leu Asp Ile Glu Ser Ile Ile Glu Ile Ala Lys Arg Asn Asp Ile
   65                  70                  75                  80
   Asp Ala Val His Pro Gly Tyr Gly Phe Leu Ala Glu Asn Ala Glu Phe
                   85                  90                  95
   Ala Lys Arg Cys Gln Glu Glu Gly Ile Ile Phe Ile Gly Pro Ser Pro
               100                 105                 110
   Ala Leu Ile Asp Lys Phe Gly Asp Lys Val Glu Ala Arg Arg Leu Ala
           115                 120                 125
   Ile Glu Ala Glu Ile Pro Val Ile Pro Gly Thr Pro Glu Pro Ile Glu
       130                 135                 140
   Thr Leu Gln Glu Ala Leu Leu Phe Ala Lys Glu Tyr Gly Tyr Pro Ile
   145                 150                 155                 160
   Ile Ile Lys Gly Val Ser Gly Gly Gly Gly Arg Gly Met Arg Ile Val
                   165                 170                 175
   Arg Ser Gln Glu Gly Leu Gln Asp Ser Leu Asp Arg Ala Arg Ser Glu
               180                 185                 190
   Ala Arg Ser Ser Phe Gly Asn Ala Lys Val Tyr Leu Glu Arg Tyr Leu
           195                 200                 205
   Glu Gln Pro Lys His Ile Glu Val Gln Ile Leu Gly Asp Asn His Gly
       210                 215                 220
   Asn Ile Val Tyr Leu Tyr Glu Arg Asp Cys Ser Val Gln Arg Arg His
   225                 230                 235                 240
   Gln Lys Val Val Glu Val Ala Pro Ser Leu Ser Leu Asp Asp Lys Leu
                   245                 250                 255
   Arg Glu Asp Ile Cys Gln Ala Ala Leu Thr Leu Met Lys Lys Ala Gly
               260                 265                 270
   Tyr Ser Asn Ala Gly Thr Val Glu Phe Leu Leu Thr Pro Asp Lys Arg
           275                 280                 285
   Phe Tyr Phe Ile Glu Val Asn Pro Arg Ile Gln Val Glu His Thr Ile
       290                 295                 300
   Thr Glu Leu Ile Thr Gly Ile Asp Ile Val Gln Ser Gln Ile Arg Val
   305                 310                 315                 320
   Ala Glu Gly His Ala Leu Ser Asp Glu Glu Ile Gly Ile Arg Ala Gln
                   325                 330                 335
   Ser Asp Ile Gln Met Ser Gly Phe Ala Ile Gln Cys Arg Val Thr Thr
               340                 345                 350
   Glu Asp Ala Glu Asn Asn Phe Leu Pro Asp Ala Gly Arg Leu Ser Ala
           355                 360                 365
   Trp Arg Ser Gly Gly Gly Phe Gly Val Arg Leu Asp Gly Gly Asn Gly
       370                 375                 380
   Tyr Pro Gly Ala Ile Ile Thr Pro Phe Tyr Asp Ser Leu Leu Val Lys
   385                 390                 395                 400
   Ile Ser Thr Tyr Gly Ala Ser Phe Asp Gln Ala Ala Arg Lys Met Leu
                   405                 410                 415
   Arg Thr Leu Arg Glu Phe Arg Ile Arg Gly Val Lys Thr Asn Leu Pro
               420                 425                 430
   Phe Leu Glu Asn Val Val Thr His Pro Asp Phe Leu Ser Gly Asn Tyr
           435                 440                 445
   Asp Thr Ser Phe Ile Asp Thr Lys Pro Glu Leu Phe Ile Phe Pro Gly
       450                 455                 460
   Arg Gln Asp Arg Gly Thr Lys Leu Leu Ser Tyr Ile Gly Asp Thr Ile
   465                 470                 475                 480
   Val Asn Gly Tyr Pro Gly Leu Pro Lys Ser Glu Lys Lys Pro His Phe
                   485                 490                 495
   Asp Ser Pro Arg Ile Pro Arg Thr Pro Phe Thr Gln Pro Tyr Pro Asp
               500                 505                 510
   Gly Thr Lys Gln Ile Leu Asp Lys Glu Gly Ala Asp Gly Leu Val Arg
   515 520 525
   Trp Ile Gln Ala Gln Lys Gln Val Leu Val Thr Asp Thr Thr Phe Arg
   530 535 540
   Asp Ala His Gln Ser Leu Phe Ala Thr Arg Val Arg Thr Tyr Asp Leu
   545 550 555 560
   Ala Ser Ile Ala Glu Ala Thr Gly Lys Leu Gly Ser Gly Leu Phe Ser
   565 570 575
   Leu Glu Met Trp Gly Gly Ala Thr Phe Asp Thr Thr Met Arg Phe Leu
   580 585 590
   Gln Glu Ser Pro Trp Glu Arg Leu Gln Ile Leu Arg Gln Arg Ile Pro
   595 600 605
   Asn Val Leu Phe Gln Met Leu Leu Arg Gly Ala Asn Gly Val Gly Tyr
   610 615 620
   Thr Asn Tyr Pro Asp Asn Ala Ile His Ala Phe Val Lys Ala Ser Ala
   625 630 635 640
   Glu Asn Gly Ile Asp Val Phe Arg Ile Phe Asp Ser Leu Asn Trp Leu
   645 650 655
   Pro Gly Met Gln Thr Ala Ile Glu Ala Val Arg Glu Ser Gly Lys Val
   660 665 670
   Ala Glu Ala Ala Ile Cys Tyr Thr Gly Asp Ile Leu Asp Pro Thr Lys
   675 680 685
   Thr Lys Tyr Thr Leu Ala Tyr Tyr Val Asn Leu Ala Lys Glu Leu Glu
   690 695 700
   Lys Ala Gly Ala His Ile Leu Ala Ile Lys Asp Met Ala Gly Leu Leu
   705 710 715 720
   Lys Pro Tyr Ala Ala Tyr Ser Leu Val Ser Ala Leu Lys Gln Glu Ile
   725 730 735
   Ser Ile Pro Ile His Leu His Thr His Asp Thr Ser Gly Asn Ala Gly
   740 745 750
   Ala Met Leu Leu Lys Ala Ile Glu Ala Gly Val Asp Ile Val Asp Ala
   755 760 765
   Cys Val Ser Ser Met Ser Gly Leu Thr Ser Gln Pro Ser Leu Asn Gly
   770 775 780
   Leu Ile Ala Ser Leu Ala His Thr Glu Arg Glu Thr Gly Leu Ser Leu
   785 790 795 800
   Glu Ser Phe Asn Lys Leu Ser Asp Tyr Trp Glu Asp Val Arg Pro Tyr
   805 810 815
   Tyr Gln Gly Phe Glu Ser Gly Met Lys Ala Ser Asn Thr Glu Val Tyr
   820 825 830
   Val His Glu Met Pro Gly Gly Gln Tyr Thr Asn Leu Glu Gln Gln Ala
   835 840 845
   Lys Ala Val Gly Leu Glu Gly Arg Trp Asp Glu Val Lys His Met Tyr
   850 855 860
   Ala Val Val Asn Gln Met Cys Gly Asp Ile Val Lys Val Thr Pro Ser
   865 870 875 880
   Ser Lys Val Val Gly Asp Met Ala Leu Phe Met Val Gln Asn Asn Leu
   885 890 895
   Asn Glu Glu Asn Ile Trp Glu Lys Gly Thr Arg Leu Asp Phe Pro Asp
   900 905 910
   Ser Val Ile Gln Phe Phe Gln Gly Tyr Leu Gly Gln Pro Pro Gly Gly
   915 920 925
   Phe Pro Lys Lys Leu Gln Glu Leu Val Leu Lys Gly Arg Asp Ala Phe
   930 935 940
   Thr Ala Arg Pro Gly Glu Leu Leu Ala Pro Ile Asp Phe Thr Gln Val
   945 950 955 960
   Ala Ala Glu Leu Glu Ala Lys Ile Gly Arg Glu Pro Ser His Leu Asp
   965 970 975
   Val Leu Ser Tyr Ile Met Tyr Pro Gln Val Tyr Leu Gln Phe Glu Gln
   980 985 990
   Arg Leu Lys Glu Tyr Gly Asp Leu Ser Val Leu Asn Thr Gly Thr Phe
           995                 1000                1005
   Phe Tyr Gly Leu Arg Pro Gly Glu Glu Thr Ala Ile Thr Ile Glu Arg
      1010                1015                1020
   Gly Lys Thr Leu Ile Ile Lys Leu Val Ala Val Gly Glu Leu His Pro
   1025                1030                1035                1040
   Asp Gly Arg Arg Ile Ile Tyr Phe Glu Leu Asn Gly Gln Pro Arg Glu
                   1045                1050                1055
   Ile Phe Ile Arg Asp Gln Ser Ala Lys Val Ser Glu Leu Ile Arg Arg
               1060                1065                1070
   Lys Ala Glu Ala Gln Asn Pro Ala His Leu Gly Ala Ser Met Pro Gly
           1075                1080                1085
   Lys Val Leu Lys Val Leu Val Ala Glu Gly Asp Lys Val Arg Lys Gly
       1090                1095                1100
   Glu His Leu Leu Val Ser Glu Ala Met Lys Met Glu Thr Thr Ile Gln
   1105                1110                1115                1120
   Ala Pro Leu Asp Gly Lys Ile Lys Ala Val Tyr Val Lys Ala Gly Glu
                   1125                1130                1135
   Ala Ile Gln Thr Gly Asp Leu Leu Ile Glu Met Glu
               1140                1145
<210> 3
   <211> 1173
   <212> PRT
   <213> Debaryomyces hansenii
<220>
   <223> DhaPYC
<400> 3
<210> 4
   <211> 1173
   <212> PRT
   <213> Kluyveromyces lactis
<220>
   <223> KlaPYC
<400> 4
   Met Ser Ser Gln Leu Ala Gly Leu Arg Asp Asn Ser Asn Leu Met Gly
   5 10 15
   Glu Lys Asn Lys Leu Leu Val Ala Asn Arg Gly Glu Ile Pro Ile Arg
   20 25 30
   Ile Phe Arg Thr Ala His Glu Leu Ser Met Lys Thr Val Ala Ile Tyr
   35 40 45
   Ser His Glu Asp Arg Leu Ser Met His Arg Leu Lys Ala Asp Glu Ala
   50 55 60
   Tyr Val Ile Gly Glu Gln Gly Lys Tyr Thr Pro Val Gly Ala Tyr Leu
   65 70 75 80
   Ala Ile Asp Glu Ile Ile Asn Ile Ala Lys Ser His Gly Val Asn Phe
   85 90 95
   Ile His Pro Gly Tyr Gly Phe Leu Ser Glu Asn Ser Glu Phe Ala Glu
   100 105 110
   Lys Val Ala Ala Ser Gly Ile Thr Trp Val Gly Pro Pro Ala Ala Val
   115 120 125
   Ile Asp Ser Val Gly Asp Lys Val Ser Ala Arg Asn Leu Ala Glu Lys
   130 135 140
   Ala Asp Val Pro Val Val Pro Gly Thr Pro Gly Pro Ile Glu Thr Val
   145 150 155 160
   Glu Glu Ala Gln Ala Phe Val Asp Lys Tyr Gly Phe Pro Val Ile Ile
   165 170 175
   Lys Ala Ala Phe Gly Gly Gly Gly Arg Gly Met Arg Val Val Arg Glu
   180 185 190
   Gly Asp Asp Ile Ser Asp Ala Phe Gln Arg Ala Lys Ser Glu Ala Ile
   195 200 205
   Thr Ala Phe Gly Asn Gly Thr Cys Phe Ile Glu Arg Phe Leu Asp Lys
   210 215 220
   Pro Lys His Ile Glu Val Gln Leu Leu Ala Asp Asn Tyr Gly Asn Val
   225 230 235 240
   Val His Leu Phe Glu Arg Asp Cys Ser Val Gln Arg Arg His Gln Lys
   245 250 255
   Val Val Glu Val Ala Pro Ala Lys Thr Leu Pro Glu Glu Val Arg Asp
   260 265 270
   Ala Ile Leu Thr Asp Ala Val Lys Leu Ala Lys Glu Ala Gly Tyr Arg
   275 280 285
   Asn Ala Gly Thr Ala Glu Phe Leu Val Asp Asn Gln Asn Arg His Tyr
   290 295 300
   Phe Ile Glu Ile Asn Pro Arg Ile Gln Val Glu His Thr Ile Thr Glu
   305 310 315 320
   Glu Ile Thr Gly Ile Asp Ile Val Ala Ala Gln Ile Gln Ile Ala Ala
   325 330 335
   Gly Ala Thr Leu Glu Gln Leu Gly Leu Met Gln Asp Lys Ile Thr Thr
   340 345 350
   Arg Gly Phe Ala Ile Gln Cys Arg Ile Thr Thr Glu Asp Pro Ser Lys
   355 360 365
   Asn Phe Gln Pro Asp Thr Gly Arg Ile Asp Val Tyr Arg Ser Ala Gly
   370 375 380
   Gly Asn Gly Val Arg Leu Asp Gly Gly Asn Ala Phe Ala Gly Ser Val
   385 390 395 400
   Ile Ser Pro His Tyr Asp Ser Met Leu Val Lys Cys Ser Cys Ser Gly
   405 410 415
   Ser Thr Tyr Glu Ile Val Arg Arg Lys Met Leu Arg Ala Leu Ile Glu
   420 425 430
   Phe Arg Ile Arg Gly Val Lys Thr Asn Ile Pro Phe Leu Leu Thr Leu
   435 440 445
   Leu Thr His Pro Val Phe Lys Ser Gly Glu Tyr Trp Thr Thr Phe Ile
   450 455 460
   Asp Asp Thr Pro Gln Leu Phe Glu Met Val Ser Ser Gln Asn Arg Ala
   465 470 475 480
   Gln Lys Leu Leu His Tyr Leu Ala Asp Leu Ala Val Asn Gly Ser Ser
                   485                 490                 495
   Ile Lys Gly Gln Ile Gly Leu Pro Lys Leu Thr Thr His Pro Thr Ile
               500                 505                 510
   Pro His Leu His Asn Glu Asp Gly Ser Leu Val Asp Val Ser Ala Lys
           515                 520                 525
   Pro Pro Ala Gly Trp Arg Asp Val Leu Leu Asp Tyr Gly Pro Glu Glu
       530                 535                 540
   Phe Ala Lys Gln Val Arg Lys Phe Asn Gly Thr Leu Leu Met Asp Thr
   545                 550                 555                 560
   Thr Trp Arg Asp Ala His Gln Ser Leu Leu Ala Thr Arg Val Arg Thr
                   565                 570                 575
   Tyr Asp Leu Ala Ala Ile Ala Pro Thr Thr Ala His Ala Met Ser Gly
               580                 585                 590
   Ala Phe Ala Leu Glu Cys Trp Gly Gly Ala Thr Phe Asp Val Ser Met
           595                 600                 605
   Arg Phe Leu His Glu Asp Pro Trp Glu Arg Leu Arg Thr Leu Arg Lys
       610                 615                 620
   Leu Val Pro Asn Ile Pro Phe Gln Met Leu Leu Arg Gly Ala Asn Gly
   625                 630                 635                 640
   Val Ala Tyr Ser Ser Leu Pro Asp Asn Ala Ile Asp His Phe Val Lys
                   645                 650                 655
   Gln Ala Lys Asp Asn Gly Val Asp Ile Phe Arg Val Phe Asp Ala Leu
               660                 665                 670
   Asn Asp Leu Glu Gln Leu Lys Val Gly Val Asp Ala Val Lys Lys Ala
           675                 680                 685
   Gly Gly Val Val Glu Ala Thr Ile Cys Tyr Ser Gly Asp Met Leu Ala
       690                 695                 700
   Pro Gly Lys Lys Tyr Asn Leu Asp Tyr Tyr Leu Asp Ile Thr Glu Lys
   705                 710                 715                 720
   Ile Val Asn Ser Gly Thr His Ile Leu Gly Ile Lys Asp Met Ala Gly
                   725                 730                 735
   Thr Leu Lys Pro Ser Ala Ala Arg Leu Leu Ile Gly Ser Ile Arg Ala
               740                 745                 750
   Lys Tyr Pro Glu Leu Pro Ile His Val His Thr His Asp Ser Ala Gly
           755                 760                 765
   Thr Gly Val Ala Ser Met Ala Ala Cys Ala Leu Ser Gly Ala Asp Val
       770                 775                 780
   Val Asp Val Ala Thr Asn Ser Met Ser Gly Leu Thr Ser Gln Pro Ser
   785                 790                 795                 800
   Ile Asn Ala Leu Leu Ala Ala Leu Asp Gly Glu Ile Asp Asn Asn Val
                   805                 810                 815
   Asn Val Gly Tyr Val Arg Glu Leu Asp Ala Tyr Trp Ala Glu Met Arg
               820                 825                 830
   Leu Leu Tyr Ser Cys Phe Glu Ala Asp Leu Lys Gly Pro Asp Pro Glu
           835                 840                 845
   Val Tyr Val His Glu Ile Pro Gly Gly Gln Leu Thr Asn Leu Leu Phe
       850                 855                 860
   Gln Ala Gln Gln Leu Gly Leu Gly Glu Lys Trp Ala Glu Thr Lys Arg
   865                 870                 875                 880
   Ala Tyr Arg Glu Ala Asn Leu Leu Leu Gly Asp Leu Val Lys Val Thr
                   885                 890                 895
   Pro Thr Ser Lys Val Val Gly Asp Leu Ala Gln Phe Met Val Thr Asn
               900                 905                 910
   Lys Leu Thr Ser Asp Asp Val Arg Arg Leu Ala Ser Ser Leu Asp Phe
           915                 920                 925
   Pro Asp Ser Val Met Asp Phe Phe Glu Gly Leu Ile Gly Gln Pro Tyr
       930                 935                 940
   Gly Gly Phe Pro Glu Pro Leu Arg Thr Asp Val Leu Arg Asn Lys Arg
   945                 950                 955                 960
   Arg Lys Leu Thr Gln Arg Pro Gly Leu Glu Leu Ala Pro Phe Glu Leu
                   965                 970                 975
   Glu Lys Ile Lys Glu Asp Leu Ser Thr Arg Phe Ser Asp Ile Asp Glu
               980                 985                 990
   Cys Asp Val Ala Ser Tyr Asn Met Tyr Pro Lys Val Tyr Glu Asp Phe
           995                 1000                1005
   Arg Lys Ile Lys Glu Lys Tyr Gly Asp Leu Ser Val Leu Pro Thr Lys
      1010                1015                1020
   Asn Phe Leu Ser Pro Pro Val Ile Gly Glu Glu Ile Val Val Thr Ile
   1025                1030                1035                1040
   Glu Gln Gly Lys Thr Leu Ile Ile Lys Pro Gln Ala Ile Gly Asp Leu
                   1045                1050                1055
   Asn Lys Glu Thr Gly Ile Arg Glu Val Tyr Phe Glu Leu Asn Gly Glu
               1060                1065                1070
   Leu Arg Lys Val Ser Val Ala Asp Arg Ser Gln Lys Val Glu Thr Val
           1075                1080                1085
   Ser Lys Pro Lys Ala Asp Ala His Asp Pro Phe Gln Ile Gly Ser Pro
       1090                1095                1100
   Met Ala Gly Val Val Val Glu Val Lys Val His Lys Gly Ser Leu Ile
   1105                1110                1115                1120
   Ala Lys Gly Gln Pro Val Ala Val Leu Ser Ala Met Lys Met Glu Met
                   1125                1130                1135
   Val Ile Ser Ser Pro Ala Asp Gly Gln Val Lys Glu Val Leu Val Lys
               1140                1145                1150
   Asp Gly Glu Asn Val Asp Ala Ser Asp Leu Leu Val Val Leu Glu Glu
           1155                1160                1165
   Ala Pro Ala Lys Glu
       1170
<210> 5
   <211> 1174
   <212> PRT
   <213> Lachancea thermotolerans
<220>
   <223> LthPYC
<400> 5
<210> 6
   <211> 1179
   <212> PRT
   <213> Lodderomyces elongisporus
<220>
   <223> LelPYC
<400> 6
   Met Thr Ser Ile Ala Ser Ser Asn Thr Gln Asp Gly Arg Ile Asn Gln
                   5                   10                  15
   Met Arg Arg Asp Ser Thr Val Leu Gly Pro Met Asn Lys Ile Leu Val
               20                  25                  30
   Ala Asn Arg Gly Glu Ile Pro Ile Arg Ile Phe Arg Thr Ala His Glu
           35                  40                  45
   Leu Ser Met Gln Thr Val Ala Ile Tyr Ser His Glu Asp Arg Leu Ser
       50                  55                  60
   Met His Arg Leu Lys Ala Asp Glu Ser Tyr Ala Ile Gly Lys Lys Gly
   65                  70                  75                  80
   Gln Phe Thr Pro Val Gly Ala Tyr Leu Gln Ile Asp Glu Ile Ile Asp
                   85                  90                  95
   Ile Ala Lys Lys His Asn Val Asn Met Ile His Pro Gly Tyr Gly Phe
               100                 105                 110
   Leu Ser Glu Asn Ser Glu Phe Ala Lys Lys Val Glu Gln Asn Gly Ile
           115                 120                 125
   Val Trp Ile Gly Pro Ser His Lys Thr Ile Asp Ala Val Gly Asp Lys
       130                 135                 140
   Val Ser Ala Arg Thr Leu Ala Ile Glu Asn Asp Val Pro Val Val Pro
   145                 150                 155                 160
   Gly Thr Pro Gly Pro Ile Asp Asp Val Ser Asp Ala Arg Lys Phe Val
                   165                 170                 175
   Glu Lys Tyr Gly Tyr Pro Val Ile Ile Lys Ala Ala Phe Gly Gly Gly
               180                 185                 190
   Gly Arg Gly Met Arg Val Val Arg Glu Gly Asp Asp Ile Glu Asp Ala
           195                 200                 205
   Phe Lys Arg Ala Thr Ser Glu Ala Lys Thr Ala Phe Gly Asn Gly Thr
       210                 215                 220
   Cys Phe Ile Glu Arg Phe Leu Asp Lys Pro Lys His Ile Glu Val Gln
   225                 230                 235                 240
   Leu Leu Ala Asp Asn Tyr Gly Asn Val Ile His Leu Phe Glu Arg Asp
                   245                 250                 255
   Cys Ser Val Gln Arg Arg His Gln Lys Val Val Glu Ile Ala Pro Ala
               260                 265                 270
   Lys Asn Leu Pro Arg Glu Val Arg Asp Ala Ile Leu Thr Asp Ala Val
           275                 280                 285
   Lys Leu Ala Lys Ser Ala Asn Tyr Arg Asn Ala Gly Thr Ala Glu Phe
       290                 295                 300
   Leu Val Asp Glu Gln Asn Arg His Tyr Phe Ile Glu Ile Asn Pro Arg
   305                 310                 315                 320
   Ile Gln Val Glu His Thr Ile Thr Glu Glu Ile Thr Gly Val Asp Ile
                   325                 330                 335
   Val Ala Ala Gln Ile Gln Ile Ala Ala Gly Ala Ser Leu Gln Gln Leu
               340                 345                 350
   Gly Leu Leu Gln Asp Lys Ile Thr Thr Arg Gly Phe Ala Ile Gln Cys
           355                 360                 365
   Arg Ile Thr Thr Glu Asp Pro Ala Lys Asn Phe Gln Pro Asp Thr Gly
       370                 375                 380
   Lys Ile Glu Val Tyr Arg Ser Ala Gly Gly Asn Gly Val Arg Leu Asp
   385                 390                 395                 400
   Gly Gly Asn Gly Phe Ala Gly Ser Ile Ile Ser Pro His Tyr Asp Ser
                   405                 410                 415
   Met Leu Val Lys Cys Ser Cys Ser Gly Ser Thr Phe Glu Ile Ala Arg
               420                 425                 430
   Arg Lys Met Ile Arg Ala Leu Ile Glu Phe Arg Ile Arg Gly Val Lys
           435                 440                 445
   Thr Asn Ile Pro Phe Leu Leu Ala Leu Leu Thr Asn Gln Thr Phe Val
       450                 455                 460
   Glu Gly Asp Cys Trp Thr Thr Phe Ile Asp Asp Thr Pro Ser Leu Phe
   465                 470                 475                 480
   Gln Met Ile Ser Ser Gln Asn Arg Ala Thr Lys Leu Leu Leu Tyr Leu
                   485                 490                 495
   Ala Asp Leu Tyr Val Asn Gly Ser Ser Ile Lys Gly Gln Ile Gly Tyr
               500                 505                 510
   Pro Lys Leu Asp Thr Glu Ala Leu Ile Pro Glu Leu His Glu Pro Arg
           515                 520                 525
   Thr Gly Ile Ala Ile Asp Val Asn His Thr Pro Pro Pro Arg Gly Trp
       530                 535                 540
   Arg Gln Val Leu Leu Glu Glu Gly Pro Ala Lys Phe Ala Lys Lys Val
   545                 550                 555                 560
   Arg Glu Phe Lys Gly Cys Leu Ile Thr Asp Thr Thr Trp Arg Asp Ala
                   565                 570                 575
   His Gln Ser Leu Leu Ala Thr Arg Val Arg Thr Ile Asp Leu Leu Asn
               580                 585                 590
   Ile Ala Pro Thr Thr Ala Phe Ala Leu Asn Gly Ala Phe Ser Leu Glu
           595                 600                 605
   Cys Trp Gly Gly Ala Thr Phe Asp Val Cys Met Arg Phe Leu Tyr Glu
       610                 615                 620
   Asp Pro Trp Val Arg Leu Arg Gln Leu Arg Ala Leu Val Pro Asn Ile
   625                 630                 635                 640
   Pro Phe Gln Met Leu Leu Arg Gly Ala Asn Gly Val Ala Tyr Ser Ser
                   645                 650                 655
   Leu Pro Asp Asn Ala Ile Asp Gln Phe Val Lys Glu Ala Lys Glu Asn
               660                 665                 670
   Gly Val Asp Ile Phe Arg Val Phe Asp Ala Leu Asn Asp Leu Glu Gln
           675                 680                 685
   Leu Lys Val Gly Val Asp Ala Val Lys Lys Ala Gly Gly Val Val Glu
       690                 695                 700
   Ala Thr Val Cys Tyr Ser Gly Asp Met Leu Lys Pro Gly Lys Lys Tyr
   705                 710                 715                 720
   Asn Leu Glu Tyr Tyr Leu Asn Phe Val Asp Glu Val Val Lys Met Gly
                   725                 730                 735
   Thr His Phe Leu Gly Ile Lys Asp Met Ala Gly Thr Leu Lys Pro Ala
               740                 745                 750
   Ala Ala Lys Leu Leu Val Gly Glu Ile Arg Lys Arg His Pro Asp Leu
           755                 760                 765
   Pro Ile His Val His Thr His Asp Ser Ala Gly Thr Gly Val Ala Ser
       770                 775                 780
   Met Thr Glu Cys Ala Lys Ala Gly Ala Asp Val Val Asp Ala Ala Ser
   785                 790                 795                 800
   Asn Ser Met Ser Gly Met Thr Ser Gln Pro Ser Ile Ser Ala Ile Leu
                   805                 810                 815
   Ala Ser Phe Glu Gly Ser Ile Glu Ser Gly Leu Ser Glu Gln Leu Val
               820                 825                 830
   Arg Glu Leu Asp Glu Tyr Trp Ala Gln Met Arg Leu Leu Tyr Ser Cys
           835                 840                 845
   Phe Glu Ala Asp Leu Lys Gly Pro Asp Pro Glu Val Tyr Ser His Glu
       850                 855                 860
   Ile Pro Gly Gly Gln Leu Thr Asn Leu Leu Phe Gln Ala Gln Gln Leu
   865                 870                 875                 880
   Gly Leu Gly Thr Lys Trp Leu Glu Thr Lys Lys Ala Tyr Arg Ile Ala
                   885                 890                 895
   Asn Lys Ile Leu Gly Asp Leu Val Lys Val Thr Pro Thr Ser Lys Val
                   900                 905                 910
   Val Gly Asp Leu Ala Gln Phe Met Val Ser Asn Ser Leu Thr Glu Glu
               915                 920                 925
   Asp Ile Asn Arg Leu Ala Gly Glu Leu Asp Phe Pro Asp Ser Val Tyr
       930                 935                 940
   Asp Phe Met Glu Gly Leu Met Gly Gln Pro Tyr Gly Gly Phe Pro Glu
   945                 950                 955                 960
   Pro Leu Arg Thr Asn Met Leu Gly Asn Lys Arg Gln Lys Leu Thr Gln
                   965                 970                 975
   Arg Pro Gly Leu Ser Leu Pro Pro Val Lys Phe Asp Glu Ile Lys Gln
               980                 985                 990
   Glu Leu Gln Ser Arg Tyr Gly Thr Gln Val Ser Glu Thr Asp Ile Ala
           995                 1000                1005
   Ser Tyr Val Met Tyr Pro Lys Val Tyr Glu Ala Tyr Arg Lys Gln Val
       1010                1015                1020
   Glu Lys Tyr Gly Asp Leu Ser Val Leu Pro Thr Arg Tyr Phe Leu Lys
   1025                1030                1035                1040
   Pro Ala Asn Ile Gly Gln Glu Ile Val Val Asp Ile Glu Gln Gly Lys
                   1045                1050                1055
   Thr Leu Ile Ile Lys Leu Leu Ala Val Gly Glu Leu Ser Glu Lys Thr
               1060                1065                1070
   Gly Ser Arg Glu Ile Phe Phe Glu Leu Asn Gly Glu Met Arg Ser Val
           1075                1080                1085
   Thr Val Glu Asp Lys Thr Ala Ser Val Glu Thr Lys Thr Arg Pro Lys
       1090                1095                1100
   Ala Gln Gln Pro Asn Asp Val Gly Ala Pro Met Ser Gly Val Val Ile
   1105                1110                1115                1120
   Glu Val Arg Thr His Lys His Gln Glu Val Lys Lys Gly Asp Pro Ile
                   1125                1130                1135
   Ala Val Leu Ser Ala Met Lys Met Glu Met Ile Ile Ser Ala Pro Val
               1140                1145                1150
   Ser Gly Val Val Gly Asp Ile Leu Val Lys Glu Gly Asp Ser Val Asp
           1155                1160                1165
   Ala Asn Asp Leu Ile Thr Ser Ile Leu Lys His
       1170                1175
<210> 7
   <211> 1174
   <212> PRT
   <213> Saccharomyces cerevisiae
<220>
   <223> ScePYC
<400> 7
<210> 8
   <211> 1216
   <212> PRT
   <213> Candida orthopsilosis
<220>
   <223> CorPYC
<400> 8
   Met Ile Phe Ile Asn Ser Ser Ser Ile Ser Thr Lys Ser Pro Leu Asn
                   5                   10                  15
   Ser Ile Ser Leu Ser Ser Thr Gln Leu Leu Phe Phe Cys Gln Ile Tyr
               20                  25                  30
   Ser Pro Pro Ile Thr Met Thr Ser Leu Gln Pro Thr Ala Thr Gln Asp
           35                  40                  45
   Gly Gln Ile Asn Gln Met Arg Arg Ala Ser Ser Val Leu Gly Pro Met
       50                  55                  60
   Asn Lys Ile Leu Val Ala Asn Arg Gly Glu Ile Pro Ile Arg Ile Phe
   65                  70                  75                  80
   Arg Thr Ala His Glu Leu Ser Met Gln Thr Val Ala Ile Tyr Ser His
                   85                  90                  95
   Glu Asp Arg Leu Ser Met His Arg Leu Lys Ala Asp Glu Ser Tyr Val
               100                 105                 110
   Ile Gly Lys Lys Gly Gln Tyr Ser Pro Val Gly Ala Tyr Leu Gln Ile
           115                 120                 125
   Asp Glu Ile Ile Asp Ile Ala Lys Gln His Asn Val Asn Met Ile His
       130                 135                 140
   Pro Gly Tyr Gly Phe Leu Ser Glu Asn Ser Glu Phe Ala Arg Lys Val
   145                 150                 155                 160
   Glu Glu Asn Gly Ile Leu Trp Val Gly Pro Ser Tyr Lys Thr Ile Asp
                   165                 170                 175
   Ala Val Gly Asp Lys Val Ser Ala Arg Thr Leu Ala Ile Glu Asn Asp
               180                 185                 190
   Val Pro Val Val Pro Gly Thr Pro Gly Pro Ile Asp Asp Val Thr Asp
           195                 200                 205
   Ala Arg Lys Phe Val Glu Lys Tyr Gly Phe Pro Val Ile Ile Lys Ala
       210                 215                 220
   Ala Phe Gly Gly Gly Gly Arg Gly Met Arg Val Val Arg Glu Gly Asp
   225                 230                 235                 240
   Asp Ile Glu Asp Ala Phe Lys Arg Ala Thr Ser Glu Ala Lys Thr Ala
                   245                 250                 255
   Phe Gly Asn Gly Thr Cys Phe Ile Glu Arg Phe Leu Asp Lys Pro Lys
               260                 265                 270
   His Ile Glu Val Gln Leu Leu Ala Asp Asn Tyr Gly Asn Val Ile His
           275                 280                 285
   Leu Phe Glu Arg Asp Cys Ser Val Gln Arg Arg His Gln Lys Val Val
       290                 295                 300
   Glu Ile Ala Pro Ala Lys Asn Leu Pro Lys Glu Val Arg Asp Ala Ile
   305                 310                 315                 320
   Leu Thr Asp Ala Val Lys Leu Ala Lys Ser Ala Asn Tyr Arg Asn Ala
                   325                 330                 335
   Gly Thr Ala Glu Phe Leu Val Asp Glu Gln Asn Arg His Tyr Phe Ile
               340                 345                 350
   Glu Ile Asn Pro Arg Ile Gln Val Glu His Thr Ile Thr Glu Glu Ile
   355 360 365
   Thr Gly Val Asp Ile Val Ala Ala Gln Ile Gln Ile Ala Ala Gly Ala
   370 375 380
   Ser Leu Gln Gln Leu Gly Leu Leu Gln Asp Lys Ile Thr Thr Arg Gly
   385 390 395 400
   Phe Ala Ile Gln Cys Arg Ile Thr Thr Glu Asp Pro Ala Lys Asn Phe
   405 410 415
   Gln Pro Asp Thr Gly Lys Ile Glu Val Tyr Lys Ser Ala Gly Gly Asn
   420 425 430
   Gly Val Arg Leu Asp Gly Gly Asn Gly Phe Ala Gly Ser Val Ile Ser
   435 440 445
   Pro His Tyr Asp Ser Met Leu Val Lys Cys Ser Cys Ser Gly Ser Thr
   450 455 460
   Phe Glu Ile Ala Arg Arg Lys Met Leu Arg Ala Leu Ile Glu Phe Arg
   465 470 475 480
   Ile Arg Gly Val Lys Thr Asn Ile Pro Phe Leu Leu Ala Leu Leu Thr
   485 490 495
   Asn Glu Thr Phe Ile Lys Gly Asp Cys Trp Thr Thr Phe Ile Asp Asp
   500 505 510
   Thr Pro Ser Leu Phe Gln Met Val Ser Ser Gln Asn Arg Ala Thr Lys
   515 520 525
   Leu Leu Ser Tyr Leu Ala Asp Leu Tyr Val Asn Gly Ser Ser Ile Lys
   530 535 540
   Gly Gln Ile Gly Tyr Pro Lys Leu Asn Glu Glu Ala Leu Ile Pro Val
   545 550 555 560
   Leu His Asp Pro Lys Thr Gly Ile Pro Ile Asp Val Ala His Ile Pro
   565 570 575
   Pro Pro Arg Gly Trp Arg Gln Val Leu Leu Glu Glu Gly Pro Asp Ala
   580 585 590
   Phe Ala Lys Lys Val Arg Gln Tyr Asn Gly Thr Leu Ile Thr Asp Thr
   595 600 605
   Thr Trp Arg Asp Ala His Gln Ser Leu Leu Ala Thr Arg Val Arg Thr
   610 615 620
   Ile Asp Leu Leu Asn Ile Ala Pro Thr Thr Ala Phe Ala Leu Asn Gly
   625 630 635 640
   Ala Phe Ser Leu Glu Cys Trp Gly Gly Ala Thr Phe Asp Val Cys Met
   645 650 655
   Arg Phe Leu Tyr Glu Asp Pro Trp Asp Arg Leu Arg Lys Leu Arg Ala
   660 665 670
   Leu Val Pro Asn Ile Pro Phe Gln Met Leu Leu Arg Gly Ala Asn Gly
   675 680 685
   Val Ala Tyr Ser Ser Leu Pro Asp Asn Ala Ile Asp Gln Phe Val Lys
   690 695 700
   Glu Ala Lys Gly Asn Gly Val Asp Ile Phe Arg Val Phe Asp Ala Leu
   705 710 715 720
   Asn Asp Leu Glu Gln Leu Lys Val Gly Ile Asp Ala Val Lys Lys Ala
   725 730 735
   Gly Gly Val Val Glu Ala Thr Val Cys Tyr Ser Gly Asp Met Leu Lys
   740 745 750
   Pro Gly Lys Lys Tyr Asn Leu Glu Tyr Tyr Leu Ser Val Val Asp Glu
   755 760 765
   Val Val Lys Met Gly Thr His Phe Leu Gly Ile Lys Asp Met Ala Gly
   770 775 780
   Thr Leu Lys Pro Ala Ala Ala Arg Leu Leu Val Gly Glu Ile Arg Asn
   785 790 795 800
   Arg Tyr Pro Glu Leu Pro Ile His Val His Thr His Asp Ser Ala Gly
   805 810 815
   Thr Gly Val Ala Ser Met Thr Glu Cys Ala Lys Ala Gly Ala Asp Val
   820 825 830
   Val Asp Ala Ala Ser Asn Ser Met Ser Gly Leu Thr Ser Gln Pro Ser
           835                 840                 845
   Ile Ser Ala Ile Leu Ala Ser Phe Glu Gly Ser Ile Asp Thr Gly Leu
       850                 855                 860
   Ser Glu Ser Leu Val Arg Glu Leu Asp Thr Tyr Trp Ala Gln Met Arg
   865                 870                 875                 880
   Leu Leu Tyr Ser Cys Phe Glu Ala Asp Leu Lys Gly Pro Asp Pro Glu
                   885                 890                 895
   Val Tyr Ser His Glu Ile Pro Gly Gly Gln Leu Thr Asn Leu Leu Phe
               900                 905                 910
   Gln Ala Gln Gln Leu Gly Leu Gly Glu Lys Trp Leu Gln Thr Lys Glu
           915                 920                 925
   Thr Tyr Lys Ile Ala Asn Lys Ile Leu Gly Asp Leu Val Lys Val Thr
       930                 935                 940
   Pro Thr Ser Lys Val Val Gly Asp Leu Ala Gln Phe Met Val Ser Asn
   945                 950                 955                 960
   Ser Leu Thr Glu Glu Asp Val Asn Arg Leu Ala Ser Glu Leu Asp Phe
                   965                 970                 975
   Pro Asp Ser Val Leu Asp Phe Met Glu Gly Leu Met Gly Lys Pro Tyr
               980                 985                 990
   Gly Gly Phe Pro Glu Pro Leu Arg Thr Asn Met Leu Gly Asn Lys Arg
           995                 1000                1005
   Gln Lys Leu Asp Lys Arg Pro Gly Leu Tyr Leu Lys Pro Val Asp Phe
      1010                1015                1020
   Ala Ala Ile Arg Lys Glu Leu Ile Ser Arg Tyr Gly Ser Gln Ile Asp
   1025                1030                1035                1040
   Glu Thr Asp Val Ala Ser Tyr Val Met Tyr Pro Lys Val Phe Glu Ala
                   1045                1050                1055
   Phe Arg Lys Gln Val Glu Lys Tyr Gly Asp Leu Ser Val Leu Pro Thr
               1060                1065                1070
   Arg Phe Phe Leu Lys Pro Ala Asn Ile Gly Glu Glu Ile Val Val Asp
           1075                1080                1085
   Ile Glu Lys Gly Lys Thr Leu Ile Ile Lys Leu Leu Ala Val Gly Glu
       1090                1095                1100
   Ile Ser Glu Lys Thr Gly Thr Arg Glu Val Phe Phe Glu Leu Asn Gly
   1105                1110                1115                1120
   Glu Met Arg Ser Val Ser Val Glu Asp Asn Thr Val Ser Val Glu Thr
                   1125                1130                1135
   Lys Thr Arg Pro Lys Ala Ser Ala Pro Asn Asp Val Gly Ala Pro Met
               1140                1145                1150
   Ala Gly Val Val Ile Glu Val Arg Thr His Lys His Gln Glu Val Lys
           1155                1160                1165
   Lys Gly Asp Pro Ile Ala Val Leu Ser Ala Met Lys Met Glu Met Val
       1170                1175                1180
   Ile Ser Ala Pro Val Ser Gly Leu Val Asp Asp Leu Leu Val Arg Glu
   1185                1190                1195                1200
   Gly Asp Ser Val Asp Ala Asn Asp Leu Ile Thr Ser Ile Leu Lys Ala
                   1205                1210                1215
<210> 9
   <211> 1180
   <212> PRT
   <213> Candida tropicalis
<220>
   <223> CtrPYC
<400> 9
   Met Ser Val Pro Val Gln Lys Ala Asn Ser Gln Asp Thr Arg Ile Ser
                   5                   10                  15
   Gln Met Arg Arg Asp Ser Thr Val Leu Gly Pro Met Asn Lys Ile Leu
               20                  25                  30
   Val Ala Asn Arg Gly Glu Ile Pro Ile Arg Ile Phe Arg Thr Ala His
           35                  40                  45
   Glu Leu Ser Met Gln Thr Val Ala Ile Tyr Ser His Glu Asp Arg Leu
       50                  55                  60
   Ser Met His Arg Leu Lys Ala Asp Glu Ser Tyr Val Ile Gly Lys Lys
   65                  70                  75                  80
   Gly Gln Phe Ser Pro Val Gly Ala Tyr Leu Gln Ile Asp Glu Ile Ile
                   85                  90                  95
   Asn Ile Ala Lys Lys His Asn Val Asn Met Ile His Pro Gly Tyr Gly
               100                 105                 110
   Phe Leu Ser Glu Asn Ser Glu Phe Ala Arg Lys Val Glu Glu Asn Gly
           115                 120                 125
   Ile Ile Trp Ile Gly Pro Ser Tyr Lys Thr Ile Asp Ala Val Gly Asp
       130                 135                 140
   Lys Val Ser Ala Arg Thr Leu Ala Ile Glu Asn Asp Val Pro Val Val
   145                 150                 155                 160
   Pro Gly Thr Pro Gly Pro Ile Glu Thr Val Glu Glu Ala Lys Ala Phe
                   165                 170                 175
   Val Asp Lys Tyr Gly Leu Pro Val Ile Ile Lys Ala Ala Phe Gly Gly
               180                 185                 190
   Gly Gly Arg Gly Met Arg Val Val Arg Glu Gly Asp Asp Ile Glu Asp
       195                 200                 205
   Ala Phe Lys Arg Ala Thr Ser Glu Ala Lys Thr Ala Phe Gly Asn Gly
       210                 215                 220
   Thr Cys Phe Ile Glu Arg Phe Leu Val Lys Pro Lys His Ile Glu Val
   225                 230                 235                 240
   Gln Leu Leu Ala Asp Asn Tyr Gly Asn Val Ile His Leu Phe Glu Arg
                   245                 250                 255
   Asp Cys Ser Val Gln Arg Arg His Gln Lys Val Val Glu Ile Ala Pro
               260                 265                 270
   Ala Lys Ser Leu Pro Lys His Val Arg Asp Ala Ile Leu Thr Asp Ala
           275                 280                 285
   Val Lys Leu Ala Lys Ser Ala Asn Tyr Arg Asn Ala Gly Thr Ala Glu
       290                 295                 300
   Phe Leu Val Asp Ala Gln Asp Arg His Tyr Phe Ile Glu Ile Asn Pro
   305                 310                 315                 320
   Arg Ile Gln Val Glu His Thr Ile Thr Glu Glu Ile Thr Gly Val Asp
                   325                 330                 335
   Leu Val Ala Ala Gln Ile Gln Ile Ala Ala Gly Ala Ser Leu Gln Gln
               340                 345                 350
   Leu Gly Leu Leu Gln Asp Lys Ile Thr Thr Arg Gly Phe Ala Ile Gln
           355                 360                 365
   Cys Arg Ile Thr Thr Glu Asp Pro Thr Lys Asn Phe Gln Pro Asp Thr
       370                 375                 380
   Gly Lys Ile Glu Val Tyr Arg Ser Ala Gly Gly Asn Gly Val Arg Leu
   385                 390                 395                 400
   Asp Gly Gly Asn Gly Phe Val Gly Ser Ile Ile Ser Pro His Tyr Asp
                   405                 410                 415
   Ser Met Leu Val Lys Cys Ser Cys Ser Gly Ser Thr Tyr Glu Ile Ala
               420                 425                 430
   Arg Arg Lys Met Leu Arg Ala Leu Ile Glu Phe Arg Ile Arg Gly Val
           435                 440                 445
   Lys Thr Asn Ile Pro Phe Leu Leu Ala Leu Leu Thr Asn Asp Ile Phe
       450                 455                 460
   Ile Lys Gly Asp Cys Trp Thr Thr Phe Ile Asp Asp Thr Pro Ser Leu
   465                 470                 475                 480
   Phe Gln Met Ile Ser Ser Gln Asn Arg Ala Thr Lys Met Leu Ser Tyr
                   485                 490                 495
   Leu Ala Asp Leu Val Val Asn Gly Ser Ser Ile Lys Gly Gln Val Gly
               500                 505                 510
   Tyr Pro Lys Leu Glu Thr Glu Pro Val Val Pro Asp Ile His Glu Pro
           515                 520                 525
   Lys Thr Gly Ile Val Ile Asp Val Asn Asn Thr Pro Ala Pro Arg Gly
           530                 535                 540
   Trp Arg Gln Val Leu Leu Glu Glu Gly Pro Glu Ala Phe Ala Lys Lys
   545                 550                 555                 560
   Val Arg Ala Phe Lys Gly Thr Leu Ile Thr Asp Thr Thr Trp Arg Asp
                   565                 570                 575
   Ala His Gln Ser Leu Leu Ala Thr Arg Val Arg Thr Ile Asp Leu Val
               580                 585                 590
   Asn Ile Ala Ser Thr Thr Ala Phe Ala Leu Asn Gly Ala Phe Ser Leu
           595                 600                 605
   Glu Cys Trp Gly Gly Ala Thr Phe Asp Val Cys Met Arg Phe Leu Tyr
       610                 615                 620
   Glu Asp Pro Trp Ala Arg Leu Arg Lys Leu Arg Ala Leu Val Pro Asn
   625                 630                 635                 640
   Ile Pro Phe Gln Met Leu Leu Arg Gly Ala Asn Gly Val Ala Tyr Ser
                   645                 650                 655
   Ser Leu Pro Asp Asn Ala Ile Asp Gln Phe Val Lys Glu Ala Lys Asp
               660                 665                 670
   Asn Gly Val Asp Ile Phe Arg Val Phe Asp Ala Leu Asn Asp Leu Glu
           675                 680                 685
   Gln Leu Lys Val Gly Ile Asp Ala Val Lys Lys Ala Gly Gly Val Val
       690                 695                 700
   Glu Ala Thr Val Cys Tyr Ser Gly Asp Met Leu Lys Pro Gly Lys Lys
   705                 710                 715                 720
   Tyr Asn Leu Glu Tyr Tyr Leu Asn Val Val Asp Glu Ile Val Lys Leu
                   725                 730                 735
   Gly Thr His Phe Leu Gly Ile Lys Asp Met Ala Gly Thr Leu Lys Pro
               740                 745                 750
   Ala Ala Ala Lys Ile Leu Ile Gly Glu Ile Arg Ser Arg Tyr Pro Asp
           755                 760                 765
   Leu Pro Ile His Val His Thr His Asp Ser Ala Gly Thr Gly Val Ala
       770                 775                 780
   Ser Met Thr Gln Cys Ala Val Ser Gly Ala Asp Val Val Asp Ala Ala
   785                 790                 795                 800
   Ser Asn Ser Met Ser Gly Met Thr Ser Gln Pro Ser Ile Ser Ala Leu
                   805                 810                 815
   Leu Ala Ser Phe Glu Gly Ser Ile Asp Thr Gly Leu Ser Glu Ser Met
               820                 825                 830
   Val Arg Glu Leu Asp Asn Tyr Trp Ala Gln Met Arg Leu Leu Tyr Ser
           835                 840                 845
   Cys Phe Asp Ala Asp Leu Lys Gly Pro Asp Pro Glu Val Tyr Gln His
       850                 855                 860
   Glu Ile Pro Gly Gly Gln Leu Thr Asn Leu Leu Phe Gln Ala Gln Gln
   865                 870                 875                 880
   Leu Gly Leu Gly Ser Gln Trp Val Gln Thr Lys Glu Ala Tyr Lys Val
                   885                 890                 895
   Ala Asn Ser Ile Leu Gly Asp Leu Val Lys Val Thr Pro Thr Ser Lys
               900                 905                 910
   Val Val Gly Asp Leu Ala Gln Phe Met Val Ser Asn Thr Leu Thr Glu
           915                 920                 925
   Glu Asp Val Asn Arg Leu Ala Ala Glu Leu Asp Phe Pro Asp Ser Val
       930                 935                 940
   Leu Asp Phe Phe Gln Gly Leu Met Gly Thr Pro Tyr Gly Gly Phe Pro
   945                 950                 955                 960
   Glu Pro Leu Arg Thr Asn Ile Leu Gly Ser Lys Arg Gln Lys Leu Asn
                   965                 970                 975
   Glu Arg Pro Gly Leu Thr Leu Pro Pro Val Asp Phe Val Gly Ile Arg
               980                 985                 990
   Glu Glu Leu Ser Ser Arg Tyr Gly Gly Gln Ile Thr Glu Thr Asp Ile
           995                 1000                1005
   Ala Ser Tyr Val Met Tyr Pro Lys Val Phe Glu Gln Phe Arg Asn Ile
       1010                1015                1020
   Ile Asp Lys Tyr Gly Asp Leu Ser Val Leu Pro Thr Lys Tyr Phe Leu
   1025 1030 1035 1040
   Lys Pro Cys Ala Ile Gly Glu Glu Leu Ser Val Asp Ile Glu Gln Gly
   1045 1050 1055
   Lys Thr Leu Ile Ile Lys Leu Leu Ala Val Gly Asp Ile Ser Asp Lys
   1060 1065 1070
   Thr Gly Asn Arg Glu Val Phe Phe Glu Leu Asn Gly Glu Met Arg Ser
   1075 1080 1085
   Val Ser Val Glu Asp Lys Ala Ala Ala Val Glu Thr Arg Val Lys Pro
   1090 1095 1100
   Lys Ala Ser Ser Pro Asn Asp Val Gly Ala Pro Met Ala Gly Val Val
   1105 1110 1115 1120
   Ile Glu Ile Arg Ala His Arg His Gln Glu Val Lys Lys Gly Asp Pro
   1125 1130 1135
   Ile Ala Val Leu Ser Ala Met Lys Met Glu Met Val Ile Ser Ser Pro
   1140 1145 1150
   Cys Asp Gly Glu Val Gly Glu Ile Val Ile His Glu Gly Asp Ser Val
   1155 1160 1165
   Asp Ala Asn Asp Leu Ile Thr Asn Ile Ile Gln His
   1170 1175 1180
<210> 10
   <211> 1176
   <212> PRT
   <213> Naumovozyma castellii
<220>
   <223> NcaPYC
<400> 10
   Met Ser Ile Asn Lys Lys Leu Asn Gly Leu Arg Asp Asn Phe Asn Leu
                       5                   10                  15
   Leu Gly Thr Arg Asn Lys Ile Leu Val Ala Asn Arg Gly Glu Ile Pro
               20                  25                  30
   Ile Arg Ile Phe Arg Ser Ala His Glu Leu Ser Met Gln Thr Val Ala
           35                  40                  45
   Ile Tyr Ser His Glu Asp Arg Leu Ser Thr His Arg Leu Lys Ala Asp
       50                  55                  60
   Glu Ser Tyr Val Ile Gly Glu Pro His Gln Phe Thr Pro Val Gly Ala
   65                  70                  75                  80
   Tyr Leu Ala Ile Asp Glu Ile Ile Asn Ile Ala Lys Arg His Gly Val
                   85                  90                  95
   Asp Tyr Ile His Pro Gly Tyr Gly Phe Leu Ser Glu Asn Ser Glu Phe
               100                 105                 110
   Ala Asp Lys Val Ala Lys Ala Gly Ile Thr Trp Ile Gly Pro Pro Ala
           115                 120                 125
   Ser Val Ile Asp Ser Val Gly Asp Lys Val Ser Ala Arg Asn Leu Ala
       130                 135                 140
   Thr Lys Ala Asn Val Pro Thr Val Pro Gly Thr Pro Gly Pro Ile Arg
   145                 150                 155                 160
   Thr Val Gln Asp Ala Gln Asp Phe Val Asn Glu Tyr Gly Tyr Pro Val
                   165                 170                 175
   Ile Ile Lys Ala Ala Phe Gly Gly Gly Gly Arg Gly Met Arg Val Val
               180                 185                 190
   Asn Glu Gly Asp Asp Leu Ala Asp Ala Phe Gln Arg Ala Ser Ser Glu
           195                 200                 205
   Ala Leu Thr Ala Phe Gly Asp Gly Thr Cys Phe Val Glu Arg Phe Leu
       210                 215                 220
   Asn Lys Pro Lys His Ile Glu Val Gln Leu Leu Ala Asp Thr His Gly
   225                 230                 235                 240
   Asn Val Ile His Leu Phe Glu Arg Asp Cys Ser Val Gln Arg Arg His
                   245                 250                 255
   Gln Lys Val Val Glu Val Ala Pro Ala Lys Thr Leu Pro Arg Glu Val
               260                 265                 270
   Arg Asp Ala Ile Leu Thr Asp Ala Val Lys Leu Ala Lys Glu Cys Gly
           275                 280                 285
   Tyr Gln Asn Ala Gly Thr Ala Glu Phe Leu Val Asp Asp Gln Asn Arg
       290                 295                 300
   His Tyr Phe Ile Glu Ile Asn Pro Arg Ile Gln Val Glu His Thr Ile
   305                 310                 315                 320
   Thr Glu Glu Ile Thr Gly Ile Asp Ile Val Ala Ala Gln Ile Gln Ile
                   325                 330                 335
   Ala Ala Gly Ala Ser Leu Glu Gln Leu Gly Leu Leu Gln Asp Lys Ile
               340                 345                 350
   Thr Thr Arg Gly Phe Ala Ile Gln Cys Arg Ile Thr Thr Glu Asp Pro
           355                 360                 365
   Ala Lys Asn Phe Gln Pro Asp Thr Gly Arg Leu Glu Val Tyr Asp Ser
       370                 375                 380
   Ala Gly Gly Asn Gly Val Arg Leu Asp Gly Gly Asn Ala Tyr Ala Gly
   385                 390                 395                 400
   Ala Ile Ile Ser Pro His Tyr Asp Ser Met Leu Val Lys Cys Ser Cys
                   405                 410                 415
   Ser Gly Ser Thr Tyr Glu Val Val Arg Arg Lys Met Ile Arg Ala Leu
               420                 425                 430
   Ile Glu Phe Arg Ile Arg Gly Val Lys Thr Asn Ile Pro Phe Leu Leu
           435                 440                 445
   Thr Leu Leu Thr His Pro Val Phe Ile Ala Gly Asp Tyr Trp Thr Thr
       450                 455                 460
   Phe Ile Asp Asp Thr Pro Gln Leu Phe Lys Met Val Ser Ser Gln Asn
   465                 470                 475                 480
   Arg Ala Gln Lys Leu Leu His Tyr Leu Ala Asp Leu Ala Val Asn Gly
                   485                 490                 495
   Ser Ser Ile Lys Gly Gln Val Gly Ile Pro Gln Leu Lys Lys Val Pro
               500                 505                 510
   Asp Ile Pro His Ile His Asp Ala Gln Gly Asn Val Ile Asp Val Thr
           515                 520                 525
   Asn Val Pro Pro Pro Ala Gly Trp Arg Gln Val Leu Leu Glu Gln Gly
       530                 535                 540
   Pro Glu Glu Phe Ala Lys Gln Val Arg Gln Phe Gln Gly Thr Leu Leu
   545                 550                 555                 560
   Met Asp Thr Thr Trp Arg Asp Ala His Gln Ser Leu Leu Ala Thr Arg
                   565                 570                 575
   Val Arg Thr His Asp Leu Ala Lys Ile Ala Pro Thr Thr Ala His Ala
               580                 585                 590
   Leu Ala Ser Ala Phe Ala Leu Glu Cys Trp Gly Gly Ala Thr Phe Asp
           595                 600                 605
   Val Ala Met Arg Phe Leu His Glu Asp Pro Trp Lys Arg Leu Arg Thr
       610                 615                 620
   Leu Arg Lys Leu Val Pro Asn Ile Pro Phe Gln Met Leu Leu Arg Gly
   625                 630                 635                 640
   Ala Asn Gly Val Ala Tyr Ser Ser Leu Pro Asp Asn Ala Ile Asp His
                   645                 650                 655
   Phe Val Lys Gln Ala Lys Asp Asn Gly Val Asp Ile Phe Arg Val Phe
               660                 665                 670
   Asp Ala Leu Asn Asp Leu Asp Gln Leu Lys Val Gly Val Asp Ala Val
           675                 680                 685
   Lys Lys Ala Gly Gly Val Val Glu Ala Thr Val Cys Tyr Ser Gly Asp
       690                 695                 700
   Met Leu Gln Pro Gly Lys Lys Tyr Asn Leu Asp Tyr Tyr Leu Glu Val
   705                 710                 715                 720
   Thr Glu Lys Ile Val Gln Met Gly Thr His Ile Leu Gly Ile Lys Asp
                   725                 730                 735
   Met Ala Gly Thr Met Lys Pro Ala Ala Ala Arg Leu Leu Ile Gly Ser
               740                 745                 750
   Ile Arg Ala Lys Tyr Pro Asp Leu Pro Ile His Val His Thr His Asp
           755                 760                 765
   Ser Ala Gly Thr Ala Val Ala Ser Met Ser Ala Ala Ala Tyr Ala Gly
       770                 775                 780
   Ala Asp Val Ile Asp Val Ala Thr Asn Ser Met Ser Gly Leu Thr Ser
   785                 790                 795                 800
   Gln Pro Ser Ile Asn Ala Leu Leu Ala Ser Leu Asp Gly Glu Ile Asn
                   805                 810                 815
   Thr Ser Ile Asn Val Gln His Val Arg Glu Leu Asp Ala Tyr Trp Ala
               820                 825                 830
   Glu Met Arg Leu Leu Tyr Ser Cys Phe Gly Ala Asp Leu Lys Gly Pro
           835                 840                 845
   Asp Pro Glu Val Tyr Glu His Glu Ile Pro Gly Gly Gln Leu Thr Asn
       850                 855                 860
   Leu Leu Phe Gln Ala Gln Gln Leu Gly Leu Gly Glu Gln Trp Thr Glu
   865                 870                 875                 880
   Thr Lys Arg Ala Tyr Lys Glu Ala Asn Met Leu Leu Gly Asp Ile Val
                   885                 890                 895
   Lys Val Thr Pro Thr Ser Lys Val Val Gly Asp Leu Ala Gln Phe Met
               900                 905                 910
   Val Ser Asn Lys Leu Thr Ser Glu Asp Val Lys Arg Leu Ala Asn Ser
           915                 920                 925
   Leu Asp Phe Pro Asp Ser Val Met Asp Phe Phe Glu Gly Leu Met Gly
       930                 935                 940
   Gln Pro Tyr Gly Gly Phe Pro Glu Pro Leu Arg Ser Asp Ile Leu Arg
   945                 950                 955                 960
   Asn Lys Arg Arg Lys Leu Thr Cys Arg Pro Gly Leu Glu Leu Val Pro
                   965                 970                 975
   Phe Glu Leu Asn Asn Ile Lys Glu Asp Leu Gln Thr Arg Phe Gly Asp
               980                 985                 990
   Ile Asn Glu Cys Asp Ile Ala Ser Tyr Asn Met Tyr Pro Lys Val Tyr
       995                 1000                1005
   Glu Asp Phe Gln Lys Met Lys Glu Lys Tyr Gly Asp Leu Ser Val Leu
      1010                1015                1020
   Pro Thr Lys Ser Phe Leu Ala Pro Ala Glu Ile Gly Glu Glu Ile Leu
   1025                1030                1035                1040
   Val Thr Ile Gln Gln Gly Lys Thr Leu Ile Ile Lys Leu Gln Ala Ile
                   1045                1050                1055
   Gly Asp Leu Asn Lys Glu Thr Gly Lys Arg Glu Val Phe Phe Glu Leu
               1060                1065                1070
   Asn Gly Glu Met Arg Lys Ile Ser Val Thr Asp Lys Ser Gln Lys Val
           1075                1080                1085
   Glu Thr Val Ala Lys Pro Lys Ala Asp Thr His Asp Pro Phe Gln Ile
       1090                1095                1100
   Gly Ala Pro Met Ala Gly Val Ile Val Glu Val Lys Val His Lys Gly
   1105                1110                1115                1120
   Ser Leu Val Lys Lys Gly Glu Ala Val Ala Val Leu Ser Ala Met Lys
                   1125                1130                1135
   Met Glu Met Val Ile Ser Ser Pro Ala Asp Gly Leu Val Lys Glu Val
                1140                1145                1150
   Leu Val Asn Glu Ser Glu Asn Val Asp Ala Ser Asp Leu Leu Val Leu
         1155                1160                1165
   Leu  Glu Glu Ser Thr Pro Pro Ser
       1170                1175
<210> 11
   <211> 1178
   <212> PRT
   <213> Naumovozyma dairenensis
<220>
   <223> NdaPYC
<400> 11
   Met Ser Asn Lys Lys Phe Asn Gly Ile Arg Asp Asn Phe Asn Leu Leu
                   5                   10                  15
   Gly Pro Lys Asn Lys Ile Leu Val Ala Asn Arg Gly Glu Ile Pro Ile
               20                  25                  30
   Arg Ile Phe Arg Thr Ser His Glu Leu Ser Met Lys Thr Val Ala Ile
           35                  40                  45
   Tyr Ser His Glu Asp Arg Leu Ser Thr His Arg Leu Lys Ala Asp Glu
       50                  55                  60
   Ser Tyr Val Ile Gly Glu Pro Asn Gln Phe Thr Pro Val Gly Ala Tyr
   65                  70                  75                  80
   Leu Ala Ile Asp Glu Ile Ile Asn Ile Ala Lys Lys His Asn Val Asp
                   85                  90                  95
   Phe Ile His Pro Gly Tyr Gly Phe Leu Ser Glu Asn Ser Glu Phe Ala
               100                 105                 110
   Asp Lys Val Ala Lys Asn Gly Ile Ala Trp Val Gly Pro Pro Ala Ser
           115                 120                 125
   Val Ile Asp Ser Val Gly Asp Lys Val Ser Ala Arg His Leu Ala Glu
       130                 135                 140
   Arg Ala Asn Val Pro Thr Val Pro Gly Thr Pro Gly Pro Ile Lys Ser
   145                 150                 155                 160
   Ala Lys Glu Ala Glu Glu Phe Val Ala Lys Tyr Gly Phe Pro Val Ile
                   165                 170                 175
   Ile Lys Ala Ala Phe Gly Gly Gly Gly Arg Gly Met Arg Val Val Arg
               180                 185                 190
   Glu Gly Asp Asp Ile Ala Asp Ala Phe Gln Arg Ala Ser Ser Glu Ala
           195                 200                 205
   Val Thr Ala Phe Gly Asn Gly Thr Cys Phe Val Glu Arg Phe Leu Asn
       210                 215                 220
   Lys Pro Lys His Ile Glu Val Gln Leu Leu Ala Asp Asn Tyr Gly Asn
   225                 230                 235                 240
   Val Ile His Leu Phe Glu Arg Asp Cys Ser Val Gln Arg Arg His Gln
                   245                 250                 255
   Lys Val Val Glu Val Ala Pro Ala Lys Thr Leu Pro Arg Asp Ile Arg
               260                 265                 270
   Asn Ala Ile Leu Thr Asp Ala Val Lys Leu Ala Lys Glu Cys Gly Tyr
           275                 280                 285
   Lys Asn Ala Gly Thr Ala Glu Phe Leu Val Asp Asp Gln Asn Arg His
       290                 295                 300
   Tyr Phe Ile Glu Ile Asn Pro Arg Ile Gln Val Glu His Thr Ile Thr
   305                 310                 315                 320
   Glu Glu Ile Thr Gly Ile Asp Ile Val Ala Ala Gln Ile Gln Ile Ala
                   325                 330                 335
   Ala Gly Ala Ser Leu Glu Gln Leu Gly Leu Leu Gln Asp Lys Ile Thr
               340                 345                 350
   Thr Arg Gly Phe Ala Ile Gln Cys Arg Ile Thr Thr Glu Asp Pro Ala
           355                 360                 365
   Lys Asn Phe Gln Pro Asp Thr Gly Arg Ile Glu Val Tyr Gly Ser Ala
       370                 375                 380
   Gly Gly Asn Gly Val Arg Leu Asp Gly Gly Asn Ala Tyr Ala Gly Ala
   385                 390                 395                 400
   Ile Ile Ser Pro His Tyr Asp Ser Met Leu Val Lys Cys Ser Cys Ser
                   405                 410                 415
   Gly Ser Thr Tyr Glu Val Val Arg Arg Lys Met Ile Arg Ala Leu Ile
               420                 425                 430
   Glu Phe Arg Ile Arg Gly Val Lys Thr Asn Ile Pro Phe Leu Leu Thr
           435                 440                 445
   Leu Leu Thr His Pro Val Phe Ile Ser Gly Gln Tyr Trp Thr Thr Phe
       450                 455                 460
   Ile Asp Asp Thr Pro Gln Leu Phe Gln Met Val Ser Ser Gln Asn Arg
   465 470 475 480
   Ala Gln Lys Leu Leu His Tyr Leu Ala Asp Ile Ala Val Asn Gly Ser
   485 490 495
   Ser Ile Lys Gly Gln Val Gly Ile Pro Arg Leu Thr Lys Ile Pro Asp
   500 505 510
   Ile Pro Pro Leu Tyr Asp Thr Asn Asn Asn Lys Ile Asp Val Leu Lys
   515 520 525
   Thr Pro Pro Pro Ala Gly Trp Arg Gln Val Leu Leu Glu Lys Gly Pro
   530 535 540
   Val Glu Phe Ala Lys Gln Val Arg Gln Phe Gln Gly Thr Leu Leu Met
   545 550 555 560
   Asp Thr Thr Trp Arg Asp Ala His Gln Ser Leu Leu Ala Thr Arg Val
   565 570 575
   Arg Thr Tyr Asp Leu Ala Thr Ile Ala Pro Thr Thr Ala His Ala Leu
   580 585 590
   Ser Gly Ala Phe Ala Leu Glu Cys Trp Gly Gly Ala Thr Phe Asp Val
   595 600 605
   Ala Met Arg Phe Leu His Glu Asp Pro Trp Ala Arg Leu Arg Thr Leu
   610 615 620
   Arg Lys Leu Val Pro Asn Ile Pro Phe Gln Met Leu Leu Arg Gly Ala
   625 630 635 640
   Asn Gly Val Ala Tyr Ser Ser Leu Pro Asp Asn Ala Ile Asp His Phe
   645 650 655
   Val Lys Gln Ala Lys Asp Asn Gly Val Asp Ile Phe Arg Val Phe Asp
   660 665 670
   Ala Leu Asn Asp Leu Asp Gln Leu Lys Val Gly Val Asp Ala Val Lys
   675 680 685
   Lys Ala Gly Gly Val Val Glu Ala Thr Leu Cys Tyr Ser Gly Asp Met
   690 695 700
   Leu Ala Lys Gly Lys Lys Tyr Asn Leu Asp Tyr Tyr Leu Glu Val Thr
   705 710 715 720
   Asp Lys Ile Val Gln Met Gly Thr His Phe Leu Gly Ile Lys Asp Met
   725 730 735
   Ala Gly Thr Met Lys Pro Ala Ala Ala Lys Leu Leu Ile Gly Ser Ile
   740 745 750
   Arg Ala Lys Tyr Pro Asp Leu Pro Ile His Val His Thr His Asp Ser
   755 760 765
   Ala Gly Thr Ala Val Ser Ser Met Ala Ala Ala Ala Val Ser Gly Ala
   770 775 780
   Asp Val Val Asp Val Ala Ile Asn Ser Met Ser Gly Leu Thr Ser Gln
   785 790 795 800
   Pro Ser Ile Asn Ala Leu Leu Ala Ser Leu Asp Gly Glu Ile Asp Thr
   805 810 815
   Gly Ile Asn Val Gln His Val Arg Asp Leu Asp Ala Tyr Trp Ala Glu
   820 825 830
   Met Arg Leu Leu Tyr Ser Cys Phe Gly Ala Asp Leu Lys Gly Pro Asp
   835 840 845
   Pro Glu Val Tyr Gln His Glu Ile Pro Gly Gly Gln Leu Thr Asn Leu
   850 855 860
   Leu Phe Gln Ala Gln Gln Leu Gly Leu Gly Glu Gln Trp Ala Glu Thr
   865 870 875 880
   Lys Arg Ala Tyr Arg Glu Ala Asn His Leu Leu Gly Asp Ile Val Lys
   885 890 895
   Val Thr Pro Thr Ser Lys Val Val Gly Asp Leu Ala Gln Phe Met Val
   900 905 910
   Ser Asn Lys Leu Thr Ser Asp Asp Val Lys Arg Leu Ala Asn Ser Leu
   915 920 925
   Asp Phe Pro Asp Ser Val Met Asp Phe Phe Glu Gly Leu Met Gly Gln
   930 935 940
   Pro Tyr Gly Gly Phe Pro Glu Pro Leu Arg Ser Asp Ile Leu Arg Asn
   945                 950                 955                 960
   Lys Arg Arg Lys Leu Thr Ser Arg Pro Gly Leu Glu Leu Ala Pro Phe
                   965                 970                 975
   Asp Leu Asn Thr Ile Arg Glu Asp Leu Gln Thr Arg Phe Gly Asp Asp
               980                 985                 990
   Ile Asp Glu Cys Asp Val Ala Ser Tyr Asn Met Tyr Pro Lys Val Tyr
           995                 1000                1005
   Glu Asp Phe Gln Lys Ile Arg Glu Thr Tyr Gly Asp Leu Ser Val Leu
      1010                1015                1020
   Pro Thr Lys Asn Phe Leu Ala Pro Ala Glu Ile Gly Glu Glu Ile Glu
   1025                1030                1035                1040
   Ile Val Ile Glu Gln Gly Lys Thr Leu Ile Val Lys Leu Gln Ala Ile
                   1045                1050                1055
   Gly Asp Leu Asn Lys Lys Thr Gly Ile Arg Glu Val Tyr Phe Glu Leu
               1060                1065                1070
   Asn Gly Glu Met Arg Lys Ile Gly Thr Leu Asp Arg Ser Gln Lys Val
           1075                1080                1085
   Glu Thr Val Ala Lys Pro Lys Ala Asp Gly His Asn Pro Phe Gln Ile
       1090                1095                1100
   Gly Ala Pro Met Ala Gly Val Ile Val Glu Val Arg Val His Lys Gly
   1105                1110                1115                1120
   Ser Leu Val Lys Lys Gly Glu Ala Val Ala Val Leu Ser Ala Met Lys
                   1125                1130                1135
   Met Glu Met Ile Ile Ser Ser Pro Thr Asp Gly Gln Val Lys Asp Val
               1140                1145                1150
   Leu Val Asn Asp Gly Glu Asn Val Glu Ala Ser Asp Leu Leu Val Leu
           1155                1160                1165
   Leu Glu Glu Thr  Glu Ile Pro Glu Glu Ala
       1170                1175
<210> 12
   <211> 1178
   <212> PRT
   <213> Saccharomyces kudriavzevii
<220>
   <223> SkuPYC
<400> 12
   Met Ser Gln Lys Lys Phe Ala Gly Leu Arg Asp Asn Phe Asn Leu Leu
                       5                  10                  15
   Gly Glu Lys Asn Lys Ile Leu Val Ala Asn Arg Gly Glu Ile Pro Ile
                   20                  25                  30
   Arg Ile Phe Arg Thr Ala His Glu Leu Ser Met Gln Thr Val Ala Ile
               35                  40                  45
   Tyr Ser His Glu Asp Arg Leu Ser Thr His Lys Gln Lys Ala Asp Glu
       50                  55                  60
   Ala Tyr Val Ile Gly Glu Ala Gly Gln Tyr Thr Pro Val Gly Ala Tyr
   65                  70                  75                  80
   Leu Ala Ile Asp Glu Ile Ile Ser Ile Ala Gln Lys His Gln Val Asp
                   85                  90                  95
   Phe Ile His Pro Gly Tyr Gly Phe Leu Ser Glu Asn Ser Glu Phe Ala
               100                 105                 110
   Asp Lys Val Ala Lys Ala Gly Ile Thr Trp Ile Gly Pro Pro Ala Glu
           115                 120                 125
   Val Ile Asn Ser Val Gly Asp Lys Val Ser Ala Arg Asn Leu Ala Ala
       130                 135                 140
   Arg Ala Asn Val Pro Thr Val Pro Gly Thr Pro Gly Pro Ile Glu Ser
   145                 150                 155                 160
   Val Gln Glu Ala Leu Asp Phe Val Gly Glu Tyr Gly Tyr Pro Val Ile
                   165                 170                 175
   Ile Lys Ala Ala Phe Gly Gly Gly Gly Arg Gly Met Arg Val Val Arg
                   180                 185                 190
   Glu Gly Asp Asp Val Ala Asp Ala Phe Gln Arg Ala Thr Ser Glu Ala
               195                 200                 205
   Arg Thr Ala Phe Gly Asn Gly Thr Cys Phe Val Glu Arg Phe Leu Val
       210                 215                 220
   Lys Pro Lys His Ile Glu Val Gln Leu Leu Ala Asp Asn His Gly Asn
   225                 230                 235                 240
   Val Val His Leu Phe Glu Arg Asp Cys Ser Val Gln Arg Arg His Gln
                   245                 250                 255
   Lys Val Val Glu Val Ala Pro Ala Lys Thr Leu Pro Arg Glu Val Arg
                   260                 265                 270
   Asp Ala Ile Leu Thr Asp Ala Val Lys Leu Ala Lys Glu Cys Gly Tyr
               275                 280                 285
   Arg Asn Ala Gly Thr Ala Glu Phe Leu Val Asp Asn Gln Asn Arg His
       290                 295                 300
   Tyr Phe Ile Glu Ile Asn Pro Arg Ile Gln Val Glu His Thr Ile Thr
   305                 310                 315                 320
   Glu Glu Ile Thr Gly Ile Asp Ile Val Ala Ala Gln Ile Gln Ile Ala
                   325                 330                 335
   Ala Gly Ala Ser Leu Pro Gln Leu Gly Leu Phe Gln Asp Lys Ile Thr
                   340                 345                 350
   Thr Arg Gly Phe Ala Ile Gln Cys Arg Ile Thr Thr Glu Asp Pro Ala
               355                 360                 365
   Lys Asn Phe Gln Pro Asp Thr Gly Arg Ile Glu Val Tyr Arg Ser Ala
       370                 375                 380
   Gly Gly Asn Gly Val Arg Leu Asp Gly Gly Asn Ala Tyr Ala Gly Thr
   385                 390                 395                 400
   Ile Ile Ser Pro His Tyr Asp Ser Met Leu Val Lys Cys Ser Cys Ser
                   405                 410                 415
   Gly Ser Thr Tyr Glu Ile Val Arg Arg Lys Met Ile Arg Ala Leu Ile
                   420                 425                 430
   Glu Phe Arg Ile Arg Gly Val Lys Thr Asn Met Pro Phe Leu Leu Thr
               435                 440                 445
   Leu Leu Thr His Pro Val Phe Ile Asp Gly Thr Tyr Trp Thr Thr Phe
       450                 455                 460
   Ile Asp Asp Thr Pro Gln Leu Phe Gln Met Val Ser Ser Gln Asn Arg
   465                 470                 475                 480
   Ala Gln Lys Leu Leu His Tyr Leu Ala Asp Val Ala Val Asn Gly Ser
                   485                 490                 495
   Ser Ile Lys Gly Gln Ile Gly Ser Pro Lys Leu Gly Ser Asn Pro Ser
                   500                 505                 510
   Ile Pro His Leu His Asp Ser Gln Gly Asn Val Ile Asn Val Thr Lys
               515                 520                 525
   Thr Thr Pro Pro Ser Gly Trp Arg Gln Val Leu Leu Glu Lys Gly Pro
       530                 535                 540
   Ala Glu Phe Ala Lys Gln Val Arg Gln Phe Asn Gly Thr Leu Leu Met
   545                 550                 555                 560
   Asp Thr Thr Trp Arg Asp Ala His Gln Ser Leu Leu Ala Thr Arg Val
                   565                 570                 575
   Arg Thr His Asp Leu Ala Thr Ile Ala Pro Thr Thr Ala His Ala Leu
                   580                 585                 590
   Ala Gly Ala Phe Ala Leu Glu Cys Trp Gly Gly Ala Thr Phe Asp Val
               595                 600                 605
   Ala Met Arg Phe Leu His Glu Asp Pro Trp Glu Arg Leu Arg Lys Leu
       610                 615                 620
   Arg Ala Leu Val Pro Asn Ile Pro Phe Gln Met Leu Leu Arg Gly Ala
   625                 630                 635                 640
   Asn Gly Val Ala Tyr Ser Ser Leu Pro Asp Asn Ala Ile Asp His Phe
                   645                 650                 655
   Val Lys Gln Ala Lys Asp Asn Gly Val Asp Ile Phe Arg Val Phe Asp
                   660                 665                 670
   Ala Leu Asn Asp Leu Asp Gln Leu Lys Val Gly Val Asp Ala Val Lys
   675 680 685
   Lys Ala Gly Gly Val Val Glu Ala Thr Val Cys Phe Ser Gly Asp Met
   690 695 700
   Leu Gln Pro Gly Lys Lys Tyr Asn Leu Asp Tyr Tyr Leu Glu Ile Ala
   705 710 715 720
   Glu Lys Ile Val Lys Met Gly Thr His Ile Leu Gly Ile Lys Asp Met
   725 730 735
   Ala Gly Thr Met Lys Pro Ala Ala Ala Lys Leu Leu Ile Gly Ser Leu
   740 745 750
   Arg Ala Lys Tyr Pro Asp Leu Pro Ile His Val His Thr His Asp Ser
   755 760 765
   Ala Gly Thr Ala Val Ala Ser Met Ala Ala Cys Ala Leu Ala Gly Ala
   770 775 780
   Asp Val Val Asp Val Ala Ile Asn Ser Met Ser Gly Leu Thr Ser Gln
   785 790 795 800
   Pro Ser Ile Asn Ala Leu Leu Ala Ser Leu Glu Gly Asn Ile Asp Thr
   805 810 815
   Gly Ile Asn Val Glu His Val Arg Glu Leu Asp Ala Tyr Trp Ala Glu
   820 825 830
   Met Arg Leu Leu Tyr Ser Cys Phe Glu Ala Asp Leu Lys Gly Pro Asp
   835 840 845
   Pro Glu Val Tyr Gln His Glu Ile Pro Gly Gly Gln Leu Thr Asn Leu
   850 855 860
   Leu Phe Gln Ala Gln Gln Leu Gly Leu Gly Glu Gln Trp Ala Glu Thr
   865 870 875 880
   Lys Arg Ala Tyr Arg Glu Ala Asn Tyr Leu Leu Gly Asp Ile Val Lys
   885 890 895
   Val Thr Pro Thr Ser Lys Val Val Gly Asp Leu Ala Gln Phe Met Val
   900 905 910
   Ser Asn Lys Leu Thr Pro Asp Asp Val Lys Arg Leu Ala Asn Ser Leu
   915 920 925
   Asp Phe Pro Asp Ser Val Met Asp Phe Phe Glu Gly Leu Ile Gly Gln
   930 935 940
   Pro Tyr Gly Gly Phe Pro Glu Pro Phe Arg Ser Asp Val Leu Arg Asn
   945 950 955 960
   Lys Arg Arg Lys Leu Thr Cys Arg Pro Gly Leu Glu Leu Glu Pro Phe
   965 970 975
   Asp Leu Glu Lys Ile Arg Glu Asp Leu Gln Asn Arg Phe Gly Asp Ile
   980 985 990
   Asn Glu Cys Asp Val Ala Ser Tyr Asn Met Tyr Pro Arg Val Tyr Glu
   995 1000 1005
   Asp Phe Gln Lys Met Arg Glu Thr Tyr Gly Asp Leu Ser Val Leu Pro
   1010 1015 1020
   Thr Arg Ser Phe Leu Ser Pro Leu Glu Thr Asp Glu Glu Ile Glu Val
   1025 1030 1035 1040
   Ile Ile Glu Gln Gly Lys Thr Leu Ile Ile Lys Leu Gln Ala Val Gly
   1045 1050 1055
   Asp Leu Asn Lys Lys Thr Gly Glu Arg Glu Val Tyr Phe Asp Leu Asn
   1060 1065 1070
   Gly Glu Met Arg Lys Ile Arg Val Val Asp Arg Ser Leu Lys Val Ala
   1075 1080 1085
   Thr Val Thr Lys Ser Lys Ala Asp Met His Asp Pro Leu His Ile Gly
   1090 1095 1100
   Ala Pro Met Ala Gly Val Ile Val Glu Val Lys Val His Lys Gly Ser
   1105 1110 1115 1120
   Leu Ile Lys Lys Gly Gln Pro Val Ala Val Leu Ser Ala Met Lys Met
   1125 1130 1135
   Glu Met Ile Ile Ser Ser Pro Ser Asp Gly Gln Val Lys Glu Val Phe
   1140 1145 1150
   Val Ser Glu Gly Glu Asn Val Asp Ser Ser Asp Leu Leu Val Leu Leu
   1155 1160 1165
   Glu Asp Gln Val Pro Ala Glu Thr Lys Ala
   1170 1175
<210> 13
   <211> 1185
   <212> PRT
   <213> Schizosaccharomyces pombe
<220>
   <223> SpoPYC
<400> 13
   Met Thr Ser Lys Tyr Asp Ala Leu Leu His Asn Gln Ser Thr Asn Thr
   5 10 15
   Asn Pro Phe Ser Lys Leu Gln Asp Arg Ser Ser Leu Leu Gly Glu Lys
   20 25 30
   Phe Thr Lys Val Leu Val Ala Asn Arg Ser Glu Ile Ala Ile Arg Val
   35 40 45
   Phe Arg Thr Ala His Glu Leu Ser Met His Thr Val Ala Ile Tyr Ser
   50 55 60
   Tyr Glu Asp Arg Leu Ser Met His Arg Gln Lys Ala Asp Glu Ser Tyr
   65 70 75 80
   Pro Ile Gly Lys Val Gly Gln Tyr Ser Pro Val Gly Ala Tyr Leu Ala
   85 90 95
   Ile Asp Glu Ile Val Ser Ile Ala Lys Arg Thr Gly Ala Asn Leu Val
   100 105 110
   His Pro Gly Tyr Gly Phe Leu Ser Glu Asn Ala Glu Phe Ala Arg Lys
   115 120 125
   Val Asn Glu Ala Gly Met Gln Phe Val Gly Pro Ser Pro Glu Val Ile
   130 135 140
   Asp Ser Leu Gly Asp Lys Thr Lys Ala Arg Ala Ile Ala Ile Arg Cys
   145 150 155 160
   Gly Val Pro Val Val Pro Gly Thr Pro Gly Pro Val Glu His Tyr Glu
   165 170 175
   Glu Ala Glu Ala Phe Val Lys Glu Tyr Gly Leu Pro Val Ile Ile Lys
   180 185 190
   Ala Ala Met Gly Gly Gly Gly Arg Gly Met Arg Val Val Arg Ser Ala
   195 200 205
   Asp Thr Leu Lys Glu Ser Phe Glu Arg Ala Arg Ser Glu Ala Leu Ala
   210 215 220
   Ser Phe Gly Asp Gly Thr Val Phe Ile Glu Arg Phe Leu Asp Lys Pro
   225 230 235 240
   Lys His Ile Glu Ile Gln Leu Met Ala Asp Lys Ala Gly Asn Val Ile
   245 250 255
   His Leu His Glu Arg Asp Cys Ser Val Gln Arg Arg His Gln Lys Val
   260 265 270
   Val Glu Ile Ala Pro Ala Lys Asp Leu Asp Pro Lys Ile Arg Gln Ala
   275 280 285
   Leu Tyr Asp Asp Ala Ile Lys Ile Ala Lys Glu Val Lys Tyr Cys Asn
   290 295 300
   Ala Gly Thr Ala Glu Phe Leu Leu Asp Gln Lys Gly Arg His Tyr Phe
   305 310 315 320
   Ile Glu Ile Asn Pro Arg Ile Gln Val Glu His Thr Ile Thr Glu Glu
   325 330 335
   Ile Thr Gly Val Asp Ile Val Ser Ala Gln Leu His Val Ala Ala Gly
   340 345 350
   Phe Thr Leu Pro Glu Ile Gly Leu Thr Gln Asp Lys Ile Ser Thr Arg
   355 360 365
   Gly Phe Ala Ile Gln Cys Arg Val Thr Thr Glu Asp Pro Asn Asn Gly
   370 375 380
   Phe Ala Pro Asp Ile Gly Lys Ile Glu Val Tyr Arg Ser Ala Gly Gly
   385                 390                 395                 400
   Asn Gly Val Arg Leu Asp Gly Ala Asn Gly Phe Ala Gly Ser Val Ile
                   405                 410                 415
   Thr Pro His Tyr Asp Ser Met Leu Val Lys Cys Thr Cys His Asp Ala
               420                 425                 430
   Thr Tyr Glu Tyr Thr Arg Arg Lys Met Ile Arg Ser Leu Ile Glu Phe
           435                 440                 445
   Arg Val Arg Gly Val Lys Thr Asn Ile Pro Phe Val Leu Arg Leu Leu
       450                 455                 460
   Met His Asp Thr Phe Ile Gln Gly Asn Cys Trp Thr Thr Phe Ile Asp
   465                 470                 475                 480
   Asp Thr Pro Glu Leu Phe Gln Leu Tyr Arg Ser Arg Asn Arg Ala Gln
                   485                 490                 495
   Lys Leu Leu Ala Tyr Leu Gly Asp Leu Ala Val Asn Gly Ser Ser Ile
               500                 505                 510
   Lys Gly Gln Asn Gly Glu Pro Ala Leu Lys Ser Glu Ile Val Met Pro
           515                 520                 525
   Val Leu Leu Asp Ser Thr Gly Asn Gln Ile Asp Val Ser His Pro Ser
       530                 535                 540
   Glu Lys Gly Trp Arg Lys Leu Leu Leu Asp Asn Gly Pro Ala Ala Phe
   545                 550                 555                 560
   Ala Lys Ala Val Arg Asn His Lys Arg Gly Leu Ile Met Asp Thr Thr
                   565                 570                 575
   Trp Arg Asp Ala His Gln Ser Leu Leu Ala Thr Arg Val Arg Thr Ile
               580                 585                 590
   Asp Leu Val Asn Ile Ala Pro Tyr Thr Ser His Ala Leu Ala Ser Ala
           595                 600                 605
   Tyr Ser Leu Glu Met Trp Gly Gly Ala Thr Phe Asp Val Ser Met Arg
       610                 615                 620
   Phe Leu His Glu Cys Pro Trp Asp Arg Leu Arg Arg Leu Arg Lys Leu
   625                 630                 635                 640
   Val Pro Asn Ile Pro Phe Gln Met Leu Leu Arg Gly Ala Asn Gly Leu
                   645                 650                 655
   Cys Tyr Ser Ser Leu Pro Asp Asn Val Ile Tyr Phe Phe Cys Glu Gln
               660                 665                 670
   Ala Lys Lys Asn Gly Ile Asp Ile Phe Arg Val Phe Asp Ala Leu Asn
           675                 680                 685
   Asp Val Asn Asn Leu Ser Leu Gly Ile Asp Ala Ala Lys Arg Ala Gly
       690                 695                 700
   Gly Val Val Glu Ala Thr Met Cys Tyr Ser Gly Asp Met Leu Asn Pro
   705                 710                 715                 720
   Lys Lys Lys Tyr Asn Leu Asp Tyr Tyr Val Asn Leu Val Asp Lys Met
                   725                 730                 735
   Val Glu Met Gly Ile His Ile Leu Gly Ile Lys Asp Met Ala Gly Val
               740                 745                 750
   Met Lys Pro Lys Ala Ala Arg Leu Leu Ile Ser Ala Ile Arg Glu Lys
           755                 760                 765
   His Pro Glu Leu Pro Ile His Val His Thr His Asp Ser Ala Gly Thr
       770                 775                 780
   Ala Val Ala Ser Met Ala Ala Ala Leu Glu Ala Gly Ala Asp Val Val
   785                 790                 795                 800
   Asp Val Ala Thr Asp Ser Met Ser Gly Leu Thr Ser Gln Pro Ser Phe
                   805                 810                 815
   Gly Ala Val Leu Ala Ser Val Asp Gly Thr Asp Lys Gln Leu Glu Phe
               820                 825                 830
   Asp Asn Asn Gln Leu Arg Glu Ile Asp Ser Tyr Trp Ala Gln Met Arg
           835                 840                 845
   Leu Leu Tyr Ser Pro Phe Glu Ser Glu Ile Lys Gly Thr Asp Ser Asp
       850                 855                 860
   Val Tyr Asn His Glu Ile Pro Gly Gly Gln Leu Thr Asn Leu Lys Phe
   865                 870                 875                 880
   Gln Ala Thr Ser Leu Gly Leu Gly Thr Gln Trp Ala Glu Thr Lys Lys
                   885                 890                 895
   Ala Tyr Ile Glu Ala Asn Lys Leu Leu Gly Asp Ile Ile Lys Val Thr
               900                 905                 910
   Pro Thr Ser Lys Val Val Gly Asp Leu Ala Gln Phe Met Val Gln Asn
           915                 920                 925
   Lys Leu Ser Ala Glu Asp Val Glu Asn Arg Ala Thr Thr Leu Asp Phe
       930                 935                 940
   Pro Ala Ser Val Leu Asp Phe Phe Gln Gly Leu Met Gly Gln Pro Tyr
   945                 950                 955                 960
   Gly Gly Phe Pro Glu Pro Leu Arg Thr Asn Val Leu Lys Gly Arg Arg
                   965                 970                 975
   Gln Pro Leu Thr Asp Arg Pro Gly Lys Phe Leu Pro Ala Ala Asp Phe
               980                 985                 990
   Asp Ala Ile Arg Lys Leu Leu Ser Glu Lys Phe Gly Val Ser Ser Asp
           995                 1000                1005
   Cys Asp Ile Ala Ala Tyr Thr Gln Phe Pro Gly Val Phe Glu Glu Tyr
      1010                1015                1020
   Arg Gln Phe Val Asp Arg Tyr Gly Asp Leu Thr Thr Val Pro Thr Lys
   1025                1030                1035                1040
   Phe Phe Leu Ser Arg Pro Glu Met Asn Glu Glu Met His Val Glu Ile
                   1045                1050                1055
   Asp Gln Gly Lys Thr Leu Ile Val Lys Phe Val Ala Leu Gly Pro Leu
               1060                1065                1070
   Asn Pro Arg Thr Gly Gln Arg Glu Val Tyr Phe Glu Leu Asn Gly Glu
           1075                1080                1085
   Asn Arg His Val Thr Val Glu Asp Lys Lys Ala Ala Ile Glu Thr Val
       1090                1095                1100
   Thr Arg Pro Arg Ala Asp Pro Gly Asn Pro Gly His Val Ala Ala Pro
   1105                1110                1115                1120
   Met Ser Gly Thr Ile Val Glu Ile Arg Val Lys Glu Gly Ala Lys Val
                   1125                1130                1135
   Lys Lys Gly Asp Ile Ile Ala Val Leu Ser Ala Met Lys Met Glu Ile
               1140                1145                1150
   Val Ile Ser Ala Pro His Ser Gly Val Leu Lys Ser Leu Ala Val Val
           1155                1160                1165
   Gln Gly Asp Ser Val Asn Gly Gly Asp Leu Cys Ala Val Leu Glu His
       1170                1175                1180
   Glu
   1185
<210> 14
   <211> 1175
   <212> PRT
   <213> Tetrapisispora blattae
<220>
   <223> TblPYC
<400> 14
   Met Thr Asn Lys Lys Phe Ser Gly Leu Arg Asp Asn Phe Ser Leu Leu
                       5                  10                  15
   Gly Glu Lys Asn Lys Val Leu Val Ala Asn Arg Gly Glu Ile Pro Ile
                   20                  25                  30
   Arg Ile Phe Arg Thr Ala His Glu Leu Ser Met Arg Thr Val Ala Ile
               35                  40                  45
   Tyr Ser His Glu Asp Arg Leu Ala Met His Arg Leu Lys Ala Asp Glu
       50                  55                  60
   Ser Tyr Val Ile Gly Ala Glu Asn Gln Tyr Thr Pro Val Gly Ala Tyr
   65                  70                  75                  80
   Leu Ala Ile Asp Glu Ile Ile Asn Ile Ala Lys Lys His Asn Val Asp
                       85                  90                  95
   Phe Ile His Pro Gly Tyr Gly Phe Leu Ser Glu Asn Ser Glu Phe Ala
               100                 105                 110
   Arg Lys Val Thr Glu Ser Gly Ile Thr Trp Ile Gly Pro Pro Ala Ala
           115                 120                 125
   Val Ile Asp Ser Val Gly Asp Lys Val Ser Ala Arg Asn Leu Ala Ala
       130                 135                 140
   Lys Val Asn Val Pro Thr Val Pro Gly Thr Pro Gly Pro Ile Asp Thr
   145                 150                 155                 160
   Val Glu Glu Ala Lys Ala Phe Val Glu Lys His Gly Phe Pro Val Ile
                   165                 170                 175
   Ile Lys Ala Ala Phe Gly Gly Gly Gly Arg Gly Met Arg Val Val Arg
               180                 185                 190
   Glu Gly Asp Asp Ile Ala Asp Ala Phe Gln Arg Ala Thr Ser Glu Ala
           195                 200                 205
   Arg Thr Ala Phe Gly Asn Gly Thr Cys Phe Ile Glu Arg Phe Leu Asp
       210                 215                 220
   His Pro Lys His Ile Glu Val Gln Leu Leu Ala Asp Asn Tyr Gly Asn
   225                 230                 235                 240
   Val Val His Leu Phe Glu Arg Asp Cys Ser Val Gln Arg Arg His Gln
                   245                 250                 255
   Lys Val Val Glu Val Ala Pro Ala Lys Thr Leu Pro Arg Glu Val Arg
               260                 265                 270
   Asp Ser Ile Leu Thr Asp Ala Val Lys Leu Ala Lys Glu Ala Gly Tyr
           275                 280                 285
   Thr Asn Ala Gly Thr Ala Glu Phe Leu Val Asp Asn Leu Gly Arg His
       290                 295                 300
   Tyr Phe Ile Glu Ile Asn Pro Arg Ile Gln Val Glu His Thr Ile Thr
   305                 310                 315                 320
   Glu Glu Ile Thr Gly Ile Asp Ile Val Ala Ala Gln Ile Gln Ile Ala
                   325                 330                 335
   Ala Gly Ala Ser Leu Glu Asp Leu Gly Leu Leu Gln Asp Arg Ile Thr
               340                 345                 350
   Tyr Arg Gly Phe Ala Ile Gln Cys Arg Ile Thr Thr Glu Asp Pro Ser
           355                 360                 365
   Lys Asn Phe Gln Pro Asp Thr Gly Arg Leu Glu Val Tyr Arg Ser Ala
       370                 375                 380
   Gly Gly Asn Gly Val Arg Leu Asp Gly Gly Asn Ala Tyr Val Gly Ala
   385                 390                 395                 400
   Thr Ile Ser Pro His Tyr Asp Ser Met Leu Val Lys Cys Ser Cys Ser
                   405                 410                 415
   Gly Ser Thr Tyr Glu Ile Val Arg Arg Lys Met Leu Arg Ser Leu Ile
               420                 425                 430
   Glu Phe Arg Ile Arg Gly Val Lys Thr Asn Ile Pro Phe Leu Leu Thr
           435                 440                 445
   Leu Leu Thr His Pro Val Phe Val Ser Gly Asp Tyr Trp Thr Thr Phe
       450                 455                 460
   Ile Asp Asp Thr Pro Gln Leu Phe Gln Met Val Ser Ser Lys Asn Arg
   465                 470                 475                 480
   Ala Gln Lys Leu Leu His Tyr Leu Ala Asp Leu Ala Val Asn Gly Ser
                   485                 490                 495
   Ser Ile Lys Gly Gln Ile Gly Leu Pro Lys Leu Ala Thr Arg Pro Asp
               500                 505                 510
   Ile Pro Ala Leu His Asp Lys Asn Gly Glu Val Ile Asn Val Thr Asn
           515                 520                 525
   Ala Glu Pro Pro Ser Gly Trp Arg Lys Ala Leu Leu Glu Lys Gly Pro
       530                 535                 540
   Lys Glu Phe Ala Lys Gln Val Arg Glu Phe Asn Gly Cys Leu Ile Met
   545                 550                 555                 560
   Asp Thr Thr Trp Arg Asp Ala His Gln Ser Leu Leu Ala Thr Arg Leu
                   565                 570                 575
   Arg Thr Tyr Asp Leu Ala Ala Ile Ala Pro Thr Thr Ser Phe Ala Leu
                   580                 585                 590
   Ala Gly Ala Phe Ala Leu Glu Cys Trp Gly Gly Ala Thr Phe Asp Val
               595                 600                 605
   Ala Met Arg Phe Leu His Glu Asp Pro Trp Glu Arg Leu Arg Thr Leu
       610                 615                 620
   Arg Lys Leu Val Pro Asn Ile Pro Phe Gln Met Leu Leu Arg Gly Ala
   625                 630                 635                 640
   Asn Gly Val Ala Tyr Ser Ser Leu Pro Asp Asn Ala Ile Asn His Phe
                   645                 650                 655
   Val Lys Gln Ala Lys Asp Asn Gly Val Asp Ile Phe Arg Val Phe Asp
               660                 665                 670
   Ser Leu Asn Asp Val Glu Gln Leu Lys Val Gly Ile Glu Ala Val Lys
           675                 680                 685
   Leu Ala Gly Gly Val Val Glu Ala Thr Met Cys Tyr Ser Gly Asp Met
       690                 695                 700
   Leu Gln Pro Gly Lys Lys Tyr Asn Leu Asp Tyr Tyr Leu Glu Leu Ser
   705                 710                 715                 720
   Glu Lys Ile Val Gly Leu Gly Thr His Ile Leu Gly Ile Lys Asp Met
                   725                 730                 735
   Ala Gly Thr Met Lys Pro Ala Ala Ala Lys Leu Leu Ile Ser Ser Leu
               740                 745                 750
   Arg Lys Lys Tyr Pro Asp Leu Pro Ile His Val His Ser His Asp Ser
           755                 760                 765
   Ala Gly Thr Ala Val Thr Ser Met Val Ala Cys Ala Lys Tyr Gly Ala
       770                 775                 780
   Asp Val Val Asp Val Ala Ile Asn Ser Met Ser Gly Leu Thr Ser Gln
   785                 790                 795                 800
   Gly Ser Ile Asn Ala Leu Leu Ala Ser Leu Asp Gly Asp Ile Glu Thr
                   805                 810                 815
   Asn Ile Asn Ala Gln His Val Arg Glu Leu Asp Ala Tyr Trp Ala Glu
               820                 825                 830
   Met Arg Leu Leu Tyr Ser Cys Phe Glu Ala Asp Leu Lys Gly Pro Asp
           835                 840                 845
   Pro Glu Val Tyr Glu His Glu Ile Pro Gly Gly Gln Leu Thr Asn Leu
       850                 855                 860
   Leu Phe Gln Ala Gln Gln Leu Gly Leu Gly Glu Lys Trp Lys Glu Thr
   865                 870                 875                 880
   Lys Arg Ala Tyr Arg Glu Ala Asn Tyr Leu Leu Gly Asp Ile Val Lys
                   885                 890                 895
   Val Thr Pro Thr Ser Lys Val Val Gly Asp Leu Ala Gln Phe Met Val
               900                 905                 910
   Thr Asn Lys Leu Thr Ser Asp Asp Val Arg Arg Leu Ala Asn Ser Leu
           915                 920                 925
   Asp Phe Pro Asp Ser Val Met Asp Phe Phe Glu Gly Leu Leu Gly Gln
       930                 935                 940
   Pro Tyr Gly Gly Phe Pro Glu Pro Leu Arg Thr Asp Ile Leu Lys Gly
   945                 950                 955                 960
   Lys Arg Lys Lys Met Thr Ser Arg Pro Gly Leu Glu Leu Gln Pro Phe
                   965                 970                 975
   Asp Ile Ala Ala Ile Lys Glu Asp Leu Gln Asn Arg Phe Gly Asp Ile
               980                 985                 990
   Asp Glu Cys Asp Val Ala Ser Tyr Asn Met Tyr Pro Lys Val Tyr Glu
           995                 1000                1005
   Asp Phe Gln Lys Ile Arg Glu Lys Tyr Gly Asp Leu Ser Val Leu Pro
      1010                 1015                1020
   Thr Lys Asn Phe Leu Ala Pro Pro Val Thr Gly Glu Glu Ile Glu Val
   1025                1030                1035                1040
   Thr Ile Glu Gln Gly Lys Thr Leu Ile Ile Lys Cys Gln Ala Val Gly
                   1045                1050                1055
   Asp Leu Asn Lys Ala Thr Gly Thr Arg Glu Val Tyr Phe Glu Leu Asn
               1060                1065                1070
   Gly Glu Leu Arg Lys Ile Pro Val Val Asp Arg Ser Leu Lys Val Asp
           1075                1080                1085
   Ile Val Ala Lys Pro Lys Ala Asp Thr His Asp Pro Tyr Gln Ile Gly
       1090                1095                1100
   Ala Pro Met Ala Gly Val Ile Val Glu Val Lys Val His Lys Gly Ser
   1105                1110                1115                1120
   Leu Val Lys Lys Gly Gln Pro Val Ala Val Leu Ser Ala Met Lys Met
                   1125                1130                1135
   Glu Met Val Ile Ser Ser Pro Ser Asp Gly Leu Val Lys Asn Val Thr
               1140                1145                1150
   Val Ala Asp Gly Glu Asn Val Asp Ala Ser Asp Leu Leu Ile Thr Leu
           1155                1160                1165
   Glu Asp Pro Glu Pro Pro Lys
       1170                1175
<210> 15
   <211> 1177
   <212> PRT
   <213> Torulaspora delbrueckii
<220>
   <223> TdePYC
<400> 15
   Met Ser Lys Asn Lys Arg Phe Ala Gly Leu Arg Asp Asn Phe Ser Leu
   5 10 15
   Leu Gly Glu Lys Asn Lys Leu Leu Val Ala Asn Arg Gly Glu Ile Pro
   20 25 30
   Ile Arg Ile Phe Arg Ser Ala His Glu Leu Ser Met Arg Thr Val Ala
   35 40 45
   Ile Tyr Ser His Glu Asp Arg Leu Ser Met His Lys Leu Lys Ser Asp
   50 55 60
   Glu Ala Tyr Val Ile Gly Glu Glu Gly Lys Tyr Thr Pro Val Gly Ala
   65 70 75 80
   Tyr Leu Ala Ile Asp Glu Ile Ile Glu Ile Ala Lys Gln His Asn Val
   85 90 95
   Asp Phe Ile His Pro Gly Tyr Gly Phe Leu Ser Glu Asn Ser Glu Phe
   100 105 110
   Ala Lys Lys Val Ala Asp Asn Gly Ile Thr Trp Ile Gly Pro Pro Ala
   115 120 125
   Asp Val Ile Asp Ala Val Gly Asp Lys Val Ser Ala Arg Asn Leu Ala
   130 135 140
   Leu Lys Ala Asp Val Pro Thr Val Pro Gly Thr Pro Gly Pro Ile Glu
   145 150 155 160
   Asn Val Gln Glu Ala Lys Ala Phe Val Glu Lys Tyr Gly Tyr Pro Val
   165 170 175
   Ile Ile Lys Ala Ala Phe Gly Gly Gly Gly Arg Gly Met Arg Val Val
   180 185 190
   Arg Glu Gly Asp Asp Ile Glu Asp Ala Phe Gln Arg Ala Thr Ser Glu
   195 200 205
   Ala Arg Thr Ala Phe Gly Asn Gly Thr Cys Phe Ile Glu Arg Phe Leu
   210 215 220
   Val Lys Pro Lys His Ile Glu Val Gln Leu Leu Ala Asp Asn Tyr Gly
   225 230 235 240
   Asn Val Val His Leu Phe Glu Arg Asp Cys Ser Val Gln Arg Arg His
   245 250 255
   Gln Lys Val Val Glu Val Ala Pro Ala Lys Thr Leu Pro Val Glu Val
   260 265 270
   Arg Asn Ala Ile Leu Thr Asp Ala Val Lys Leu Ala His Thr Ala Gly
   275 280 285
   Tyr Arg Asn Ala Gly Thr Ala Glu Phe Leu Val Asp Asn Gln Asn Arg
   290 295 300
   His Tyr Phe Ile Glu Ile Asn Pro Arg Ile Gln Val Glu His Thr Ile
   305                 310                 315                 320
   Thr Glu Glu Ile Thr Gly Ile Asp Ile Val Ala Ala Gln Ile Gln Ile
                   325                 330                 335
   Ala Ala Gly Ala Ser Leu Glu Gln Leu Gly Leu Met Gln Asp Arg Ile
               340                 345                 350
   Thr Thr Arg Gly Phe Ala Ile Gln Cys Arg Ile Thr Thr Glu Asp Pro
           355                 360                 365
   Ser Lys Asn Phe Gln Pro Asp Thr Gly Arg Leu Glu Val Tyr Arg Ser
       370                 375                 380
   Ala Gly Gly Asn Gly Val Arg Leu Asp Gly Gly Asn Ala Phe Ala Gly
   385                 390                 395                 400
   Ala Ile Ile Ser Pro His Tyr Asp Ser Met Leu Val Lys Cys Ser Cys
                   405                 410                 415
   Ser Gly Ser Thr Tyr Glu Ile Val Arg Arg Lys Met Ile Arg Ala Leu
               420                 425                 430
   Ile Glu Phe Arg Ile Arg Gly Val Lys Thr Asn Ile Pro Phe Leu Leu
           435                 440                 445
   Thr Leu Leu Thr Asn Pro Val Phe Val Asn Gly Asp Tyr Trp Thr Thr
       450                 455                 460
   Phe Ile Asp Asp Thr Pro Gln Leu Phe Gln Met Ile Ser Ser Gln Asn
   465                 470                 475                 480
   Arg Ala Gln Lys Leu Leu His Tyr Leu Ala Asp Leu Ala Val Asn Gly
                   485                 490                 495
   Ser Ser Ile Lys Gly Gln Leu Gly Val Pro Lys Leu Thr Thr His Pro
               500                 505                 510
   Thr Ile Pro His Leu His Asp Ala Gln Gly Glu Ile Ile Asn Val Asn
           515                 520                 525
   Ser Ala Ala Pro Pro Ala Gly Trp Arg Gln Ile Leu Leu Asp Tyr Gly
       530                 535                 540
   Pro Lys Glu Phe Ala Lys Gln Val Arg Asp Phe Asp Gly Thr Leu Ile
   545                 550                 555                 560
   Met Asp Thr Thr Trp Arg Asp Ala His Gln Ser Leu Leu Ala Thr Arg
                   565                 570                 575
   Val Arg Thr Tyr Asp Leu Ala Ala Ile Ala Pro Thr Thr Ala His Ala
               580                 585                 590
   Leu Ser Gly Ala Phe Ala Leu Glu Cys Trp Gly Gly Ala Thr Phe Asp
           595                 600                 605
   Val Ala Met Arg Phe Leu His Glu Asp Pro Trp Glu Arg Leu Arg Ile
       610                 615                 620
   Leu Arg Lys Leu Val Pro Asn Ile Pro Phe Gln Met Leu Leu Arg Gly
   625                 630                 635                 640
   Ala Asn Gly Val Ala Tyr Ser Ser Leu Pro Asp Asn Ala Ile Asp His
                   645                 650                 655
   Phe Val Lys Gln Ala Lys Glu Asn Gly Val Asp Ile Phe Arg Val Phe
               660                 665                 670
   Asp Ala Leu Asn Asp Leu Glu Gln Leu Lys Val Gly Val Asp Ala Val
           675                 680                 685
   Lys Lys Ala Gly Gly Val Val Glu Ala Thr Val Cys Tyr Ser Gly Asp
       690                 695                 700
   Met Leu Gln Pro Gly Lys Lys Tyr Asn Leu Asp Tyr Tyr Leu Glu Val
   705                 710                 715                 720
   Thr Gly Lys Ile Val Glu Met Gly Thr His Ile Leu Gly Ile Lys Asp
                   725                 730                 735
   Met Ala Gly Thr Met Lys Pro Ser Ala Ala Arg Leu Leu Ile Gly Ser
               740                 745                 750
   Ile Arg Ala Lys Tyr Pro Asp Leu Pro Ile His Val His Thr His Asp
           755                 760                 765
   Ser Ala Gly Thr Ala Val Ala Ser Met Ala Ala Cys Ala Leu Ala Gly
       770                 775                 780
   Ala Asp Val Val Asp Val Ala Thr Asn Ser Met Ser Gly Leu Thr Ser
   785                 790                 795                 800
   Gln Pro Ser Met Asn Ala Leu Leu Ala Ser Leu Asp Gly Gln Ile Asn
                   805                 810                 815
   Thr Asn Ile Asp Val Gln His Thr Arg Glu Leu Asp Ala Tyr Trp Ala
               820                 825                 830
   Glu Met Arg Leu Leu Tyr Ser Cys Phe Glu Ala Asp Leu Lys Gly Pro
           835                 840                 845
   Asp Pro Glu Val Tyr Glu His Glu Ile Pro Gly Gly Gln Leu Thr Asn
       850                 855                 860
   Leu Leu Phe Gln Ala Gln Gln Leu Gly Leu Gly Glu Lys Trp Ala Glu
   865                 870                 875                 880
   Thr Lys Arg Ala Tyr Lys Glu Ala Asn Tyr Leu Leu Gly Asp Leu Val
                   885                 890                 895
   Lys Val Thr Pro Thr Ser Lys Val Val Gly Asp Leu Ala Gln Phe Met
               900                 905                 910
   Val Ser Asn Lys Leu Thr Ser Gly Asp Val Lys Arg Leu Ala Ser Ser
           915                 920                 925
   Leu Asp Phe Pro Asp Ser Val Met Asp Phe Phe Glu Gly Leu Ile Gly
       930                 935                 940
   Gln Pro Tyr Gly Gly Phe Pro Glu Pro Leu Arg Thr Asp Ile Leu Lys
   945                 950                 955                 960
   Asn Lys Arg Arg Lys Leu Thr Cys Arg Pro Gly Leu Glu Leu Ala Pro
                   965                 970                 975
   Phe Asp Leu Glu Lys Ile Arg Glu Asp Leu Gln Asp Arg Phe Gly Asp
               980                 985                 990
   Ile Asp Glu Cys Asp Val Ala Ser Tyr Asn Met Tyr Pro Lys Val Tyr
           995                 1000                1005
   Glu Asp Phe His Lys Ile Lys Glu Arg Tyr Gly Asp Leu Ser Val Leu
      1010                1015                1020
   Pro Thr Lys Asn Phe Leu Ser Pro Pro Lys Ile Gly Glu Glu Ile Glu
   1025                1030                1035                1040
   Val Thr Ile Glu Lys Gly Lys Thr Leu Ile Ile Lys His Gln Ala Val
                   1045                1050                1055
   Gly Asp Leu Asn Lys Glu Thr Gly Val Arg Glu Val Tyr Phe Glu Leu
               1060                1065                1070
   Asn Gly Glu Leu Arg Lys Ile Pro Val Val Asp Lys Ser Gln Lys Val
           1075                1080                1085
   Glu Ser Val Ala Lys Pro Lys Ala Asp Gly His Asp Pro Leu Gln Val
       1090                1095                1100
   Gly Ala Pro Met Ala Gly Val Ile Val Glu Val Lys Val His Lys Gly
   1105                1110                1115                1120
   Ser Leu Val Lys Lys Gly Glu Pro Val Ala Val Leu Ser Ala Met Lys
                   1125                1130                1135
   Met Glu Met Val Ile Ser Ala Gln Ala Asp Gly Gln Val Lys Glu Val
               1140                1145                1150
   Tyr Ile Asn Asp Gly Asp Asn Val Asp Ala Ser Asp Leu Leu Val Val
           1155                1160                1165
   Leu Glu Glu Ala Val Pro Ala Glu Asn
       1170                1175
<210> 16
   <211> 1177
   <212> PRT
   <213> Zygosaccharomyces rouxii
<220>
   <223> ZroPYC
<400> 16
   Met Ser Thr Lys Lys Phe Ser Gly Leu Arg Asp Asn Phe Ser Leu Leu
                   5                   10                  15
   Gly Glu Lys Asn Lys Ile Leu Val Ala Asn Arg Gly Glu Ile Pro Ile
               20                  25                  30
   Arg Ile Phe Arg Ser Ala His Glu Leu Ser Met Arg Ser Val Ala Val
               35                  40                  45
   Tyr Ser His Glu Asp Arg Leu Ser Met His Arg Leu Lys Ala Asp Glu
       50                  55                  60
   Ala Tyr Val Ile Gly Glu Glu Gly Lys Tyr Thr Pro Val Gly Ala Tyr
   65                  70                  75                  80
   Leu Ala Ile Asp Glu Ile Ile Ala Ile Ala Lys Lys His Asn Val Asp
                   85                  90                  95
   Phe Ile His Pro Gly Tyr Gly Phe Leu Ser Glu Asn Ser Glu Phe Ala
               100                 105                 110
   Lys Lys Val Glu Ala Asn Gly Ile Thr Trp Ile Gly Pro Pro Ala Glu
           115                 120                 125
   Val Ile Asp Ser Val Gly Asp Lys Val Ser Ala Arg Asn Leu Ala Ala
       130                 135                 140
   Lys Ala His Val Ala Thr Val Pro Gly Thr Pro Gly Pro Ile Glu Ser
   145                 150                 155                 160
   Val Asp Glu Ala Ile Glu Phe Thr Lys Lys Tyr Gly Phe Pro Val Ile
                   165                 170                 175
   Ile Lys Ala Ala Phe Gly Gly Gly Gly Arg Gly Met Arg Val Val Arg
               180                 185                 190
   Glu Gly Asp Asp Val Ala Asp Ala Phe Gln Arg Ala Ser Ser Glu Ala
           195                 200                 205
   Arg Ser Ser Phe Gly Asn Gly Thr Cys Phe Met Glu Arg Phe Leu Val
       210                 215                 220
   Lys Pro Lys His Ile Glu Val Gln Leu Leu Ala Asp Asn Tyr Gly Asn
   225                 230                 235                 240
   Val Val His Leu Phe Glu Arg Asp Cys Ser Val Gln Arg Arg His Gln
                   245                 250                 255
   Lys Val Val Glu Leu Ala Pro Ala Lys Ala Leu Pro Val Glu Thr Arg
               260                 265                 270
   Asn Ala Ile Leu Thr Asp Ala Val Lys Leu Ala Lys Thr Ala Gly Tyr
           275                 280                 285
   Arg Asn Ala Gly Thr Ala Glu Phe Leu Val Asp Asn Glu Asn Arg His
       290                 295                 300
   Tyr Phe Ile Glu Ile Asn Pro Arg Ile Gln Val Glu His Thr Ile Thr
   305                 310                 315                 320
   Glu Glu Ile Thr Gly Ile Asp Ile Val Ala Ala Gln Ile Gln Ile Ala
                   325                 330                 335
   Ala Gly Ala Ser Leu Asp Gln Leu Gly Leu Leu Gln Asp Arg Ile Thr
               340                 345                 350
   Thr Arg Gly Phe Ala Ile Gln Cys Arg Ile Thr Thr Glu Asp Pro Ser
           355                 360                 365
   Lys Asn Phe Gln Pro Asp Thr Gly Arg Ile Glu Val Tyr Arg Ser Ser
       370                 375                 380
   Gly Gly Asn Gly Val Arg Leu Asp Gly Gly Asn Thr Tyr Ala Gly Ala
   385                 390                 395                 400
   Val Ile Ser Pro His Tyr Asp Ser Met Leu Val Lys Cys Ser Cys Ser
                   405                 410                 415
   Gly Ser Thr Tyr Glu Ile Val Arg Arg Lys Met Ile Arg Ala Leu Val
               420                 425                 430
   Glu Phe Arg Ile Arg Gly Val Lys Thr Asn Ile Pro Phe Leu Leu Thr
           435                 440                 445
   Leu Leu Thr His Pro Thr Phe Val Gln Gly Gln Phe Trp Thr Thr Phe
       450                 455                 460
   Ile Asp Asp Thr Pro Gln Leu Phe Gln Met Ile Ser Ser Gln Asn Arg
   465                 470                 475                 480
   Ala Gln Lys Leu Leu His Tyr Leu Ala Asp Leu Ala Val Asn Gly Ser
                   485                 490                 495
   Ser Ile Lys Gly Gln Val Gly Leu Pro Lys Leu Lys Thr His Pro Thr
               500                 505                 510
   Val Pro Arg Leu His Gly Ser Asn Gly Glu Val Ile Asp Val Leu Ala
           515                 520                 525
   Thr Pro Pro Pro Ser Gly Trp Arg Gln Val Leu Leu Glu His Gly Pro
       530                 535                 540
   Glu Glu Phe Ala Lys Arg Val Arg Gln Phe Asn Gly Cys Leu Ile Met
   545                 550                 555                 560
   Asp Thr Thr Trp Arg Asp Ala His Gln Ser Leu Leu Ala Thr Arg Val
                   565                 570                 575
   Arg Thr Tyr Asp Leu Ala Ala Ile Ala Pro Thr Thr Ala His Ala Leu
               580                 585                 590
   Ser Gly Ala Phe Ser Leu Glu Cys Trp Gly Gly Ala Thr Phe Asp Val
           595                 600                 605
   Ala Met Arg Phe Leu His Glu Asp Pro Trp Glu Arg Leu Arg Ile Leu
       610                 615                 620
   Arg Arg Leu Val Pro Asn Val Pro Phe Gln Met Leu Leu Arg Gly Ala
   625                 630                 635                 640
   Asn Gly Val Ala Tyr Ser Ser Leu Pro Asp Asn Ala Ile Asp His Phe
                   645                 650                 655
   Val Lys Gln Ala Lys Glu Asn Gly Val Asp Ile Phe Arg Val Phe Asp
               660                 665                 670
   Ala Leu Asn Asp Leu Asp Gln Leu Lys Val Gly Val Asp Ala Val Lys
           675                 680                 685
   Lys Ala Gly Gly Val Val Glu Ala Thr Val Cys Tyr Ser Gly Asp Met
       690                 695                 700
   Leu Gln Pro Gly Lys Lys Tyr Asn Leu Asp Tyr Tyr Leu Glu Val Thr
   705                 710                 715                 720
   Glu Gln Ile Val Lys Met Gly Thr His Phe Leu Gly Ile Lys Asp Met
                   725                 730                 735
   Ala Gly Thr Met Lys Pro Ala Ala Ala Lys Leu Leu Val Gly Ser Ile
               740                 745                 750
   Arg Ala Lys Tyr Pro Asn Leu Pro Ile His Ile His Thr His Asp Ser
           755                 760                 765
   Ala Gly Thr Gly Val Ala Ser Met Ala Ala Cys Ala Val Ser Gly Ala
       770                 775                 780
   Asp Val Val Asp Val Ala Val Asn Ser Met Ser Gly Leu Thr Ser Gln
   785                 790                 795                 800
   Pro Cys Met Asn Ala Leu Leu Ala Ser Leu Asp Gly Glu Ile Asn Thr
                   805                 810                 815
   Gly Ile Asn Ala Asn Tyr Ala Arg Glu Leu Asp Ala Tyr Trp Ala Glu
               820                 825                 830
   Met Arg Leu Leu Tyr Ser Cys Phe Asp Thr Asp Leu Lys Gly Pro Asp
           835                 840                 845
   Pro Glu Val Tyr Glu His Glu Ile Pro Gly Gly Gln Leu Thr Asn Leu
       850                 855                 860
   Leu Phe Gln Ala Gln Gln Leu Gly Leu Gly Glu Lys Trp Ile Glu Thr
   865                 870                 875                 880
   Lys Lys Ala Tyr Lys Glu Ala Asn Tyr Leu Leu Gly Asp Leu Val Lys
                   885                 890                 895
   Val Thr Pro Thr Ser Lys Val Val Gly Asp Leu Ala Gln Phe Met Val
               900                 905                 910
   Ser Asn Lys Leu Thr Ser Asp Asp Val Arg Arg Leu Ala Asn Ser Leu
           915                 920                 925
   Asp Phe Pro Asp Ser Val Met Asp Phe Phe Glu Gly Leu Ile Gly Gln
       930                 935                 940
   Pro Tyr Gly Gly Phe Pro Glu Pro Leu Arg Ser Asp Val Leu Arg Asn
   945                 950                 955                 960
   Lys Arg Arg Lys Leu Thr Asn Arg Pro Gly Leu Asp Leu Ala Pro Phe
                   965                 970                 975
   Asp Leu Glu Ala Val Arg Glu Asp Leu Gln Asp Arg Phe Gly Asp Thr
               980                 985                 990
   Asp Glu Cys Asp Val Ala Ser Tyr Asn Met Tyr Pro Lys Val Tyr Glu
           995                 1000                1005
   Asp Phe Gln Lys Ile Lys Glu Gln Phe Gly Asp Leu Ser Val Leu Pro
   1010 1015 1020
   Thr Lys Asn Phe Leu Ser Pro Pro Gly Ile Gly Glu Glu Ile Glu Val
   1025 1030 1035 1040
   Thr Ile Glu Gln Gly Lys Thr Leu Ile Ile Lys His Gln Ala Val Gly
   1045 1050 1055
   Asp Leu Asn Lys Glu Thr Gly Met Arg Glu Val Tyr Phe Glu Leu Asn
   1060 1065 1070
   Gly Glu Leu Arg Lys Ile Pro Val Val Asp Arg Ser Gln Lys Val Glu
   1075 1080 1085
   Ile Val Ser Lys Pro Lys Ala Asp Gly His Asp Pro Tyr Gln Val Gly
   1090 1095 1100
   Ala Pro Met Ala Gly Val Ile Val Glu Ile Lys Val His Lys Gly Ser
   1105 1110 1115 1120
   Leu Ile Lys Lys Gly Gln Pro Val Ala Val Leu Ser Ala Met Lys Met
   1125 1130 1135
   Glu Met Val Ile Ser Ala Gln Thr Asp Gly Gln Val Lys Glu Val Leu
   1140 1145 1150
   Ala Asn Gly Gly Asp Asn Val Asp Ala Ser Asp Leu Leu Val Thr Leu
   1155 1160 1165
   Glu Asp Ala Val Pro Pro Lys Glu Asn
   1170 1175
<210> 17
   <211> 294
   <212> PRT
   <213> Archaeoglobus fulgidus
<220>
   <223> AfuMDH
<400> 17
   Met Lys Leu Gly Phe Val Gly Ala Gly Arg Val Gly Ser Thr Ser Ala
                   5                   10                  15
   Phe Thr Cys Leu Leu Asn Leu Asp Val Asp Glu Ile Ala Leu Val Asp
               20                  25                  30
   Ile Ala Glu Asp Leu Ala Val Gly Glu Ala Met Asp Leu Ala His Ala
           35                  40                  45
   Ala Ala Gly Ile Asp Lys Tyr Pro Lys Ile Val Gly Gly Ala Asp Tyr
       50                  55                  60
   Ser Leu Leu Lys Gly Ser Glu Ile Ile Val Val Thr Ala Gly Leu Ala
   65                  70                  75                  80
   Arg Lys Pro Gly Met Thr Arg Leu Asp Leu Ala His Lys Asn Ala Gly
                   85                  90                  95
   Ile Ile Lys Asp Ile Ala Lys Lys Ile Val Glu Asn Ala Pro Glu Ser
               100                 105                 110
   Lys Ile Leu Val Val Thr Asn Pro Met Asp Val Met Thr Tyr Ile Met
           115                 120                 125
   Trp Lys Glu Ser Gly Lys Pro Arg Asn Glu Val Phe Gly Met Gly Asn
       130                 135                 140
   Gln Leu Asp Ser Gln Arg Leu Lys Glu Arg Leu Tyr Asn Ala Gly Ala
   145                 150                 155                 160
   Arg Asn Ile Arg Arg Ala Trp Ile Ile Gly Glu His Gly Asp Ser Met
                   165                 170                 175
   Phe Val Ala Lys Ser Leu Ala Asp Phe Asp Gly Glu Val Asp Trp Glu
               180                 185                 190
   Ala Val Glu Asn Asp Val Arg Phe Val Ala Ala Glu Val Ile Lys Arg
           195                 200                 205
   Lys Gly Ala Thr Ile Phe Gly Pro Ala Val Ala Ile Tyr Arg Met Val
       210                 215                 220
   Lys Ala Val Val Glu Asp Thr Gly Glu Ile Ile Pro Thr Ser Met Ile
   225                 230                 235                 240
   Leu Gln Gly Glu Tyr Gly Ile Glu Asn Val Ala Val Gly Val Pro Ala
                   245                 250                 255
   Lys Leu Gly Lys Asn Gly Ala Glu Val Ala Asp Ile Lys Leu Ser Asp
               260                 265                 270
   Glu Glu Ile Glu Lys Leu Arg Asn Ser Ala Lys Ile Leu Arg Glu Arg
           275                 280                 285
   Leu Glu Glu Leu Gly Tyr
       290
<210> 18
   <211> 326
   <212> PRT
   <213> Congregibacter litoralis
<220>
   <223> CliMDH
<400> 18
   Met Lys Ala Pro Val Arg Val Thr Val Thr Gly Ala Ala Gly Gln Ile
   5 10 15
   Gly Tyr Ala Leu Leu Phe Arg Ile Ala Ser Gly Ala Met Leu Gly Asp
   20 25 30
   Asp Gln Pro Val Ile Leu His Leu Leu Asp Ile Thr Pro Ala Met Asp
   35 40 45
   Ala Leu Glu Gly Val Arg Met Glu Leu Asp Asp Cys Ala Phe Pro Leu
   50 55 60
   Leu Ala Gly Ile Val Cys Thr Asp Asp Pro Asn Val Gly Phe Lys Asp
   65 70 75 80
   Ala Asp Tyr Ala Leu Leu Val Gly Ala Arg Pro Arg Gly Pro Gly Met
   85 90 95
   Glu Arg Lys Asp Leu Leu Glu Ala Asn Ala Ala Ile Phe Ser Val Gln
   100 105 110
   Gly Lys Ala Ile Asn Asp His Ala Ser Arg Asp Ile Arg Val Leu Val
   115 120 125
   Val Gly Asn Pro Ala Asn Thr Asn Ala Leu Ile Thr Gln Arg Asn Ala
   130 135 140
   Pro Asp Ile Asp Pro Arg Asn Phe Thr Ala Met Met Arg Leu Asp His
   145 150 155 160
   Asn Arg Ala Lys Thr Gln Ile Ala Gln Lys Leu Asp Ala Pro Val Thr
   165 170 175
   Ala Val Ser Thr Met Thr Val Trp Gly Asn His Ser Ala Thr Gln Tyr
   180 185 190
   Pro Asp Leu His His Ser Thr Val Asn Gly Lys Val Ala Val Asp Ala
   195 200 205
   Val Glu Gln Gln Trp Tyr Glu Asp Glu Phe Ile Pro Thr Val Gln Gln
   210 215 220
   Arg Gly Ala Ala Ile Ile Lys Ala Arg Gly Ala Ser Ser Ala Ala Ser
   225 230 235 240
   Ala Ala Asn Ala Ala Ile Asp His Met Arg Asp Trp Ala Leu Gly Thr
   245 250 255
   Pro Glu Gly Asp Trp Val Ser Met Gly Val Tyr Ser Asp Gly Ser Tyr
   260 265 270
   Gly Ile Ala Glu Gly Leu Ile Tyr Ser Phe Pro Cys Arg Cys Ser Gly
   275 280 285
   Gly Asp Trp Ser Ile Val Gln Gly Leu Glu Ile Asn Asp Phe Ser Arg
   290 295 300
   Gly Arg Met Gln Ala Thr Glu Gln Glu Leu Thr Glu Glu Arg Asp Ala
   305 310 315 320
   Val Ala Asp Leu Leu Pro
   325
<210> 19
   <211> 328
   <212> PRT
   <213> Delftia acidovorans
<220>
   <223> DacMDH
<400> 19
   Met Ser Lys Lys Pro Val Arg Val Ala Val Thr Gly Ala Ala Gly Gln
                   5                   10                  15
   Ile Gly Tyr Ala Leu Leu Phe Arg Ile Ala Ser Gly Glu Met Leu Gly
               20                  25                  30
   Lys Asp Gln Pro Val Ile Leu Gln Leu Leu Glu Ile Pro Asp Glu Lys
           35                  40                  45
   Ala Gln Asn Ala Leu Lys Gly Val Ile Met Glu Leu Glu Asp Cys Ala
       50                  55                  60
   Phe Pro Leu Leu Ala Gly Ile Glu Ala His Ser Asp Pro Leu Gln Ala
   65                  70                  75                  80
   Phe Lys Asp Thr Asp Tyr Ala Leu Leu Val Gly Ala Arg Pro Arg Gly
                   85                  90                  95
   Pro Gly Met Glu Arg Ala Asp Leu Leu Ala Ala Asn Ala Gln Ile Phe
               100                 105                 110
   Thr Ala Gln Gly Lys Ala Leu Asn Ala Ala Ala Ser Arg Asn Val Lys
           115                 120                 125
   Val Leu Val Val Gly Asn Pro Ala Asn Thr Asn Ala Tyr Ile Ala Met
       130                 135                 140
   Lys Ser Ala Pro Asp Leu Pro Ala Lys Asn Phe Thr Ala Met Leu Arg
   145                 150                 155                 160
   Leu Asp His Asn Arg Ala Ala Ser Gln Leu Ala Ala Lys Ala Gly Phe
                   165                 170                 175
   Lys Val Gly Asp Ile Arg Lys Leu Thr Val Trp Gly Asn His Ser Pro
               180                 185                 190
   Thr Met Tyr Ala Asp Tyr Arg Phe Ala Thr Val Asn Gly Glu Ser Val
           195                 200                 205
   Lys Ala Lys Ile Asn Asp Gln Ala Trp Asn Lys Asp Val Phe Leu Pro
       210                 215                 220
   Thr Val Gly Lys Arg Gly Ala Ala Ile Ile Ala Ala Arg Gly Leu Ser
   225                 230                 235                 240
   Ser Ala Ala Ser Ala Ala Asn Ala Ala Ile Asp His Met Arg Asp Trp
                   245                 250                 255
   Ala Leu Gly Ser Gly Gly Glu Trp Val Thr Met Gly Val Pro Ser Asn
               260                 265                 270
   Gly Glu Tyr Gly Ile Pro Ala Gly Ile Val Phe Gly Phe Pro Val Thr
           275                 280                 285
   Thr Glu Asn Gly Glu Tyr Lys Ile Val Glu Gly Leu Glu Ile Asp Ala
       290                 295                 300
   Phe Ser Gln Glu Cys Ile Asp Lys Thr Leu Ala Glu Leu Gln Gly Glu
   305                 310                 315                 320
   Gln Asp Gly Val Lys His Leu Leu
                   325
<210> 20
   <211> 324
   <212> PRT
   <213> Halomonas elongata
<220>
   <223> HelMDH
<400> 20
   Met Lys Asp Pro Val Arg Ile Ala Ile Thr Gly Ala Ala Gly Gln Ile
                   5                   10                  15
   Ser Tyr Ser Leu Ala Phe Arg Ile Ala Ser Gly Asp Met Leu Gly Lys
   20 25 30
   Asp Gln Pro Val Ile Leu Gln Leu Leu Glu Ile Pro Pro Ala Met Asp
   35 40 45
   Ala Leu Asn Gly Val Val Met Glu Leu Asn Asp Cys Ala Phe Pro Leu
   50 55 60
   Val Gln Asp Ile Val Ala Ser Asp Asp Pro Asn Val Ala Phe Lys Asp
   65 70 75 80
   Ala Asp Phe Ala Leu Leu Val Gly Ala Arg Pro Arg Gly Pro Gly Met
   85 90 95
   Glu Arg Lys Asp Leu Leu Glu Ala Asn Ala Ala Ile Phe Ser Val Gln
   100 105 110
   Gly Lys Ala Leu Asn Asp His Ala Ser Arg Asp Val Arg Val Leu Val
   115 120 125
   Val Gly Asn Pro Ala Asn Thr Asn Ala Leu Ile Ala Ser Cys Asn Ala
   130 135 140
   Pro Asp Leu Asp Ala Gly Gln Phe Thr Ala Met Met Arg Leu Asp His
   145 150 155 160
   Asn Arg Ala Lys Thr Gln Leu Ala Gln Lys Val Gly Lys His Val Thr
   165 170 175
   Asp Val Glu Ser Met Ile Val Trp Gly Asn His Ser Ala Thr Gln Tyr
   180 185 190
   Pro Asp Leu Ala His Cys Lys Val Asp Gly Lys Pro Ala Leu Glu Leu
   195 200 205
   Val Asp Gln Ala Trp Tyr Glu Asn Asp Phe Ile Pro Thr Val Gln Gln
   210 215 220
   Arg Gly Ala Ala Ile Ile Lys Ala Arg Gly Ala Ser Ser Ala Ala Ser
   225 230 235 240
   Ala Ala Ser Ala Ala Ile Asp His Met Arg Asp Trp Ala Leu Gly Ser
   245 250 255
   Asp Gly Ile Val Ser Met Gly Ile Pro Ala Asp Gly Ser Tyr Gly Ile
   260 265 270
   Glu Pro Gly Ile Met Tyr Ser Tyr Pro Val Val Cys Lys Asn Gly Gln
   275 280 285
   Tyr Glu Ile Val Gln Asp Leu Glu Ile Gly Asp Phe Ser Arg Glu Arg
   290 295 300
   Met Asn Ala Thr Glu Gln Glu Leu Arg Glu Glu Arg Asp Ala Val Glu
   305 310 315 320
   His Leu Leu Gly
<210> 21
   <211> 311
   <212> PRT
   <213> Shewanella putrefaciens
<220>
   <223> SpuMDH
<400> 21
   Met Lys Val Ala Val Leu Gly Ala Ala Gly Gly Ile Gly Gln Ala Leu
                   5                   10                  15
   Ala Leu Leu Leu Lys Thr Gln Leu Pro Ala Gly Ser Lys Leu Ser Leu
               20                  25                  30
   Tyr Asp Ile Ala Pro Val Thr Pro Gly Val Ala Val Asp Leu Ser His
           35                  40                  45
   Ile Pro Thr Ala Val Glu Ile Lys Gly Phe Ala Gly Glu Asp Pro Thr
       50                  55                  60
   Pro Ala Leu Val Gly Ala Asp Val Val Leu Ile Ser Ala Gly Val Ala
   65                  70                  75                  80
   Arg Lys Pro Gly Met Asp Arg Ser Asp Leu Phe Asn Ile Asn Ala Gly
                   85                  90                  95
   Ile Val Arg Asn Leu Ile Glu Lys Val Ala Ala Thr Cys Pro Lys Ala
               100                 105                 110
   Leu Val Gly Ile Ile Thr Asn Pro Val Asn Thr Thr Val Ala Ile Ala
           115                 120                 125
   Ala Glu Val Met Lys Lys Ala Gly Val Tyr Asp Lys Asn Arg Leu Phe
       130                 135                 140
   Gly Val Thr Thr Leu Asp Val Ile Arg Ser Glu Thr Phe Ile Ala Glu
   145                 150                 155                 160
   Leu Lys Gly Leu Asn Val Ala Asp Val Lys Ile Asn Val Ile Gly Gly
                   165                 170                 175
   His Ser Gly Val Thr Ile Leu Pro Leu Leu Ser Gln Val Glu Gly Val
               180                 185                 190
   Thr Phe Ser Asp Glu Glu Val Ala Ser Leu Thr Lys Arg Ile Gln Asn
           195                 200                 205
   Ala Gly Thr Glu Val Val Glu Ala Lys Ala Gly Gly Gly Ser Ala Thr
       210                 215                 220
   Leu Ser Met Gly Gln Ala Ala Cys Arg Phe Gly Met Ser Leu Val Arg
   225                 230                 235                 240
   Gly Leu Gln Gly Glu Ala Asn Val Val Glu Cys Ala Tyr Val Asp Gly
                   245                 250                 255
   Gly Ser Glu His Ala Glu Phe Phe Ala Gln Pro Val Leu Leu Gly Lys
               260                 265                 270
   Asn Gly Ile Glu Lys Val Leu Pro Tyr Gly Glu Val Ser Ala Phe Glu
           275                 280                 285
   Ala Asn Ala Arg Asp Ser Met Leu Asp Thr Leu Lys Gly Asp Ile Lys
       290                 295                 300
   Leu Gly Val Asp Phe Val Lys
   305                 310
<210> 22
   <211> 569
   <212> PRT
   <213> Schizosaccharomyces pombe
<220>
   <223> SpoPDC2
<400> 22
   Met Thr Lys Asp Ala Glu Ser Thr Met Thr Val Gly Thr Tyr Leu Ala
                   5                   10                  15
   Gln Arg Leu Val Glu Ile Gly Ile Lys Asn His Phe Val Val Pro Gly
               20                  25                  30
   Asp Tyr Asn Leu Arg Leu Leu Asp Phe Leu Glu Tyr Tyr Pro Gly Leu
           35                  40                  45
   Ser Glu Ile Gly Cys Cys Asn Glu Leu Asn Cys Ala Phe Ala Ala Glu
       50                  55                  60
   Gly Tyr Ala Arg Ser Asn Gly Ile Ala Cys Ala Val Val Thr Tyr Ser
   65                  70                  75                  80
   Val Gly Ala Leu Thr Ala Phe Asp Gly Ile Gly Gly Ala Tyr Ala Glu
                   85                  90                  95
   Asn Leu Pro Val Ile Leu Val Ser Gly Ser Pro Asn Thr Asn Asp Leu
               100                 105                 110
   Ser Ser Gly His Leu Leu His His Thr Leu Gly Thr His Asp Phe Glu
           115                 120                 125
   Tyr Gln Met Glu Ile Ala Lys Lys Leu Thr Cys Ala Ala Val Ala Ile
       130                 135                 140
   Lys Arg Ala Glu Asp Ala Pro Val Met Ile Asp His Ala Ile Arg Gln
   145                 150                 155                 160
   Ala Ile Leu Gln His Lys Pro Val Tyr Ile Glu Ile Pro Thr Asn Met
                   165                 170                 175
   Ala Asn Gln Pro Cys Pro Val Pro Gly Pro Ile Ser Ala Val Ile Ser
               180                 185                 190
   Pro Glu Ile Ser Asp Lys Glu Ser Leu Glu Lys Ala Thr Asp Ile Ala
           195                 200                 205
   Ala Glu Leu Ile Ser Lys Lys Glu Lys Pro Ile Leu Leu Ala Gly Pro
       210                 215                 220
   Lys Leu Arg Ala Ala Gly Ala Glu Ser Ala Phe Val Lys Leu Ala Glu
   225                 230                 235                 240
   Ala Leu Asn Cys Ala Ala Phe Ile Met Pro Ala Ala Lys Gly Phe Tyr
                   245                 250                 255
   Ser Glu Glu His Lys Asn Tyr Ala Gly Val Tyr Trp Gly Glu Val Ser
               260                 265                 270
   Ser Ser Glu Thr Thr Lys Ala Val Tyr Glu Ser Ser Asp Leu Val Ile
           275                 280                 285
   Gly Ala Gly Val Leu Phe Asn Asp Tyr Ser Thr Val Gly Trp Arg Ala
       290                 295                 300
   Ala Pro Asn Pro Asn Ile Leu Leu Asn Ser Asp Tyr Thr Ser Val Ser
   305                 310                 315                 320
   Ile Pro Gly Tyr Val Phe Ser Arg Val Tyr Met Ala Glu Phe Leu Glu
                   325                 330                 335
   Leu Leu Ala Lys Lys Val Ser Lys Lys Pro Ala Thr Leu Glu Ala Tyr
               340                 345                 350
   Asn Lys Ala Arg Pro Gln Thr Val Val Pro Lys Ala Ala Glu Pro Lys
           355                 360                 365
   Ala Ala Leu Asn Arg Val Glu Val Met Arg Gln Ile Gln Gly Leu Val
       370                 375                 380
   Asp Ser Asn Thr Thr Leu Tyr Ala Glu Thr Gly Asp Ser Trp Phe Asn
   385                 390                 395                 400
   Gly Leu Gln Met Lys Leu Pro Ala Gly Ala Lys Phe Glu Val Glu Met
                   405                 410                 415
   Gln Trp Gly His Ile Gly Trp Ser Val Pro Ser Ala Met Gly Tyr Ala
               420                 425                 430
   Val Ala Ala Pro Glu Arg Arg Thr Ile Val Met Val Gly Asp Gly Ser
           435                 440                 445
   Phe Gln Leu Thr Gly Gln Glu Ile Ser Gln Met Ile Arg His Lys Leu
       450                 455                 460
   Pro Val Leu Ile Phe Leu Leu Asn Asn Arg Gly Tyr Thr Ile Glu Ile
   465                 470                 475                 480
   Gln Ile His Asp Gly Pro Tyr Asn Arg Ile Gln Asn Trp Asp Phe Ala
                   485                 490                 495
   Ala Phe Cys Glu Ser Leu Asn Gly Glu Thr Gly Lys Ala Lys Gly Leu
               500                 505                 510
   His Ala Lys Thr Gly Glu Glu Leu Thr Ser Ala Ile Lys Val Ala Leu
           515                 520                 525
   Gln Asn Lys Glu Gly Pro Thr Leu Ile Glu Cys Ala Ile Asp Thr Asp
       530                 535                 540
   Asp Cys Thr Gln Glu Leu Val Asp Trp Gly Lys Ala Val Arg Ser Ala
   545                 550                 555                 560
   Asn Ala Arg Pro Pro Thr Ala Asp Asn
                   565
<210> 23
   <211> 565
   <212> PRT
   <213> Schizosaccharomyces pombe
<220>
   <223> Spomae2
<400> 23
   Met Pro Ala Gly Thr Lys Glu Gln Ile Glu Cys Pro Leu Lys Gly Val
                   5                   10                  15
   Thr Leu Leu Asn Ser Pro Arg Tyr Asn Lys Asp Thr Ala Phe Thr Pro
               20                  25                  30
   Glu Glu Arg Gln Lys Phe Glu Ile Ser Ser Arg Leu Pro Pro Ile Val
           35                  40                  45
   Glu Thr Leu Gln Gln Gln Val Asp Arg Cys Tyr Asp Gln Tyr Lys Ala
       50                  55                  60
   Ile Gly Asp Glu Pro Leu Gln Lys Asn Leu Tyr Leu Ser Gln Leu Ser
   65                  70                  75                  80
   Val Thr Asn Gln Thr Leu Phe Tyr Ala Leu Ile Ser Gln His Leu Ile
                   85                  90                  95
   Glu Met Ile Pro Ile Ile Tyr Thr Pro Thr Glu Gly Asp Ala Ile Lys
               100                 105                 110
   Gln Phe Ser Asp Ile Tyr Arg Tyr Pro Glu Gly Cys Tyr Leu Asp Ile
           115                 120                 125
   Asp His Asn Asp Leu Ser Tyr Ile Lys Gln Gln Leu Ser Glu Phe Gly
       130                 135                 140
   Lys Ser Asp Ser Val Glu Tyr Ile Ile Ile Thr Asp Ser Glu Gly Ile
   145                 150                 155                 160
   Leu Gly Ile Gly Asp Gln Gly Val Gly Gly Val Leu Ile Ser Val Ala
                   165                 170                 175
   Lys Gly His Leu Met Thr Leu Cys Ala Gly Leu Asp Pro Asn Arg Phe
               180                 185                 190
   Leu Pro Ile Val Leu Asp Val Gly Thr Asn Asn Glu Thr His Arg Lys
           195                 200                 205
   Asn His Gln Tyr Met Gly Leu Arg Lys Asp Arg Val Arg Gly Glu Gln
       210                 215                 220
   Tyr Asp Ser Phe Leu Asp Asn Val Ile Lys Ala Ile Arg Glu Val Phe
   225                 230                 235                 240
   Pro Glu Ala Phe Ile His Phe Glu Asp Phe Gly Leu Ala Asn Ala Lys
                   245                 250                 255
   Arg Ile Leu Asp His Tyr Arg Pro Asp Ile Ala Cys Phe Asn Asp Asp
               260                 265                 270
   Ile Gln Gly Thr Gly Ala Val Ala Leu Ala Ala Ile Ile Gly Ala Leu
           275                 280                 285
   His Val Thr Lys Ser Pro Leu Thr Glu Gln Arg Ile Met Ile Phe Gly
       290                 295                 300
   Ala Gly Thr Ala Gly Val Gly Ile Ala Asn Gln Ile Val Ala Gly Met
   305                 310                 315                 320
   Val Thr Asp Gly Leu Ser Leu Asp Lys Ala Arg Gly Asn Leu Phe Met
                   325                 330                 335
   Ile Asp Arg Cys Gly Leu Leu Leu Glu Arg His Ala Lys Ile Ala Thr
               340                 345                 350
   Asp Gly Gln Lys Pro Phe Leu Lys Lys Asp Ser Asp Phe Lys Glu Val
           355                 360                 365
   Pro Ser Gly Asp Ile Asn Leu Glu Ser Ala Ile Ala Leu Val Lys Pro
       370                 375                 380
   Thr Ile Leu Leu Gly Cys Ser Gly Gln Pro Gly Lys Phe Thr Glu Lys
   385                 390                 395                 400
   Ala Ile Arg Glu Met Ser Lys His Val Glu Arg Pro Ile Ile Phe Pro
                   405                 410                 415
   Ile Ser Asn Pro Thr Thr Leu Met Glu Ala Lys Pro Asp Gln Ile Asp
               420                 425                 430
   Lys Trp Ser Asp Gly Lys Ala Leu Ile Ala Thr Gly Ser Pro Leu Pro
           435                 440                 445
   Pro Leu Asn Arg Asn Gly Lys Lys Tyr Val Ile Ser Gln Cys Asn Asn
       450                 455                 460
   Ala Leu Leu Tyr Pro Ala Leu Gly Val Ala Cys Val Leu Ser Arg Cys
   465                 470                 475                 480
   Lys Leu Leu Ser Asp Gly Met Leu Lys Ala Ala Ser Asp Ala Leu Ala
                   485                 490                 495
   Thr Val Pro Arg Ser Leu Phe Ala Ala Asp Glu Ala Leu Leu Pro Asp
               500                 505                 510
   Leu Asn Asn Ala Arg Glu Ile Ser Arg His Ile Val Phe Ala Val Leu
           515                 520                 525
   Lys Gln Ala Val Ser Glu Gly Met Ser Thr Val Asp Leu Pro Lys Asp
       530                 535                 540
   Asp Ala Lys Leu Lys Glu Trp Ile Ile Glu Arg Glu Trp Asn Pro Glu
   545                 550                 555                 560
   Tyr Lys Pro Phe Val
                   565
<210> 24
   <211> 21
   <212> DNA
   <213> Artificial Sequence
<220>
   <223> Description of Artificial Sequence: UF for pdc2-deleted fragment
<400> 24
   ctctccagct ccatccataa g 21
<210> 25
   <211> 45
   <212> DNA
   <213> Artificial Sequence
<220>
   <223> Description of Artificial Sequence: UR for pdc2-deleted fragment
<400> 25
   gacacaactt cctaccaaaa agcctttctg cccatgtttt ctgtc 45
<210> 26
   <211> 23
   <212> DNA
   <213> Artificial Sequence
<220>
   <223> Description of Artificial Sequence: OF for pdc2-deleted fragment
<400> 26
   gctttttggt aggaagttgt gtc 23
<210> 27
   <211> 43
   <212> DNA
   <213> Artificial Sequence
<220>
   <223> Description of Artificial Sequence: OR for pdc2-deleted fragment
<400> 27
   agtgggattt gtagctaagc tgtatccatt tcagccgttt gtg 43
<210> 28
   <211> 45
   <212> DNA
   <213> Artificial Sequence
<220>
   <223> Description of Artificial Sequence: DF for pdc2-deleted fragment
<400> 28
   aagtttcgtc aatatcacaa gctgacagaa aacatgggca gaaag 45
<210> 29
   <211> 24
   <212> DNA
   <213> Artificial Sequence
<220>
   <223> Description of Artificial Sequence: DR for pdc2-deleted fragment
<400> 29
   gttccttaga aaaagcaact ttgg 24
<210> 30
   <211> 20
   <212> DNA
   <213> Artificial Sequence
<220>
   <223> Description of Artificial Sequence: FF for pdc2-deleted fragment
<400> 30
   cataagcttg ccaccacttc 20
<210> 31
   <211> 24
   <212> DNA
   <213> Artificial Sequence
<220>
   <223> Description of Artificial Sequence: FR for pdc2-deleted fragment
<400> 31
   gaaaaagcaa ctttggtatt ctgc 24
<210> 32
   <211> 21
   <212> DNA
   <213> Artificial Sequence
<220>
   <223> Description of Artificial Sequence: F for ura4 fragment
<400> 32
   agcttagcta caaatcccac t 21
<210> 33
   <211> 23
   <212> DNA
   <213> Artificial Sequence
<220>
   <223> Description of Artificial Sequence: R for ura4 fragment
<400> 33
   agcttgtgat attgacgaaa ctt 23
<210> 34
   <211> 5400
   <212> DNA
   <213> Artificial Sequence
<220>
   <223> Description of Artificial Sequence: Fr.1 for pSMh
<400> 34
   tggggagcct ggggactttc cacaccctaa ctgacacaca ttccacagga cattgattat      60
   tgactaggtt taaaccacgt gggtgaccaa attgtcaagc gtgagcttgc cactggtgtc     120
   cccattgtct accacttgga caaggacggc aagtacgtct ccaaggagct cattgacaac     180
   tagatttcct actagatttt agtcgcctat tttaacgaca tatacactgt ttttctacac     240
   taactcattt ctatgatgtt gtataatgca atttcttttt ttgaaatcaa atcaaactac     300
   aaggtagacg aaataataga gtaattatga gggagtaaca agggagtaac ggggtggtgg     360
   aagaagtgag tgagttggta gtgcaaggag agagaatcgt accaatacat taggaggaag     420
   aaaaagtatc gatttagtag aaagaaatag cattatcgta ctgtgtgaag agtttacagt     480
   cttgctagtt attaatagta atcaattacg gggtcattag ttcatagccc atatatggag     540
   ttccgcgtta cataacttac ggtaaatggc ccgcctggct gaccgcccaa cgacccccgc     600
   ccattgacgt caataatgac gtatgttccc atagtaacgc caatagggac tttccattga     660
   cgtcaatggg tggagtattt acggtaaact gcccacttgg cagtacatca agtgtatcat     720
   atgccaagta cgccccctat tgacgtcaat gacggtaaat ggcccgcctg gcattttgcc     780
   cagtacatga ccttatggga ctttcctact tggcagtaca tctacgtatt agtcatcgct     840
   attaccatgg tgatgcggtt ttggcagtac atcaatgggc gtggatagcg gtttgactca     900
   cggggatttc caagtctcca ccccattgac gtcaatggga gtttgttttg gcaccaaaat     960
   caacgggact ttccaaaatg tcgtaacaac tccgccccat tgacgcaaat gggcggtagg 1020
   cgtgtacggt gggaggtcta tataagcaga tttctcttta gttctttgca agaaggtaga 1080
   gataaagaca ctttttcaaa tatgagcgaa aaacaggttg tagggatcct tggaggtggt 1140
   caattgggcc gaatgatggt agaggcagcc catcgcttaa acatcaaatg catcatcttg 1200
   gatgcagcaa attctcctgc caaacaaatt gatggaggac gtgagcacat tgatgcatca 1260
   tttactgacc ccgatgcaat tgttgaactg tctaagaagt gcacgttatt aacaactgaa 1320
   attgagcata ttaacactga tgccttggca gccgttacga aatctgttgc tgttgaaccc 1380
   tctcctgcaa ctctgcgatg cattcaagac aaatatcttc aaaaacagca tttacaggtt 1440
   tttaagattg cacttcctga attttgcgat gcacctgacc aggaaagtgt tgaaaaagca 1500
   ggccaagagt ttggttatcc ttttgtactg aaaagtaaaa cattggctta cgacggtcgt 1560
   ggaaattacg ttgttcatca accatctgag attcctactg ccatcaaagc acttggtgat 1620
   cgtccgcttt atgttgaaaa gttcgttcct ttctccatgg aaattgcagt gatggtagta 1680
   cgcagtttag acggaaaagt ttatgcttat cctacaactg agaccattca aaaggataat 1740
   gtttgtcatt tagtatatgc ccctgctcgt cttcccttct caattcaaca gcgtgctcaa 1800
   acccttgcca tggatgcggt gcgcactttt gaaggcgctg gtatatatgg tgtagagatg 1860
   tttgttttgc aagatggtga gaccatttta ctcaacgaaa ttgctcctcg gcctcacaat 1920
   tcaggtcact acaccattga agcttgccca acttctcagt ttgaagctca cttacgggcc 1980
   atatgtggtc ttcctttcag cgaaatcaac acccaactct cgacttccac aactcatgcg 2040
   ttgatggtaa atattttagg tactgatgat cctgattatg tttcaaagat cgctaaacgt 2100
   tctctgtcca ttcccggtgc aactttgcat ctttatggta aagctgaatc tagaaagggt 2160
   cgcaagatgg gacacgttac catcatttct gattcacctc aagaatgtga acgtaggtat 2220
   cagatgcttc ttgacgtcaa agatcctgtc gaatcacctg ttgttggtat tatcatgggt 2280
   tcggattctg atttaagcaa gatgaaagat gctgccgtca ttttagatga attcaaggtg 2340
   ccttacgaac ttactattgt ttcagctcac cgcacaccag atcgcatggt tacttatgct 2400
   cgtaccgcag cttcaagagg gttgcgtgtg attattgctg gtgctggtgg tgccgctcat 2460
   ttgcctggta tggttgctgc aatgacacct cttccagtaa tcggcgttcc tgtaaaagga 2520
   agcactcttg acggagttga ctctcttcac tctattgttc agatgcctcg aggtgtccct 2580
   gtcgccactg ttgctatcaa taatagccaa aatgccggta ttttagcctg tcgtatactt 2640
   gctacatttc aaccctccct tttggctgct atggagagct ttatggacaa tatgaaagag 2700
   attgttttag aaaaggctga taaattagaa aaagttggtt ggaaaaatta ttctgcatag 2760
   atggatgcta gagtatttca aagctattca gctagagctg aggggatgaa aaatcccatt 2820
   gccaaggaat tgttggcttt gatggaagaa aagcaaagca acttgtcagt cgcggtcgat 2880
   ttgacgaaga aatccgaaat cttagaattg gtagataaaa ttggacccta tgtctgtgtt 2940
   atcaagacac atattgacgt tgtcgaggat ttcgaccagg atatggtaga aaaactggtg 3000
   gccttaggta aaaagcatcg ttttcttatc tttgaggatc gcaaattcgc agacattgga 3060
   aataccgtca agctacaata tgcatctggt gtgtacaaaa ttgcttcttg ggctcatatc 3120
   acaaattgcc atacagtgcc aggcgagggt attatacaag gcctcaaaga agttggttta 3180
   cctttgggac gtggtctctt gcttttggct gaaatgtctt ccaaaggctc tttggctact 3240
   ggttcctaca cagagaaaac cttagaatgg tttgagaagc ataccgattt ttgctttggc 3300
   tttatagctg gtcgtcgatt tcctaacctt caaagcgact acataactat gtcccctggt 3360
   atcggcttgg atgttaaagg agacgggctg ggacagcaat atcgtactcc tgaagaagtg 3420
   attgtaaact gcggtagcga tatcatcatt gttggtcgtg gagtctatgg agctggtcgt 3480
   aatcctgttg tcgaagccaa gagatataga gaagctggtt ggaaggcata tcagcaaaga 3540
   ctttctcagc attaaaaaaa gactaatgta aaattttttt ggttggttat tgaaaaagtc 3600
   gatgccttgt ttgcgtttgt tttcctaggc gttttatgtc agaaggcatt tagaattagt 3660
   atacaagtac tctttggtaa aattttatgt agcgactaaa atattaacta ttatagataa 3720
   acaccttggg aataaaaagt aatttgctat agtaatttat taaacatgct cctacaacat 3780
   tacctctagt tattaatagt aatcaattac ggggtcatta gttcatagcc catatatgga 3840
   gttccgcgtt acataactta cggtaaatgg cccgcctggc tgaccgccca acgacccccg 3900
   cccattgacg tcaataatga cgtatgttcc catagtaacg ccaataggga ctttccattg 3960
   acgtcaatgg gtggagtatt tacggtaaac tgcccacttg gcagtacatc aagtgtatca 4020
   tatgccaagt acgcccccta ttgacgtcaa tgacggtaaa tggcccgcct ggcattttgc 4080
   ccagtacatg accttatggg actttcctac ttggcagtac atctacgtat tagtcatcgc 4140
   tattaccatg gtgatgcggt tttggcagta catcaatggg cgtggatagc ggtttgactc 4200
   acggggattt ccaagtctcc accccattga cgtcaatggg agtttgtttt ggcaccaaaa 4260
   tcaacgggac tttccaaaat gtcgtaacaa ctccgcccca ttgacgcaaa tgggcggtag 4320
   gcgtgtacgg tgggaggtct atataagcag atttctcttt agttctttgc aagaaggtag 4380
   agataaagac actttttcaa acatgtcgat tacaaggatg atgatgataa gtaagaattc 4440
   gagctcggta cccggggatc ctctagagtc gacctgcagg catgcaagct taaataggaa 4500
   agtttcttca acaggattac agtgtagcta cctacatgct gaaaaatata gcctttaaat 4560
   catttttata ttataactct gtataataga gataagtcca ttttttaaaa atgttttccc 4620
   caaaccataa aaccctatac aagttgttct agtaacaata catgagaaag atgtctatgt    4680
   agctgaaaat aaaatgacgt cacaagacga tctgcctcgc gcgtttcggt gatgacggtg    4740
   aaaacctctg acacatgcag ctcccggaga cggtcacagc ttgtctgtaa gcggatgccg    4800
   ggagcagaca agcccgtcag ggcgcgtcag cgggtgttgg cgggtgtcgg ggcgcagcca    4860
   tgacccagtc acgtagcgat agcggagtgt aatcgtttgt gaagagttta cagtcttgca    4920
   gcaagtttct caatgcttta gagtgcctat gtctacgatg gatgctacgt cttcctctga    4980
   atagtctatt aaaaatcaac aatacctttt tgttacaagg tgttgagtgt cattaaaaga    5040
   caaagtgaaa tacaagggga ttattaagaa attatagtgc ggcggagcaa agtagactag    5100
   tagtccattt tctatgcaca gtagaaaaca aaaagtattc aacaaaaaaa aaaaaaaaca    5160
   gaatacattg caaggattag aaagcaaaca acccttcttt acaaggatgt aatgaagcat    5220
   atttttatta tcaaaaacat tgatgatgaa attggtatgc ttttggatac tactattgct    5280
   ttttactcta cgacaatgac atttcacatt caattggttt aaaccacgtg gattactggc    5340
   ttaactatgc ggcatcagag cagattgtac tgagagtgca ccatatgcgg tgtgaaatac    5400
<210> 35
   <211> 8849
   <212> DNA
   <213> Artificial Sequence
<220>
   <223> Description of Artificial Sequence: pSMh
<400> 35
   gaattgttgt tgttaacttg tttattgcag cttataatgg ttacaaataa agcaatagca      60
   tcacaaattt cacaaataaa gcattttttt cactgcattc tagttgtggt ttgtccaaac     120
   tcatcaatgt atcttatcat gtctggatcg atcccggcag gttgggcgtc gcttggtcgg     180
   tcatttcgaa ccccagagtc ccgctcagaa gaactcgtca agaaggcgat agaaggcgat     240
   gcgctgcgaa tcgggagcgg cgataccgta aagcacgagg aagcggtcag cccattcgcc     300
   gccaagctct tcagcaatat cacgggtagc caacgctatg tcctgatagc ggtccgccac     360
   acccagccgg ccacagtcga tgaatccaga aaagcggcca ttttccacca tgatattcgg     420
   caagcaggca tcgccatggg tcacgacgag atcctcgccg tcgggcatgc gcgccttgag     480
   cctggcgaac agttcggctg gcgcgagccc ctgatgctct tcgtccagat catcctgatc     540
   gacaagaccg gcttccatcc gagtacgtgc tcgctcgatg cgatgtttcg cttggtggtc     600
   gaatgggcag gtagccggat caagcgtatg cagccgccgc attgcatcag ccatgatgga     660
   tactttctcg gcaggagcaa ggtgagatga caggagatcc tgccccggca cttcgcccaa     720
   tagcagccag tcccttcccg cttcagtgac aacgtcgagc acagctgcgc aaggaacgcc     780
   cgtcgtggcc agccacgata gccgcgctgc ctcgtcctgc agttcattca gggcaccgga     840
   caggtcggtc ttgacaaaaa gaaccgggcg cccctgcgct gacagccgga acacggcggc     900
   atcagagcag ccgattgtct gttgtgccca gtcatagccg aatagcctct ccacccaagc     960
   ggccggagaa cctgcgtgca atccatcttg ttcaatcatg cgaaacgatc ctcatcctgt    1020
   ctcttgatca gatccgggac ctgaaataaa agacaaaaag actaaactta ccagttaact    1080
   ttctggtttt tcagttcctc gaggagcttt ttgcaaaagc ctaggcctcc aaaaaagcct    1140
   cctcactact tctggaatag ctcagaggcc gaggcggcct cggcctctgc ataaataaaa    1200
   aaaattagtc agccatgggg cggagaatgg gcggaactgg gcggagttag gggcgggatg    1260
   ggcggagtta ggggcgggac tatggttgct gactaattga gatgcatgct ttgcatactt    1320
   ctgcctgctg gggagcctgg ggactttcca cacctggttg ctgactaatt gagatgcatg    1380
   ctttgcatac ttctgcctgc tggggagcct ggggactttc cacaccctaa ctgacacaca    1440
   ttccacagga cattgattat tgactaggtt taaaccacgt gggtgaccaa attgtcaagc    1500
   gtgagcttgc cactggtgtc cccattgtct accacttgga caaggacggc aagtacgtct    1560
   ccaaggagct cattgacaac tagatttcct actagatttt agtcgcctat tttaacgaca    1620
   tatacactgt ttttctacac taactcattt ctatgatgtt gtataatgca atttcttttt    1680
   ttgaaatcaa atcaaactac aaggtagacg aaataataga gtaattatga gggagtaaca    1740
   agggagtaac ggggtggtgg aagaagtgag tgagttggta gtgcaaggag agagaatcgt    1800
   accaatacat taggaggaag aaaaagtatc gatttagtag aaagaaatag cattatcgta    1860
   ctgtgtgaag agtttacagt cttgctagtt attaatagta atcaattacg gggtcattag    1920
   ttcatagccc atatatggag ttccgcgtta cataacttac ggtaaatggc ccgcctggct    1980
   gaccgcccaa cgacccccgc ccattgacgt caataatgac gtatgttccc atagtaacgc    2040
   caatagggac tttccattga cgtcaatggg tggagtattt acggtaaact gcccacttgg    2100
   cagtacatca agtgtatcat atgccaagta cgccccctat tgacgtcaat gacggtaaat    2160
   ggcccgcctg gcattttgcc cagtacatga ccttatggga ctttcctact tggcagtaca    2220
   tctacgtatt agtcatcgct attaccatgg tgatgcggtt ttggcagtac atcaatgggc    2280
   gtggatagcg gtttgactca cggggatttc caagtctcca ccccattgac gtcaatggga    2340
   gtttgttttg gcaccaaaat caacgggact ttccaaaatg tcgtaacaac tccgccccat 2400
   tgacgcaaat gggcggtagg cgtgtacggt gggaggtcta tataagcaga tttctcttta 2460
   gttctttgca agaaggtaga gataaagaca ctttttcaaa tatgagcgaa aaacaggttg 2520
   tagggatcct tggaggtggt caattgggcc gaatgatggt agaggcagcc catcgcttaa 2580
   acatcaaatg catcatcttg gatgcagcaa attctcctgc caaacaaatt gatggaggac 2640
   gtgagcacat tgatgcatca tttactgacc ccgatgcaat tgttgaactg tctaagaagt 2700
   gcacgttatt aacaactgaa attgagcata ttaacactga tgccttggca gccgttacga 2760
   aatctgttgc tgttgaaccc tctcctgcaa ctctgcgatg cattcaagac aaatatcttc 2820
   aaaaacagca tttacaggtt tttaagattg cacttcctga attttgcgat gcacctgacc 2880
   aggaaagtgt tgaaaaagca ggccaagagt ttggttatcc ttttgtactg aaaagtaaaa 2940
   cattggctta cgacggtcgt ggaaattacg ttgttcatca accatctgag attcctactg 3000
   ccatcaaagc acttggtgat cgtccgcttt atgttgaaaa gttcgttcct ttctccatgg 3060
   aaattgcagt gatggtagta cgcagtttag acggaaaagt ttatgcttat cctacaactg 3120
   agaccattca aaaggataat gtttgtcatt tagtatatgc ccctgctcgt cttcccttct 3180
   caattcaaca gcgtgctcaa acccttgcca tggatgcggt gcgcactttt gaaggcgctg 3240
   gtatatatgg tgtagagatg tttgttttgc aagatggtga gaccatttta ctcaacgaaa 3300
   ttgctcctcg gcctcacaat tcaggtcact acaccattga agcttgccca acttctcagt 3360
   ttgaagctca cttacgggcc atatgtggtc ttcctttcag cgaaatcaac acccaactct 3420
   cgacttccac aactcatgcg ttgatggtaa atattttagg tactgatgat cctgattatg 3480
   tttcaaagat cgctaaacgt tctctgtcca ttcccggtgc aactttgcat ctttatggta 3540
   aagctgaatc tagaaagggt cgcaagatgg gacacgttac catcatttct gattcacctc 3600
   aagaatgtga acgtaggtat cagatgcttc ttgacgtcaa agatcctgtc gaatcacctg 3660
   ttgttggtat tatcatgggt tcggattctg atttaagcaa gatgaaagat gctgccgtca 3720
   ttttagatga attcaaggtg ccttacgaac ttactattgt ttcagctcac cgcacaccag 3780
   atcgcatggt tacttatgct cgtaccgcag cttcaagagg gttgcgtgtg attattgctg 3840
   gtgctggtgg tgccgctcat ttgcctggta tggttgctgc aatgacacct cttccagtaa 3900
   tcggcgttcc tgtaaaagga agcactcttg acggagttga ctctcttcac tctattgttc 3960
   agatgcctcg aggtgtccct gtcgccactg ttgctatcaa taatagccaa aatgccggta 4020
   ttttagcctg tcgtatactt gctacatttc aaccctccct tttggctgct atggagagct 4080
   ttatggacaa tatgaaagag attgttttag aaaaggctga taaattagaa aaagttggtt 4140
   ggaaaaatta ttctgcatag atggatgcta gagtatttca aagctattca gctagagctg 4200
   aggggatgaa aaatcccatt gccaaggaat tgttggcttt gatggaagaa aagcaaagca 4260
   acttgtcagt cgcggtcgat ttgacgaaga aatccgaaat cttagaattg gtagataaaa 4320
   ttggacccta tgtctgtgtt atcaagacac atattgacgt tgtcgaggat ttcgaccagg 4380
   atatggtaga aaaactggtg gccttaggta aaaagcatcg ttttcttatc tttgaggatc 4440
   gcaaattcgc agacattgga aataccgtca agctacaata tgcatctggt gtgtacaaaa 4500
   ttgcttcttg ggctcatatc acaaattgcc atacagtgcc aggcgagggt attatacaag 4560
   gcctcaaaga agttggttta cctttgggac gtggtctctt gcttttggct gaaatgtctt 4620
   ccaaaggctc tttggctact ggttcctaca cagagaaaac cttagaatgg tttgagaagc 4680
   ataccgattt ttgctttggc tttatagctg gtcgtcgatt tcctaacctt caaagcgact 4740
   acataactat gtcccctggt atcggcttgg atgttaaagg agacgggctg ggacagcaat 4800
   atcgtactcc tgaagaagtg attgtaaact gcggtagcga tatcatcatt gttggtcgtg 4860
   gagtctatgg agctggtcgt aatcctgttg tcgaagccaa gagatataga gaagctggtt 4920
   ggaaggcata tcagcaaaga ctttctcagc attaaaaaaa gactaatgta aaattttttt 4980
   ggttggttat tgaaaaagtc gatgccttgt ttgcgtttgt tttcctaggc gttttatgtc 5040
   agaaggcatt tagaattagt atacaagtac tctttggtaa aattttatgt agcgactaaa 5100
   atattaacta ttatagataa acaccttggg aataaaaagt aatttgctat agtaatttat 5160
   taaacatgct cctacaacat tacctctagt tattaatagt aatcaattac ggggtcatta 5220
   gttcatagcc catatatgga gttccgcgtt acataactta cggtaaatgg cccgcctggc 5280
   tgaccgccca acgacccccg cccattgacg tcaataatga cgtatgttcc catagtaacg 5340
   ccaataggga ctttccattg acgtcaatgg gtggagtatt tacggtaaac tgcccacttg 5400
   gcagtacatc aagtgtatca tatgccaagt acgcccccta ttgacgtcaa tgacggtaaa 5460
   tggcccgcct ggcattttgc ccagtacatg accttatggg actttcctac ttggcagtac 5520
   atctacgtat tagtcatcgc tattaccatg gtgatgcggt tttggcagta catcaatggg 5580
   cgtggatagc ggtttgactc acggggattt ccaagtctcc accccattga cgtcaatggg 5640
   agtttgtttt ggcaccaaaa tcaacgggac tttccaaaat gtcgtaacaa ctccgcccca 5700
   ttgacgcaaa tgggcggtag gcgtgtacgg tgggaggtct atataagcag atttctcttt 5760
   agttctttgc aagaaggtag agataaagac actttttcaa acatgtcgat tacaaggatg 5820
   atgatgataa gtaagaattc gagctcggta cccggggatc ctctagagtc gacctgcagg 5880
   catgcaagct taaataggaa agtttcttca acaggattac agtgtagcta cctacatgct 5940
   gaaaaatata gcctttaaat catttttata ttataactct gtataataga gataagtcca 6000
   ttttttaaaa atgttttccc caaaccataa aaccctatac aagttgttct agtaacaata 6060
   catgagaaag atgtctatgt agctgaaaat aaaatgacgt cacaagacga tctgcctcgc 6120
   gcgtttcggt gatgacggtg aaaacctctg acacatgcag ctcccggaga cggtcacagc 6180
   ttgtctgtaa gcggatgccg ggagcagaca agcccgtcag ggcgcgtcag cgggtgttgg 6240
   cgggtgtcgg ggcgcagcca tgacccagtc acgtagcgat agcggagtgt aatcgtttgt 6300
   gaagagttta cagtcttgca gcaagtttct caatgcttta gagtgcctat gtctacgatg 6360
   gatgctacgt cttcctctga atagtctatt aaaaatcaac aatacctttt tgttacaagg 6420
   tgttgagtgt cattaaaaga caaagtgaaa tacaagggga ttattaagaa attatagtgc 6480
   ggcggagcaa agtagactag tagtccattt tctatgcaca gtagaaaaca aaaagtattc 6540
   aacaaaaaaa aaaaaaaaca gaatacattg caaggattag aaagcaaaca acccttcttt 6600
   acaaggatgt aatgaagcat atttttatta tcaaaaacat tgatgatgaa attggtatgc 6660
   ttttggatac tactattgct ttttactcta cgacaatgac atttcacatt caattggttt 6720
   aaaccacgtg gattactggc ttaactatgc ggcatcagag cagattgtac tgagagtgca 6780
   ccatatgcgg tgtgaaatac cgcacagatg cgtaaggaga aaataccgca tcaggcgctc 6840
   ttccgcttcc tcgctcactg actcgctgcg ctcggtcgtt cggctgcggc gagcggtatc 6900
   agctcactca aaggcggtaa tacggttatc cacagaatca ggggataacg caggaaagaa 6960
   catgcatgtg agcaaaaggc cagcaaaagg ccaggaaccg taaaaaggcc gcgttgctgg 7020
   cgtttttcca taggctccgc ccccctgacg agcatcacaa aaatcgacgc tcaagtcaga 7080
   ggtggcgaaa cccgacagga ctataaagat accaggcgtt tccccctgga agctccctcg 7140
   tgcgctctcc tgttccgacc ctgccgctta ccggatacct gtccgccttt ctcccttcgg 7200
   gaagcgtggc gctttctcat agctcacgct gtaggtatct cagttcggtg taggtcgttc 7260
   gctccaagct gggctgtgtg cacgaacccc ccgttcagcc cgaccgctgc gccttatccg 7320
   gtaactatcg tcttgagtcc aacccggtaa gacacgactt atcgccactg gcagcagcca 7380
   ctggtaacag gattagcaga gcgaggtatg taggcggtgc tacagagttc ttgaagtggt 7440
   ggcctaacta cggctacact agaaggacag tatttggtat ctgcgctctg ctgaagccag 7500
   ttaccttcgg aaaaagagtt ggtagctctt gatccggcaa acaaaccacc gctggtagcg 7560
   gtggtttttt tgtttgcaag cagcagatta cgcgcagaaa aaaaggatct caagaagatc 7620
   ctttgatctt ttctacgggg tctgacgctc agtggaacga aaactcacgt taagggattt 7680
   tggtcatgag attatcaaaa aggatcttca cctagatcct tttaaattaa aaatgaagtt 7740
   ttaaatcaat ctaaagtata tatgagtaaa cttggtctga cagttaccaa tgcttaatca 7800
   gtgaggcacc tatctcagcg atctgtctat ttcgttcatc catagttgcc tgactccccg 7860
   tcgtgtagat aactacgata cgggagggct taccatctgg ccccagtgct gcaatgatac 7920
   cgcgagaccc acgctcaccg gctccagatt tatcagcaat aaaccagcca gccggaaggg 7980
   ccgagcgcag aagtggtcct gcaactttat ccgcctccat ccagtctatt aattgttgcc 8040
   gggaagctag agtaagtagt tcgccagtta atagtttgcg caacgttgtt gccattgctg 8100
   caggcatcgt ggtgtcacgc tcgtcgtttg gtatggcttc attcagctcc ggttcccaac 8160
   gatcaaggcg agttacatga tcccccatgt tgtgcaaaaa agcggttagc tccttcggtc 8220
   ctccgatcgt tgtcagaagt aagttggccg cagtgttatc actcatggtt atggcagcac 8280
   tgcataattc tcttactgtc atgccatccg taagatgctt ttctgtgact ggtgagtact 8340
   caaccaagtc attctgagaa tagtgtatgc ggcgaccgag ttgctcttgc ccggcgtcaa 8400
   cacgggataa taccgcgcca catagcagaa ctttaaaagt gctcatcatt ggaaaacgtt 8460
   cttcggggcg aaaactctca aggatcttac cgctgttgag atccagttcg atgtaaccca 8520
   ctcgtgcacc caactgatct tcagcatctt ttactttcac cagcgtttct gggtgagcaa 8580
   aaacaggaag gcaaaatgcc gcaaaaaagg gaataagggc gacacggaaa tgttgaatac 8640
   tcatactctt cctttttcaa tattattgaa gcatttatca gggttattgt ctcatgagcg 8700
   gatacatatt tgaatgtatt tagaaaaata aacaaatagg ggttccgcgc acatttcccc 8760
   gaaaagtgcc acctgacgtc taagaaacca ttattatcat gacattaacc tataaaaata 8820
   ggcgtatcac gaggcccttt cgtcttcaa 8849
<210> 36
   <211> 31
   <212> DNA
   <213> Artificial Sequence
<220>
   <223> Description of Artificial Sequence: AniPYC-F
<400> 36
   gacacttttt caaacatggc tgctccccgc c 31
<210> 37
   <211> 36
   <212> DNA
   <213> Artificial Sequence
<220>
   <223> Description of Artificial Sequence: AniPYC-R
<400> 37
   atcatccttg taatcggcct tgacgatctt gcagac 36
<210> 38
   <211> 45
   <212> DNA
   <213> Artificial Sequence
<220>
   <223> Description of Artificial Sequence: BbrPYC-F
<400> 38
   gacacttttt caaacatgaa aaagagaaaa atcaatcgcc tactg 45
<210> 39
   <211> 40
   <212> DNA
   <213> Artificial Sequence
<220>
   <223> Description of Artificial Sequence: BbrPYC-R
<400> 39
   atcatccttg taatcctcca tttcaatgag cagatcacca 40
<210> 40
   <211> 44
   <212> DNA
   <213> Artificial Sequence
<220>
   <223> Description of Artificial Sequence: DhaPYC-F
<400> 40
   gacacttttt caaacatgag tgacggtaat gaacataaga tcaa 44
<210> 41
   <211> 45
   <212> DNA
   <213> Artificial Sequence
<220>
   <223> Description of Artificial Sequence: DhaPYC-R
<400> 41
   atcatccttg taatctttaa caacacttgc aatcaaatcg ttagc 45
<210> 42
   <211> 40
   <212> DNA
   <213> Artificial Sequence
<220>
   <223> Description of Artificial Sequence: KlaPYC-F
<400> 42
   gacacttttt caaacatgtc atctcaacta gctggattgc 40
<210> 43
   <211> 37
   <212> DNA
   <213> Artificial Sequence
<220>
   <223> Description of Artificial Sequence: KlaPYC-R
<400> 43
   atcatccttg taatcttcct tagctggagc ttcttcc 37
<210> 44
   <211> 36
   <212> DNA
   <213> Artificial Sequence
<220>
   <223> Description of Artificial Sequence: LthPYC-F
<400> 44
   gacacttttt caaacatgtc caaactggca ggactg 36
<210> 45
   <211> 39
   <212> DNA
   <213> Artificial Sequence
<220>
   <223> Description of Artificial Sequence: LthPYC-R
<400> 45
   atcatccttg taatcttctt ttggtgggac agattcctc 39
<210> 46
   <211> 43
   <212> DNA
   <213> Artificial Sequence
<220>
   <223> Description of Artificial Sequence: LelPYC-F
<400> 46
   gacacttttt caaacatgac atcaattgct tcatcaaaca ctc 43
<210> 47
   <211> 47
   <212> DNA
   <213> Artificial Sequence
<220>
   <223> Description of Artificial Sequence: LelPYC-R
<400> 47
   atcatccttg taatcatgtt tcaaaatact ggtgatcaaa tcatttg 47
<210> 48
   <211> 37
   <212> DNA
   <213> Artificial Sequence
<220>
   <223> Description of Artificial Sequence: ScePYC-F
<400> 48
   gacacttttt caaacatgtc gcaaagaaaa ttcgccg 37
<210> 49
   <211> 40
   <212> DNA
   <213> Artificial Sequence
<220>
   <223> Description of Artificial Sequence: ScePYC-R
<400> 49
   atcatccttg taatctgcct tagtttcaac aggaacttgg 40
<210> 50
   <211> 54
   <212> DNA
   <213> Artificial Sequence
<220>
   <223> Description of Artificial Sequence: CorPYC-F
<400> 50
   cactttttca aacatgattt ttataaattc atcatcaatt tctacaaaat ctcc 54
<210> 51
   <211> 47
   <212> DNA
   <213> Artificial Sequence
<220>
   <223> Description of Artificial Sequence: CorPYC-R
<400> 51
   atcatccttg taatcggctt tcaaaatact ggtgattaaa tcattag 47
<210> 52
   <211> 40
   <212> DNA
   <213> Artificial Sequence
<220>
   <223> Description of Artificial Sequence: CtrPYC-F
<400> 52
   cactttttca aacatgtctg taccagtcca aaaagctaac 40
<210> 53
   <211> 51
   <212> DNA
   <213> Artificial Sequence
<220>
   <223> Description of Artificial Sequence: CtrPYC-R
<400> 53
   atcatccttg taatcatgtt gaataatgtt agtaatcaaa tcattagcat c 51
<210> 54
   <211> 41
   <212> DNA
   <213> Artificial Sequence
<220>
   <223> Description of Artificial Sequence: NcaPYC-F
<400> 54
   cactttttca aacatgtcca taaacaaaaa actaaacggc c 41
<210> 55
   <211> 42
   <212> DNA
   <213> Artificial Sequence
<220>
   <223> Description of Artificial Sequence: NcaPYC-R
<400> 55
   atcatccttg taatcagatg gaggcgtaga ttcttctaaa ag 42
<210> 56
   <211> 44
   <212> DNA
   <213> Artificial Sequence
<220>
   <223> Description of Artificial Sequence: NdaPYC-F
<400> 56
   cactttttca aacatgtcga acaaaaaatt caatggtatc agag 44
<210> 57
   <211> 41
   <212> DNA
   <213> Artificial Sequence
<220>
   <223> Description of Artificial Sequence: NdaPYC-R
<400> 57
   atcatccttg taatctgctt cttccggaat ttcagtttct t 41
<210> 58
   <211> 36
   <212> DNA
   <213> Artificial Sequence
<220>
   <223> Description of Artificial Sequence: SkuPYC-F
<400> 58
   cactttttca aacatgtcgc aaaagaaatt cgctgg 36
<210> 59
   <211> 41
   <212> DNA
   <213> Artificial Sequence
<220>
   <223> Description of Artificial Sequence: SkuPYC-R
<400> 59
   atcatccttg taatctgctt tagtttcagc tggaacttga t 41
<210> 60
   <211> 41
   <212> DNA
   <213> Artificial Sequence
<220>
   <223> Description of Artificial Sequence: SpoPYC-F
<400> 60
   cactttttca aacatgactt ctaaatatga tgcgttgttg c 41
<210> 61
   <211> 38
   <212> DNA
   <213> Artificial Sequence
<220>
   <223> Description of Artificial Sequence: SpoPYC-R
<400> 61
   atcatccttg taatcctcgt gttcaagaac agcacaca 38
<210> 62
   <211> 45
   <212> DNA
   <213> Artificial Sequence
<220>
   <223> Description of Artificial Sequence: TblPYC-F
<400> 62
   cactttttca aacatgacaa ataagaaatt ctctggtcta agaga 45
<210> 63
   <211> 43
   <212> DNA
   <213> Artificial Sequence
<220>
   <223> Description of Artificial Sequence: TblPYC-R
<400> 63
   atcatccttg taatcttttg gtggttctgg atcttctaat gtg 43
<210> 64
   <211> 37
   <212> DNA
   <213> Artificial Sequence
<220>
   <223> Description of Artificial Sequence: TdePYC-F
<400> 64
   cactttttca aacatgtcca agaacaagag gtttgct 37
<210> 65
   <211> 36
   <212> DNA
   <213> Artificial Sequence
<220>
   <223> Description of Artificial Sequence: TdePYC-R
<400> 65
   atcatccttg taatcgtttt cagctgggac agcctc 36
<210> 66
   <211> 39
   <212> DNA
   <213> Artificial Sequence
<220>
   <223> Description of Artificial Sequence: ZroPYC-F
<400> 66
   cactttttca aacatgagta ccaagaagtt ctctggtct 39
<210> 67
   <211> 35
   <212> DNA
   <213> Artificial Sequence
<220>
   <223> Description of Artificial Sequence: ZroPYC-R
<400> 67
   atcatccttg taatcattct cctttggtgg gacgg 35
<210> 68
   <211> 35
   <212> DNA
   <213> Artificial Sequence
<220>
   <223> Description of Artificial Sequence: AfuMDH-F
<400> 68
   actttttcaa acatgaaact cggttttgtt ggtgc 35
<210> 69
   <211> 45
   <212> DNA
   <213> Artificial Sequence
<220>
   <223> Description of Artificial Sequence: AfuMDH-R
<400> 69
   atcatcatca tccttgtaat catatccgag ttcctcaagc ctctc 45
<210> 70
   <211> 32
   <212> DNA
   <213> Artificial Sequence
<220>
   <223> Description of Artificial Sequence: CliMDH-F
<400> 70
   actttttcaa acatgaaagc acctgttcgc gt 32
<210> 71
   <211> 40
   <212> DNA
   <213> Artificial Sequence
<220>
   <223> Description of Artificial Sequence: CliMDH-R
<400> 71
   atcatcatca tccttgtaat cgggcagaag gtctgccacg 40
<210> 72
   <211> 32
   <212> DNA
   <213> Artificial Sequence
<220>
   <223> Description of Artificial Sequence: DacMDH-F
<400> 72
   actttttcaa acatgagcaa gaagcccgtt cg 32
<210> 73
   <211> 39
   <212> DNA
   <213> Artificial Sequence
<220>
   <223> Description of Artificial Sequence: DacMDH-R
<400> 73
   atcatcatca tccttgtaat ccagcaggtg cttgacgcc 39
<210> 74
   <211> 35
   <212> DNA
   <213> Artificial Sequence
<220>
   <223> Description of Artificial Sequence: HelMDH-F
<400> 74
   actttttcaa acatgaaaga ccccgtccgt atagc 35
<210> 75
   <211> 40
   <212> DNA
   <213> Artificial Sequence
<220>
   <223> Description of Artificial Sequence: HelMDH-R
<400> 75
   atcatcatca tccttgtaat cgccaagcag atgctcgacg 40
<210> 76
   <211> 35
   <212> DNA
   <213> Artificial Sequence
<220>
   <223> Description of Artificial Sequence: SpuMDH-F
<400> 76
   actttttcaa acatgaaagt tgctgtactt ggtgc 35
<210> 77
   <211> 46
   <212> DNA
   <213> Artificial Sequence
<220>
   <223> Description of Artificial Sequence: SpuMDH-R
<400> 77
   atcatcatca tccttgtaat ctttaacgaa atcaacgcct agtttg 46
<210> 78
   <211> 20
   <212> DNA
   <213> Artificial Sequence
<220>
   <223> Description of Artificial Sequence: UF for mae2-deleted fragment
<400> 78
   aggctttgat agccactggt 20
<210> 79
   <211> 40
   <212> DNA
   <213> Artificial Sequence
<220>
   <223> Description of Artificial Sequence: UR for mae2-deleted fragment
<400> 79
   tgggatttgt agctaagctt taaaataaaa ggctttatac 40
<210> 80
   <211> 39
   <212> DNA
   <213> Artificial Sequence
<220>
   <223> Description of Artificial Sequence: OF for mae2-deleted fragment
<400> 80
   ttcgtcaata tcacaagctt aggtcgactg ggctaatcg 39
<210> 81
   <211> 40
   <212> DNA
   <213> Artificial Sequence
<220>
   <223> Description of Artificial Sequence: OR for mae2-deleted fragment
<400> 81
   taaaataaaa ggctttatac aggcattttc aaacttcaag 40
<210> 82
   <211> 40
   <212> DNA
   <213> Artificial Sequence
<220>
   <223> Description of Artificial Sequence: DF for mae2-deleted fragment
<400> 82
   cttgaagttt gaaaatgcct gtataaagcc ttttatttta 40
<210> 83
   <211> 27
   <212> DNA
   <213> Artificial Sequence
<220>
   <223> Description of Artificial Sequence: DR for mae2-deleted fragment
<400> 83
   ttcattcaat acataacggt ttacggt 27
<210> 84
   <211> 20
   <212> DNA
   <213> Artificial Sequence
<220>
   <223> Description of Artificial Sequence: FF for mae2-deleted fragment
<400> 84
   attcgtgaaa tgagcaagca 20
<210> 85
   <211> 25
   <212> DNA
   <213> Artificial Sequence
<220>
   <223> Description of Artificial Sequence: FR for mae2-deleted fragment
<400> 85
   tgcgatttac tatttgtttg tttca 25
<210> 86
   <211> 24
   <212> DNA
   <213> Artificial Sequence
<220>
   <223> Description of Artificial Sequence: UF for fum1-deleted fragment
<400> 86
   ctcaagtaat gggcaatcat gcca 24
<210> 87
   <211> 40
   <212> DNA
   <213> Artificial Sequence
<220>
   <223> Description of Artificial Sequence: UR for fum1-deleted fragment
<400> 87
   tgggatttgt agctaagctt agagtggtaa aaaattatac 40
<210> 88
   <211> 40
   <212> DNA
   <213> Artificial Sequence
<220>
   <223> Description of Artificial Sequence: OF for fum1-deleted fragment
<400> 88
   ttcgtcaata tcacaagctt aataaaatac acaaaactgt 40
<210> 89
   <211> 40
   <212> DNA
   <213> Artificial Sequence
<220>
   <223> Description of Artificial Sequence: OR for fum1-deleted fragment
<400> 89
   agagtggtaa aaaattatac agccatgtgg gttattttaa 40
<210> 90
   <211> 40
   <212> DNA
   <213> Artificial Sequence
<220>
   <223> Description of Artificial Sequence: DF for fum1-deleted fragment
<400> 90
   ttaaaataac ccacatggct gtataatttt ttaccactct 40
<210> 91
   <211> 28
   <212> DNA
   <213> Artificial Sequence
<220>
   <223> Description of Artificial Sequence: DR for fum1-deleted fragment
<400> 91
   gagcaaccga atatcaagga aatacaca 28
<210> 92
   <211> 23
   <212> DNA
   <213> Artificial Sequence
<220>
   <223> Description of Artificial Sequence: FF for fum1-deleted fragment
<400> 92
   aggcgaattg atccttcctg cta 23
<210> 93
   <211> 29
   <212> DNA
   <213> Artificial Sequence
<220>
   <223> Description of Artificial Sequence: FR for fum1-deleted fragment
<400> 93
   tggtaagcct ggtatgagtt ctatactat 29
<210> 94
   <211> 544
   <212> PRT
   <213> Candida glabrata
<220>
   <223> CglPCK
<400> 94
   Met Pro Ser Lys Ser Ser Val Ser Gly Asn Ser Val Glu Glu Arg Ile
                       5                  10                  15
   Arg Ser Glu Leu Gly Leu Ser Lys Glu Val Thr Leu Ile Arg Arg Asn
                   20                  25                  30
   Ala Pro Ala Ala Val Leu Tyr Gln Asp Ala Leu Lys Glu Lys Lys Thr
           35                  40                  45
   Val Ile Ser Ser Ala Gly Ala Leu Ile Ala Tyr Ser Gly Glu Lys Thr
       50                  55                  60
   Gly Arg Ser Pro Lys Asp Lys Arg Ile Val Glu Glu Glu Thr Ser Arg
   65                  70                  75                  80
   Asp Asn Val Trp Trp Gly Pro Val Asn Lys Pro Cys Ser Glu Arg Thr
                   85                  90                  95
   Trp Ala Ile Asn Arg Glu Arg Ala Ala Asp Tyr Leu Arg Thr Arg Glu
               100                 105                 110
   Thr Leu Tyr Val Val Asp Ala Phe Ala Gly Trp Asp Pro Lys Tyr Arg
           115                 120                 125
   Ile Lys Val Arg Val Val Cys Ala Arg Ala Tyr His Ala Leu Phe Met
       130                 135                 140
   Thr Asn Met Leu Ile Arg Pro Thr Glu Glu Glu Leu Ala Asn Phe Gly
   145                 150                 155                 160
   Glu Pro Asp Phe Thr Val Trp Asn Ala Gly Gln Phe Pro Ala Asn Ala
                   165                 170                 175
   Arg Thr Gln Asp Met Thr Ser Lys Thr Thr Ile Glu Ile Asn Phe Lys
               180                 185                 190
   Ala Met Glu Met Val Ile Leu Gly Thr Glu Tyr Ala Gly Glu Met Lys
           195                 200                 205
   Lys Gly Ile Phe Thr Val Met Phe Tyr Leu Met Pro Val His His Asn
       210                 215                 220
   Val Leu Thr Leu His Ser Ser Cys Asn Gln Gly Ile Lys Asn Gly Asp
   225                 230                 235                 240
   Val Thr Leu Phe Phe Gly Leu Ser Gly Thr Gly Lys Thr Thr Leu Ser
                   245                 250                 255
   Ala Asp Pro His Arg Leu Leu Ile Gly Asp Asp Glu His Cys Trp Ser
   260 265 270
   Asp Glu Gly Val Phe Asn Ile Glu Gly Gly Cys Tyr Ala Lys Cys Ile
   275 280 285
   Asn Leu Ser Arg Glu Lys Glu Pro Glu Ile Phe Asp Ala Ile Arg Phe
   290 295 300
   Gly Ser Val Leu Glu Asn Val Ile Tyr Asp Ser Glu Ser His Glu Val
   305 310 315 320
   Asp Tyr Asp Asp Ser Ser Ile Thr Glu Asn Thr Arg Cys Ala Tyr Pro
   325 330 335
   Ile Asp Phe Ile Pro Ser Ala Lys Ile Pro Cys Leu Ala Pro Ala His
   340 345 350
   Pro Lys Asn Ile Ile Leu Leu Thr Cys Asp Ala Ser Gly Val Leu Pro
   355 360 365
   Pro Val Ser Lys Leu Thr Pro Glu Gln Val Met Tyr His Phe Ile Ser
   370 375 380
   Gly Tyr Thr Ser Lys Met Ala Gly Thr Glu Gln Gly Val Thr Glu Pro
   385 390 395 400
   Glu Pro Thr Phe Ser Ser Cys Phe Gly Gln Pro Phe Leu Ser Leu His
   405 410 415
   Pro Met Arg Tyr Ala Thr Met Leu Ala Glu Lys Met His Glu His Ser
   420 425 430
   Ala Asn Ala Trp Leu Ile Asn Thr Gly Trp Thr Gly Ser Ser Tyr Val
   435 440 445
   Ser Gly Gly Lys Arg Cys Ala Leu Lys Tyr Thr Arg Ala Ile Leu Asp
   450 455 460
   Ala Ile His Asp Gly Ser Leu Ala Lys Ala Glu Phe Glu Ser Leu Pro
   465 470 475 480
   Ile Phe Asn Leu Gln Val Pro Lys Ala Val Glu Gly Val Pro Ser Glu
   485 490 495
   Leu Leu Asn Pro Ala Arg Asn Trp Ser Glu Gly Glu Ala Lys Tyr Gln
   500 505 510
   Ser Ala Val Ser Lys Leu Ala Gly Leu Phe Val Glu Asn Phe Lys Thr
   515 520 525
   Tyr Gln Asp Lys Ala Thr Ser Asp Val Leu Ala Ala Gly Pro Gln Leu
   530 535 540
<210> 95
   <211> 539
   <212> PRT
   <213> Citrobacter koseri
<220>
   <223> CkoPCK
<400> 95
   Met Arg Val Asn Ser Leu Thr Pro Gln Asp Leu Lys Ala Tyr Gly Ile
                   5                   10                  15
   Asn Asp Val Gln Asp Ile Val Tyr Asn Pro Ser Tyr Asp Leu Leu Tyr
               20                  25                  30
   Gln Glu Glu Leu Asp Pro Thr Leu Glu Gly Tyr Glu Arg Gly Val Leu
           35                  40                  45
   Thr Asn Leu Gly Ala Val Ala Val Asp Thr Gly Ile Phe Thr Gly Arg
       50                  55                  60
   Ser Pro Lys Asp Lys Tyr Ile Val Arg Asp Asp Thr Thr Arg Asp Thr
   65                  70                  75                  80
   Leu Trp Trp Ser Asp Lys Gly Lys Gly Lys Asn Asp Asn Lys Pro Leu
                   85                  90                  95
   Ser Gln Glu Thr Trp Gln His Leu Lys Gly Leu Val Thr Gln Gln Leu
               100                 105                 110
   Ser Gly Lys Arg Leu Phe Ile Val Asp Ala Phe Cys Gly Ala Asn Ala
           115                 120                 125
   Asp Thr Arg Leu Ser Val Arg Phe Ile Thr Glu Val Ala Trp Gln Ala
       130                 135                 140
   His Phe Val Lys Asn Met Phe Ile Arg Pro Ala Asp Glu Glu Leu Val
   145                 150                 155                 160
   Asp Phe Lys Pro Asp Phe Ile Val Met Asn Gly Ala Lys Cys Thr Asn
                   165                 170                 175
   Pro Gln Trp Lys Glu Gln Gly Leu Asn Ser Glu Asn Phe Val Ala Phe
               180                 185                 190
   Asn Leu Thr Glu Arg Ile Gln Leu Ile Gly Gly Thr Trp Tyr Gly Gly
           195                 200                 205
   Glu Met Lys Lys Gly Met Phe Ser Val Met Asn Tyr Leu Leu Pro Leu
       210                 215                 220
   Lys Gly Ile Ala Ser Met His Cys Ser Ala Asn Val Gly Glu Lys Gly
   225                 230                 235                 240
   Asp Val Ala Val Phe Phe Gly Leu Ser Gly Thr Gly Lys Thr Thr Leu
                   245                 250                 255
   Ser Thr Asp Pro Lys Arg Arg Leu Ile Gly Asp Asp Glu His Gly Trp
               260                 265                 270
   Asp Asp Asp Gly Val Phe Asn Phe Glu Gly Gly Cys Tyr Ala Lys Thr
           275                 280                 285
   Ile Lys Leu Ser Lys Glu Ala Glu Pro Glu Ile Tyr Asn Ala Ile Arg
       290                 295                 300
   Arg Asp Ala Leu Leu Glu Asn Val Thr Val Arg Glu Asp Gly Ser Ile
   305                 310                 315                 320
   Asp Phe Asp Asp Gly Ser Lys Thr Glu Asn Thr Arg Val Ser Tyr Pro
                   325                 330                 335
   Ile Tyr His Ile Asp Asn Ile Val Lys Pro Val Ser Lys Ala Gly His
               340                 345                 350
   Ala Thr Lys Val Ile Phe Leu Thr Ala Asp Ala Phe Gly Val Leu Pro
           355                 360                 365
   Pro Val Ser Arg Leu Thr Ala Asp Gln Thr Gln Tyr His Phe Leu Ser
       370                 375                 380
   Gly Phe Thr Ala Lys Leu Ala Gly Thr Glu Arg Gly Val Thr Glu Pro
   385                 390                 395                 400
   Thr Pro Thr Phe Ser Ala Cys Phe Gly Ala Ala Phe Leu Ser Leu His
                   405                 410                 415
   Pro Thr Gln Tyr Ala Glu Val Leu Val Lys Arg Met Gln Ala Ser Gly
               420                 425                 430
   Ala Gln Ala Tyr Leu Val Asn Thr Gly Trp Asn Gly Thr Gly Lys Arg
           435                 440                 445
   Ile Ser Ile Lys Asp Thr Arg Ala Ile Ile Asp Ala Ile Leu Asn Gly
       450                 455                 460
   Ser Leu Asp Asn Ala Glu Thr Phe Asn Leu Pro Met Phe Asp Leu Ala
   465                 470                 475                 480
   Ile Pro Thr Glu Leu Pro Gly Val Glu Thr Arg Ile Leu Asp Pro Arg
                   485                 490                 495
   Asn Thr Tyr Ala Ser Pro Glu Gln Trp Gln Glu Lys Ala Thr Ala Leu
               500                 505                 510
   Ala Lys Leu Phe Val Glu Asn Phe Glu Lys Tyr Thr Asp Thr Pro Ala
           515                 520                 525
   Gly Glu Ala Leu Val Ser Ala Gly Pro Lys Leu
       530                 535
<210> 96
   <211> 539
   <212> PRT
   <213> Cronobacter sakazakii
<220>
   <223> CsaPCK
<400> 96
<210> 97
   <211> 553
   <212> PRT
   <213> Debaryomyces hansenii
<220>
   <223> DhaPCK
<400> 97
   Met Thr Pro Pro Thr Ala Val Glu Ser Ser Ile Asn Phe Gly Gly His
                   5                   10                  15
   Pro Thr Ile Lys Ser Thr Ala Glu Pro Val Val Lys Gln Leu Ser Leu
               20                  25                  30
   Ala Asp Asp Thr Val Ile Arg His Asn Ala Pro Pro Pro Thr Leu Tyr
           35                  40                  45
   Glu Asp Gly Leu Leu Glu Lys Gly Thr Val Ile Ser Ser Thr Gly Ala
       50                  55                  60
   Leu Met Ala Tyr Ser Gly Lys Lys Thr Gly Arg Ser Pro Lys Asp Lys
   65                  70                  75                  80
   Arg Ile Val Asp Glu Glu Thr Ser Thr Gln Asn Ile Trp Trp Gly Pro
                   85                  90                  95
   Val Asn Lys Gln Val Asp Glu Leu Thr Trp Lys Ile Ser Arg Ser Arg
               100                 105                 110
   Ala Leu Asp Tyr Leu Arg Thr Arg Glu Lys Leu Phe Val Val Asp Ala
           115                 120                 125
   Tyr Ala Gly Trp Asp Pro Arg Tyr Arg Leu Lys Ile Arg Val Ile Cys
       130                 135                 140
   Ala Arg Ala Tyr His Ala Leu Phe Met Thr Asn Met Leu Ile Arg Pro
   145                 150                 155                 160
   Thr Glu Glu Glu Leu Lys Asn Phe Gly Glu Pro Asp Phe Thr Ile Tyr
                   165                 170                 175
   Asn Ala Gly Gln Phe Pro Ala Asn Val His Thr Lys Gly Met Thr Ser
               180                 185                 190
   Ser Thr Ser Val Glu Ile Asn Phe Lys Asp Met Glu Met Val Ile Leu
           195                 200                 205
   Gly Thr Glu Tyr Ala Gly Glu Met Lys Lys Gly Ile Phe Thr Val Met
       210                 215                 220
   Phe Tyr Leu Met Pro Ile Lys His Lys Ile Leu Thr Leu His Ser Ser
   225                 230                 235                 240
   Ala Asn Gln Gly Glu Gln Lys Gly Asp Val Thr Leu Phe Phe Gly Leu
                   245                 250                 255
   Ser Gly Thr Gly Lys Thr Thr Leu Ser Ala Asp Pro Asn Arg Lys Leu
               260                 265                 270
   Ile Gly Asp Asp Glu His Cys Trp Ser Asp Asn Gly Val Phe Asn Ile
           275                 280                 285
   Glu Gly Gly Cys Tyr Ala Lys Cys Leu Asp Leu Ser Ala Glu Lys Glu
       290                 295                 300
   Pro Glu Ile Phe Asn Ser Ile Lys Phe Gly Ala Ile Leu Glu Asn Val
   305                 310                 315                 320
   Val Tyr Asp Gln Asn Ser Lys Val Val Asp Tyr Ala Asp Ser Thr Ile
                   325                 330                 335
   Thr Glu Asn Thr Arg Cys Ala Tyr Pro Ile Asp Phe Ile Pro Ser Ala
               340                 345                 350
   Lys Ile Pro Cys Leu Ala Asp Glu His Pro Thr Asn Ile Val Leu Leu
           355                 360                 365
   Thr Cys Asp Ala Ser Gly Val Leu Pro Pro Val Ser Lys Leu Thr Asn
       370                 375                 380
   Ala Gln Val Met Tyr His Phe Ile Ser Gly Tyr Thr Ser Lys Met Ala
   385 390 395 400
   Gly Thr Glu Glu Gly Val Thr Glu Pro Gln Ala Thr Phe Ser Ala Cys
   405 410 415
   Phe Gly Gln Pro Phe Leu Val Leu His Pro Met Lys Tyr Ala Gln Gln
   420 425 430
   Leu Ser Asp Lys Ile Ser Lys His Asn Ala Asn Ala Trp Leu Leu Asn
   435 440 445
   Thr Gly Trp Val Gly Ala Ser Ala Ala Gln Gly Gly Asn Arg Cys Ser
   450 455 460
   Leu Lys Tyr Thr Arg Ala Ile Leu Asp Ala Ile His Ser Gly Glu Leu
   465 470 475 480
   Ser Lys Val Glu Phe Glu Asn Phe Pro Thr Phe Asn Leu Asn Val Pro
   485 490 495
   Lys Ser Cys Pro Asn Val Pro Ser Glu Ile Leu Asn Pro Thr Lys Ala
   500 505 510
   Trp Thr Glu Gly Asp Ala Ser Phe Gln Lys Glu Ile Lys Ser Leu Ala
   515 520 525
   Gly Lys Phe Ala Glu Asn Phe Thr Lys Tyr Ala Asp Gln Ala Thr Thr
   530 535 540
   Glu Val Lys Ala Ala Gly Pro Asp Ala
   545 550
<210> 98
   <211> 540
   <212> PRT
   <213> Escherichia fergusonii
<220>
   <223> EfePCK
<400> 98
   Met Arg Val Asn Lys Ser Leu Thr Pro Gln Asp Leu Met Ala Tyr Gly
                   5                   10                  15
   Ile Asn Asp Val Gln Asp Ile Val Tyr Asn Pro Ser Tyr Asp Leu Leu
               20                  25                  30
   Phe Gln Glu Glu Leu Asp Pro Asn Leu Lys Gly Tyr Glu Arg Gly Val
           35                  40                  45
   Leu Thr Asn Leu Gly Ala Val Ala Val Asp Thr Gly Val Phe Thr Gly
       50                  55                  60
   Arg Ser Pro Lys Asp Lys Tyr Ile Val Arg Asp Asp Thr Thr Arg Asp
   65                  70                  75                  80
   Thr Phe Trp Trp Ala Asp Lys Gly Lys Gly Lys Asn Asp Asn Lys Pro
                   85                  90                  95
   Leu Ser Pro Glu Thr Trp Gln His Leu Lys Gly Leu Val Thr Asn Gln
               100                 105                 110
   Leu Ser Gly Lys Arg Leu Phe Val Val Asp Ala Phe Cys Gly Ala Asn
           115                 120                 125
   Ala Asp Thr Arg Leu Ser Val Arg Phe Ile Thr Glu Val Ala Trp Gln
       130                 135                 140
   Ala His Phe Val Lys Asn Met Phe Ile Arg Pro Thr Asp Glu Glu Leu
   145                 150                 155                 160
   Val Asp Phe Glu Pro Asp Phe Ile Val Met Asn Gly Ala Lys Cys Thr
                   165                 170                 175
   Asn Pro Gln Trp Lys Glu Gln Gly Leu Asn Ser Glu Asn Phe Val Ala
               180                 185                 190
   Phe Asn Leu Thr Glu Arg Met Gln Leu Ile Gly Gly Thr Trp Tyr Gly
           195                 200                 205
   Gly Glu Met Lys Lys Gly Met Phe Ser Met Met Asn Tyr Leu Leu Pro
       210                 215                 220
   Leu Lys Gly Ile Ala Ser Met His Cys Ser Ala Asn Val Gly Glu Lys
   225                 230                 235                 240
   Gly Asp Val Ala Val Phe Phe Gly Leu Ser Gly Thr Gly Lys Thr Thr
                   245                 250                 255
   Leu Ser Thr Asp Pro Lys Arg Arg Leu Ile Gly Asp Asp Glu His Gly
               260                 265                 270
   Trp Asp Asp Asp Gly Val Phe Asn Phe Glu Gly Gly Cys Tyr Ala Lys
           275                 280                 285
   Thr Ile Lys Leu Ser Lys Glu Ala Glu Pro Glu Ile Tyr Asn Ala Ile
       290                 295                 300
   Arg Arg Asp Ala Leu Leu Glu Asn Val Thr Val Arg Glu Asp Gly Thr
   305                 310                 315                 320
   Ile Asp Phe Asp Asp Gly Ser Lys Thr Glu Asn Thr Arg Val Ser Tyr
                   325                 330                 335
   Pro Ile Tyr His Ile Asp Asn Ile Val Lys Pro Val Ser Lys Ala Gly
               340                 345                 350
   His Ala Thr Lys Val Ile Phe Leu Thr Ala Asp Ala Phe Gly Val Leu
           355                 360                 365
   Pro Pro Val Ser Arg Leu Thr Ala Asp Gln Thr Gln Tyr His Phe Leu
       370                 375                 380
   Ser Gly Phe Thr Ala Lys Leu Ala Gly Thr Glu Arg Gly Ile Thr Glu
   385                 390                 395                 400
   Pro Thr Pro Thr Phe Ser Ala Cys Phe Gly Ala Ala Phe Leu Ser Leu
                   405                 410                 415
   His Pro Thr Gln Tyr Ala Glu Val Leu Val Lys Arg Met Gln Ala Ala
               420                 425                 430
   Gly Ala Gln Ala Tyr Leu Val Asn Thr Gly Trp Asn Gly Thr Gly Lys
           435                 440                 445
   Arg Ile Ser Ile Lys Asp Thr Arg Ala Ile Ile Asp Ala Ile Leu Asn
       450                 455                 460
   Gly Ser Leu Asp Asn Ala Glu Thr Phe Thr Leu Pro Met Phe Asn Leu
   465                 470                 475                 480
   Ala Ile Pro Thr Glu Leu Pro Gly Val Asp Thr Glu Ile Leu Asp Pro
                   485                 490                 495
   Arg Asn Thr Tyr Ala Ser Pro Glu Gln Trp Gln Glu Lys Ala Glu Thr
               500                 505                 510
   Leu Ala Lys Leu Phe Ile Asp Asn Phe Asp Lys Tyr Thr Asp Thr Pro
           515                 520                 525
   Ala Gly Ala Ala Leu Val Glu Ala Gly Pro Lys Met
       530                 535                 540
<210> 99
   <211> 539
   <212> PRT
   <213> Edwardsiella tarda
<220>
   <223> EtaPCK
<400> 99
   Met His Ala Thr Gly Leu Thr Ala Ala Asp Leu Ala Ser Tyr Gly Ile
                   5                   10                  15
   Ser Asp Val Thr Glu Ile Val Tyr Asn Pro Asp Tyr Asp Leu Leu Phe
               20                  25                  30
   Lys Glu Glu Thr Ala Pro Gly Leu Gln Gly Tyr Glu Arg Gly Thr Val
           35                  40                  45
   Thr Thr Leu Gly Ala Val Ala Val Asp Thr Gly Ile Phe Thr Gly Arg
       50                  55                  60
   Ser Pro Lys Asp Lys Tyr Leu Val Arg Asp Asp Thr Thr Arg Asp Thr
   65                  70                  75                  80
   Val Trp Trp Ser Asp Gln Gly Lys Gly Lys Asn Asp Asn His Pro Leu
                   85                  90                  95
   Ser Pro Glu Ile Trp Gln His Leu Lys Gly Leu Val Thr Arg Gln Leu
               100                 105                 110
   Ser Asn Lys Arg Leu Phe Val Val Asp Ala Tyr Cys Gly Ala Asn Pro
           115                 120                 125
   Asp Ser Arg Leu Lys Val Arg Phe Ile Thr Glu Val Ala Trp Gln Ala
       130                 135                 140
   His Phe Val Lys Asn Met Phe Ile Arg Pro Thr Glu Asp Glu Leu Ala
   145                 150                 155                 160
   Gly Phe Glu Pro Asp Phe Ile Val Met Asn Gly Ala Lys Cys Thr Asn
                   165                 170                 175
   Pro Gln Trp Gln Glu Gln Gly Leu Asn Ser Glu Asn Phe Val Ala Phe
               180                 185                 190
   Asn Leu Thr Glu Arg Ile Gln Leu Ile Gly Gly Thr Trp Tyr Gly Gly
           195                 200                 205
   Glu Met Lys Lys Gly Met Phe Ser Ile Met Asn Tyr Leu Leu Pro Leu
       210                 215                 220
   Lys Gly Ile Ala Ser Met His Cys Ser Ala Asn Val Gly Glu Lys Gly
   225                 230                 235                 240
   Asp Val Ala Val Phe Phe Gly Leu Ser Gly Thr Gly Lys Thr Thr Leu
                   245                 250                 255
   Ser Thr Asp Pro Lys Arg Arg Leu Ile Gly Asp Asp Glu His Gly Trp
               260                 265                 270
   Asp Asp Asp Gly Val Phe Asn Phe Glu Gly Gly Cys Tyr Ala Lys Thr
           275                 280                 285
   Ile Lys Leu Ser Ala Glu Ala Glu Pro Asp Ile Tyr His Ala Ile Thr
       290                 295                 300
   Arg Asp Ala Leu Leu Glu Asn Val Thr Val Arg Ala Asp Gly Ser Val
   305                 310                 315                 320
   Asp Phe Asp Asp Gly Ser Lys Thr Glu Asn Thr Arg Val Ser Tyr Pro
                   325                 330                 335
   Ile Tyr His Ile Lys Asn Ile Val Lys Pro Val Ser Lys Ala Gly His
               340                 345                 350
   Ala Lys Lys Val Ile Phe Leu Thr Ala Asp Ala Phe Gly Val Leu Pro
           355                 360                 365
   Pro Val Ser Arg Leu Thr Ala Asp Gln Thr Gln Tyr His Phe Leu Ser
       370                 375                 380
   Gly Phe Thr Ala Lys Leu Ala Gly Thr Glu Arg Gly Val Thr Glu Pro
   385                 390                 395                 400
   Thr Pro Thr Phe Ser Ala Cys Phe Gly Ala Ala Phe Leu Thr Leu His
                   405                 410                 415
   Pro Thr Gln Tyr Ala Glu Val Leu Val Lys Arg Met Gln Ala Ala Gly
               420                 425                 430
   Ala Gln Ala Tyr Leu Val Asn Thr Gly Trp Asn Gly Ser Gly Lys Arg
           435                 440                 445
   Ile Ser Ile Lys Asp Thr Arg Ala Ile Ile Asp Ala Ile Leu Asn Gly
       450                 455                 460
   Asp Ile Asp Lys Glu Glu Thr Phe Thr Leu Pro Ile Phe Asn Leu Ala
   465                 470                 475                 480
   Val Pro Thr Ala Leu Pro Gly Val Asn Pro Ala Ile Leu Asp Pro Arg
                   485                 490                 495
   Asp Thr Tyr Ala Ser Pro Glu Met Trp Gln Glu Lys Ala Asp Asp Leu
               500                 505                 510
   Ala Gln Arg Phe Ile Thr Asn Phe Asp Lys Tyr Thr Asp Thr Pro Ala
           515                 520                 525
   Gly Ala Ala Leu Val His Ala Gly Pro Gln Arg
       530                 535
<210> 100
   <211> 543
   <212> PRT
   <213> Kluyveromyces lactis
<220>
   <223> KlaPCK
<400> 100
   Met Ser Pro Thr Lys Thr Gln Asn Ser Ala Glu Glu Asn Ile Arg Ala
                   5                   10                  15
   Glu Leu Gly Leu Ser Ala Glu Val Val Thr Ile Arg Arg Asn Ala Pro
               20                  25                  30
   Ala Ala Leu Leu Tyr Glu Asp Ala Leu Lys Glu Arg Asp Thr Ala Ile
           35                  40                  45
   Ser Asn Ala Gly Ala Leu Ile Ala Tyr Ser Gly Asp Lys Thr Gly Arg
       50                  55                  60
   Ser Pro Arg Asp Lys Arg Ile Val Glu Glu Glu Thr Ser Lys Asp Asn
   65                  70                  75                  80
   Val Trp Trp Gly Pro Val Asn Lys Pro Cys Ser Glu Arg Thr Trp Glu
                   85                  90                  95
   Ile Asn Arg Glu Arg Ala Ala Asp Tyr Leu Arg Thr Arg Asp His Ile
               100                 105                 110
   Tyr Ile Val Asp Ala Tyr Ala Gly Trp Asp Pro Arg Tyr Arg Ile Lys
           115                 120                 125
   Val Arg Val Val Cys Ala Arg Ala Tyr His Ala Leu Phe Met Thr Asn
       130                 135                 140
   Met Leu Ile Arg Pro Ser Lys Glu Glu Leu Glu Asn Phe Gly Glu Pro
   145                 150                 155                 160
   Asp Phe Thr Val Trp Asn Ala Gly Gln Phe Pro Ala Asn Thr His Thr
                   165                 170                 175
   Ser Gly Met Thr Ser Lys Thr Thr Val Glu Ile Asn Phe Lys Gln Met
               180                 185                 190
   Glu Met Val Ile Leu Gly Thr Glu Tyr Ala Gly Glu Met Lys Lys Gly
           195                 200                 205
   Ile Phe Thr Val Met Phe Tyr Leu Met Pro Val Asn His Asn Val Leu
       210                 215                 220
   Thr Leu His Ser Ser Ala Asn Gln Gly Ile Gln Asp Asn Asp Val Thr
   225                 230                 235                 240
   Leu Phe Phe Gly Leu Ser Gly Thr Gly Lys Thr Thr Leu Ser Ala Asp
                   245                 250                 255
   Pro His Arg Leu Leu Ile Gly Asp Asp Glu His Cys Trp Ser Asp His
               260                 265                 270
   Gly Val Phe Asn Ile Glu Gly Gly Cys Tyr Ala Lys Cys Leu Gly Leu
           275                 280                 285
   Ser Ala Glu Lys Glu Pro Glu Ile Phe Asn Ala Ile Lys Phe Gly Ser
       290                 295                 300
   Val Leu Glu Asn Ile Ile Tyr Asp Pro Asn Thr Arg Glu Val Asp Tyr
   305                 310                 315                 320
   Glu Asp Ser Thr Ile Thr Glu Asn Thr Arg Cys Ala Tyr Pro Ile Glu
                   325                 330                 335
   Tyr Ile Pro Ser Ala Lys Ile Pro Cys Leu Ala Asp His His Pro Lys
               340                 345                 350
   Asn Ile Val Leu Leu Thr Cys Asp Ala Ser Gly Val Leu Pro Pro Val
           355                 360                 365
   Ser Lys Leu Thr Pro Asp Gln Val Met Tyr His Phe Ile Ser Gly Tyr
       370                 375                 380
   Thr Ser Lys Met Ala Gly Thr Glu Gln Gly Val Thr Glu Pro Glu Ala
   385                 390                 395                 400
   Thr Phe Ser Ser Cys Phe Gly Gln Pro Phe Leu Ser Leu His Pro Met
                   405                 410                 415
   Lys Tyr Ala Thr Met Leu Ala Glu Lys Met Ala Glu His Asn Ala Asn
               420                 425                 430
   Ala Trp Leu Ile Asn Thr Gly Trp Thr Gly Ser Ser Tyr Val Ala Gly
           435                 440                 445
   Gly Lys Arg Cys Pro Leu Lys Tyr Thr Arg Ala Ile Leu Asp Ala Ile
       450                 455                 460
   His Asp Gly Ser Leu Ala Lys Glu Glu Tyr Glu Val Leu Pro Ile Phe
   465                 470                 475                 480
   Asn Leu Gln Ile Pro Lys Ala Val Gly Asp Lys Val Pro Ala Ser Leu
                   485                 490                 495
   Leu Asn Pro Ser Lys Asn Trp Ala Glu Gly Glu Ala Lys Tyr Thr Ser
               500                 505                 510
   Asn Val Lys Ser Leu Ala Asn Leu Phe Val Glu Asn Phe Lys Thr Tyr
           515                 520                 525
   Gln Asp Lys Ala Thr Glu Gln Val Leu Ala Ala Gly Pro Gln Leu
       530                 535                 540
<210> 101
   <211> 542
   <212> PRT
   <213> Lodderomyces elongisporus
<220>
   <223> LelPCK
<400> 101
   Met Ala Pro Pro Ala Val Glu Ser Thr Ile Asn Phe Glu Gly His Pro
   5 10 15
   Ser Ile Lys Thr Thr Val Asp Pro Leu Val Lys Lys Leu Asn Leu Lys
   20 25 30
   Gly Asp Thr Val Ile Arg His Asn Ala Pro Pro Pro Thr Leu Tyr Glu
   35 40 45
   Asp Gly Leu Leu Glu Lys Gly Thr Thr Ile Ser Ser Thr Gly Ala Leu
   50 55 60
   Met Ala Tyr Ser Gly Glu Lys Thr Gly Arg Ser Pro Lys Asp Lys Arg
   65 70 75 80
   Ile Val Asp Glu Glu Thr Ser Ser Gln His Ile Trp Trp Gly Pro Val
   85 90 95
   Asn Lys Gln Val Asp Glu Leu Thr Trp Lys Ile Ser Arg Ser Arg Ala
   100 105 110
   Leu Asp Tyr Leu Arg Thr Arg Glu Lys Leu Phe Val Val Asp Ala Phe
   115 120 125
   Ala Gly Trp Asp Pro Lys Tyr Arg Ile Lys Val Arg Ile Ile Cys Ala
   130 135 140
   Arg Ala Tyr His Ala Leu Phe Met Thr Asn Met Leu Ile Arg Pro Thr
   145 150 155 160
   Lys Glu Glu Leu Glu Asn Phe Gly Glu Pro Asp Phe Thr Ile Tyr Asn
   165 170 175
   Ala Gly Gln Phe Pro Ala Asn Val His Thr Lys Gly Met Thr Ser Ser
   180 185 190
   Thr Ser Val Glu Ile Asn Phe Lys Asp Met Glu Met Val Ile Leu Gly
   195 200 205
   Thr Glu Tyr Ala Gly Glu Met Lys Lys Gly Ile Phe Thr Val Met Phe
   210 215 220
   Tyr Leu Met Pro Ile Lys Asn Lys Val Leu Thr Leu His Ser Ser Ala
   225 230 235 240
   Asn Glu Gly Glu Ser Gly Asp Val Thr Leu Phe Phe Gly Leu Ser Gly
   245 250 255
   Thr Gly Lys Thr Thr Leu Ser Ala Asp Pro Asn Arg Lys Leu Ile Gly
   260 265 270
   Asp Asp Glu His Cys Trp Ser Asp His Gly Val Phe Asn Ile Glu Gly
   275 280 285
   Gly Cys Tyr Ala Lys Cys Leu Asp Leu Ser Ala Glu Lys Glu Pro Glu
   290 295 300
   Ile Phe Asn Ser Ile Arg Phe Gly Ser Val Leu Glu Asn Val Val Tyr
   305 310 315 320
   Asp Pro Ala Thr Lys Val Val Asp Tyr Glu Asp Ser Ser Ile Thr Glu
   325 330 335
   Asn Thr Arg Cys Ser Tyr Pro Ile Glu Tyr Ile Pro Ser Leu Lys Ile
   340 345 350
   Pro Cys Val Gly Gly His Pro Lys Asn Ile Val Leu Leu Thr Cys Asp
   355 360 365
   Ala Ser Gly Val Leu Pro Pro Val Ser Lys Leu Thr Pro Ala Gln Val
       370                 375                 380
   Met Tyr His Phe Ile Ser Gly Tyr Thr Ser Lys Met Ala Gly Thr Glu
   385                 390                 395                 400
   Met Gly Ile Thr Glu Pro Thr Pro Ala Phe Ser Ala Cys Phe Gly Gln
                   405                 410                 415
   Pro Phe Leu Val Leu His Pro Met Lys Tyr Ala Gln Gln Leu Ala Glu
               420                 425                 430
   Lys Ile Glu Gln His Lys Ala Asn Ala Tyr Leu Leu Asn Thr Gly Trp
           435                 440                 445
   Ser Gly Val Gly Ala Ala Lys Gly Gly Lys Arg Cys Pro Leu Lys Tyr
       450                 455                 460
   Thr Arg Ala Ile Leu Asp Ala Ile His Ser Gly Glu Leu Asp Ser Ala
   465                 470                 475                 480
   Asp Thr Gln Val Phe Ser Thr Phe Asn Leu His Val Pro Lys Glu Ile
                   485                 490                 495
   Lys Gly Val Pro Leu Glu Val Leu His Pro Glu Val Asp Lys Lys Glu
               500                 505                 510
   Val Ala Ala Leu Ala Ala Lys Phe Ala Glu Asn Phe Val Lys Tyr Ala
           515                 520                 525
   Asp Gln Ala Thr Ala Glu Val Lys Ala Ala Gly Pro Asp Val
       530                 535                 540
<210> 102
   <211> 542
   <212> PRT
   <213> Pectobacterium carotovorum
<220>
   <223> PcaPCK
<400> 102
   Met Ala Pro Pro Ala Val Glu Ser Thr Ile Asn Phe Glu Gly His Pro
                   5                   10                  15
   Ser Ile Lys Thr Thr Val Asp Pro Leu Val Lys Lys Leu Asn Leu Lys
               20                  25                  30
   Gly Asp Thr Val Ile Arg His Asn Ala Pro Pro Pro Thr Leu Tyr Glu
           35                  40                  45
   Asp Gly Leu Leu Glu Lys Gly Thr Thr Ile Ser Ser Thr Gly Ala Leu
       50                  55                  60
   Met Ala Tyr Ser Gly Glu Lys Thr Gly Arg Ser Pro Lys Asp Lys Arg
   65                  70                  75                  80
   Ile Val Asp Glu Glu Thr Ser Ser Gln His Ile Trp Trp Gly Pro Val
                   85                  90                  95
   Asn Lys Gln Val Asp Glu Leu Thr Trp Lys Ile Ser Arg Ser Arg Ala
               100                 105                 110
   Leu Asp Tyr Leu Arg Thr Arg Glu Lys Leu Phe Val Val Asp Ala Phe
           115                 120                 125
   Ala Gly Trp Asp Pro Lys Tyr Arg Ile Lys Val Arg Ile Ile Cys Ala
       130                 135                 140
   Arg Ala Tyr His Ala Leu Phe Met Thr Asn Met Leu Ile Arg Pro Thr
   145                 150                 155                 160
   Lys Glu Glu Leu Glu Asn Phe Gly Glu Pro Asp Phe Thr Ile Tyr Asn
                   165                 170                 175
   Ala Gly Gln Phe Pro Ala Asn Val His Thr Lys Gly Met Thr Ser Ser
               180                 185                 190
   Thr Ser Val Glu Ile Asn Phe Lys Asp Met Glu Met Val Ile Leu Gly
           195                 200                 205
   Thr Glu Tyr Ala Gly Glu Met Lys Lys Gly Ile Phe Thr Val Met Phe
       210                 215                 220
   Tyr Leu Met Pro Ile Lys Asn Lys Val Leu Thr Leu His Ser Ser Ala
   225                 230                 235                 240
   Asn Glu Gly Glu Ser Gly Asp Val Thr Leu Phe Phe Gly Leu Ser Gly
                   245                 250                 255
   Thr Gly Lys Thr Thr Leu Ser Ala Asp Pro Asn Arg Lys Leu Ile Gly
               260                 265                 270
   Asp Asp Glu His Cys Trp Ser Asp His Gly Val Phe Asn Ile Glu Gly
           275                 280                 285
   Gly Cys Tyr Ala Lys Cys Leu Asp Leu Ser Ala Glu Lys Glu Pro Glu
       290                 295                 300
   Ile Phe Asn Ser Ile Arg Phe Gly Ser Val Leu Glu Asn Val Val Tyr
   305                 310                 315                 320
   Asp Pro Ala Thr Lys Val Val Asp Tyr Glu Asp Ser Ser Ile Thr Glu
                   325                 330                 335
   Asn Thr Arg Cys Ser Tyr Pro Ile Glu Tyr Ile Pro Ser Leu Lys Ile
               340                 345                 350
   Pro Cys Val Gly Gly His Pro Lys Asn Ile Val Leu Leu Thr Cys Asp
           355                 360                 365
   Ala Ser Gly Val Leu Pro Pro Val Ser Lys Leu Thr Pro Ala Gln Val
       370                 375                 380
   Met Tyr His Phe Ile Ser Gly Tyr Thr Ser Lys Met Ala Gly Thr Glu
   385                 390                 395                 400
   Met Gly Ile Thr Glu Pro Thr Pro Ala Phe Ser Ala Cys Phe Gly Gln
                   405                 410                 415
   Pro Phe Leu Val Leu His Pro Met Lys Tyr Ala Gln Gln Leu Ala Glu
               420                 425                 430
   Lys Ile Glu Gln His Lys Ala Asn Ala Tyr Leu Leu Asn Thr Gly Trp
           435                 440                 445
   Ser Gly Val Gly Ala Ala Lys Gly Gly Lys Arg Cys Pro Leu Lys Tyr
       450                 455                 460
   Thr Arg Ala Ile Leu Asp Ala Ile His Ser Gly Glu Leu Asp Ser Ala
   465                 470                 475                 480
   Asp Thr Gln Val Phe Ser Thr Phe Asn Leu His Val Pro Lys Glu Ile
                   485                 490                 495
   Lys Gly Val Pro Leu Glu Val Leu His Pro Glu Val Asp Lys Lys Glu
               500                 505                 510
   Val Ala Ala Leu Ala Ala Lys Phe Ala Glu Asn Phe Val Lys Tyr Ala
           515                 520                 525
   Asp Gln Ala Thr Ala Glu Val Lys Ala Ala Gly Pro Asp Val
       530                 535                 540
<210> 103
   <211> 543
   <212> PRT
   <213> Photobacterium leiognathi
<220>
   <223> PlePCK
<400> 103
   Met Ser Thr Met Cys Val Asp Asn Lys Val Ala Thr Asn Met Asp Leu
                   5                   10                  15
   Ser Gln Tyr Gly Ile Leu Asn Val Thr Asp Val Ile Arg Asn Pro Ser
               20                  25                  30
   Tyr Glu Met Leu Phe Glu Glu Glu Thr Lys Ala Glu Leu Lys Gly Phe
           35                  40                  45
   Glu Lys Gly Ile Val Thr Glu Leu Gly Ala Val Ala Val Asp Thr Gly
       50                  55                  60
   Ile Phe Thr Gly Arg Ser Pro Gln Asp Lys Phe Ile Val Lys Asp Asp
   65                  70                  75                  80
   Thr Thr Lys Asp Thr Leu Trp Trp Ser Asp Gln Gly Lys Asn Asp Asn
                   85                  90                  95
   Lys Ala Leu Ser Gln Gly Ala Trp Asn Asp Leu Lys Ser Leu Val Thr
               100                 105                 110
   Thr Gln Leu Ser Asn Lys Arg Leu Phe Val Val Asp Gly Tyr Cys Gly
           115                 120                 125
   Ala Asn Pro Asp Thr Arg Leu Cys Ile Arg Val Ile Thr Glu Val Ala
           130                 135                 140
   Trp Gln Ala His Phe Val Lys Asn Met Phe Ile Arg Pro Thr Glu Ala
   145                 150                 155                 160
   Glu Leu Asp Thr Phe Glu Pro Asp Phe Val Ile Met Asn Gly Ser Lys
                   165                 170                 175
   Cys Thr Asn Pro Lys Trp Gln Glu His Gly Met Asn Ser Glu Asn Phe
               180                 185                 190
   Thr Val Phe Asn Leu Ser Glu Lys Met Gln Leu Ile Gly Gly Thr Trp
           195                 200                 205
   Tyr Gly Gly Glu Met Lys Lys Gly Met Phe Ala Met Met Asn Tyr Phe
       210                 215                 220
   Leu Pro Leu Lys Asp Ile Ala Ser Met His Cys Ser Ala Asn Met Gly
   225                 230                 235                 240
   Glu Ala Gly Asp Val Ala Ile Phe Phe Gly Leu Ser Gly Thr Gly Lys
                   245                 250                 255
   Thr Thr Leu Ser Thr Asp Pro Lys Arg Ala Leu Ile Gly Asp Asp Glu
               260                 265                 270
   His Gly Trp Asn Asp Asn Gly Val Phe Asn Phe Glu Gly Gly Cys Tyr
           275                 280                 285
   Ala Lys Thr Ile Asn Leu Ser Lys Glu Ala Glu Pro Asp Ile Tyr Asn
       290                 295                 300
   Ala Ile Arg Arg Asp Ala Leu Leu Glu Asn Val Thr Val Arg Gly Asp
   305                 310                 315                 320
   Gly Ser Ile Asp Phe Asn Asp Asn Ser Lys Thr Glu Asn Thr Arg Val
                   325                 330                 335
   Ser Tyr Pro Ile Tyr His Ile Asp Asn Ile Val Lys Pro Val Ser Lys
               340                 345                 350
   Gly Gly His Ala Asn Lys Val Ile Phe Leu Ser Ala Asp Ala Phe Gly
           355                 360                 365
   Val Leu Pro Pro Val Ser Lys Leu Thr Pro Glu Gln Thr Lys Tyr His
       370                 375                 380
   Phe Leu Ser Gly Phe Thr Ala Lys Leu Ala Gly Thr Glu Arg Gly Ile
   385                 390                 395                 400
   Thr Glu Pro Thr Pro Thr Phe Ser Ala Cys Phe Gly Asn Ala Phe Leu
                   405                 410                 415
   Thr Leu His Pro Thr Gln Tyr Ala Glu Val Leu Val Lys Arg Met Glu
               420                 425                 430
   Ala Ala Gly Ala Glu Ala Tyr Leu Val Asn Thr Gly Trp Asn Gly Thr
           435                 440                 445
   Gly Lys Arg Ile Ser Ile Gln Asp Thr Arg Gly Ile Ile Asp Ala Ile
       450                 455                 460
   Leu Asp Gly Ser Ile Asp Thr Ala Pro Thr Lys Gln Ile Pro Ile Phe
   465                 470                 475                 480
   Asn Leu Thr Val Pro Thr Ala Leu Pro Gly Val Asp Pro Glu Ile Leu
                   485                 490                 495
   Asp Pro Arg Asp Thr Tyr Thr Asp Pro Leu Gln Trp Glu Ser Lys Ala
               500                 505                 510
   Thr Asp Leu Ala Gly Arg Phe Ile Ala Asn Phe Asp Lys Tyr Thr Asp
           515                 520                 525
   Thr Thr Glu Gly Lys Ser Leu Val Lys Ala Gly Pro Gln Leu Asp
       530                 535                 540
<210> 104
   <211> 539
   <212> PRT
   <213> Providencia rettgeri
<220>
   <223> PrePCK
<400> 104
   Met Ser Ala Lys Ser Ile Thr Leu Lys Glu Leu Glu Lys Tyr Gly Ile
                   1                   5                  10                  15
   His Asp Val Thr Glu Val Val Tyr Asn Pro Ser Tyr Glu Leu Leu Phe
               20                  25                  30
   Thr Glu Glu Thr Lys Pro Gly Leu Glu Gly Tyr Glu Arg Gly Thr Val
           35                  40                  45
   Thr Thr Leu Gly Ala Val Ala Val Asp Thr Gly Ile Phe Thr Gly Arg
       50                  55                  60
   Ser Pro Lys Asp Lys Tyr Ile Val Arg Asp Asp Val Thr Arg Asp Thr
   65                  70                  75                  80
   Val Trp Trp Ala Asp Gln Gly Lys Gly Lys Asn Asp Asn Lys Pro Met
                   85                  90                  95
   Ser Gln Glu Val Trp Ala Asp Leu Lys His Leu Val Thr Glu Gln Leu
               100                 105                 110
   Ser Gly Lys Arg Leu Phe Ile Ile Asp Ala Phe Cys Gly Ala Asn Ala
           115                 120                 125
   Asp Thr Arg Leu Lys Val Arg Phe Ile Thr Glu Val Ala Trp Gln Ala
       130                 135                 140
   His Phe Val Lys Asn Met Phe Ile Arg Pro Ser Asp Glu Glu Leu Val
   145                 150                 155                 160
   Gly Phe Glu Pro Asp Phe Ile Val Met Asn Gly Ala Lys Cys Thr Asn
                   165                 170                 175
   Pro Asn Trp Lys Ala Gln Gly Leu Asn Ser Glu Asn Phe Val Ala Phe
               180                 185                 190
   Asn Leu Thr Glu Arg Met Gln Leu Ile Gly Gly Ser Trp Tyr Gly Gly
           195                 200                 205
   Glu Met Lys Lys Gly Met Phe Ser Met Met Asn Tyr Leu Leu Pro Leu
       210                 215                 220
   Lys Gly Ile Ala Ser Met His Cys Ser Ala Asn Val Gly Glu Lys Gly
   225                 230                 235                 240
   Asp Val Ala Ile Phe Phe Gly Leu Ser Gly Thr Gly Lys Thr Thr Leu
                   245                 250                 255
   Ser Thr Asp Pro Lys Arg Lys Leu Ile Gly Asp Asp Glu His Gly Trp
               260                 265                 270
   Asp Asp Asp Gly Val Phe Asn Phe Glu Gly Gly Cys Tyr Ala Lys Thr
           275                 280                 285
   Ile Asn Leu Ser Lys Glu Ala Glu Pro Asp Ile Tyr Gly Ala Ile Lys
       290                 295                 300
   Arg Asp Ala Leu Leu Glu Asn Val Met Val Leu Ala Asp Gly Ser Val
   305                 310                 315                 320
   Asp Phe Asn Asp Gly Ser Lys Thr Glu Asn Thr Arg Val Ser Tyr Pro
                   325                 330                 335
   Ile Tyr His Ile Glu Asn Ile Val Lys Pro Val Ser Lys Ala Gly His
               340                 345                 350
   Ala Thr Lys Val Ile Phe Leu Thr Ala Asp Ala Phe Gly Val Leu Pro
           355                 360                 365
   Pro Val Ser Arg Leu Thr Pro Glu Gln Thr Gln Tyr His Phe Leu Ser
       370                 375                 380
   Gly Phe Thr Ala Lys Leu Ala Gly Thr Glu Arg Gly Val Thr Glu Pro
   385                 390                 395                 400
   Thr Pro Thr Phe Ser Ala Cys Phe Gly Ala Ala Phe Leu Ser Leu His
                   405                 410                 415
   Pro Thr Gln Tyr Ala Glu Val Leu Val Lys Arg Met Glu Ala Ala Gly
               420                 425                 430
   Ala Lys Ala Tyr Leu Val Asn Thr Gly Trp Asn Gly Thr Gly Lys Arg
           435                 440                 445
   Ile Ser Ile Lys Asp Thr Arg Ala Ile Ile Asp Ala Ile Leu Ser Gly
       450                 455                 460
   Asp Ile Glu Lys Ala Asp Met Ile Lys Leu Pro Val Phe Asp Leu Glu
   465                 470                 475                 480
   Ile Pro Thr Ala Leu Pro Gly Val Asp Thr Asn Ile Leu Asp Pro Arg
                   485                 490                 495
   Asn Thr Tyr Ala Asp Lys Ala Gln Trp Asp Glu Lys Ala Gln Asp Leu
                   500                     505                     510
   Ala Glu Arg Phe Val Asn Asn Phe Asp Lys Tyr Thr Asp Thr Pro Ala
               515                     520                     525
   Gly Ala Ala Leu Val Lys Ala Gly Pro Lys Leu
           530                     535
<210> 105
   <211> 549
   <212> PRT
   <213> Saccharomyces cerevisiae
<220>
   <223> ScePCK
<400> 105
   Met Ser Pro Ser Lys Met Asn Ala Thr Val Gly Ser Thr Ser Glu Val
                   5                   10                  15
   Glu Gln Lys Ile Arg Gln Glu Leu Ala Leu Ser Asp Glu Val Thr Thr
               20                  25                  30
   Ile Arg Arg Asn Ala Pro Ala Ala Val Leu Tyr Glu Asp Gly Leu Lys
           35                  40                  45
   Glu Asn Lys Thr Val Ile Ser Ser Ser Gly Ala Leu Ile Ala Tyr Ser
       50                  55                  60
   Gly Val Lys Thr Gly Arg Ser Pro Lys Asp Lys Arg Ile Val Glu Glu
   65                  70                  75                  80
   Pro Thr Ser Lys Asp Glu Ile Trp Trp Gly Pro Val Asn Lys Pro Cys
                   85                  90                  95
   Ser Glu Arg Thr Trp Ser Ile Asn Arg Glu Arg Ala Ala Asp Tyr Leu
               100                 105                 110
   Arg Thr Arg Asp His Ile Tyr Ile Val Asp Ala Phe Ala Gly Trp Asp
           115                 120                 125
   Pro Lys Tyr Arg Ile Lys Val Arg Val Val Cys Ala Arg Ala Tyr His
       130                 135                 140
   Ala Leu Phe Met Thr Asn Met Leu Ile Arg Pro Thr Glu Glu Glu Leu
   145                 150                 155                 160
   Ala His Phe Gly Glu Pro Asp Phe Thr Val Trp Asn Ala Gly Gln Phe
                   165                 170                 175
   Pro Ala Asn Leu His Thr Gln Asp Met Ser Ser Lys Ser Thr Ile Glu
               180                 185                 190
   Ile Asn Phe Lys Ala Met Glu Met Ile Ile Leu Gly Thr Glu Tyr Ala
           195                 200                 205
   Gly Glu Met Lys Lys Gly Ile Phe Thr Val Met Phe Tyr Leu Met Pro
       210                 215                 220
   Val His His Asn Val Leu Thr Leu His Ser Ser Ala Asn Gln Gly Ile
   225                 230                 235                 240
   Gln Asn Gly Asp Val Thr Leu Phe Phe Gly Leu Ser Gly Thr Gly Lys
                   245                 250                 255
   Thr Thr Leu Ser Ala Asp Pro His Arg Leu Leu Ile Gly Asp Asp Glu
               260                 265                 270
   His Cys Trp Ser Asp His Gly Val Phe Asn Ile Glu Gly Gly Cys Tyr
           275                 280                 285
   Ala Lys Cys Ile Asn Leu Ser Ala Glu Lys Glu Pro Glu Ile Phe Asp
       290                 295                 300
   Ala Ile Lys Phe Gly Ser Val Leu Glu Asn Val Ile Tyr Asp Glu Lys
   305                 310                 315                 320
   Ser His Val Val Asp Tyr Asp Asp Ser Ser Ile Thr Glu Asn Thr Arg
                   325                 330                 335
   Cys Ala Tyr Pro Ile Asp Tyr Ile Pro Ser Ala Lys Ile Pro Cys Leu
               340                 345                 350
   Ala Asp Ser His Pro Lys Asn Ile Ile Leu Leu Thr Cys Asp Ala Ser
           355                 360                 365
   Gly Val Leu Pro Pro Val Ser Lys Leu Thr Pro Glu Gln Val Met Tyr
       370                 375                 380
   His Phe Ile Ser Gly Tyr Thr Ser Lys Met Ala Gly Thr Glu Gln Gly
   385                 390                 395                 400
   Val Thr Glu Pro Glu Pro Thr Phe Ser Ser Cys Phe Gly Gln Pro Phe
                   405                 410                 415
   Leu Ala Leu His Pro Ile Arg Tyr Ala Thr Met Leu Ala Thr Lys Met
               420                 425                 430
   Ser Gln His Lys Ala Asn Ala Tyr Leu Ile Asn Thr Gly Trp Thr Gly
           435                 440                 445
   Ser Ser Tyr Val Ser Gly Gly Lys Arg Cys Pro Leu Lys Tyr Thr Arg
       450                 455                 460
   Ala Ile Leu Asp Ser Ile His Asp Gly Ser Leu Ala Asn Glu Thr Tyr
   465                 470                 475                 480
   Glu Thr Leu Pro Ile Phe Asn Leu Gln Val Pro Thr Lys Val Asn Gly
                   485                 490                 495
   Val Pro Ala Glu Leu Leu Asn Pro Ala Lys Asn Trp Ser Gln Gly Glu
               500                 505                 510
   Ser Lys Tyr Arg Gly Ala Val Thr Asn Leu Ala Asn Leu Phe Val Gln
           515                 520                 525
   Asn Phe Lys Ile Tyr Gln Asp Arg Ala Thr Pro Asp Val Leu Ala Ala
       530                 535                 540
   Gly Pro Gln Phe Glu
   545
<210> 106
   <211> 539
   <212> PRT
   <213> Serratia odorifera
<220>
   <223> SodPCK
<400> 106
   Met Arg Ala Lys Gly Ile Thr His Gln Asp Leu Ala Ala Tyr Gly Ile
   5 10 15
   His Asp Ile Gly Glu Ile Ile His Asn Pro Ser Tyr Asp Leu Leu Phe
   20 25 30
   Glu Glu Glu Thr Asp Pro Ser Leu Thr Gly Tyr Glu Arg Gly Val Val
   35 40 45
   Thr Lys Leu Gly Ala Val Ala Val Asp Thr Gly Ile Phe Thr Gly Arg
   50 55 60
   Ser Pro Lys Asp Lys Tyr Ile Val Arg Asp Asp Thr Thr Arg Asp Thr
   65 70 75 80
   Val Trp Trp Ala Asp Gln Gly Lys Gly Lys Asn Asp Asn Lys Pro Met
   85 90 95
   Ser Pro Glu Val Trp Ala Ser Leu Lys Thr Leu Val Thr Glu Gln Leu
   100 105 110
   Ser Gly Lys Arg Leu Phe Val Val Asp Thr Phe Cys Gly Ala Asn Ala
   115 120 125
   Asp Ser Arg Leu Lys Val Arg Phe Ile Thr Glu Val Ala Trp Gln Ala
   130 135 140
   His Phe Val Lys Asn Met Phe Ile Arg Pro Ser Asp Asp Glu Leu Ala
   145 150 155 160
   Asp Phe Glu Pro Asp Phe Val Val Met Asn Gly Ala Lys Cys Thr Asn
   165 170 175
   Pro Asp Trp Gln Gln Gln Gly Leu Asn Ser Glu Asn Phe Val Ala Phe
   180 185 190
   Asn Leu Thr Glu Arg Met Gln Leu Ile Gly Gly Thr Trp Tyr Gly Gly
   195 200 205
   Glu Met Lys Lys Gly Met Phe Ser Ile Met Asn Tyr Leu Leu Pro Leu
   210 215 220
   Lys Gly Ile Ala Ser Met His Cys Ser Ala Asn Val Gly Glu Gln Gly
   225 230 235 240
   Asp Val Ala Ile Phe Phe Gly Leu Ser Gly Thr Gly Lys Thr Thr Leu
                   245                 250                 255
   Ser Thr Asp Pro Lys Arg Gln Leu Ile Gly Asp Asp Glu His Gly Trp
               260                 265                 270
   Asp Asp Asp Gly Val Phe Asn Phe Glu Gly Gly Cys Tyr Ala Lys Thr
           275                 280                 285
   Ile Lys Leu Ser Glu Gln Ala Glu Pro Asp Ile Tyr Arg Ala Ile Lys
       290                 295                 300
   Arg Asp Ala Leu Leu Glu Asn Val Thr Val Leu Ala Asp Gly Ser Val
   305                 310                 315                 320
   Asp Phe Asn Asp Gly Ser Lys Thr Glu Asn Thr Arg Val Ser Tyr Pro
                   325                 330                 335
   Ile Tyr His Ile Gln Asn Ile Val Lys Pro Val Ser Lys Ala Gly His
               340                 345                 350
   Ala Thr Lys Val Ile Phe Leu Thr Ala Asp Ala Phe Gly Val Leu Pro
           355                 360                 365
   Pro Val Ser Arg Leu Thr Ala Asp Gln Thr Gln Tyr His Phe Leu Ser
       370                 375                 380
   Gly Phe Thr Ala Lys Leu Ala Gly Thr Glu Arg Gly Val Thr Glu Pro
   385                 390                 395                 400
   Thr Pro Thr Phe Ser Ala Cys Phe Gly Ala Ala Phe Leu Ser Leu His
                   405                 410                 415
   Pro Thr Gln Tyr Ala Glu Val Leu Val Lys Arg Met Gln Ala Ala Gly
               420                 425                 430
   Ala Gln Ala Tyr Leu Val Asn Thr Gly Trp Asn Gly Ser Gly Lys Arg
           435                 440                 445
   Ile Ser Ile Gln Asp Thr Arg Gly Ile Ile Asp Ala Ile Leu Ser Gly
       450                 455                 460
   Glu Ile Asp Asn Ala Glu Thr Ile Thr Leu Pro Ile Phe Asp Leu Ala
   465                 470                 475                 480
   Val Pro Thr Ala Leu Pro Gly Val Asn Pro Ala Ile Leu Asp Pro Arg
                   485                 490                 495
   Ala Thr Tyr Gln Ser Glu Ala Gln Trp Glu Asp Lys Ala Lys Asp Leu
               500                 505                 510
   Ala Gln Arg Phe Ile Thr Asn Phe Asp Lys Tyr Thr Asp Thr Pro Ala
           515                 520                 525
   Gly Glu Ala Leu Ile Gly Ala Gly Pro Lys Leu
       530                 535
<210> 107
   <211> 543
   <212> PRT
   <213> Vibrio orientalis
<220>
   <223> VorPCK
<400> 107
<210> 108
   <211> 558
   <212> PRT
   <213> Vanderwaltozyma polyspora
<220>
   <223> VpoPCK
<400> 108
   Met Ala Pro Thr Lys Ile Glu Asp Tyr Ser Leu Asn Glu Arg Glu Asn
                   5                   10                  15
   Ile Ile Lys Asn Leu Lys Ser Asn Ser Asn Val Lys Leu Asp Asn Val
               20                  25                  30
   Met Lys Cys Leu Tyr Asn Lys Asp Ile Thr Ile Lys Arg Asn Pro Pro
           35                  40                  45
   Ile Ala Glu Leu Tyr Glu Asp Gly Leu Lys Glu Asp Ser Thr Thr Leu
       50                  55                  60
   Ser Ser Thr Gly Ala Leu Met Ala Tyr Ser Gly Val Lys Thr Gly Arg
   65                  70                  75                  80
   Ser Pro Lys Asp Lys Arg Ile Val Glu Glu Pro Ser Ser Lys Asp Asn
                   85                  90                  95
   Ile Trp Trp Gly Pro Val Asn Lys Pro Cys Ser Glu Leu Thr Trp Ser
               100                 105                 110
   Ile Asn Arg Glu Arg Ala Ile Asp Tyr Leu Asn Thr Arg Lys Ile Leu
           115                 120                 125
   Tyr Val Ile Asp Ala Tyr Ala Gly Trp Asp Ala Asn Tyr Arg Ile Lys
       130                 135                 140
   Ile Arg Val Val Cys Ala Arg Pro Tyr His Ala Leu Phe Met Asn Asn
   145                 150                 155                 160
   Met Leu Ile Arg Pro Thr Thr Glu Glu Leu Glu Asn Phe Gly Glu Pro
                   165                 170                 175
   Asp Phe Thr Ile Trp Asn Ala Gly Ser Phe Tyr Ala Asn Ser Leu Thr
               180                 185                 190
   Lys Asp Met Thr Ser Lys Thr Ser Ile Glu Ile Asn Phe Lys Ser Met
           195                 200                 205
   Glu Met Val Ile Leu Gly Thr Glu Tyr Ala Gly Glu Met Lys Lys Gly
       210                 215                 220
   Ile Phe Thr Val Met Phe Tyr Leu Met Pro Val Tyr His Asn Val Leu
   225                 230                 235                 240
   Thr Leu His Ser Ser Ala Asn Gln Gly Val Asn Asn Gly Asp Val Thr
                   245                 250                 255
   Leu Phe Phe Gly Leu Ser Gly Thr Gly Lys Thr Thr Leu Ser Ala Asp
               260                 265                 270
   Pro His Arg Glu Leu Ile Gly Asp Asp Glu His Cys Trp Ser Asp His
           275                 280                 285
   Gly Val Phe Asn Ile Glu Gly Gly Cys Tyr Ala Lys Cys Ile Gly Leu
       290                 295                 300
   Thr Glu Glu Lys Glu Pro Glu Ile Phe Asn Ala Ile Lys Phe Gly Ser
   305                 310                 315                 320
   Val Leu Glu Asn Cys Val Tyr Asp Glu Asn Thr His Val Val Asp Tyr
                   325                 330                 335
   Asp Asp Ser Arg Ile Thr Glu Asn Thr Arg Cys Ala Tyr Pro Ile Asp
               340                 345                 350
   Phe Ile Glu Ser Ala Lys Ile Pro Cys Leu Thr Asp Lys His Pro Thr
           355                 360                 365
   Asn Leu Ile Leu Leu Thr Cys Asp Ala Ser Gly Val Leu Pro Pro Val
       370                 375                 380
   Ser Lys Leu Thr Pro Glu Gln Val Met Tyr His Phe Ile Ser Gly Tyr
   385                 390                 395                 400
   Thr Ser Lys Met Ala Gly Thr Glu Gln Gly Val Thr Glu Pro Glu Pro
                   405                 410                 415
   Thr Phe Ser Ala Cys Phe Gly Gln Pro Phe Leu Ala Leu His Pro Met
               420                 425                 430
   Lys Tyr Ala Gln Met Leu Ala Asp Lys Met Asn His His Lys Ala Ser
           435                 440                 445
   Ala Trp Leu Ile Asn Thr Gly Trp Thr Gly Ala Ser Tyr Thr Ala Gly
       450                 455                 460
   Gly Lys Arg Cys Pro Leu Lys Tyr Thr Arg Ala Ile Leu Asp Ala Ile
   465                 470                 475                 480
   His Asn Gly Ser Leu Ala Asn Glu Glu Tyr Glu Thr Leu Pro Val Phe
                       485                 490                 495
   Asn Leu Pro Ile Pro Lys Asn Val Glu Asn Val Pro Ser Glu Leu Leu
                   500                 505                 510
   Asn Pro Ser Lys Thr Trp Ser Asp Pro Asp Val Thr Tyr Ser Gln Ala
               515                 520                 525
   Val Lys Lys Leu Gly Ser Leu Phe Val Gln Asn Phe Thr Ile Tyr Gln
       530                 535                 540
   Asp Gln Ala Thr Asp Asp Val Ile Ala Ala Gly Pro Ala Leu
   545                 550                 555
<210> 109
   <211> 543
   <212> PRT
   <213> Vibrio tubiashii
<220>
   <223> VtuPCK
<400> 109
   Met Thr Val Met Glu His Thr Lys Ala Ala Thr Ile Asp Leu Thr Lys
                   5                   10                  15
   His Gly Leu Gln Asn Val Lys Glu Val Ile Arg Asn Pro Ser Tyr Glu
               20                  25                  30
   Met Leu Phe Glu Glu Glu Thr Arg Ala Asp Leu Glu Gly Tyr Glu Lys
           35                  40                  45
   Gly Val Val Thr Glu Leu Gly Ala Val Ala Val Asp Thr Gly Ile Phe
       50                  55                  60
   Thr Gly Arg Ser Pro Lys Asp Lys Tyr Ile Val Lys Asp Ala Thr Thr
   65                  70                  75                  80
   Glu Glu His Met Trp Trp Thr Ser Asp Ala Val Lys Asn Asp Asn Lys
                   85                  90                  95
   Pro Ile Asp Gln Ala Val Trp Asn Asp Leu Lys Gln Leu Val Thr Asn
               100                 105                 110
   Gln Leu Ser Gly Lys Arg Val Phe Val Ile Asp Gly Tyr Cys Gly Ala
           115                 120                 125
   Asn Pro Asp Thr Arg Leu Ser Ile Arg Val Ile Thr Glu Val Ala Trp
       130                 135                 140
   Gln Ala His Phe Val Lys Asn Met Phe Ile Arg Pro Ser Glu Lys Glu
   145                 150                 155                 160
   Leu Ala Thr Phe Glu Pro Asp Phe Val Val Met Asn Gly Ala Lys Cys
                   165                 170                 175
   Thr Asn Gln Lys Trp Glu Glu His Gly Leu Asn Ser Glu Asn Phe Thr
               180                 185                 190
   Val Phe Asn Leu Thr Glu Arg Met Gln Leu Ile Gly Gly Thr Trp Tyr
           195                 200                 205
   Gly Gly Glu Met Lys Lys Gly Met Phe Ala Met Met Asn Tyr Phe Leu
       210                 215                 220
   Pro Leu Lys Asp Ile Ala Ser Met His Cys Ser Ala Asn Lys Cys Lys
   225                 230                 235                 240
   Glu Asn Gly Asp Val Ala Val Phe Phe Gly Leu Ser Gly Thr Gly Lys
                   245                 250                 255
   Thr Thr Leu Ser Thr Asp Pro Lys Arg Glu Leu Ile Gly Asp Asp Glu
               260                 265                 270
   His Gly Trp Asp Asp Asp Gly Ile Phe Asn Phe Glu Gly Gly Cys Tyr
           275                 280                 285
   Ala Lys Thr Ile Lys Leu Ser Lys Glu Ala Glu Pro Asp Ile Tyr Asn
       290                 295                 300
   Ala Ile Arg Arg Asp Ala Leu Leu Glu Asn Val Thr Val Arg Ser Asp
   305                 310                 315                 320
   Gly Ser Ile Asp Phe Asp Asp Gly Ser Lys Thr Glu Asn Thr Arg Val
                   325                 330                 335
   Ser Tyr Pro Ile Glu His Ile Glu Asn Ile Val Lys Pro Val Ser Lys
               340                 345                 350
   Ala Gly His Ala Asn Lys Val Ile Phe Leu Ser Ala Asp Ala Phe Gly
           355                 360                 365
   Val Leu Pro Pro Val Ser Lys Leu Thr Pro Glu Gln Thr Lys Tyr His
       370                 375                 380
   Phe Leu Ser Gly Phe Thr Ala Lys Leu Ala Gly Thr Glu Arg Gly Ile
   385                 390                 395                 400
   Thr Glu Pro Thr Pro Thr Phe Ser Ala Cys Phe Gly Ala Ala Phe Leu
                   405                 410                 415
   Thr Leu His Pro Thr Lys Tyr Ala Glu Val Leu Val Lys Arg Met Glu
               420                 425                 430
   Ala Ala Gly Ala Glu Ala Tyr Leu Val Asn Thr Gly Trp Asn Gly Ser
           435                 440                 445
   Gly Lys Arg Ile Ser Ile Gln Asp Thr Arg Gly Ile Ile Asp Ala Ile
       450                 455                 460
   Leu Asp Gly Ser Ile Glu Asn Ala Glu Thr Lys His Ile Pro Ile Phe
   465                 470                 475                 480
   Asn Leu Glu Val Pro Thr Ala Leu His Asp Val Asp Pro Ala Ile Leu
                   485                 490                 495
   Asp Pro Arg Asp Thr Tyr Thr Asp Pro Leu Gln Trp Glu Ser Lys Ala
               500                 505                 510
   Lys Asp Leu Ala Glu Arg Phe Ile Asn Asn Phe Asp Lys Tyr Thr Asp
           515                 520                 525
   Asn Asp Glu Gly Lys Ser Leu Val Ala Ala Gly Pro Gln Leu Asp
       530                 535                 540
<210> 110
   <211> 539
   <212> PRT
   <213> Yersinia bercovieri
<220>
   <223> YbePCK
<400> 110
   Met Ser Val Lys Gly Ile Thr Pro Gln Glu Leu Ala Ala Tyr Gly Ile
                       5                  10                  15
   His Asn Val Ser Glu Ile Val Tyr Asn Pro Ser Tyr Asp Leu Leu Phe
                   20                  25                  30
   Gln Glu Glu Thr Lys Pro Thr Leu Glu Gly Tyr Glu Arg Gly Thr Leu
               35                  40                  45
   Thr Thr Thr Gly Ala Ile Ala Val Asp Thr Gly Ile Phe Thr Gly Arg
       50                  55                  60
   Ser Pro Lys Asp Lys Tyr Ile Val Arg Asp Ala Ile Thr Gln Asp Thr
   65                  70                  75                  80
   Val Trp Trp Ala Asp Gln Gly Lys Gly Lys Asn Asp Asn Lys Pro Leu
                       85                  90                  95
   Ser Gln Glu Thr Trp Ser His Leu Lys Gly Leu Val Thr Glu Gln Leu
                   100                 105                 110
   Ser Gly Lys Arg Leu Phe Val Val Asp Thr Phe Cys Gly Ala Asn Ala
           115                 120                 125
   Asp Ser Arg Leu Gln Val Arg Phe Val Thr Glu Val Ala Trp Gln Ala
       130                 135                 140
   His Phe Val Lys Asn Met Phe Ile Arg Pro Thr Asp Glu Glu Leu Ala
   145                 150                 155                 160
   Asp Phe Glu Pro Asp Phe Ile Val Met Asn Gly Ala Lys Cys Thr Asn
                   165                 170                 175
   Pro Asn Trp Lys Glu Gln Gly Leu Asn Ser Glu Asn Phe Val Ala Phe
               180                 185                 190
   Asn Leu Thr Glu Arg Met Gln Leu Ile Gly Gly Thr Trp Tyr Gly Gly
           195                 200                 205
   Glu Met Lys Lys Gly Met Phe Ser Met Met Asn Tyr Leu Leu Pro Leu
       210                 215                 220
   Lys Gly Ile Ala Ser Met His Cys Ser Ala Asn Val Gly Glu Lys Gly
   225                 230                 235                 240
   Asp Val Ala Ile Phe Phe Gly Leu Ser Gly Thr Gly Lys Thr Thr Leu
                   245                 250                 255
   Ser Thr Asp Pro Lys Arg Lys Leu Ile Gly Asp Asp Glu His Gly Trp
               260                 265                 270
   Asp Asp Asp Gly Val Phe Asn Phe Glu Gly Gly Cys Tyr Ala Lys Thr
           275                 280                 285
   Ile Lys Leu Ser Glu Glu Ala Glu Pro Asp Ile Tyr His Ala Ile Arg
       290                 295                 300
   Arg Asp Ala Leu Leu Glu Asn Val Val Val Leu Pro Asp Gly Thr Val
   305                 310                 315                 320
   Asp Phe Asn Asp Ser Ser Lys Thr Glu Asn Thr Arg Val Ser Tyr Pro
                   325                 330                 335
   Ile Tyr His Ile Glu Asn Ile Val Lys Pro Val Ser Lys Ala Gly His
               340                 345                 350
   Ala Thr Lys Val Ile Phe Leu Thr Ala Asp Ala Phe Gly Val Leu Pro
           355                 360                 365
   Pro Val Ser Arg Leu Thr Ala Asn Gln Thr Gln Tyr His Phe Leu Ser
       370                 375                 380
   Gly Phe Thr Ala Lys Leu Ala Gly Thr Glu Arg Gly Val Thr Glu Pro
   385                 390                 395                 400
   Thr Pro Thr Phe Ser Ala Cys Phe Gly Ala Ala Phe Leu Ser Leu His
                   405                 410                 415
   Pro Thr Gln Tyr Ala Glu Val Leu Val Lys Arg Met Gln Ala Val Gly
               420                 425                 430
   Ala Gln Ala Tyr Leu Val Asn Thr Gly Trp Asn Gly Thr Gly Lys Arg
           435                 440                 445
   Ile Ser Ile Lys Asp Thr Arg Ala Ile Ile Asp Ala Ile Leu Asn Gly
       450                 455                 460
   Glu Ile Asp Lys Ala Glu Thr Phe Thr Leu Pro Ile Phe Asp Leu Ala
   465                 470                 475                 480
   Val Pro Met Ala Leu Pro Gly Val Asp Pro Ala Ile Leu Asp Pro Arg
                   485                 490                 495
   Asp Thr Tyr Ala Asp Ile Ala Gln Trp Gln Glu Lys Ala Glu Asp Leu
               500                 505                 510
   Ala Arg Arg Phe Thr Thr Asn Phe Asp Lys Tyr Thr Asp Thr Pro Ala
           515                 520                 525
   Gly Ala Ala Leu Met Ser Ala Gly Pro Lys Ile
       530                 535
<210> 111
   <211> 556
   <212> PRT
   <213> Yarrowia lipolytica
<220>
   <223> YliPCK
<400> 111
   Met Ser Pro Pro Val Asn Gln Thr Gln Leu His Pro Lys Gly Val Glu
                   5                   10                  15
   Pro Thr Pro Ala Ala Glu Glu Ile Glu Lys Glu Leu Tyr Asp Ile Ala
               20                  25                  30
   His Ile Glu Tyr Glu Arg Val Asn Ile Asp His Asn Pro Ser Val Ala
           35                  40                  45
   Ser Leu Tyr Glu Asp Ala Leu Val Gln Glu Ser Gly Ser Ala Ile Asn
       50                  55                  60
   Ser Thr Gly Ala Leu Val Ala Ser Ser Gly Lys Lys Thr Gly Arg Ser
   65                  70                  75                  80
   Pro Lys Asp Lys Arg Ile Val Glu Glu Pro Gly Ser Val Glu His Ile
                   85                  90                  95
   Trp Trp Gly Pro Val Asn Lys Lys Leu Ser Glu Gln Ser Trp Leu Ile
               100                 105                 110
   Asn Arg Glu Arg Ala Val Asp Tyr Leu Asn Thr Arg Lys Arg Ile Tyr
           115                 120                 125
   Val Val Asp Ala Tyr Ala Gly Trp Asp Pro Lys Tyr Arg Ile Lys Val
       130                 135                 140
   Arg Ile Val Cys Ala Arg Ala Tyr His Ala Leu Phe Met Arg Asn Met
   145                 150                 155                 160
   Leu Ile Arg Pro Thr Ala Glu Glu Leu Lys Asn Phe Gly Lys Pro Asp
                   165                 170                 175
   Phe Thr Ile Tyr Asn Ala Gly Ala Phe Pro Ala Asn Arg Tyr Thr Ser
               180                 185                 190
   Gly Met Thr Ser Asn Thr Ser Ile Asp Ile Asn Phe Lys Ser Met Glu
           195                 200                 205
   Met Leu Ile Leu Gly Thr Glu Tyr Ala Gly Glu Met Lys Lys Gly Ile
       210                 215                 220
   Phe Thr Val Met Phe Tyr Leu Met Pro Val Lys His Gln Val Leu Thr
   225                 230                 235                 240
   Leu His Ser Ser Ala Asn Glu Gly Lys Asp Gly Asp Val Thr Leu Phe
                   245                 250                 255
   Phe Gly Leu Ser Gly Thr Gly Lys Thr Thr Leu Ser Ala Asp Pro Asn
               260                 265                 270
   Arg Leu Leu Ile Gly Asp Asp Glu His Cys Trp Ser Asp Thr Gly Val
           275                 280                 285
   Phe Asn Ile Glu Gly Gly Cys Tyr Ala Lys Cys Leu Gly Leu Ser Arg
       290                 295                 300
   Glu Lys Glu Pro Asp Ile Phe Asp Ala Ile Lys Phe Gly Ser Val Leu
   305                 310                 315                 320
   Glu Asn Val Val Phe Asp Pro Ile Thr Arg Glu Val Asp Tyr Asp Asp
                   325                 330                 335
   Ala Thr Leu Thr Glu Asn Thr Arg Cys Ser Tyr Pro Ile Glu Tyr Ile
               340                 345                 350
   Pro Asn Ala Lys Leu Pro Cys Val Thr Glu Ser His Pro Thr Asn Ile
           355                 360                 365
   Ile Leu Leu Thr Cys Asp Ala Arg Gly Val Ile Pro Pro Ile Ser Lys
       370                 375                 380
   Leu Thr Asn Glu Gln Val Met Tyr His Phe Ile Ser Gly Tyr Thr Ser
   385                 390                 395                 400
   Lys Met Ala Gly Thr Glu Glu Gly Val Thr Glu Pro Glu Ala Thr Phe
                   405                 410                 415
   Ser Ala Cys Phe Gly Gln Pro Phe Leu Val Leu His Pro Met Gln Tyr
               420                 425                 430
   Ala Lys Met Leu Ser Asp Lys Met Thr Gln His Asn Ala Asn Ala Trp
           435                 440                 445
   Leu Leu Asn Thr Gly Trp Thr Gly Gln Ser Tyr Thr Asn Gly Gly Lys
       450                 455                 460
   Arg Cys Pro Leu Lys Tyr Thr Arg Ser Ile Leu Asp Ala Ile His Ser
   465                 470                 475                 480
   Gly Glu Leu Ala Lys Val Glu Tyr Glu Asn Phe Asp Val Phe Asn Leu
                   485                 490                 495
   Gln Val Pro Lys Thr Ala Pro Asn Val Pro Ser Glu Ile Leu Asn Pro
               500                 505                 510
   Ala Arg Ala Trp Ser Gly Gly Lys Glu Gln Phe Thr Glu Glu Val Thr
           515                 520                 525
   Lys Leu Gly Lys Leu Phe Asn Glu Asn Phe Glu Lys Tyr Lys Asp Met
       530                 535                 540
   Ala Ile Pro Glu Val Ile Ala Ala Gly Pro Lys Ala
   545                 550                 555
<210> 112
   <211> 539
   <212> PRT
   <213> Yersinia rohdei
<220>
   <223> YroPCK
<400> 112
   Met Ser Val Lys Gly Ile Thr Pro Gln Glu Leu Ala Ala Tyr Gly Ile
                   5                   10                  15
   His Asn Val Ser Glu Ile Val Tyr Asn Pro Ser Tyr Asp Leu Leu Phe
               20                  25                  30
   Gln Glu Glu Thr Lys Pro Thr Leu Glu Gly Tyr Glu Arg Gly Thr Leu
           35                  40                  45
   Thr Thr Thr Gly Ala Ile Ala Val Asp Thr Gly Ile Phe Thr Gly Arg
       50                  55                  60
   Ser Pro Lys Asp Lys Tyr Ile Val Arg Asp Asp Ile Thr Arg Asp Thr
   65                  70                  75                  80
   Val Trp Trp Ala Asp Gln Gly Lys Gly Lys Asn Asp Asn Lys Pro Leu
                   85                  90                  95
   Ser Gln Glu Thr Trp Ala His Leu Lys Gly Leu Val Thr Gln Gln Leu
               100                 105                 110
   Ser Gly Lys Arg Leu Phe Val Val Asp Thr Phe Cys Gly Ala Asn Ala
           115                 120                 125
   Asp Thr Arg Leu Gln Val Arg Phe Val Thr Glu Val Ala Trp Gln Ala
       130                 135                 140
   His Phe Val Lys Asn Met Phe Ile Arg Pro Thr Asp Glu Glu Leu Ala
   145                 150                 155                 160
   His Phe Glu Pro Asp Phe Ile Val Met Asn Gly Ala Lys Cys Thr Asn
                   165                 170                 175
   Pro Asn Trp Lys Glu Gln Gly Leu Asn Ser Glu Asn Phe Val Ala Phe
               180                 185                 190
   Asn Leu Thr Glu Arg Met Gln Leu Ile Gly Gly Thr Trp Tyr Gly Gly
           195                 200                 205
   Glu Met Lys Lys Gly Met Phe Ser Met Met Asn Tyr Leu Leu Pro Leu
       210                 215                 220
   Lys Gly Ile Ala Ser Met His Cys Ser Ala Asn Val Gly Glu Lys Gly
   225                 230                 235                 240
   Asp Val Ala Ile Phe Phe Gly Leu Ser Gly Thr Gly Lys Thr Thr Leu
                   245                 250                 255
   Ser Thr Asp Pro Lys Arg Lys Leu Ile Gly Asp Asp Glu His Gly Trp
               260                 265                 270
   Asp Asp Asp Gly Val Phe Asn Phe Glu Gly Gly Cys Tyr Ala Lys Thr
           275                 280                 285
   Ile Lys Leu Ser Glu Glu Ala Glu Pro Asp Ile Tyr His Ala Ile Lys
       290                 295                 300
   Arg Asp Ala Leu Leu Glu Asn Val Val Val Leu Pro Asp Gly Thr Val
   305                 310                 315                 320
   Asp Phe Asn Asp Gly Ser Lys Thr Glu Asn Thr Arg Val Ser Tyr Pro
                   325                 330                 335
   Ile Tyr His Ile Glu Asn Ile Val Lys Pro Val Ser Lys Ala Gly His
               340                 345                 350
   Ala Thr Lys Val Ile Phe Leu Thr Ala Asp Ala Phe Gly Val Leu Pro
           355                 360                 365
   Pro Val Ser Arg Leu Thr Ala Ser Gln Thr Gln Tyr His Phe Leu Ser
       370                 375                 380
   Gly Phe Thr Ala Lys Leu Ala Gly Thr Glu Arg Gly Val Thr Glu Pro
   385                 390                 395                 400
   Thr Pro Thr Phe Ser Ala Cys Phe Gly Ala Ala Phe Leu Ser Leu His
                   405                 410                 415
   Pro Thr Gln Tyr Ala Glu Val Leu Leu Lys Arg Met Gln Ala Val Gly
               420                 425                 430
   Ala Gln Ala Tyr Leu Val Asn Thr Gly Trp Asn Gly Thr Gly Lys Arg
           435                 440                 445
   Ile Ser Ile Lys Asp Thr Arg Gly Ile Ile Asp Ala Ile Leu Asn Gly
       450                 455                 460
   Glu Ile Asp Lys Ala Glu Thr Phe Thr Leu Pro Ile Phe Asp Leu Ala
   465                 470                 475                 480
   Val Pro Met Ala Leu Pro Gly Val Asn Pro Asp Ile Leu Asp Pro Arg
                   485                 490                 495
   Asp Thr Tyr Ala Asp Val Ala Gln Trp Gln Glu Lys Ala Glu Asp Leu
               500                 505                 510
   Ala Lys Arg Phe Thr Thr Asn Phe Asp Lys Tyr Thr Asp Thr Pro Ala
           515                 520                 525
   Gly Ala Ala Leu Val Ser Ala Gly Pro Lys Val
       530                 535
<210> 113
   <211> 548
   <212> PRT
   <213> Zygosaccharomyces rouxii
<220>
   <223> ZroPCK
<400> 113
   Met Ser Pro Ser Lys Val Pro Val Pro Ile Thr Asp Thr Ala Glu Val
                   5                   10                  15
   Glu Glu Gln Ile Arg Lys Glu Leu Ala Leu Gly Ser Glu Val Val Thr
               20                  25                  30
   Ile Arg His Asn Ala Pro Ala Ala Thr Leu Tyr Glu Asp Gly Leu Lys
           35                  40                  45
   Glu Lys Lys Thr Ala Val Val Ser Ser Gly Ala Leu Ala Ala Tyr Ser
       50                  55                  60
   Gly Ser Lys Thr Gly Arg Ser Pro Lys Asp Lys Arg Ile Ile Glu Glu
   65                  70                  75                  80
   Glu Thr Ser Lys Asn Glu Val Trp Trp Gly Pro Val Asn Lys Pro Ala
                   85                  90                  95
   Ser Glu Lys Thr Trp Ser Ile Asn Arg Glu Arg Ala Ala Asp Tyr Leu
               100                 105                 110
   Arg Thr Arg Asp His Leu Tyr Ile Ile Asp Ala Tyr Ala Gly Trp Asp
           115                 120                 125
   Pro Arg Tyr Arg Ile Lys Val Arg Val Ile Cys Ala Arg Ala Tyr His
       130                 135                 140
   Ala Leu Phe Met Thr Asn Met Leu Ile Arg Pro Ser Pro Glu Glu Leu
   145                 150                 155                 160
   Ala Asn Phe Gly Glu Pro Asp Phe Thr Val Trp Asn Ala Gly Gln Phe
                   165                 170                 175
   Pro Ala Asn Arg Asn Thr Glu Gly Met Thr Ser Lys Thr Thr Val Glu
               180                 185                 190
   Ile Asn Phe Lys Ala Met Glu Met Ile Ile Leu Gly Thr Glu Tyr Ala
           195                 200                 205
   Gly Glu Met Lys Lys Gly Ile Phe Thr Val Met Phe Tyr Leu Met Pro
       210                 215                 220
   Val His His Asn Val Leu Thr Leu His Ser Ser Ala Asn Gln Gly Ile
   225                 230                 235                 240
   Glu Arg Gly Asp Val Thr Leu Phe Phe Gly Leu Ser Gly Thr Gly Lys
                   245                 250                 255
   Thr Thr Leu Ser Ala Asp Pro His Arg Ala Leu Ile Gly Asp Asp Glu
               260                 265                 270
   His Cys Trp Ser Asp His Gly Val Phe Asn Ile Glu Gly Gly Cys Tyr
           275                 280                 285
   Ala Lys Cys Ile Asp Leu Thr Ala Glu Lys Glu Pro Glu Ile Tyr Asn
       290                 295                 300
   Ala Ile Lys Phe Gly Ser Val Leu Glu Asn Val Val Phe Asp Ala Ser
   305                 310                 315                 320
   Asn Arg Glu Val Asp Tyr Thr Asp Asn Ser Ile Thr Glu Asn Thr Arg
                   325                 330                 335
   Cys Ala Tyr Pro Ile Asp Tyr Ile Pro Ser Ala Lys Ile Pro Cys Leu
                   340                 345                 350
   Ala Asp Lys His Pro Ser Asn Ile Val Leu Leu Thr Cys Asp Ala Ser
           355                 360                 365
   Gly Val Leu Pro Pro Val Ser Lys Leu Thr Pro Glu Gln Val Met Tyr
       370                 375                 380
   His Phe Ile Ser Gly Tyr Thr Ser Lys Met Ala Gly Thr Glu Gln Gly
   385                 390                 395                 400
   Val Thr Glu Pro Glu Thr Thr Phe Ser Ser Cys Phe Gly Gln Pro Phe
                   405                 410                 415
   Leu Ala Leu His Pro Met Arg Tyr Ala Thr Met Leu Ala Lys Lys Met
               420                 425                 430
   His Gln His Asn Ala Asn Ala Trp Leu Val Asn Thr Gly Trp Thr Gly
           435                 440                 445
   Ser Ser Tyr Thr Ala Gly Gly Lys Arg Cys Pro Leu Lys Tyr Thr Arg
       450                 455                 460
   Ala Ile Leu Asp Ser Ile His Asp Gly Ser Leu Ser Lys Ala Asp Tyr
   465                 470                 475                 480
   Glu Thr Leu Pro Ile Phe Asn Leu His Thr Pro Ala Lys Val Asn Gly
                   485                 490                 495
   Val Pro Asp Ser Leu Leu Asn Pro Ala Lys Ser Trp Val Glu Gly Pro
               500                 505                 510
   Thr Lys Tyr Asn Ser Ala Val Lys Thr Leu Ala Thr Lys Phe Thr Glu
           515                 520                 525
   Asn Phe Lys Ile Tyr Gln Asp Gln Ala Thr Pro Asp Val Leu Ala Ala
       530                 535                 540
   Gly Pro Gln Phe
   545
<210> 114
   <211> 41
   <212> DNA
   <213> Artificial Sequence
<220>
   <223> Description of Artificial Sequence: CglPCK-F
<400> 114
   gacacttttt caaaatgcca tctaaatcta gtgtttctgg g 41
<210> 115
   <211> 38
   <212> DNA
   <213> Artificial Sequence
<220>
   <223> Description of Artificial Sequence: CglPCK-R
<400> 115
   gaaatcaact tttgttccaa ttgtggacca gcagccaa 38
<210> 116
   <211> 36
   <212> DNA
   <213> Artificial Sequence
<220>
   <223> Description of Artificial Sequence: CkoPCK-F
<400> 116
   gacacttttt caaaatgcgc gttaacagct taaccc 36
<210> 117
   <211> 32
   <212> DNA
   <213> Artificial Sequence
<220>
   <223> Description of Artificial Sequence: CkoPCK-R
<400> 117
   gaaatcaact tttgttccag cttcggcccg gc 32
<210> 118
   <211> 36
   <212> DNA
   <213> Artificial Sequence
<220>
   <223> Description of Artificial Sequence: CsaPCK-F
<400> 118
   gacacttttt caaaatgcga gttacaggca taaccc 36
<210> 119
   <211> 32
   <212> DNA
   <213> Artificial Sequence
<220>
   <223> Description of Artificial Sequence: CsaPCK-R
<400> 119
   gaaatcaact tttgttcgcg ctgtgggcct gc 32
<210> 120
   <211> 37
   <212> DNA
   <213> Artificial Sequence
<220>
   <223> Description of Artificial Sequence: DhaPCK-F
<400> 120
   gacacttttt caaaatgaca cctcctactg ctgttga 37
<210> 121
   <211> 35
   <212> DNA
   <213> Artificial Sequence
<220>
   <223> Description of Artificial Sequence: DhaPCK-R
<400> 121
   gaaatcaact tttgttcagc atcaggacca gcagc 35
<210> 122
   <211> 38
   <212> DNA
   <213> Artificial Sequence
<220>
   <223> Description of Artificial Sequence: EfePCK-F
<400> 122
   gacacttttt caaaatgcgc gtgaacaaaa gtttaacc 38
<210> 123
   <211> 40
   <212> DNA
   <213> Artificial Sequence
<220>
   <223> Description of Artificial Sequence: EfePCK-R
<400> 123
   gaaatcaact tttgttccat cttcggacct gcttctacca 40
<210> 124
   <211> 35
   <212> DNA
   <213> Artificial Sequence
<220>
   <223> Description of Artificial Sequence: EtaPCK-F
<400> 124
   gacacttttt caaaatgcat gccactggct taact 35
<210> 125
   <211> 33
   <212> DNA
   <213> Artificial Sequence
<220>
   <223> Description of Artificial Sequence: EtaPCK-R
<400> 125
   gaaatcaact tttgttcacg ttgtgggccg gca 33
<210> 126
   <211> 42
   <212> DNA
   <213> Artificial Sequence
<220>
   <223> Description of Artificial Sequence: KlaPCK-F
<400> 126
   gacacttttt caaaatgtca ccaactaaga cacaaaattc tg 42
<210> 127
   <211> 39
   <212> DNA
   <213> Artificial Sequence
<220>
   <223> Description of Artificial Sequence: KlaPCK-R
<400> 127
   gaaatcaact tttgttctaa ttgcggacca gcagctaag 39
<210> 128
   <211> 32
   <212> DNA
   <213> Artificial Sequence
<220>
   <223> Description of Artificial Sequence: LelPCK-F
<400> 128
   gacacttttt caaaatggca cccccagcag ta 32
<210> 129
   <211> 36
   <212> DNA
   <213> Artificial Sequence
<220>
   <223> Description of Artificial Sequence: LelPCK-R
<400> 129
   gaaatcaact tttgttcaac atcgggtcca gcagct 36
<210> 130
   <211> 37
   <212> DNA
   <213> Artificial Sequence
<220>
   <223> Description of Artificial Sequence: PcaPCK-F
<400> 130
   gacacttttt caaaatgcag atcaacggta ttacccc 37
<210> 131
   <211> 32
   <212> DNA
   <213> Artificial Sequence
<220>
   <223> Description of Artificial Sequence: PcaPCK-R
<400> 131
   gaaatcaact tttgttcgcg cttcggccct gc 32
<210> 132
   <211> 40
   <212> DNA
   <213> Artificial Sequence
<220>
   <223> Description of Artificial Sequence: PlePCK-F
<400> 132
   gacacttttt caaaatgagc acgatgtgtg tcgataataa 40
<210> 133
   <211> 41
   <212> DNA
   <213> Artificial Sequence
<220>
   <223> Description of Artificial Sequence: PlePCK-R
<400> 133
   gaaatcaact tttgttcgtc taactgtggt ccggctttaa c 41
<210> 134
   <211> 37
   <212> DNA
   <213> Artificial Sequence
<220>
   <223> Description of Artificial Sequence: PrePCK-F
<400> 134
   gacacttttt caaaatgagc gctaaaagca ttaccct 37
<210> 135
   <211> 38
   <212> DNA
   <213> Artificial Sequence
<220>
   <223> Description of Artificial Sequence: PrePCK-R
<400> 135
   gaaatcaact tttgttccag ttttggccct gctttcac 38
<210> 136
   <211> 42
   <212> DNA
   <213> Artificial Sequence
<220>
   <223> Description of Artificial Sequence: ScePCK-F
<400> 136
   gacacttttt caaaatgtcc ccttctaaaa tgaatgctac ag 42
<210> 137
   <211> 38
   <212> DNA
   <213> Artificial Sequence
<220>
   <223> Description of Artificial Sequence: ScePCK-R
<400> 137
   gaaatcaact tttgttcctc gaattgagga ccagcggc 38
<210> 138
   <211> 36
   <212> DNA
   <213> Artificial Sequence
<220>
   <223> Description of Artificial Sequence: SodPCK-F
<400> 138
   gacacttttt caaaatgcgt gctaaaggta tcaccc 36
<210> 139
   <211> 33
   <212> DNA
   <213> Artificial Sequence
<220>
   <223> Description of Artificial Sequence: SodPCK-R
<400> 139
   gaaatcaact tttgttccag cttcggaccg gcg 33
<210> 140
   <211> 40
   <212> DNA
   <213> Artificial Sequence
<220>
   <223> Description of Artificial Sequence: VorPCK-F
<400> 140
   gacacttttt caaaatgacc gttatggaac atactaaggc 40
<210> 141
   <211> 38
   <212> DNA
   <213> Artificial Sequence
<220>
   <223> Description of Artificial Sequence: VorPCK-R
<400> 141
   gaaatcaact tttgttcatc tagctgagga cctgcagc 38
<210> 142
   <211> 44
   <212> DNA
   <213> Artificial Sequence
<220>
   <223> Description of Artificial Sequence: VpoPCK-F
<400> 142
   gacacttttt caaaatggct ccaactaaaa tagaagatta ctct 44
<210> 143
   <211> 35
   <212> DNA
   <213> Artificial Sequence
<220>
   <223> Description of Artificial Sequence: VpoPCK-R
<400> 143
   gaaatcaact tttgttctag ggctggacca gctgc 35
<210> 144
   <211> 40
   <212> DNA
   <213> Artificial Sequence
<220>
   <223> Description of Artificial Sequence: VtuPCK-F
<400> 144
   gacacttttt caaaatgacc gttatggaac atacaaaggc 40
<210> 145
   <211> 37
   <212> DNA
   <213> Artificial Sequence
<220>
   <223> Description of Artificial Sequence: VtuPCK-R
<400> 145
   gaaatcaact tttgttcatc tagctgaggt ccagccg 37
<210> 146
   <211> 39
   <212> DNA
   <213> Artificial Sequence
<220>
   <223> Description of Artificial Sequence: YbePCK-F
<400> 146
   gacacttttt caaaatgagt gttaaaggaa ttaccccgc 39
<210> 147
   <211> 35
   <212> DNA
   <213> Artificial Sequence
<220>
   <223> Description of Artificial Sequence: YbePCK-R
<400> 147
   gaaatcaact tttgttcgat cttcggccct gcgct 35
<210> 148
   <211> 33
   <212> DNA
   <213> Artificial Sequence
<220>
   <223> Description of Artificial Sequence: YliPCK-F
<400> 148
   gacacttttt caaaatgtct ccccccgtca acc 33
<210> 149
   <211> 33
   <212> DNA
   <213> Artificial Sequence
<220>
   <223> Description of Artificial Sequence: YliPCK-R
<400> 149
   gaaatcaact tttgttcagc cttagggccg gca 33
<210> 150
   <211> 39
   <212> DNA
   <213> Artificial Sequence
<220>
   <223> Description of Artificial Sequence: YroPCK-F
<400> 150
   gacacttttt caaaatgagt gttaaaggaa ttaccccgc 39
<210> 151
   <211> 37
   <212> DNA
   <213> Artificial Sequence
<220>
   <223> Description of Artificial Sequence: YroPCK-R
<400> 151
   gaaatcaact tttgttcgac ttttggccca gcactga 37
<210> 152
   <211> 34
   <212> DNA
   <213> Artificial Sequence
<220>
   <223> Description of Artificial Sequence: ZroPCK-F
<400> 152
   gacacttttt caaaatgtcg ccctccaaag ttcc 34
<210> 153
   <211> 40
   <212> DNA
   <213> Artificial Sequence
<220>
   <223> Description of Artificial Sequence: ZroPCK-R
<400> 153
   gaaatcaact tttgttcgaa ttgaggacca gctgcaagaa 40
<210> 154
   <211> 540
   <212> PRT
   <213> Escherichia coli
<220>
   <223> EcoPCK
<400> 154
   Met Arg Val Asn Asn Gly Leu Thr Pro Gln Glu Leu Glu Ala Tyr Gly
   1               5                   10                  15
   Ile Ser Asp Val His Asp Ile Val Tyr Asn Pro Ser Tyr Asp Leu Leu
               20                  25                  30
   Tyr Gln Glu Glu Leu Asp Pro Ser Leu Thr Gly Tyr Glu Arg Gly Val
           35                  40                  45
   Leu Thr Asn Leu Gly Ala Val Ala Val Asp Thr Gly Ile Phe Thr Gly
       50                  55                  60
   Arg Ser Pro Lys Asp Lys Tyr Ile Val Arg Asp Asp Thr Thr Arg Asp
   65                  70                  75                  80
   Thr Phe Trp Trp Ala Asp Lys Gly Lys Gly Lys Asn Asp Asn Lys Pro
                   85                  90                  95
   Leu Ser Pro Glu Thr Trp Gln His Leu Lys Gly Leu Val Thr Lys Gln
               100                 105                 110
   Leu Ser Gly Lys Arg Leu Phe Val Val Asp Ala Phe Cys Gly Ala Asn
           115                 120                 125
   Pro Asp Thr Arg Leu Ser Val Arg Phe Ile Thr Glu Val Ala Trp Gln
       130                 135                 140
   Ala His Phe Val Lys Asn Met Phe Ile Arg Pro Ser Asp Glu Glu Leu
   145                 150                 155                 160
   Ala Gly Phe Lys Pro Asp Phe Ile Val Met Asn Gly Ala Lys Cys Thr
                   165                 170                 175
   Asn Pro Gln Trp Lys Glu Gln Gly Leu Asn Ser Glu Asn Phe Val Ala
               180                 185                 190
   Phe Asn Leu Thr Glu Arg Met Gln Leu Ile Gly Gly Thr Trp Tyr Gly
           195                 200                 205
   Gly Glu Met Lys Lys Gly Met Phe Ser Met Met Asn Tyr Leu Leu Pro
       210                 215                 220
   Leu Lys Gly Ile Ala Ser Met His Cys Ser Ala Asn Val Gly Glu Lys
   225                 230                 235                 240
   Gly Asp Val Ala Val Phe Phe Gly Leu Ser Gly Thr Gly Lys Thr Thr
                   245                 250                 255
   Leu Ser Thr Asp Pro Lys Arg Arg Leu Ile Gly Asp Asp Glu His Gly
               260                 265                 270
   Trp Asp Asp Asp Gly Val Phe Asn Phe Glu Gly Gly Cys Tyr Ala Lys
           275                 280                 285
   Thr Ile Lys Leu Ser Lys Glu Ala Glu Pro Glu Ile Tyr Asn Ala Ile
       290                 295                 300
   Arg Arg Asp Ala Leu Leu Glu Asn Val Thr Val Arg Glu Asp Gly Thr
   305                 310                 315                 320
   Ile Asp Phe Asp Asp Gly Ser Lys Thr Glu Asn Thr Arg Val Ser Tyr
                   325                 330                 335
   Pro Ile Tyr His Ile Asp Asn Ile Val Lys Pro Val Ser Lys Ala Gly
               340                 345                 350
   His Ala Thr Lys Val Ile Phe Leu Thr Ala Asp Ala Phe Gly Val Leu
           355                 360                 365
   Pro Pro Val Ser Arg Leu Thr Ala Asp Gln Thr Gln Tyr His Phe Leu
   370 375 380
   Ser Gly Phe Thr Ala Lys Leu Ala Gly Thr Glu Arg Gly Ile Thr Glu
   385 390 395 400
   Pro Thr Pro Thr Phe Ser Ala Cys Phe Gly Ala Ala Phe Leu Ser Leu
   405 410 415
   His Pro Ile Gln Tyr Ala Glu Val Leu Val Lys Arg Met Gln Ala Ala
   420 425 430
   Gly Ala Gln Ala Tyr Leu Val Asn Thr Gly Trp Asn Gly Thr Gly Lys
   435 440 445
   Arg Ile Ser Ile Lys Asp Thr Arg Ala Ile Ile Asp Ala Ile Leu Asn
   450 455 460
   Gly Ser Leu Asp Asn Ala Glu Thr Phe Thr Leu Pro Met Phe Asn Leu
   465 470 475 480
   Ala Ile Pro Thr Glu Leu Pro Gly Val Asp Thr Lys Ile Leu Asp Pro
   485 490 495
   Arg Asn Thr Tyr Ala Ser Pro Glu Gln Trp Gln Glu Lys Ala Glu Thr
   500 505 510
   Leu Ala Lys Leu Phe Ile Asp Asn Phe Asp Lys Tyr Thr Asp Thr Pro
   515 520 525
   Ala Gly Ala Ala Leu Val Ala Ala Gly Pro Lys Leu
   530 535 540
<210> 155
   <211> 1178
   <212> PRT
   <213> Gallus gallus
<220>
   <221> misc_feature
   <222> (566) .. (566)
   <223> Xaa can be any naturally occurring amino acid
   <223> GglPYC
<400> 155
   Met Ser Gln Leu Cys Val Pro Arg Gly Gly Arg Ala Leu Leu Gly Ala
   1 5 10 15
   Trp Arg Leu Pro Leu Leu Arg Pro Pro Pro Gly Ser Val Arg Ser Ala
   20 25 30
   Ser Cys Gln Pro Ile Arg Lys Val Leu Val Ala Asn Arg Gly Glu Ile
   35 40 45
   Ala Ile Arg Val Phe Arg Ala Cys Thr Glu Leu Gly Leu Arg Thr Val
   50 55 60
   Ala Val Tyr Ser Glu Gln Asp Thr Gly Gln Met His Arg Gln Lys Ala
   65 70 75 80
   Asp Glu Ala Tyr Leu Val Gly Arg Gly Leu Pro Pro Val Gln Ala Tyr
   85 90 95
   Leu His Val Pro Asp Ile Ile Arg Val Ala Arg Glu Asn Ala Val Asp
   100 105 110
   Ala Ile His Pro Gly Tyr Gly Phe Leu Ser Glu Arg Ala Asp Phe Ala
   115 120 125
   Gln Ala Cys Val Asp Ala Gly Val Arg Phe Val Gly Pro Pro Pro Glu
   130 135 140
   Val Val Arg Lys Met Gly Asp Lys Val Glu Ala Arg Ser Ile Ala Ile
   145 150 155 160
   Ala Ala Gly Val Pro Val Val Pro Gly Thr Ser Ala Pro Val Ala Thr
   165 170 175
   Leu Gly Glu Ala Gln Asp Phe Ala Ala Arg Val Gly Phe Pro Ile Ile
   180 185 190
   Phe Lys Ala Ala His Gly Gly Gly Gly Arg Gly Met Arg Ala Val Arg
   195 200 205
   Gly Pro Gln Glu Leu Glu Glu Ser Phe Ser Arg Ala Ser Ser Glu Ala
   210 215 220
   Leu Ala Ala Phe Gly Asp Gly Ala Leu Phe Val Glu Lys Leu Met Glu
   225 230 235 240
   Arg Pro Arg His Ile Glu Gly Gln Ile Leu Gly Asp Lys His Gly Asn
   245 250 255
   Val Val His Leu Tyr Glu Arg Asp Cys Ser Ile Gln Arg Arg His Gln
   260 265 270
   Lys Val Val Glu Ile Ala Pro Ala Ala Arg Leu Asp Pro Gln Leu Arg
   275 280 285
   Ala Gln Leu Ala Ser Asp Ala Val Arg Ile Ala Gln Gln Val Gly Tyr
   290 295 300
   Glu Asn Ala Gly Thr Val Glu Phe Leu Val Asp Arg Asp Gly Lys His
   305 310 315 320
   Tyr Phe Ile Glu Val Asn Ser Arg Leu Gln Val Glu His Thr Val Thr
   325 330 335
   Glu Glu Ile Thr Gly Val Asp Leu Val Gln Ala Gln Leu Leu Val Ala
   340 345 350
   Ala Gly Arg Ser Leu Ser Glu Leu Gly Leu Gln Gln Asp Ser Val Arg
   355 360 365
   Val Asn Gly Cys Ala Ile Gln Cys Arg Val Thr Thr Glu Asp Pro Ala
   370 375 380
   Arg Gly Phe Gln Pro Asp Thr Gly Arg Ile Glu Val Phe Arg Ser Gly
   385 390 395 400
   Glu Gly Met Gly Ile Arg Leu Asp Gly Ala Ser Ala Phe Gln Gly Ala
   405 410 415
   Leu Ile Ser Pro His Tyr Asp Ser Leu Leu Val Lys Val Ile Ala His
   420 425 430
   Gly Pro Asp Gln Pro Ser Ala Ala Ala Lys Met Ser Arg Ala Leu Gly
   435 440 445
   Glu Phe Arg Ile Arg Gly Val Lys Thr Asn Ile Pro Phe Leu Gln Asn
   450 455 460
   Val Leu Ala His Pro Gln Phe Leu Gly Gly Ala Val Asp Thr Gln Phe
   465 470 475 480
   Ile Asp Glu Asn Pro Glu Leu Phe His Leu Arg Pro Ser Gln Asn Arg
   485 490 495
   Ala Gln Lys Leu Leu His Tyr Leu Gly His Val Met Val Asn Gly Pro
               500                 505                 510
   Ser Thr Pro Leu Pro Val Lys Ala Lys Ala Ala Val Val Glu Pro Val
           515                 520                 525
   Pro Pro Pro Val Pro Met Gly Ser Pro Pro Glu Gly Leu Arg Ala Val
       530                 535                 540
   Leu Gln Arg Glu Gly Pro Ala Gly Phe Ala Arg Ala Leu Arg Gly His
   545                 550                 555                 560
   Arg Gly Leu Leu Leu Xaa Asp Thr Thr Phe Arg Asp Ala His Gln Ser
                   565                 570                 575
   Leu Leu Ala Thr Arg Val Arg Thr Arg Asp Leu Ala Arg Ile Ala Pro
               580                 585                 590
   Phe Val Ala His Ser Leu Ser Pro Leu Cys Ser Met Glu Thr Trp Gly
           595                 600                 605
   Gly Ala Thr Phe Asp Val Ala Met Arg Phe Leu His Glu Cys Pro Trp
       610                 615                 620
   Glu Arg Leu Arg Glu Leu Arg Arg Leu Val Pro Asn Ile Pro Phe Gln
   625                 630                 635                 640
   Met Leu Leu Arg Gly Ala Asn Ala Val Gly Tyr Thr Asn Tyr Pro Asp
                   645                 650                 655
   Asn Val Ile Tyr Arg Phe Cys Glu Val Ala Ala Ala Asn Gly Met Asp
               660                 665                 670
   Ile Phe Arg Ile Phe Asp Ala Leu Asn Tyr Leu Pro Asn Leu Leu Leu
           675                 680                 685
   Gly Val Glu Ala Val Gly Arg Ala Gly Ala Val Val Glu Ala Ala Leu
       690                 695                 700
   Ser Tyr Thr Gly Asp Val Ala Asp Pro Thr Arg Thr Lys Tyr Ser Leu
   705                 710                 715                 720
   Asp Tyr Tyr Leu Gly Leu Ala Lys Glu Leu Val Ala Ala Gly Thr His
                   725                 730                 735
   Ile Leu Cys Ile Lys Asp Met Ala Gly Leu Leu Thr Pro Ala Ala Ala
   740 745 750
   Arg Leu Leu Val Ser Ser Leu Arg Asp Arg Phe Pro Asp Val Pro Ile
   755 760 765
   His Val His Thr His Asp Thr Ala Gly Ala Ala Ile Ala Thr Leu Leu
   770 775 780
   Ala Ala Ala Asn Ala Asp Ala Asp Val Val Asp Val Ala Val Asp Ala
   785 790 795 800
   Met Ser Gly Met Thr Ser Gln Pro Ser Met Gly Ala Leu Val Ala Cys
   805 810 815
   Ala Arg Gly Thr Pro Leu Asp Thr Gly Ile Ala Leu Glu Arg Val Phe
   820 825 830
   Glu Tyr Ser Glu Tyr Trp Glu Gly Ala Arg Gly Leu Tyr Ala Ala Phe
   835 840 845
   Asp Cys Thr Ala Thr Met Lys Ser Gly Asn Ala Asp Val Tyr Glu Asn
   850 855 860
   Glu Ile Pro Gly Gly Gln Tyr Thr Asn Leu His Phe Gln Ala His Ala
   865 870 875 880
   Met Gly Leu Gly His Lys Phe Lys Glu Val Lys Lys Ala Tyr Ala Glu
   885 890 895
   Ala Asn Lys Leu Leu Gly Asp Leu Ile Lys Val Thr Pro Ser Ser Lys
   900 905 910
   Val Val Gly Asp Leu Ala Gln Phe Met Val Gln Asn Gly Leu Ser Arg
   915 920 925
   Glu Glu Ala Glu Ala Arg Ala Asp Glu Leu Ser Phe Pro Leu Ser Val
   930 935 940
   Val Glu Phe Leu Gln Gly Tyr Ile Gly Thr Pro Pro Gly Gly Phe Pro
   945 950 955 960
   Glu Pro Phe Arg Ser Lys Val Leu Lys Asp Leu Pro Arg Val Glu Gly
   965 970 975
   Arg Pro Gly Ala Ser Leu Pro Pro Leu Asp Phe Glu Ala Leu Ser Gln
   980 985 990
   Glu Leu Gly Ala Arg Asp Gly Thr Pro Pro Ser Pro Glu Asp Leu Leu
   995 1000 1005
   Ser Ala Ala Leu Tyr Pro Lys Val Tyr Ala Glu Phe Arg Asp Phe
   1010 1015 1020
   Thr Ser Thr Phe Gly Pro Val Ser Cys Leu Gly Thr Arg Leu Phe
   1025 1030 1035
   Leu Glu Gly Pro Thr Ile Ala Glu Glu Phe Glu Val Glu Leu Glu
   1040 1045 1050
   Arg Gly Lys Thr Leu His Ile Lys Ala Leu Ala Leu Gly Asp Leu
   1055 1060 1065
   Asn Ala Ala Gly Gln Arg Glu Ala Phe Phe Glu Leu Asn Gly Gln
   1070 1075 1080
   Leu Arg Ser Ile Leu Val Arg Asp Thr Gln Ala Leu Lys Glu Met
   1085 1090 1095
   His Val His Pro Lys Ala Asp Arg Ser Ala Lys Gly Gln Val Gly
   1100 1105 1110
   Ala Pro Met Pro Gly Glu Val Val Glu Val Arg Val Lys Glu Gly
   1115 1120 1125
   Glu Ala Val Glu Lys Gly Ala Pro Leu Cys Val Leu Ser Ala Met
   1130 1135 1140
   Lys Met Glu Thr Val Val Thr Ala Pro Arg Gly Gly Thr Val Ser
   1145 1150 1155
   Arg Leu His Val Arg Pro Gly Met Ser Leu Glu Gly Asp Asp Leu
   1160 1165 1170
   Ile Ala Glu Ile Glu
   1175
<210> 156
   <211> 312
   <212> PRT
   <213> Escherichia coli
<220>
   <223> EcoMDH
<400> 156
   Met Lys Val Ala Val Leu Gly Ala Ala Gly Gly Ile Gly Gln Ala Leu
   1               5                   10                  15
   Ala Leu Leu Leu Lys Thr Gln Leu Pro Ser Gly Ser Glu Leu Ser Leu
               20                  25                  30
   Tyr Asp Ile Ala Pro Val Thr Pro Gly Val Ala Val Asp Leu Ser His
           35                  40                  45
   Ile Pro Thr Ala Val Lys Ile Lys Gly Phe Ser Gly Glu Asp Ala Thr
       50                  55                  60
   Pro Ala Leu Glu Gly Ala Asp Val Val Leu Ile Ser Ala Gly Val Ala
   65                  70                  75                  80
   Arg Lys Pro Gly Met Asp Arg Ser Asp Leu Phe Asn Val Asn Ala Gly
                   85                  90                  95
   Ile Val Lys Asn Leu Val Gln Gln Val Ala Lys Thr Cys Pro Lys Ala
               100                 105                 110
   Cys Ile Gly Ile Ile Thr Asn Pro Val Asn Thr Thr Val Ala Ile Ala
           115                 120                 125
   Ala Glu Val Leu Lys Lys Ala Gly Val Tyr Asp Lys Asn Lys Leu Phe
       130                 135                 140
   Gly Val Thr Thr Leu Asp Ile Ile Arg Ser Asn Thr Phe Val Ala Glu
   145                 150                 155                 160
   Leu Lys Gly Lys Gln Pro Gly Glu Val Glu Val Pro Val Ile Gly Gly
                   165                 170                 175
   His Ser Gly Val Thr Ile Leu Pro Leu Leu Ser Gln Val Pro Gly Val
               180                 185                 190
   Ser Phe Thr Glu Gln Glu Val Ala Asp Leu Thr Lys Arg Ile Gln Asn
           195                 200                 205
   Ala Gly Thr Glu Val Val Glu Ala Lys Ala Gly Gly Gly Ser Ala Thr
       210                 215                 220
   Leu Ser Met Gly Gln Ala Ala Ala Arg Phe Gly Leu Ser Leu Val Arg
   225                 230                 235                 240
   Ala Leu Gln Gly Glu Gln Gly Val Val Glu Cys Ala Tyr Val Glu Gly
                   245                 250                 255
   Asp Gly Gln Tyr Ala Arg Phe Phe Ser Gln Pro Leu Leu Leu Gly Lys
               260                 265                 270
   Asn Gly Val Glu Glu Arg Lys Ser Ile Gly Thr Leu Ser Ala Phe Glu
           275                 280                 285
   Gln Asn Ala Leu Glu Gly Met Leu Asp Thr Leu Lys Lys Asp Ile Ala
       290                 295                 300
   Leu Gly Glu Glu Phe Val Asn Lys
   305                 310
<210> 157
   <211> 38
   <212> DNA
   <213> Artificial Sequence
<220>
   <223> Description of Artificial Sequence: EcoPCK-F
<400> 157
   gacacttttt caaaatgcgc gttaacaatg gtttgacc 38
<210> 158
   <211> 32
   <212> DNA
   <213> Description of Artificial Sequence: EcoPCK-R
<400> 158
   gaaatcaact tttgttccag cttcggacca gc 32
<210> 159
   <211> 34
   <212> DNA
   <213> Description of Artificial Sequence: GglPYC-F
<400> 159
   catctttttc aaacatgtcg cagctgtgtg tccc 34
<210> 160
   <211> 37
   <212> DNA
   <213> Description of Artificial Sequence: GglPYC-R
<400> 160
   atcatcatca tccttgtaat cctcgatctc ggcgatg 37
<210> 161
   <211> 32
   <212> DNA
   <213> Description of Artificial Sequence: EcoMDH-F
<400> 161
   actttttcaa acatgaaagt cgcagtcctc gg 32
<210> 162
   <211> 43
   <212> DNA
   <213> Description of Artificial Sequence: EcoMDH-R
<400> 162
   atcatcatca tccttgtaat ccttattaac gaactcttcg ccc 43

## Description

### Technical Field

The present application discloses a transformant, a method for manufacturing the same, and a method for manufacturing a dicarboxylic acid having 4 carbon atoms (C4 dicarboxylic acid). More specifically, the present application discloses a transformant, which is obtained by incorporating one or more foreign genes of one or more kinds selected from the group consisting of a Phosphoenolpyruvate carboxykinase (hereinafter, referred to PCK as well) gene, a Pyruvate carboxylase (hereinafter, referred to as PYC as well) gene, and a Malate dehydrogenase (hereinafter, referred to as MDH as well) gene into Schizosaccharomyces pombe (hereinafter, referred to as S. pombe as well) and in which some of the genes in a group of Pyruvate decarboxylase (hereinafter, referred to as PDC as well)-encoding genes and Malic enzyme (hereinafter, referred to as MAE as well) genes have undergone deletion or deactivation, a method for manufacturing the transformant, and a method for manufacturing a C4 dicarboxylic acid in which the transformant is cultured in a culture solution and a C4 dicarboxylic acid is obtained from the culture solution.

### Background Art

Malic acid (HOOCCH(OH)CH₂COOH) is a dicarboxylic acid consisting of 4 carbon atoms (C4 dicarboxylic acid). Generally, malic acid is commercially manufactured through chemical synthesis from raw materials derived from petroleum or through microbial fermentation from renewable feedstocks.

Many natural or genetic recombinant microorganisms are known to be able to produce malic acid as the main fermentative organisms. For example, as a method of using wild-type microorganisms, a method of producing malic acid by culturing Aspergillus flavus (Non-Patent Document 1) and Penicillium sclerotiorum (Non-Patent Document 2) has been disclosed.

Regarding a microorganism whose malic acid producibility is improved by gene recombination, Patent Document 1 describes that a C4 dicarboxylic acid producibility is improved by introducing a C4 dicarboxylic acid transporter into filamentous bacteria such as Aspergillus oryzae.

In Patent Document 2, malic acid is produced using a transformant which is obtained by deleting a PDC gene from Saccharomyces cerevisiae and introducing a PYC gene or a Phosphoenolpyruvate carboxykinase (hereinafter, referred to PCK as well) gene, an MDH gene, and a malic acid transporter protein gene into the Saccharomyces cerevisiae.

In Non-Patent Document 3, malic acid is produced using a transformant which is obtained by deleting a PDC gene from Saccharomyces cerevisiae and introducing a PYC gene, an MDH gene, and a malic acid transporter protein gene into the Saccharomyces cerevisiae. In Patent Document 3, malic acid is produced using a transformant which is obtained by deleting a PDC enzyme gene, a pyruvate kinase (PYK) gene, and a hexokinase (HXK) gene from Saccharomyces cerevisiae and introducing a PYC gene, an MDH gene, a malic acid transporter protein gene, and a PCK gene into the Saccharomyces cerevisiae.

In Non-Patent Document 4, a transformant is manufactured whose malic acid producibility is improved by deactivating a plurality of enzymes in E. coli that is involved in pathways other than a pathway through which malic acid is produced from pyruvic acid. Non-Patent Document 4 discloses that between a deletion strain of a malic enzyme gene maeB and a non-deletion strain thereof, the maeB gene non-deletion strain has a higher malic acid production rate. The maeB gene of E. coli corresponds to a malic enzyme gene mae2 of S. Pombe.

### Citation List

### Patent Literature

[Patent Document 1] Published Japanese Translation No. 2013-503631 of the PCT International Publication
[Patent Document 2] Published Japanese Translation No. 2009-516526 of the PCT International Publication
[Patent Document 3] PCT International Publication No. WO2009/011974

### Non-patent Literature

[Non-Patent Document 1] Battat et al., Biotechnology and Bioengineering, 1991, vol. 37, p. 1108-1116.
[Non-Patent Document 2] Wang et al., Bioresource Technology, 2013, vol. 143, p. 674-677.
[Non-Patent Document 3] Zelle et al., Applied and Environmental Microbiology, 2008, vol. 74, p. 2766-2777.
[Non-Patent Document 4] Zhang et al., Applied and Environmental Microbiology, 2011, vol. 77, p. 427-434.

### Summary of Invention

### Technical Problem

All of the transformants disclosed in Patent Document 1 to 3 and Non-Patent Document 3 and 4 have a malic acid production rate of less than 1.0 g/(L·h). Therefore, the development of a microorganism having higher malic acid producibility is desired.

An object of the present invention is to provide a Schizosaccharomyces pombe transformant, which can excellently produce a C4 dicarboxylic acid through microbial fermentation from renewable feedstocks. A method for manufacturing the transformant is also described.

Another object of the present invention is to provide a method for manufacturing a C4 dicarboxylic acid including malic acid by using the transformant.

### Solution to Problem

A transformant according to the present invention is a transformant which uses Schizosaccharomyces pombe as a host and, into which one or more foreign genes of one or more kinds selected from the group consisting of a phosphoenolpyruvate carboxykinase gene and a pyruvate carboxylase gene are incorporated, and in which some of the genes in a group of pyruvate decarboxylase-encoding genes of the Schizosaccharomyces pombe host have undergone deletion or deactivation. The Phosphoenolpyruvate carboxykinase gene encodes a polypeptide which includes an amino acid sequence represented by any of SEQ ID NOS: 94 to 113, a polypeptide which includes an amino acid sequence obtained by substitution, addition, or deletion of one or a plurality of amino acids in an amino acid sequence represented by any of SEQ ID NOS: 94 to 113 and has a phosphoenolpyruvate carboxykinase activity, or a polypeptide which includes an amino acid sequence sharing a sequence identity of equal to or higher than 80% with an amino acid sequence represented by any of SEQ ID NOS: 94 to 113 and has phosphoenolpyruvate carboxykinase activity, the pyruvate carboxylase gene encodes a polypeptide which includes an amino acid sequence represented by any of SEQ ID NOS: 1 to 16, a polypeptide which includes an amino acid sequence obtained by the substitution, addition, or deletion of one or a plurality of amino acids in an amino acid sequence represented by any of SEQ ID NOS: 1 to 16 and has pyruvate carboxylase activity, or a polypeptide which includes an amino acid sequence sharing a sequence identity of equal to or higher than 80% with an amino acid sequence represented by any of SEQ ID NOS: 1 to 16 and has pyruvate carboxylase activity, and the pyruvate decarboxylase-endocing genes that have undergone deletion or deactivation are pdc2 genes.

It is preferable that one or more foreign malate dehydrogenase genes are further incorporated into the transformant according to the present invention.

It is preferable that in the transformant, the malate dehydrogenase genes are genes encoding a polypeptide which includes an amino acid sequence represented by any of SEQ ID NOS: 17 to 21, a polypeptide which includes an amino acid sequence obtained by the substitution, addition, or deletion of one or a plurality of amino acids in an amino acid sequence represented by any of SEQ ID NOS: 17 to 21 and has malate dehydrogenase activity, or a polypeptide which includes an amino acid sequence sharing sequence identity of equal to or higher than 80% with an amino acid sequence represented by any of SEQ ID NOS: 17 to 21 and has malate dehydrogenase activity.

It is preferable that in the transformant, malic enzyme genes have also undergone deletion or deactivation.

It is preferable that in the transformant, one or more foreign phosphoenolpyruvate carboxykinase genes or one or more foreign pyruvate carboxylase genes and one or more foreign malate dehydrogenase genes are incorporated, and all of the pdc2 genes and the malic enzyme gene have undergone deletion or deactivation.

In the transformant according to the present invention, the foreign genes are incorporated into a chromosome of the host.

A method for manufacturing a dicarboxylic acid having 4 carbon atoms according to the present invention includes culturing the transformant in a culture solution and obtaining a dicarboxylic acid having 4 carbon atoms from the cultured transformant or a culture supernatant.

It is preferable that in the method for manufacturing a dicarboxylic acid having 4 carbon atoms according to the present invention, the dicarboxylic acid having 4 carbon atoms is malic acid or oxaloacetic acid.

It is preferable that in the method for manufacturing a dicarboxylic acid having 4 carbon atoms according to the present invention, culturing is continued even after the pH of the culture solution becomes equal to or less than 3.5.
The invention is defined by the claims.

### Advantageous Effects of Invention

The schizosaccharomyces pombe transformant according to the present invention can produce a C4 dicarboxylic acid including malic acid at a high production rate that was not achieved in the related art.

The transformant can be obtained in a more simple manner by the method for manufacturing a transformant.

The method for manufacturing a C4 dicarboxylic acid according to the present invention makes it possible to manufacture a C4 dicarboxylic acid with higher produtivity through microbial fermentation.

### Brief Description of Drawings

FIG. 1 is a view showing a constitution of a monodentate integrative recombinant vector pSMh.
FIG. 2 is a view showing relative values of a PYC expression amount of 16 kinds of transformant into which a PYC gene is introduced.
FIG. 3 is a view showing relative PYC activity per enzyme liquid (mU/mL) of 8 kinds of transformant into which a PYC gene is introduced.
FIG. 4 is a view showing relative values of an MDH expression amount of 5 kinds of transformant into which an MDH gene is introduced.
FIG. 5 is a view showing MDH activity per enzyme liquid (mU/mL) of 3 kinds of transformant into which an MDH gene is introduced.
FIG. 6 is a view showing how a glucose concentration (g/L), an ethanol concentration (g/L), and a malic acid concentration (g/L) of a wild-type strain (ARC010 strain) change over time.
FIG. 7 is a view showing how a glucose concentration (g/L), an ethanol concentration (g/L), and a malic acid concentration (g/L) of an ASP4590 strain (Δpdc2) change over time.
FIG. 8 is a view showing how a glucose concentration (g/L), an ethanol concentration (g/L), and a malic acid concentration (g/L) of an ASP4491 strain (Δpdc2, +ScePYC, +DacMDH) change over time.
FIG. 9 is a view showing how a glucose concentration (g/L), an ethanol concentration (g/L), and a malic acid concentration (g/L) of an ASP4964 strain (Δpdc2, Δmae2, +ScePYC, +DacMDH) change over time.
FIG. 10 is a view showing how a glucose concentration (g/L), an ethanol concentration (g/L), and a malic acid concentration (g/L) of an ASP4933 strain (Δpdc2, Δfum1, +ScePYC, +DacMDH) change over time.
FIG. 11 is a view how a glucose concentration (g/L) and a malic acid concentration (g/L) of a culture solution of each of an ASP4491 strain and an ASP4892 strain change over time.
FIG. 12 is a view showing relative values of a PCK expression amount of 20 kinds of transformant into which a PCK gene is introduced.

### Description of Embodiments

### [Gene recombinant]

The transformant according described herein is a transformant which uses schizosaccharomyces pombe as a host, into which one or more foreign genes selected from the group consisting of a phosphoenolpyruvate carboxykinase (PCK) gene and a pyruvate carboxylase (PYC) gene are incorporated, and in which some of the genes in gene group pyruvate decarboxylase-encoding genes of the schizosaccharomyces pombe host have undergone deletion or deactivation.

In the present invention and the specification of the present application, a foreign gene means a structural gene which is not a structure gene inherent to a host (a structural gene contained in a chromosome of a natural-type host having not undergone transformation) but a structural gene introduced into a host through a transformation operation or the like. A gene inherent to a host is also included in a foreign gene of the present invention as long as the gene is introduced into the host through a transformation operation or the like.

Oxaloacetic acid is a sort of C4 dicarboxylic acid. In S. pombe, oxaloacetic acid is important as a raw material for the synthesis of other C4 dicarboxylic acids such as malic acid. In wild-type S. pombe, oxaloacetic acid is synthesized mainly through a pathway in which oxaloacetic acid is directly synthesized from phosphoenolpyruvic acid by PCK and synthesized by PYC through pyruvic acid. Therefore, if PCK activity and PYC activity are enhanced, an amount of oxaloacetic acid produced from S. pombe could be increased.

Pyruvic acid is important in ethanol fermentation. Pyruvic acid is converted into acetaldehyde by pyruvate decarboxylase, and then the acetaldehyde is converted into ethanol by alcohol dehydrogenase. That is, because pyruvic acid is used as a raw material of ethanol fermentation, in order to increase an amount of oxaloacetic acid produced, not only the enhancement of PCK activity and PYC activity, but also the inhibition of ethanol fermentation needs to be achieved.

Into the transformant according to the present invention, at least one of the foreign genes including the PCK gene and the PYC gene is incorporated. Furthermore, in the transformant according to the present invention, at least one of the PCK activity and the PYC activity is enhanced, some or the genes in a group of pyruvate decarboxylase-encoding genes have undergone deletion or deactivation, and ethanol fermentation efficiency is reduced.

That is, in the transformant according to the present invention, ethanol fermentation is inhibited, and an amount of pyruvic acid usable as a matrix of PYC is increased. Therefore, the transformant has excellent oxaloacetic acid producibility and excellent producibility of other C4 dicarboxylic acids synthesized from oxaloacetic acid.

In wild-type S. pombe, through a pathway in which aerobic pyruvic acid is converted into acetyl CoA, citric acid, succinic acid, and the like, malic acid is produced through fumaric acid hydration by fumarate dehydrase. Oxaloacetic acid is produced by anaerobic PYC, and malic acid is produced from the oxaloacetic acid by malate dehydrogenase (MDH). In the transformant according to the present invention, an amount of oxaloacetic acid produced is increased. Therefore, the transformant also has excellent malic acid producibility.

Into the transformant according to the present invention, at least one PCK gene or at least one PYC gene should be introduced as a foreign gene. The number of PYC genes to be introduced is not limited to 1 and may be equal to or greater than 2. The more the foreign genes to be introduced, the higher then PYC activity of the obtained transformant.

In a case where 2 or more PYC genes are introduced, PYC genes of the same kind may be introduced, or two or more kinds of PYC genes may be introduced. Similarly, into the transformant according to the present invention, 1 PCK gene may be introduced, or 2 or more PCK genes of the same kind or different kinds may be introduced.

In order to cause the transformant according to the present invention to have particularly high malic acid producibility, it is preferable to inhibit the decomposition of malic acid by enhancing MDH activity.

Specifically, a transformant is preferable which is obtained by incorporating at least one of the foreign genes including a PCK gene and a PYC gene and one or more foreign MDH genes into S. pombe as a host, and in which some of the genes in a group of pyruvate decarboxylase-encoding genes and malic enzyme genes have undergone deletion or deactivation. The aforementioned transformant is a transformant into which foreign genes such as at least either the PCK gene or the PYC gene and the MDH genes are incorporated, and in which at least either the PCK activity or the PYC activity and the MDH activity are enhanced, some of the genes in a group pyruvate decarboxylase-encoding genes have undergone deletion or deactivation, and ethanol fermentation efficiency is reduced. That is, in the transformant, malic acid fermentation is accelerated while ethanol fermentation is inhibited. Therefore, the transformant has excellent malic acid producibility.

In a case where at least 1 MDH gene is introduced as a foreign gene into the transformant according to the present invention, 1 MDH gene or 2 or more MDH genes of the same kind or different kinds may be introduced into the transformant according to the present invention.

It is preferable that at least 1 PCK gene, at least 1 PYC gene, and at least 1 MDH gene are introduced as foreign genes into the transformant according to the present invention. By the incorporation of foreign genes such as the PCK gene, the PYC gene, and the MDH gene, malic acid producibility is further improved.

### <S. pombe>

S. pombe used as a host is yeast (fission yeast) of the genus Schizosaccharomyces, and is a microorganism having particularly excellent acid resistance compared to other yeasts. Because S. pombe inherently has an mae1 (C4 dicarboxylic acid transporter) gene, even in a case where an amount of C4 dicarboxylic acid produced in the microbial cells is increased, an excess of C4 dicarboxylic acid is inhibited from affecting the growth or the like of the microbial cells. Therefore, compared to other yeasts not having the Mae1 gene, such as Saccharomyces cerevisiae, S. pombe is suitable for producing a C4 dicarboxylic acid.

The entire base sequence of a chromosomes of S. pombe has been publicized by being listed as "Schizosaccharomyces pombe Gene DB (http://www.genedb.org/genedb/pombe/)" in the database "Gene DB" of Sanger Institute. The gene sequence data of S. pombe described in the present specification can be obtained through the search by the gene name or the aforementioned strain name from the database described above.

### <Pyruvate decarboxylase-encoding gene>

The group of pyruvate decarboxylase-encoding genes (pyruvate decarboxylase genes, hereinafter, referred to as "PDC genes" as well) in S. pombe consists of 4 kinds of genes including a gene encoding pyruvate decarboxylase 1 (hereinafter, referred to as a "PDC1 gene"), a gene encoding pyruvate decarboxylase 2 (hereinafter, referred to as a "PDC2 gene"), a gene encoding pyruvate decarboxylase 3 (hereinafter, referred to as a "PDC3 gene"), and a gene encoding pyruvate decarboxylase 4 (hereinafter, referred to as a "PDC4 gene"). Among these, the PDC2 gene and the PDC4 gene are PDC genes that play a key functional role in S. pombe.

The strain name of each of the PDC genes is as follows.
PDC1 gene (pdc1); SPAC13A11. 06
PDC2 gene (pdc2); SPAC1F8. 07c
PDC3 gene (pdc3); SPAC186. 09
PDC4 gene (pdc4); SPAC3G9. 11c

The PDC gene sequence data can be obtained through the search by the gene name or the strain name from the aforementioned S. pombe gene database.

The transformant according to the present invention has a chromosome in which some of the genes in a group of pyruvate decarboxylase-encoding genes have undergone deletion or deactivation. Due to the deletion or deactivation of some of the genes in the group of PDC genes of the transformant, the ethanol fermentation efficiency of the transformant is reduced, and the amount of pyruvic acid to be converted into ethanol is decreased. Therefore, the productivity of a C4 dicarboxylic acid including malic acid is improved. Here, if the group of PDC genes is totally deleted or deactivated, ethanol fermentation is not performed at all, and the growth of the transformant is inhibited. Accordingly, only some of the genes in the group of PDC genes should be deleted or deactivated.

The PDC genes to be deleted or deactivated are particularly preferably PDC2 genes. The PDC2 genes are PDC genes that particularly play a key functional role. An amino acid sequence of PDC2 (SpoPDC2) encoded by the PDC2 genes of S. pombe is represented by SEQ ID NO: 22.

As described above, if all of the PDC genes are deleted or deactivated, the transformant does not perform ethanol fermentation, and thus the growth of the transformant is hindered. Therefore, the deletion or deactivation of the PDC genes must be performed by maintaining an ethanol fermentation ability necessary for the growth so as to obtain a sufficient amount of transformant and simultaneously by lowering the ethanol fermentation ability so as to improve fermentation efficiency of a C4 dicarboxylic acid.

In order to delete or deactivate the PDC genes as described above, the inventors of the present invention conducted investigation. As a result, they found that if PDC2 genes are deleted or deactivated, PDC4 genes are activated to some extent, and the ethanol fermentation ability enough for obtaining a sufficient amount of transformant and the production of a C4 dicarboxylic acid with high fermentation efficiency can be accomplished simultaneously.

### <Malic enzyme gene>

A malic enzyme-endcoding gene (malic enzyme gene, hereinafter, referred to as an "mae gene" as well) in S. pombe is a gene encoding malic enzyme 2 (hereinafter, referred to as an "mae2 gene"). The strain name of the mae2 gene is as follows.
mae2 gene (mae2); SPCC794. 12c

The mae2 gene sequence data can be obtained through the search by the gene name or the strain name from the aforementioned S. pombe gene database. An amino acid sequence of mae2 (Spomae2) encoded by the mae2 gene of S. pombe is represented by SEQ ID NO: 23.

In the wild-type S. pombe, some of malic acids produced are converted into pyruvic acid by mae2. In the transformant according to the present invention in which the mae2 gene has undergone deletion or deactivation, malic acid produced in the transformant is not converted into pyruvic acid, and hence the amount of malic acid produced significantly increases.

### <Deletion or deactivation of gene>

The deletion or deactivation of the PDC genes and the mae2 genes can be performed by a known method. For example, by using a Latour method (described in the journal of Nucleic Acids Research, 2006, Vol. 34, p. e11, PCT International Publication No. WO2007/063919, and the like), the PDC genes and the like can be deleted.

Furthermore, through the deletion, insertion, substitution, or addition induced in a portion of a base sequence of the PDC genes and the like, the PDC genes and the like can be deactivated. Only one of the mutations including deletion, insertion, substitution, and addition may be induced, or two or more mutations among the above may be induced.

As the method for introducing the mutation into a portion of the PDC genes and the like, a known method can be used.

For example, a mutation separation method using a mutagen (Experimental Method of Yeast Molecular Genetics, 1996, Gakkai Shuppan Center) and a random mutation method using a polymerase chain reaction (PCR) (the journal of PCR Methods Application, Vol. 2, pp. 28-33, 1992) can be used.

The PDC genes that carry the mutation introduced into a portion thereof may be genes expressing temperature-sensitive mutant-type pyruvate decarboxylase. The temperature-sensitive mutant-type pyruvate decarboxylase is an enzyme which shows activity equivalent to the activity of wild-type pyruvate decarboxylase at a certain culture temperature but undergoes the loss or deterioration of the activity at a temperature equal to or higher than a specific culture temperature.

A mutant strain expressing the mutant-type pyruvate decarboxylase can be obtained by being selected from strains whose growth rate is equivalent to the growth rate of the wild-type yeast under the conditions in which the activity is not limited by the temperature but is greatly reduced under specific temperature conditions in which the activity is limited.

Similarly, the mae2 genes that carry the mutation introduced into a portion thereof may be genes expressing temperature-sensitive mutant-type mae2.

The deletion or deactivation of genes does not mean only the deletion or deactivation of structural genes. Not only a case where structural genes are deleted, but also a case where even if a protein is encoded by a structural gene and the structural gene is expressed, the protein is not an enzyme having activity means that the genes are deactivated.

In a case where even if a structural domain of a gene encodes an enzyme having activity, a protein of the structural domain of the gene is not expressed due to the deletion of genes encoding a regulatory domain or the mutation of the sequence of the genes, it means that the genes are "deleted or deactivated". Therefore, the PDC genes or the mae2 genes to be deleted or deactivated may be either or both of a structural domain and a regulatory domain of the PDC genes or the mae2 genes.

### <PYC gene>

The PYC genes introduced as foreign genes into the transformant according to the present invention should be structural genes which can express a protein performing PYC activity in a case where the structural genes are introduced into S. pombe, and may be derived from any of the biospecies.

Examples of PYC encoded by the PYC genes introduced as foreign genes include PYC derived from Aspergillus niger (AniPYC) (AC. No. : CAC19838. 1.) (SEQ ID NO: 1), PYC derived from Brevibacillus brevis (BbrPYC) (SEQ ID NO: 2), PYC derived from Debaryomyces hansenii (DhaPYC) (AC. No. : CAG86153. 2.) (SEQ ID NO: 3), PYC derived from Kluyveromyces lactis (KlaPYC) (SEQ ID NO: 4), PYC derived from Lachancea thermotolerans (LthPYC) (SEQ ID NO: 5), PYC derived from Lodderomyces elongisporus (LelPYC) (SEQ ID NO: 6), PYC derived from Saccharomyces cerevisiae (ScePYC) (SEQ ID NO: 7), PYC derived from Candida orthopsilosis (CorPYC) (AC. No. : CCG23801. 1.) (SEQ ID NO: 8), PYC derived from Candida tropicalis (CtrPYC) (AC. No. : EER35520. 1.) (SEQ ID NO: 9), PYC derived from Naumovozyma castellii (NcaPYC) (AC. No. : CCC71457. 1.) (SEQ ID NO: 10), PYC derived from Naumovozyma dairenensis (NdaPYC) (AC. No. : CCD26740. 1.) (SEQ ID NO: 11), PYC derived from Saccharomyces kudriavzevii (SkuPYC) (AC. No. :EJT41260. 1.) (SEQ ID NO: 12), PYC derived from S. pombe (SpoPYC) (AC. No. :BAA11239. 1., CAB52809. 1.) (SEQ ID NO: 13), PYC derived from Tetrapisispora blattae (TblPYC) (AC. No. :CCH58779. 1.) (SEQ ID NO: 14), PYC derived from Torulaspora delbrueckii (TdePYC) (AC. No. :CCE93722. 1.) (SEQ ID NO: 15), and PYC derived from Zygosaccharomyces rouxii (ZroPYC) (AC. No. :CAR26934. 1.) (SEQ ID NO: 16).

The PYC genes may be genes encoding a polypeptide which includes an amino acid sequence obtained by the substitution, addition, or deletion of one or a plurality of amino acids in amino acid sequences (SEQ ID NOS: 1 to 16) of the above PYCs and has PYC activity.

The PYC genes may be genes encoding a polypeptide which includes an amino acid sequence sharing a sequence identity of equal to or higher than 80%, preferably equal to or higher than 85%, more preferably equal to or higher than 90%, and even more preferably equal to or higher than 95% with the amino acid sequences (SEQ ID NOS: 1 to 16) of the above PYCs and has PYC activity.

In the specification of the present application, "AC. No." means an accession number of a database GenBank of National Center for Biotechnology Information (NCBI).

In the present invention and the specification of the present application, "plurality of amino acids" mean 2 to 20 amino acids and are preferably 2 to 10 amino acids.

The PYC genes introduced into the transformant according to the present invention are preferably genes encoding PYC which is the same as BbrPYC, KlaPYC, LelPYC, ScePYC, SpoPYC, or TblPYC or has an amino acid sequence similar to that of the above PYCs.

Specifically, the PYC genes are preferably genes encoding a polypeptide which includes amino acid sequences represented by SEQ ID NOS: 2, 4 to 7, and 12 to 14, a polypeptide which includes an amino acid sequence obtained by the substitution, addition, or deletion of one or a plurality of amino acids in amino acid sequences represented by SEQ ID NOS: 2, 4 to 7, and 12 to 14 and has PYC activity, or a polypeptide which includes an amino acid sequence sharing a sequence identity of equal to or higher than 80% with amino acid sequences represented by SEQ ID NOS: 2, 4 to 7, and 12 to 14 and has PYC activity.

The PYC genes are more preferably a polypeptide which is represented by SEQ ID NO: 7, a polypeptide which includes an amino acid sequence obtained by the substitution, addition, or deletion of one or a plurality of amino acids in an amino acid sequence represented by SEQ ID NO: 7 and has PYC activity, or a polypeptide which includes an amino acid sequence sharing a sequence identity of equal to or higher than 80% with an amino acid sequence represented by SEQ ID NO: 7 and has PYC activity. The PYC genes are even more preferably a polypeptide which is represented by SEQ ID NO: 7 or a polypeptide which includes an amino acid sequence obtained by the substitution, addition, or deletion of one or a plurality of amino acids in an amino acid sequence represented by SEQ ID NO: 7 and has PYC activity.

### <MDH gene>

The MDH genes introduced as foreign genes into the transformant according to the present invention should be structural genes which can express a protein performing MDH activity in a case where the structural genes are introduced into S. pombe, and may be derived from any biospecies.

Examples of MDH encoded by the MDH genes introduced as foreign genes include MDH derived from Archaeoglobus fulgidus (AfuMDH) (SEQ ID NO: 17), MDH derived from Congregibacter litoralis (CliMDH) (SEQ ID NO: 18), MDH derived from Delftia acidovorans (DacMDH) (SEQ ID NO: 19), MDH derived from Halomonas elongata (HelMDH) (SEQ ID NO: 20), and MDH derived from Shewanella putrefaciens (SpuMDH) (SEQ ID NO: 21).

The MDH genes may be genes encoding a polypeptide which includes an amino acid sequence obtained by the substitution, addition, or deletion of one or a aplurality of amino acids in amino acid sequences (SEQ ID NOS: 17 to 21) of the above MDHs and has MDH activity. Furthermore, the MDH genes may be genes encoding a polypeptide which includes an amino acid sequence sharing a sequence identity of equal to or higher than 80%, preferably equal to or higher than 85%, more preferably equal to or higher than 90%, and even more preferably equal to or higher than 95% with amino acid sequences (SEQ ID NOS: 17 to 21) of the above MDHs and has MDH activity.

The MDH genes introduced into the transformant according to the present invention are preferably genes encoding MDH which is the same as CliMDH, DacMDH, or HelMDH or has an amino acid sequence similar to that of the above MDHs.

Specifically, the MDH genes are preferably genes encoding a polypeptide which includes amino acid sequences represented by SEQ ID NOS: 18 to 20, a polypeptide which includes an amino acid sequence obtained by the substitution, addition, or deletion of one or a plurality of amino acids in amino acid sequences represented by SEQ ID NOS: 18 to 20 and has MDH activity, or a polypeptide which includes an amino acid sequence sharing a sequence identity of equal to or higher than 80% with amino acid sequences represented by SEQ ID NOS: 18 to 20 and has MDH activity.

The MDH genes are more preferably genes encoding a polypeptide represented by SEQ ID NO: 19, a polypeptide which includes an amino acid sequence obtained by the substitution, addition, or deletion of one or a plurality of amino acids in an amino acid sequence represented by SEQ ID NO: 19 and has MDH activity, or a polypeptide which includes an amino acid sequence sharing a sequence identity of equal to or higher than 80% with an amino acid sequence represented by SEQ ID NO: 19 and has MDH activity. The MDH genes are even more preferably a polypeptide represented by SEQ ID NO: 19 or a polypeptide which includes an amino acid sequence obtained by the substitution, addition, or deletion of one or a plurality of amino acids in an amino acid sequence represented by SEQ ID NO: 19 and has MDH activity.

### <PCK genes>

The PCK genes introduced as foreign genes into the transformant according the present invention should be structural genes which can express a protein performing PCK activity in a case where the structural genes are introduced into S. pombe, and may be derived from any biospecies.

Examples of PCK encoded by the PCK genes introduced as foreign genes include PCK derived from Candida glabrata (CglPCK) (AC. No. :CAG60017. 1) (SEQ ID NO: 94), PCK derived from Citrobacter koseri (CkoPCK) (AC. No. :KGY18702. 1) (SEQ ID NO: 95), PCK derived from Cronobacter sakazakii (CsaPCK) (AC. No. :EGL73852. 1) (SEQ ID NO: 96), PCK derived from Debaryomyces hansenii (DhaPCK) (AC. No. :CAG88379. 1) (SEQ ID NO: 97), PCK derived from Escherichia fergusonii (EfePCK) (AC. No. :EGC96802. 1) (SEQ ID NO: 98), PCK derived from Edwardsiella tarda (EtaPCK) (Ac. No. :GAC66070. 1) (SEQ ID NO: 99), PCK derived from Kluyveromyces lactis (KlaPCK) (AC. No. :AAC27661. 1) (SEQ ID NO: 100), PCK derived from Lodderomyces elongisporus (LelPCK) (Ac. No. :EDK41877. 1) (SEQ ID NO: 101), PCK derived from Pectobacterium carotovorum (PcaPCK) (Ac. No. :ACT14911. 1) (SEQ ID NO: 102), PCK derived from Photobacterium leiognathi (PlePCK) (Ac. No. :GAA03015. 1) (SEQ ID NO: 103), PCK derived from Providencia rettgeri (PrePCK) (Ac. No. :EFE52670. 1) (SEQ ID NO: 104), PCK derived from Saccharomyces cerevisiae (ScePCK) (Ac. No. :CAA31488. 1) (SEQ ID NO: 105), PCK derived from Serratia odorifera (SodPCK) (Ac. No. :EFE97343. 1) (SEQ ID NO: 106), PCK derived from Vibrio orientalis (VorPCK) (Ac. No. :EGU52194. 1) (SEQ ID NO: 107), PCK derived from Vanderwaltozyma polyspora (VpoPCK) (Ac. No. :EDO17884. 1) (SEQ ID NO: 108), PCK derived from Vibrio tubiashii (VtuPCK) (Ac. No. :EIF04922. 1) (SEQ ID NO: 109), PCK derived from Yersinia bercovieri (YbePCK) (Ac. No. :EEQ07887. 1) (SEQ ID NO: 110), PCK derived from Yarrowia lipolytica (YliPCK) (Ac. No. :CAG82248. 1) (SEQ ID NO: 111), PCK derived from Yersinia rohdei (YroPCK) (Ac. No. :AJJ11585. 1) (SEQ ID NO: 112), and PCK derived from Zygosaccharomyces rouxii (ZroPCK) (Ac. No. :CAR26716. 1) (SEQ ID NO: 113).

The PCK genes may be genes encoding a polypeptide which includes an amino acid sequence obtained by the substitution, addition, or deletion of one or a plurality of amino acids in amino acid sequences (SEQ ID NOS: 94 to 113) of the above PCKs and has PCK activity. Furthermore, the PCK genes may be genes encoding a polypeptide which includes an amino acid sequence sharing a sequence identity of equal to or higher than 80%, preferably equal to or higher than 85%, more preferably equal to or higher than 90%, and even more preferably equal to or higher than 95% with amino acid sequences (SEQ ID NOS: 94 to 113) of the above PCKs and has PCK activity.

As PCK, PYC, and MDH having high activity, PCK derived from Escherichia coli (EcoPCK) (AC. No. :AAA58200. 1) (SEQ ID NO: 154), PYC derived from Gallus gallus (GglPYC) (AC. No. :AAM92771. 1.) (SEQ ID NO: 155), and MDH derived from Escherichia coli (EcoMDH) (AC. No. :AAA58038. 1) (SEQ ID NO: 156) are known.

### [Manufacturing of transformant]

The transformant according to the present invention is obtained by using S. pombe, in which some of the genes in a group of PDC genes have undergone deletion or deactivation, as a host and introducing at least one of the foreign genes including a PCK gene and a PYC gene into the host by a genetic engineering method. Furthermore, the transformant according to the present invention can also be obtained by deleting or deactivating some of the genes in a group of PDC genes of a transformant which is obtained by introducing at least one of the foreign genes including a PCK gene and a PYC gene into S. pombe used as a host by a genetic engineering method.

The transformant according to the present invention into which foreign MDH genes are introduced and in which mae2 genes have undergone deletion or the like can be obtained by using S. pombe, in which some of the genes in a group of PDC genes have undergone deletion or deactivation, as a host, introducing at least one of the foreign genes including a PCK gene and a PYC gene and at least one foreign MDH gene into the host by a genetic engineering method, and deleting or deactivating an mae2 gene of the host.

In addition, S. pombe in which some of the genes in a group of PDC genes have undergone deletion or deactivation may be used as a host, an mae2 gene of the host may be deleted or deactivated, and then at least one of the foreign genes including a PCK gene and a PYC gene and at least one foreign MDH gene may be incorporated into the host by a genetic engineering method.

Furthermore, the transformant according to the present invention can also be obtained by deleting or deactivating some of the genes in a group of PDC genes and an mae2 gene of a transformant which is obtained by introducing at least one of the foreign genes including a PCK gene and a PYC gene and at least one foreign MDH gene into S. pombe used as a host by genetic engineering method. In a case where a plurality of foreign genes is introduced, all of the foreign genes may be introduced into the host at the same time or sequentially introduced into the host (in random order).

The method for manufacturing the transformant will be described based on, for example, a method of using S. pombe, in which some of the genes in a group of PDC genes have undergone deletion or deactivation, as a host, introducing a PYC gene and an MDH gene into the host, and then deleting or deactivating an mae2 gene.

### <Host>

The S. pombe used as a host may be a wild type or a mutant type in which specific genes have undergone deletion or deactivation according to the purpose. As a method for deleting or deactivating the specific genes, a known method can be used. Specifically, by using the Latour method described above, the genes can be deleted.

Furthermore, by a mutation separation method using a mutagen (Experimental Method of Yeast Molecular Genetics, 1996, Gakkai Shuppan Center), a random mutation method using PCR (the journal of PCR Methods Application, Vol. 2, pp. 28-33, 1992), and the like, a mutation is introduced into some of the genes, thereby deactivating the genes. The yeast host of the genus Schizosaccharomyces in which specific genes have undergone deletion or deactivation is described in, for example, PCT International Publication No. WO2002/101038 and PCT International Publication No. WO2007/015470.

The portion in which the deletion or deactivation of specific genes is performed may be an open reading frame (ORF) portion or an expression regulatory sequence portion. It is particularly preferable to use a deletion or deactivation method by a PCR-mediated homologous recombination method (the journal of Yeast, Vol. 14, pp. 943-951, 1998) in which an ORF portion of a structural gene is substituted with a marker gene.

A mutant in which PDC genes have undergone deletion or deactivation can be preferably used as a host for manufacturing the transformant according to the present invention. Furthermore, the S. pombe, in which the PDC genes and specific genes other than PDC genes have undergone deletion or deactivation, can also be used as a host. By the deletion or deactivation of a protease gene and the like, the expression efficiency of heterologous proteins can be improved, and if the host obtained in this way is used as a host in the present invention, the production efficiency of a C4 dicarboxylic acid such as malic acid could be improved.

As S. pombe used as a host, it is preferable to use those having a marker for selecting a transformant. For example, it is preferable to use a host which essentially requires a specific nutritional component for growth due to the lack of certain genes. In a case where a transformant is prepared by transformation using a vector including a target gene sequence, if the lacked gene (complementary auxotrophic marker) is incorporated in advance into the vector, the auxotrophy of the host disappears in the transformant. By the difference in auxotrophy between the host and the transformant, it is possible to make a differentiation between a host and a transformant and to obtain a transformant.

For example, by using S. pombe, which becomes uracil auxotrophic due to the deletion or deactivation of an orotidine phosphate decarboxylase gene (ura4 gene), as a host, performing transformation using a vector having a ura4 gene (complementary auxotrophic marker), and then selecting a transformant in which uracil auxotrophy has disappeared, a transformant into which the vector is incorporated can be obtained. The missing gene that makes the host auxotrophic is not limited to the ura4 gene as long as the gene can be used for selecting a transformant, and may be an isopropylmalate dehydrogenase gene (leu1 gene) or the like.

In addition, the S. pombe in which a group of PDC genes have not undergone deletion or deactivation can be used as a host for manufacturing a transformant. In this case, as a host, it is possible to use a host in which the aforementioned gene (an auxotrophic marker, a protease gene, or the like) other than the PDC genes has undergone deletion or deactivation. By manufacturing a transformant by using the host and then deleting or deactivating some of the genes in a group of PDC genes of the obtained transformant, the transformant according to the present invention can be obtained.

### <Method for introducing foreign genes>

As a method for introducing foreign genes into a host by a genetic engineering method, a known method can be used. As a method in which S. pombe is used as a host and structural genes of heterologous proteins are introduced into the host, for example, it is possible to use the methods described in Japanese Unexamined Patent Application, First Publication No. H05-15380, PCT International Publication No. WO95/09914, Japanese Unexamined Patent Application, First Publication No. H10-234375, Japanese Unexamined Patent Application, First Publication No. 2000-262284, Japanese Unexamined Patent Application, First Publication No. 2005-198612, PCT International Publication No. WO2011/021629, and the like.

### <Expression cassette>

An expression cassette is a combination of DNA necessary for expressing a target protein, and includes a structural gene which encodes the target protein and a promoter and a terminator which function in a host. An expression cassette used for manufacturing the transformant according to the present invention includes at least either a PYC gene or an MDH gene as well as a promoter and a terminator which function in S. pombe.

The expression cassette may include any one or more domains among a 5'-untraslated domain and a 3'-untraslated domain. Furthermore, the cassette may include the aforementioned complementary auxotrophic marker. A plurality of foreign genes may be present in a single expression cassette. The number of foreign genes in a single expression cassette is preferably 1 to 8, and more preferably 1 to 5.

In a case where a plurality of foreign genes is included in a single expression cassette, the cassette may include two or more kinds of foreign genes. As the expression cassette, an expression cassette is preferable which includes one or a plurality of PYC genes and MDH genes, a promoter, a terminator, a 5'-untraslated domain, a 3'-untraslated domain, and a complementary auxotrophic marker.

In manufacturing the transformant according to the present invention, the PYC genes and the MDH genes may be introduced into the host by different expression cassettes or by a single expression cassette. As the expression cassette including the PYC genes and the MDH genes, for example, an expression cassette is preferable which includes a promoter, PYC genes, a cleavage sequence, a complementary auxotrophic marker (for example, a Ura4 gene), MDH genes, and a terminator from the 5' terminal side.

As a gene sequence of the PYC genes or the MDH genes included in the expression cassette, a gene encoded by a wild type may be used as it is. In order to increase the expression amount in S. pombe used as a host, the gene sequence of the wild type may be modified into a codon used at a high frequency in S. pombe.

The promoter and the terminator functioning in S. pombe should be able to function in a transformant and maintain the expression of a protein encoded by foreign genes, even if an acidic condition (pH of equal to or less than 6) is created due to the accumulation of a C4 dicarboxylic acid by the transformant according to the present invention. As the promoter functioning in S. pombe, it is possible to use a promoter (preferably a promoter having high transcription initiation activity) inherent to S. pombe or a promoter (such as a promoter derived from a virus) not being inherent to S. pombe. Herein, two or more kinds of promoter may be present in a vector.

Examples of the promoter inherent to S. pombe include an alcohol dehydrogenase gene promoter, an nmt1 gene promoter involved in the thiamine metabolism, fructose-1,6-bisphosphatase gene promoter involved in the glucose metabolism, an invertase gene promoter involved in the catabolite repression (see PCT International Publication No. WO99/23223), a heat-shock protein gene promoter (see PCT International Publication No. WO2007/26617), and the like.

Examples of the promoter not being inherent to S. pombe include promoters derived from animal cell viruses, described in Japanese Unexamined Patent Application, First Publication No. H05-15380, Japanese Unexamined Patent Application, First Publication No. H07-163373, and Japanese Unexamined Patent Application, First Publication No. H10-234375. As such promoters, an hCMV promoter and an SV40 promoter are preferable.

As the terminator functioning in S. pombe, it is possible to use a terminator inherent to S. pombe or a terminator not being inherent to S. pombe. Herein, two or more kinds of terminator may be present in a vector.

Examples of the terminator include terminators derived from human beings, described in Japanese Unexamined Patent Application, First Publication No. H05-15380, Japanese Unexamined Patent Application, First Publication No. H07-163373, and Japanese Unexamined Patent Application, First Publication No. H10-234375. As such terminators, a terminator of human lipocortin I is preferable.

### <Vector>

The transformant described herein has an expression cassette, which includes foreign genes, in a chromosome or has an expression cassette as an extrachromosomal gene. Having the expression cassette in a chromosome means a state where the expression cassette is incorporated into one or more sites in a chromosome of the host cell. Having the expression cassette as an extrachromosomal gene means a state where the transformant has a plasmid including the expression cassette in a cell. The transformant having each expression cassette is obtained by transforming S. pombe as a host by using a vector including each expression cassette.

The vector can be manufactured by incorporating the expression cassette into a vector having a cyclic DNA structure or a linear DNA structure. In a case where a transformant in which the expression cassette is retained as an extrachromosomal gene in a host cell is prepared, the vector is preferably a plasmid including a sequence to be replicated in the host cell, that is, an Autonomously Replicating Sequence (ARS).

In contrast, in a case where a transformant in which the expression cassette is incorporated into a chromosome of a host cell is prepared, the vector is preferably a vector which has a linear DNA structure, does not have ARS, and is introduced into the host cell. For example, the vector may be a vector consisting of linear DNA or a vector having a cyclic DNA structure that has a restriction enzyme recognition sequence for cutting and opening the vector into linear DNA when being introduced into the host. In a case where the vector is a plasmid having ARS, a linear DNA structure can be established by deleting the ARS portion or by deactivating the function of ARS by cleaving the ARS portion, and then the vector can be introduced into a host.

The vector preferably has a marker for selecting a transformant. Examples of the marker include a ura4 gene (complementary auxotrophic marker) and an isopropylmalate dehydrogenase gene (leu1 gene).

Each foreign gene is preferably introduced into a chromosome of S. pombe. By the introduction of the foreign gene into the chromosome, a transformant which is excellently stably maintained in a passage is obtained. Furthermore, a plurality of foreign genes can be introduced into the chromosome. In the transformant according to the present invention, the number of PYC genes incorporated into a chromosome is preferably 1 to 20 and particularly preferably 1 to 8. In addition, the number of MDH genes incorporated into a chromosome of the transformant is preferably 1 to 20 and particularly preferably 1 to 8.

As a method for introducing foreign genes into a chromosome, a known method can be used. For example, by the method described in Japanese Unexamined Patent Application, First Publication No. 2000-262284, a plurality of foreign genes can be introduced into a chromosome. By the same method, a single foreign gene can be introduced into a chromosome. Furthermore, as will be described later, one or a plurality of foreign genes can be introduced into a plurality of sites of a chromosome.

As a method for introducing foreign genes into a chromosome of S. pombe, a method is preferable in which the foreign genes are introduced by a homologous recombination method by using a vector having an expression cassette, which has the foreign genes, and a recombination site.

The recombination site of a vector is a site having a base sequence that can cause homologous recombination with a target site of homologous recombination in a chromosome of S. pombe. The target site is a site into which an expression cassette is incorporated in a chromosome of S. pombe. The target site can be freely set by designing the base sequence of the recombination site of the vector such that the recombination site can cause homologous recombination with the target site.

The base sequence of the recombination site and the base sequence of the target site need to share identity of equal to or higher than 70%. Furthermore, in view of facilitating the occurrence of homologous recombination, the identity shared between the base sequence of the recombination site and the base sequence of the target site is preferably equal to or higher than 90%, and more preferably equal to or higher than 95%. By using the vector having the recombination site described above, an expression cassette can be incorporated into the target site through homologous recombination.

The length (number of bases) of the recombination site is preferably 20 bp to 2,000 bp. If the length of the recombination site is equal to or greater than 20 bp, homologous recombination easily occurs. If the length of the recombination site is equal to or less than 2,000 bp, it is easy to prevent a case where the vector becomes too long and thus the homologous recombination does not easily occur. The length of the recombination site is more preferably equal to or greater than 100 bp, and even more preferably equal to or greater than 200 bp. In addition, the length of the recombination site is more preferably equal to or less than 800 bp, and even more preferably equal to or less than 400 bp.

The vector may have other DNA domains in addition to the aforementioned expression cassette and recombination site. Examples of the DNA domains include a replication initiation domain called "ori" that is necessary for the replication in E. coli and an antibiotic resistance gene (a neomycin resistance gene or the like). These are genes generally required in a case where a vector is constructed using E. coli. Here, it is preferable that the replication initiation domain is removed when the vector is incorporated into a chromosome of a host as will be described later.

In a case where foreign genes are incorporated into a chromosome, the vector preferably has a linear DNA structure when being introduced into a cell of S. pombe. That is, in a case where the vector has a cyclic DNA structure such as plasmid DNA that is generally used, it is preferable that the vector is introduced into the cell of S. pombe after being cut and opened to become linear by a restriction enzyme.

In this case, a position in which the vector having a cyclic DNA structure is cut and opened is in the recombination site. As a result, in each of both ends of the cut and opened vector, the recombination site is partially present, and through homologous recombination, the entirety of the vector is incorporated into a target site of a chromosome.

As long as a linear DNA structure can be established for the vector such that a portion of the recombination site is present in each of both ends thereof, the vector may be constructed by a method other than the method of cutting and opening the vector having a cyclic DNA structure.

As the vector, for example, plasmids derived from E. coli, such as pBR 322, pBR 325, pUC 118, pUC 119, pUC 18, and pUC 19, can be suitably used.

In this case, it is preferable that a replication initiation domain called "ori" necessary for the replication in E. coli is removed from the plasmid vector used at the time of homologous recombination. In this way, when the aforementioned vector is incorporated into a chromosome, the incorporation efficiency can be improved.

A method for constructing a vector, from which the replication initiation domain has been removed, is not particularly limited, but it is preferable to use the method described in Japanese Unexamined Patent Application, First Publication No. 2000-262284. That is, it is preferable to use a method of constructing in advance a precursor vector in which a replication initiation domain is inserted into a cleavage site in a recombination site such that the vector has the linear DNA structure described above and the replication initiation domain is cut off. By this method, a vector from which a replication initiation domain has been removed can be easily obtained.

Furthermore, it is also preferable to use a method in which a precursor vector having an expression cassette and a recombination site is constructed by using the expression vector described in Japanese Unexamined Patent Application, First Publication No. H05-15380, Japanese Unexamined Patent Application, First Publication No. H07-163373, PCT International Publication No. WO96/23890, Japanese Unexamined Patent Application, First Publication No. H10-234375, and the like or using the construction method thereof, and a replication initiation domain is removed from the precursor vector by a general genetic engineering method so as to obtain a vector used for homologous recombination.

### <Target site>

The target site into which the vector is incorporated may be present in only one site or two or more sites in a chromosome of S. pombe. In a case where two or more target sites are present, the vector is incorporated into two or more sites of a chromosome of S. pombe. In a case where a plurality of foreign genes is included in a single expression cassette, the plurality of foreign genes can be incorporated into one target site.

In addition, by using two or more kinds of vector having a recombination site corresponding to each of the target sites, the expression cassette can be incorporated into two or more kinds of target site. By this method, a plurality of foreign genes can be incorporated into a chromosome of S. pombe. As a result, an expression amount of PYC or MDH encoded by the foreign genes can be increased, and the productivity of a C4 dicarboxylic acid can be improved.

For example, by incorporating an expression cassette including a PYC gene into a vector having a first target site, incorporating an expression cassette including an MDH gene into a vector having a second target site, performing transformation by using the vectors and S. pombe, in which some of the genes in a group of PDC genes have undergone deletion or deactivation, as a host, and then deleting or deactivating mae2 genes of the obtained transformant, the transformant described herein is obtained.

In a case where an expression cassette is incorporated into one target site, for example, it is possible to use the target site shown in the method described in Japanese Unexamined Patent Application, First Publication No. 2000-262284. By using two or more kinds of vector having different incorporation sites, the vectors can be incorporated into different target sites respectively. However, the above method is complicated for incorporating vectors into two or more sites of a chromosome.

As long as a plurality of portions, which is present in a chromosome and has base sequences substantially the same as each other, can be used as target sites, and a vector can be incorporated into each of the plurality of target sites, the vector can be incorporated into two or more sites in the chromosome by using one kind of vector.

The base sequences substantially the same as each other mean that the sequences share identity of equal to or higher than 90%. The identity shared between the target sites is preferably equal to or higher than 95%. The length of each of the base sequences substantially the same as each other is a length including the recombination site of the aforementioned vector, which is preferably equal to or greater than 1,000 bp.

In a case where foreign genes are incorporated into a plurality of target sites in a dispersed state, even if the same number of foreign genes are incorporated into the target sites, a phenomenon in which the foreign genes are broken away all at once from a chromosome when the transformant grows occurs less than in a case where a plurality of foreign genes is incorporated into a single target site. Therefore, the transformant is more stably maintained in passage.

As the plurality of target sites present in a chromosome, a transposon gene Tf2 is preferable. Tf2 is a transposon gene present in a total of 13 sites in each triple-strand (monoploid) chromosome of S. pombe. The length (number of bases) thereof is known to be about 4,900 bp, and the base sequence identity shared between the genes thereof is known to be 99.7% (see the following documents).

Nathan J. Bowen et al, "Retrotransposons and Their Recognition of pol II Promoters: A Comprehensive Survey of the Transposable Elements from the Complete Genome Sequence of Schizosaccharomyces pombe", Genome Res. 2003 13: 1984-1997

It is possible to incorporate a vector into only one of the Tf2's present in 13 sites in a chromosome. In this case, by incorporating a vector having two or more foreign genes, a transformant having two or more foreign genes can be obtained. Furthermore, by incorporating a vector into two or more Tf2's, a transformant having two or more foreign genes can be obtained. In this case, by incorporating the vector having two or more foreign genes, a transformant having more foreign genes can be obtained. If a vector is incorporated into all of the 13 Tf2's, too much burden may be imposed on the survival or growth of the transformant. Therefore, the vector is preferably incorporated into 8 or less of the 13 Tf2's, and more preferably incorporated into 5 or less Tf2's.

### <Transformation method>

As a transformation method, any of known transformation methods can be used. Examples of the transformation method include the methods known in the related art, such as a lithium acetate method, an electroporation method, a spheroplast method, and a glass bead method, and the method described in Japanese Unexamined Patent Application, First Publication No. 2005-198612. Furthermore, commercially available yeast transformation kits may be used.

As a method for transforming the S. pombe host by a homologous recombination method, a known homologous recombination method can be used. As a transformation method at the time of manufacturing the transformant according to the present invention, a method is preferable wherein S. pombe in which some of the genes in a group of PDC genes described above have undergone deletion or deactivation is used as a host, and an expression cassette is incorporated into a chromosome of S. pombe through homologous recombination by using the vector described above.

At the time of manufacturing the transformant, generally, after homologous recombination is performed, the obtained transformant is selected. As the selection method, for example, the following method can be used. By using a medium which makes it possible to select transformants by using the aforementioned auxotrophic marker, screening is carried out, thereby selecting a plurality of transformants from the obtained colony.

Then, each of the transformants is individually subjected to liquid culture. Thereafter, an expression amount of a heterologous protein in each culture solution is investigated, and transformants showing a greater expression amount of the heterologous protein are selected. Through a pulse field gel electrophoresis method, genomic analysis is performed on the selected transformants, and in this way, the number of vectors or expression cassettes incorporated into a chromosome is investigated.

The number of vectors incorporated into a chromosome can be adjusted to some extent by adjusting the incorporation conditions or the like. It is considered that the incorporation efficiency or the number of vectors incorporated may vary with the size (number of bases) or structure of the vector.

The transformant according to the present invention has malic acid producibility that is better than ever. A malic acid production rate of the transformant is preferably equal to or higher than 2.0 g/(L·h), more preferably equal to or higher than 5.0 g/(L·h), even more preferably equal to or higher than 10 g/(L·h), and particularly preferably 15 g/(L·h) to 30 g/(L·h).

### [Method for manufacturing C4 dicarboxylic acid]

A method for manufacturing a C4 dicarboxylic acid according to the present invention includes culturing the transformant according to the present invention in a culture solution and obtaining a C4 dicarboxylic acid from the culture solution.

By culturing of the transformant according to the present invention in a sugar-containing culture solution, oxaloacetic acid is produced from pyruvic acid obtained from the sugar by a glycolytic system with the aid of PYC or produced from phosphoenolpyruvic acid with the aid of PCK. From the produced oxaloacetic cid, malic acid is produced with the aid of MDH, and from the produced malic acid, other C4 dicarboxylic acids are produced as well.

Although the C4 dicarboxylic acids including the produced malic acid are accumulated in the microbial cell, some of them are released to a culture supernatant due to Mae1 (C4 dicarboxylic acid transporter). By obtaining C4 dicarboxylic acids from the cultured transformant or the culture supernatant, it is possible to manufacture C4 dicarboxylic acids. Examples of C4 dicarboxylic acids manufactured from the transformant include oxaloacetic acid, malic acid, fumaric acid, succinic acid, and the like, and among these, oxaloacetic acid or malic acid is preferable.

As the culture solution used for manufacturing a C4 dicarboxylic acid, a known sugar-containing culture medium for yeast can be used. Furthermore, the culture medium should contain a nitrogen source, inorganic salts, and the like that S. pombe can utilize and should enable S. pombe to be efficiently cultured. As the culture solution, a natural medium or a synthetic medium may be used.

Examples of the sugar as a carbon source include sugars such as glucose, fructose, sucrose, and maltose. Examples of the nitrogen source include ammonia, an ammonium salt of an inorganic or organic acid, such as ammonium chloride or ammonium acetate, peptone, casamino acid, yeast extract, and the like. Examples of the inorganic salts include magnesium phosphate, magnesium sulfate, sodium chloride, and the like. It is also possible to further add a fermentation accelerating factor such as proteolipid.

In the method for manufacturing a C4 dicarboxylic acid according to the present invention, it is preferable to use a culture solution particularly containing glucose or sucrose as sugar. A concentration of glucose or sucrose in the culture solution (100% by mass) at the initial stage of culture is preferably equal to or greater than 1% by mass, more preferably 1% by mass to 50% by mass, and even more preferably 2% by mass to 16% by mass. After the glucose concentration or the sucrose concentration is reduced due to culture, it is preferable to continue the culture by adding glucose as necessary. At the final stage of culture, the glucose concentration or the like may become equal to or less than 1% by mass.

In a case where continuous culture is performed by circulating the culture solution in a state of separating a C4 dicarboxylic acid, it is preferable to maintain the glucose concentration or the like. If the glucose concentration is set to be equal to or greater than 2% by mass, the productivity of a C4 dicarboxylic acid is further improved. Furthermore, if the concentration of glucose or sucrose in the culture solution is set to be equal to or less than 16% by mass, the production efficiency of a C4 dicarboxylic acid is further improved.

In order to improve the productivity of manufacturing of a C4 dicarboxylic acid, it is preferable to perform high-density culture. During the high-density culture, the initial microbial cell concentration of the transformant in the culture solution, expressed in terms of a weight of dry microbial cells, is preferably set to be 0.1 g/L to 100 g/L. The initial microbial cell concentration of the transformant in the culture solution, expressed in terms of a weight of dry microbial cells, is more preferably set to be 20 g/L to 60 g/L. If the initial microbial cell concentration is set to be high, high productivity can be achieved within a short period of time. Furthermore, if the initial microbial cell concentration is too high, a problem such as the aggregation of microbial cells or the reduction of purification efficiency may occur.

The microbial cell concentration described in examples and the like, which will be described later, is a value converted from an absorbance (OD₆₆₀) of light having a wavelength of 660 nm measured by a visible-ultraviolet spectrometer V550 manufactured by JASCO Corporation. The value of 1 that equals OD₆₆₀ at 660 nm corresponds to a dry weight of 0.2 g and a wet weight of 0.8 g of fission yeast.

For the culture, a known yeast culture method can be used. For example, shake culture or stirring culture can be performed.

The culture temperature is preferably 23°C to 37°C, and the culture time can be appropriately determined.

The culture may be batch culture or continuous culture. For example, after culture is performed by batch culture, by separating the microbial cells from the culture solution, a culture solution containing a C4 dicarboxylic acid can be obtained. In addition, examples of the continuous culture method include a method of continuously performing culture by repeating a process of taking out a portion of the culture solution from a culture tank in which culture is being performed, separating a C4 dicarboxylic acid from the taken culture solution and recovering the culture supernatant, adding glucose or a new culture solution to the culture supernatant, and returning the culture supernatant to the culture tank. By performing the continuous culture, the productivity of a C4 dicarboxylic acid is further improved.

The transformant according to the present invention has particularly excellent acid resistance. Therefore, even at a low pH (about pH 2 to 4) resulting from the accumulation of a C4 dicarboxylic acid, the transformant can produce a C4 dicarboxylic acid without being subjected to neutralization. Accordingly, even after the pH of the culture solution becomes equal to or less than 3.5, it is possible to manufacture a C4 dicarboxylic acid by continuous culture in which culture is continued. The pH of the last stage of culture and the pH during the continuous culture is preferably equal to or less than 3.5 and particularly preferably 2.3 to 3.5. In some cases, culture may be finished before the pH of the culture solution becomes equal to or less than 3.5.

A C4 dicarboxylic acid can be obtained from the culture solution by a known method. Examples of the method include a method in which microbial cells are separated by centrifugation from the culture solution after the end of the culture, and a C4 dicarboxylic acid is extracted using diethyl ether or ethyl acetate after the pH becomes equal to or less than 1; a method in which the culture solution is absorbed onto an ion exchange resin and washed, and then a C4 dicarboxylic acid is eluted; a method in which impurities are removed using activated carbon; a method in which the culture solution is reacted with alcohol in the presence of an acid catalyst and then subjected to distillation; and a method in which a C4 dicarboxylic acid is separated using a separation membrane.

Furthermore, in some cases, by neutralizing a C4 dicarboxylic acid in the culture solution and then separating a C4 dicarboxylic acid salt from the culture solution, a C4 dicarboxylic acid can be obtained. For example, by a method of converting a C4 dicarboxylic acid in the culture solution into a calcium salt or a lithium salt and crystallizing the neutralized salt, a C4 dicarboxylic acid can also be obtained.

The method for manufacturing a C4 dicarboxylic acid according to the present invention described above makes it possible to manufacture a C4 dicarboxylic acid with high productivity in a simple manner by using a transformant that uses S. pombe as a host. According to the manufacturing method, a production rate of a C4 dicarboxylic acid easily becomes equal to or higher than 5 g/L/h, and in some cases, the production rate of a C4 dicarboxylic acid becomes equal to or higher than 15 g/L/h. The method for manufacturing a C4 dicarboxylic acid according to the present invention is also suitable for high-density culture that is performed in the presence of high-concentration glucose by using a high-concentration transformant.

### [Examples]

Hereinafter, the present invention will be specifically described by showing examples and comparative examples. In the present examples, unless otherwise specified, "%" means "% by mass".

### <Preparation of PDC2 gene deletion strain of S. pombe>

A uracil auxotrophic ARC019 strain of S. pombe (genotype: h⁻, leu1-32, ura4-D18, Ade6-M216) (Strain name: JY741, NBRPID: FY7512) was transformed according to a Latour method (described in the journal of Nucleic Acids Research, 2006, Vol. 34, p. e11, PCT International Publication No. WO2007/063919), thereby preparing a deletion strain (IGF836 strain) from which PDC2 genes (strain name: SPAC1F8. 07c) were deleted.

For preparing a deletion fragment, total genomic DNA prepared from an ARC032 strain of S. pombe (genotype: h⁻) (see PCT International Publication No. WO2007/015470) by using DNeasy (manufactured by QUIAGEN) was used as a template, and 8 kinds of synthetic oligo DNA (manufactured by Operon Biotechnologies) having the base sequences shown in Table 1 were used.

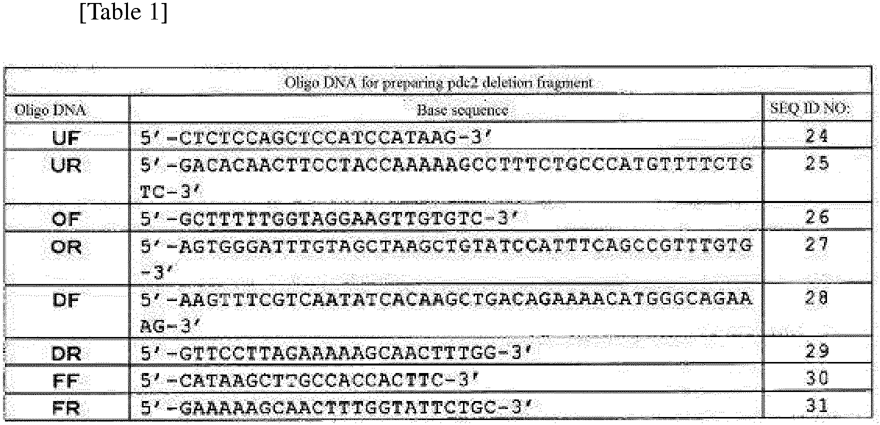

Specifically, by a PCR method using KOD-Dash (manufactured by TOYOBO CO., LTD.), a UP domain was prepared using UF and UR, an OL domain was prepared using OF and OR, and a DN domain was prepared using DF and DR. Then, by using these as templates, a full-length deletion fragment was prepared by the same PCR method using FF and FR respectively. At the time of preparing the full-length deletion fragment, 2 kinds of synthetic oligo DNA (manufactured by Operon Biotechnologies) having the base sequences shown in Table 2 were used, and the total genomic DNA prepared from the ARC032 strain in the same manner as above was used as a template. Furthermore, a fragment of a domain of a uracil auxotrophic marker ura4 of S. pombe (strain name listed in GeneDB: SPCC330.05c, orotidine-5'-phosphate decarboxylase gene) prepared by the same PCR method was also used in combination as a template.

**[Table 2]**

| Oligo DNA for preparing um4 fragment | | |
|---|---|---|
| Oligo DNA | Base sequence | SEQ ID NO |
| F | 5'-AGCTTAGCTACAAATCCCACT-3' | 32 |
| R | 5'-AGCTTGTGATATTGACGAAACTT-3' | 33 |

By using a DNA fragment obtained by treating a pSHh vector with a restriction enzyme PmaCI, the obtained PDC2 gene deletion strain of S. pombe (IGF836 strain, h⁻leu1-32 ura4-D18 ade6-M216 pdc2-D23) was transformed, thereby obtaining an ASP4590 strain in which uracil auxotrophy and adenine auxotrophy were restored.

### [Example 2]

### <Preparation of foreign gene introduction strain>

Transformants of pombe were prepared (Table 24) into which PYC derived from Aspergillus niger (AniPYC) (SEQ ID NO: 1), PYC derived from Brevibacillus brevis (BbrPYC) (SEQ ID NO: 2), PYC derived from Debaryomyces hansenii (DhaPYC) (SEQ ID NO: 3), PYC derived from Kluyveromyces lactis (KlaPYC) (SEQ ID NO: 4), PYC derived from Lachancea thermotolerans (LthPYC) (SEQ ID NO: 5), PYC derived from Lodderomyces elongisporus (LelPYC) (SEQ ID NO: 6), PYC derived from Saccharomyces cerevisiae (ScePYC) (SEQ ID NO: 7), PYC derived from Candida orthopsilosis (CorPYC) (SEQ ID NO: 8), PYC derived from Candida tropicalis (CtrPYC) (SEQ ID NO: 9), PYC derived from Naumovozyma castellii (NcaPYC) (SEQ ID NO: 10), PYC derived from Naumovozyma dairenensis (NdaPYC) (SEQ ID NO: 11), PYC derived from Saccharomyces kudriavzevii (SkuPYC) (SEQ ID NO: 12), PYC derived from S. pombe (SpoPYC) (SEQ ID NO: 13), PYC derived from Tetrapisispora blattae (TblPYC) (SEQ ID NO: 14), PYC derived from Torulaspora delbrueckii (TdePYC) (SEQ ID NO: 15), PYC derived from Zygosaccharomyces rouxii (ZroPYC) (SEQ ID NO: 16), MDH derived from Archaeoglobus fulgidus (AfuMDH) (SEQ ID NO: 17), MDH derived from Congregibacter litoralis (CliMDH) (SEQ ID NO: 18), MDH derived from Delftia acidovorans (DacMDH) (SEQ ID NO: 19), MDH derived from Halomonas elongata (HelMDH) (SEQ ID NO: 20), or MDH derived from Shewanella putrefaciens (SpuMDH) (SEQ ID NO: 21) was introduced.

Specifically, The IGF836 strain (gene deletion strain of S. pombe) prepared in Example 1 was transformed according to the method of Bahler et al. (the journal of Yeast, 1998, Vol. 14, pp. 943-951) by using a digest of a restriction enzyme BsiWI of a monodentate integrative recombinant vector pSLh-AniPYC retaining an AniPYC gene expression cassette, a monodentate integrative recombinant vector pSLh-BbrPYC retaining a BbrPYC gene expression cassette, a monodentate integrative recombinant vector pSLh-DhaPYC retaining a DhaPYC gene expression cassette, a monodentate integrative recombinant vector pSLh-KlaPYC retaining a KlaPYC gene expression cassette, a monodentate integrative recombinant vector pSLh-LthPYC retaining an LthPYC gene expression cassette, a monodentate integrative recombinant vector pSLh-LelPYC retaining an LelPYC gene expression cassette, a monodentate integrative recombinant vector pSLh-ScePYC retaining an ScePYC gene expression cassette, a monodentate integrative recombinant vector pSLh-CorPYC retaining a CorPYC gene expression cassette, a monodentate integrative recombinant vector pSLh-CtrPYC retaining a CtrPYC gene expression cassette, a monodentate integrative recombinant vector pSLh-NcaPYC retaining an NcaPYC gene expression cassette, a monodentate integrative recombinant vector pSLh-NdaPYC retaining an NdaPYC gene expression cassette, a monodentate integrative recombinant vector pSLh-SkuPYC retaining an SkuPYC gene expression cassette, a monodentate integrative recombinant vector pSLh-SpoPYC retaining an SpoPYC gene expression cassette, a monodentate integrative recombinant vector pSLh-TblPYC retaining a TblPYC gene expression cassette, a monodentate integrative recombinant vector pSLh-TdePYC retaining a TdePYC gene expression cassette, a monodentate integrative recombinant vector pSLh-ZroPYC retaining a ZroPYC gene expression cassette, a monodentate integrative recombinant vector pSMh-AfuMDH retaining an AfuMDH gene expression cassette, a monodentate integrative recombinant vector pSMh-CliMDH retaining a CliMDH gene expression cassette, a monodentate integrative recombinant vector pSMh-DacMDH retaining a DacMDH gene expression cassette, a monodentate integrative recombinant vector pSMh-HelMDH retaining an HelMDH gene expression cassette, or a monodentate integrative recombinant vector pSMh-SpuMDH retaining an SpuMDH gene expression cassette.

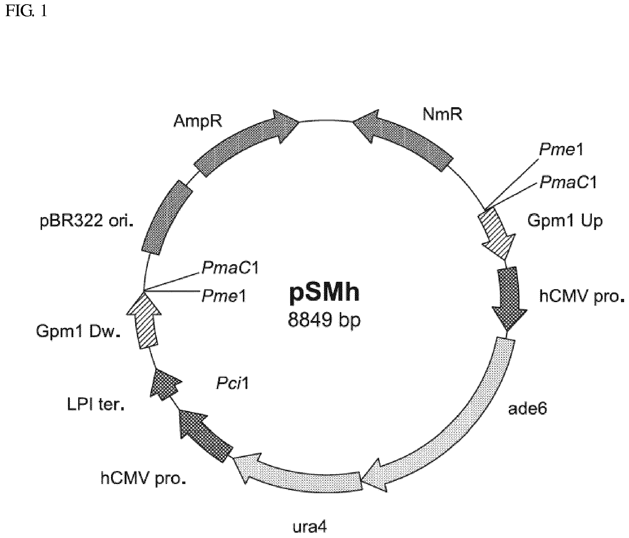

The monodentate integrative recombinant vector pSMh can be prepared through the following process. First, a fragment obtained by digestion of a DNA fragment (Fr. 1), which was prepared by total synthesis of DNA and had a base sequence represented by SEQ ID NO: 34, with a restriction enzyme BsiWI and a DNA fragment, which was obtained by the digestion of a pSE vector with a restriction enzyme BsiWI and the double digestion of the obtained digest with restriction enzymes Kpnl and SnaBI, were subjected to ligation, thereby preparing pSMh (8,849 bp, FIG. 1) having a base sequence (5'→3', cyclic) represented by SEQ ID NO: 35.

pSLh-AniPYC was prepared through the following process. First, by using total genomic DNA prepared by DNeasy (manufactured by QUIAGEN) from the culture of Aspergillus niger as a template and using 2 kinds of synthetic oligo DNA (AniPYC-F and AniPYC-R manufactured by Operon Biotechnologies) described in Table 3, an ORF fragment of an AniPYC gene was obtained by a PCR method using KOD-Dash (manufactured by TOYOBO CO., LTD.). The ORF fragment encoded AniPYC (SEQ ID NO: 1).

**[Table 3]**

| | Sequence | SEQ ID NO: |
|---|---|---|
| AniPYC-F | 5'-GACACTTTTTCAAACATGGCTGCTCCCCGCC-3' | 36 |
| AniPYC-R | 5'-ATCATCCTTGTAATCGGCCTTGACGATCTTGCAGAC-3' | 37 |
| AniPYC | | 1 |

By using an In-Fusion (registered trademark) HD Cloning Kit (manufactured by Clontech Laboratories, Inc.), the obtained amplified fragment was incorporated into pSLh, thereby preparing pSLh-AniPYC. The In-Fusion method was performed according to the manual included in the kit. That is, the obtained PCR product was purified using a spin column, added to an In-Fusion reaction solution together with pSLh, and reacted for 15 minutes at 50°C.

By the same method as described above, a monodentate integrative recombinant vector pSLh-BbrPYC, a monodentate integrative recombinant vector pSLh-DhaPYC, a monodentate integrative recombinant vector pSLh-KlaPYC, a monodentate integrative recombinant vector pSLh-LthPYC, a monodentate integrative recombinant vector pSLh-LelPYC, a monodentate integrative recombinant vector pSLh-ScePYC, a monodentate integrative recombinant vector pSLh-CorPYC, a monodentate integrative recombinant vector pSLh-CtrPYC, a monodentate integrative recombinant vector pSLh-NcaPYC, a monodentate integrative recombinant vector pSLh-NdaPYC, a monodentate integrative recombinant vector pSLh-SkuPYC, a monodentate integrative recombinant vector pSLh-SpoPYC, a monodentate integrative recombinant vector pSLh-TblPYC, a monodentate integrative recombinant vector pSLh-TdePYC, a monodentate integrative recombinant vector pSLh-ZroPYC, a monodentate integrative recombinant vector pSMh-AfuMDH, a monodentate integrative recombinant vector pSMh-CliMDH, a monodentate integrative recombinant vector pSMh-DacMDH, a monodentate integrative recombinant vector pSMh-HelMDH, and a monodentate integrative recombinant vector pSMh-SpuMDH were prepared. Primer sets using the respective vectors will be listed below.

The ASP4590 strain was transformed using the monodentate integrative recombinant vector pSLh-ScePYC and the monodentate integrative recombinant vector pSMh-DacMDH, thereby obtaining an ASP4491 strain into which an ScePYC gene and a DacMDH gene were introduced.

**[Table 4]**

| | Sequence | SEQ ID NO: |
|---|---|---|
| BbrPYC-F | | 38 |
| BbrPYC-R | 5'-ATCATCCTTGTAATCCTCCATTTCAATGAGCAGATCACCA-3' | 39 |
| BbrPYC | | 2 |

**[Table 5]**

| | Sequence | SEQ ID NO: |
|---|---|---|
| DhaPYC-F | | 40 |
| DhaPYC-R | | 41 |
| DhaPYC | | 3 |

**[Table 6]**

| | Sequence | SEQ ID NO: |
|---|---|---|
| KlaPYC-F | 5'-GACACTTTTTCAAACATGTCATCTCAACTAGCTGGATTGC-3' | 42 |
| KlaPYC-R | 5'-ATCATCCTTGTAATCTTCCTTAGCTGGAGCTTCTTCC-3' | 43 |
| KlaPYC | | 4 |

**[Table 7]**

| | Sequence | SEQ ID NO: |
|---|---|---|
| LthPYC-F | 5'-GACACTTTTTCAAACATGTCCAAACTGGCAGGACTG-3' | 44 |
| LthPYC-R | 5'-ATCATCCTTGTAATCTTCTTTTGGTGGGACAGATTCCTC-3' | 45 |
| LthPYC | | 5 |

**[Table 8]**

| | Sequence | SEQ ID NO: |
|---|---|---|
| LelPYC-F | | 46 |
| LelPYC-R | | 47 |
| LelPYC | | 6 |

**[Table 9]**

| | Sequence | SEQ ID NO. |
|---|---|---|
| ScePYC-F | 5'-GACACTTTTTCAAACATGTCGCAAAGAAAATTCGCCG-3' | 48 |
| ScePYC-R | 5'-ATCATCCTTGTAATCTGCCTTAGTTTCAACAGGAACTTGG-3' | 49 |
| ScePYC | | 7 |

**[Table 10]**

| | Sequence | SEQ ID NO. |
|---|---|---|
| CorPYC-F | | 50 |
| CorPYC-R | | 51 |
| CorPYC | | 8 |

**[Table 11]**

| | Sequence | SEQ ID NO. |
|---|---|---|
| CtrPYC-F | 5'-CACTTTTTCAAAcATGTCTGTACCAGTCCAAAAAGCTAAC-3' | 52 |
| CtrPYC-R | | 53 |
| CtrPYC | | 9 |

**[Table 12]**

| | Sequence | SEQ ID NO. |
|---|---|---|
| NcaPYC-F | | 54 |
| NcaPYC-R | | 55 |
| NcaPYC | | 10 |

**[Table 13]**

| | Sequence | SEQ ID NO. |
|---|---|---|
| NdaPYC-F | | 56 |
| SdaPYC-a | | 57 |
| NdaPYC | | 11 |

**[Table 14]**

| | Sequence | SEQ ID NO: |
|---|---|---|
| SkuPYC-F | 5'-CACTTTTTCAAAcATGTCGCAAAAGAAATTCGCTGG-3' | 58 |
| SkuPYC-R | | 59 |
| SkuPYC | | 12 |

**[Table 15]**

| | Sequence | SEQ ID NO. |
|---|---|---|
| SpoPYC-F | | 60 |
| SpoPYC-R | 5'-ATCATCCTTGTAATCCTCGTGTTCAAGAACAGCACACA-3' | 61 |
| SpoPYC | | 13 |

**[Table 16]**

| | Sequence | SEQ ID NO. |
|---|---|---|
| TblPYC-F | | 62 |
| TblPYC-R | | 63 |
| TblPYC | | 14 |

**[Table 17]**

| | Sequence | SEQ ID NO: |
|---|---|---|
| TdePYC-F | 5'-CACTTTTTCAAAcATGTCCAAGAACAAGAGGTTTGCT-3' | 64 |
| TdaPYC-R | 5'-ATCATCCTTGTAATCGTTTTCAGCTGGGACAGCCTC-3' | 65 |
| TdePYC | | 15 |

**[Table 18]**

| | Sequence | SEQ ID NO. |
|---|---|---|
| ZroPYC-F | 5'-CACTTTTTCAAAcATGAGTACCAAGAAGTTCTCTGGTCT-3' | 66 |
| ZroPYC-R | 5'-ATCATCCTTGTAATCATTCTCCTTTGGTGGGACGG-3' | 67 |
| ZroPYC | | 16 |

**[Table 19]**

| | Sequence | SEQ ID NO: |
|---|---|---|
| AfuMDH-F | 5'-ACTTTTTCAAACATGAAACTCGGTTTTGTTGGTGC-3' | 68 |
| AfuMDH-R | | 69 |
| AfuMDH | | 17 |

**[Table 20]**

| | Sequence | SEQ ID NO: |
|---|---|---|
| CliMDH-F | 5'-ACTTTTTCAAACATGAAAGCACCTGTTCGCGT-3' | 70 |
| CliNDH-R | 5'-ATCATCATCATCCTTGTAATCGGGCAGAAGGTCTGCCACG-3' | 71 |
| CliMDH | | 18 |

**[Table 21]**

| | Sequence | SEQ ID NO: |
|---|---|---|
| DacMDH-F | 5' -ACTTTTTCAAACATGAGCAAGAAGCCCGTTCG-3' | 72 |
| DacMDH-R | 5'-ATCATCATCATCCTTGTAATCCAGCAGGTGCTTGACGCC-3' | 73 |
| DacMDH | | 19 |

**[Table 22]**

| | Sequence | SEQ ID NO: |
|---|---|---|
| HelMDH-F | 5'-ACTTTTTCAAACATGAAAGACCCCGTCCGTCCGTATAGC-3' | 74 |
| HelMDH-R | 5'-ATCATCATCATCCTTGTAATCGCCAAGCAGATGCTCGACG-3' | 75 |
| HelMDH | | 20 |

**[Table 23]**

| | Sequence | SEQ ID NO: |
|---|---|---|
| SpuMDH-F | 5'-ACTTTTTCAAACATGAAAGTTGCTGTACTTGGTGC-3' | 76 |
| SpuMDH-R | | 77 |
| SpuMDH | | 21 |

The name and host of each of the manufactured transformants and the name of the introduced foreign gene are shown in Table 24.

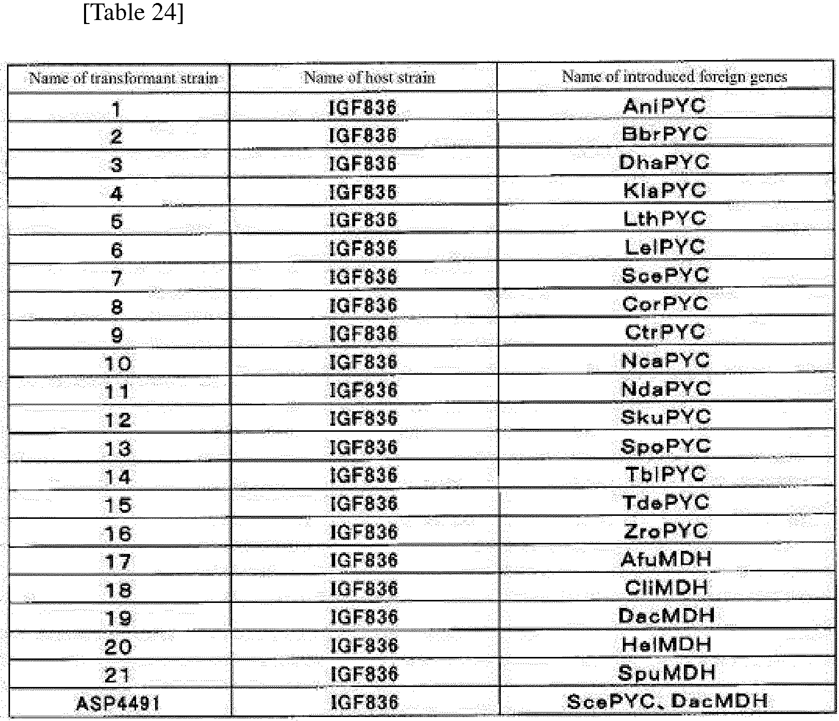

### <Confirmation of PYC expression>

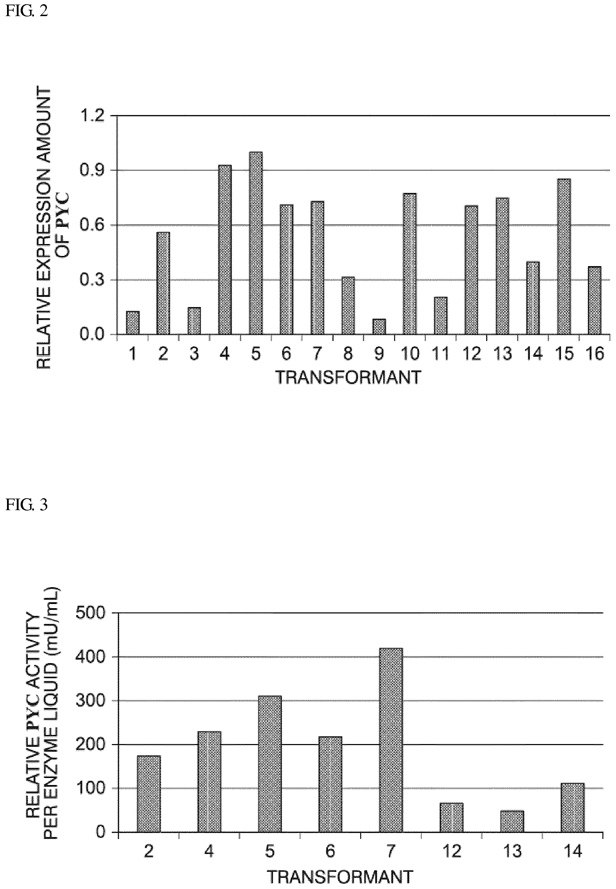

For 16 kinds of transformant into which only the PYC gene was introduced, an expression amount of PYC was confirmed. Specifically, each of the transformants was shake-cultured for 44 hours in 20 mL of a YPD6 medium, and the grown microbial cells were then collected and pulverized using a multibead shocker. The obtained microbial cell lysate was purified according to a protocol by using ANTI-FLAG (registered trademark) M1 Agarose Affinity Gel (manufactured by Sigma-Aldrich Co. LLC.). SDS-PAGE was performed using a purified enzyme liquid, and the detected band was digitized using an image analysis device LAS 4000, thereby calculating a relative expression amount. The results of the PYC expression amount (relative values) for each of the transformants are shown in FIG. 2. The results show that the expression of PYC was confirmed in all of the transformants.

### <Confirmation of PYC activity>

Among the 16 kinds of transformant into which only the PYC gene was introduced, 8 kinds of transformant caused to express BbrPYC, KluPYC, LthPYC, LelPYC, ScePYC, SkuPYC, SpoPYC, or TblPYC were investigated regarding PYC activity. Specifically, the aforementioned purified enzyme liquid was mixed with a reaction solution kept at 30°C (100 mM Tris-HCl (pH 7.5), 2 mM ATP, 100 mM MgCl₂, 100 mM KCl, 5 mM Pyruvic acid, 5 mM NaHCO₃, 0.1 mM Acetyl-CoA, 0.15 mM NADH), and then measured over time by using an absorptiometer having a mixing function. From the obtained temporal change of absorbance, a relative activity (mU/mL) per enzyme liquid was calculated. The results of the PYC activity per enzyme liquid calculated for each of the transformants are shown in FIG. 3. The results show that the PYC activity was confirmed in all of the transformants.

### <Confirmation of MDH expression>

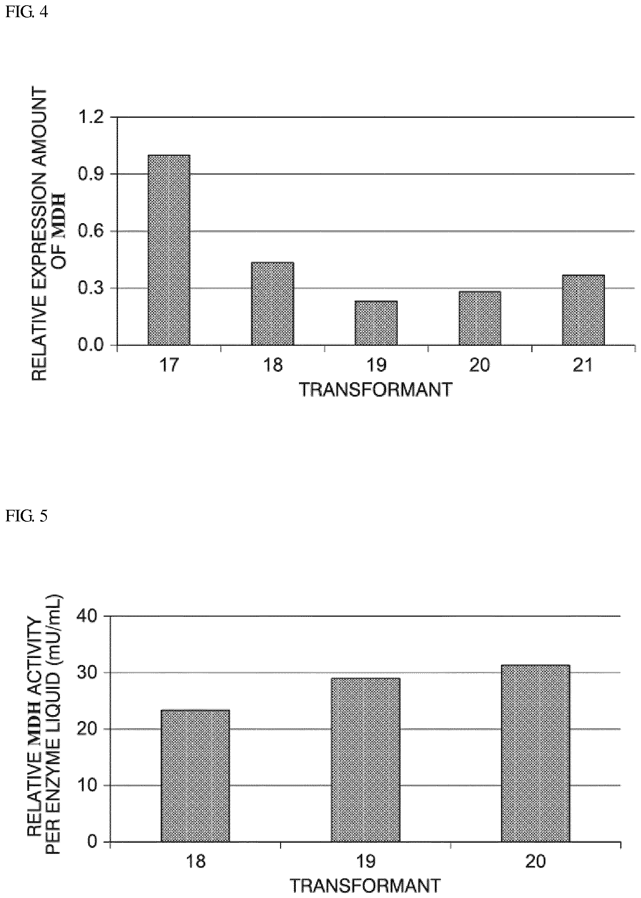

For 5 kinds of transformant into which only the MDH gene was introduced, an expression amount of MDH was confirmed. Specifically, each of the transformants was cultured in the same manner as described in <Confirmation of PYC expression>, a purified enzyme liquid was prepared, SDS-PAGE was performed using the enzyme liquid, and a relative expression amount was calculated. The results of the MDH expression amount (relative values) for each of the transformants are shown in FIG. 4. The results show that the expression of MDH was confirmed in all of the transformants.

### <Confirmation of MDH activity>

Among 5 kinds of transformant into which only the MDH gene was introduced, 3 kinds of transformant caused to express CliMDH, DacMDH, or HelMDH were investigated regarding MDH activity. Specifically, the aforementioned purified enzyme liquid was mixed with a reaction solution kept at 30°C (90 mM Tris-HCl (pH 9.0), 0.5 mM Oxaloacetate, 0.25 mM NADH) and then measured over time by using an absorptiometer having a mixing function. From the obtained temporal change of absorbance, a relative activity (mU/mL) per enzyme liquid was calculated. The results of the MDH activity per enzyme liquid calculated for each of the transformants are shown in FIG. 5. The results show that the MDH activity was confirmed in all of the transformants.

### [Example 3] Preparation of mae2 deletion strain

By deleting an mae2 gene from the ASP4491 strain into which an ScePYC gene and a DacMDH gene were introduced, a deletion strain (ASP4964 strain) was prepared. A method for preparing an mae2 deletion fragment was the same as the method for preparing the deletion fragment from which pdc2 was deleted.

The ASP4491 strain (genotype: h⁻, leu1-32, ura4-D18, Ade6-M216, Δpdc2, +ScePYC, +DacMDH) of S. pombe was transformed according to the Latour method, thereby preparing an ASP4964 strain from which an mae2 gene (strain name: SPCC794. 12c) was deleted.

For preparing a deletion fragment, total genomic DNA prepared from an ARC032 strain of S. pombe by using DNeasy (manufactured by QUIAGEN) was used as a template, and 8 kinds of synthetic oligo DNA (manufactured by Operon Biotechnologies) having the base sequences shown in Table 25 were used.

**[Table 25]**

| Oligo DNA for preparing name2 deletion fragment | | |
|---|---|---|
| Oligo DNA | Base sequence | SEQ ID NO: |
| UF | 5'-AGGCTTTGATAGCCACTGGT-3' | 78 |
| UR | 5' -TGGGATTTGTAGCTAAGCTTTAAAATAAAAGGCFTTTATAC-3' | 79 |
| OF | 5'-TTCGTCAATATCACAAGCTTAGGTCGACTGGGCTAATCG-3' | 80 |
| OR | 5'-TAAAATAAAAGGCTTTATACAGGCATTTTCAAACTTCAAG-3' | 81 |
| DF | 5'-CTTGAAGTTTGAAAATGCCTGTATAAAGCCTTTTATTTTA-3' | 82 |
| DR | 5' -TTCATTCAATACATAACGGTTTACGGT-3' | 83 |
| FF | 5'-ATTCGTGAAATGAGCAAGCA-3' | 84 |
| FR | 5'-TGCGATTTACTATTTGTTTGTTTCA-3' | 85 |

### [Example 4] Preparation of fum1 deletion strain

By deleting a fum1 gene from the ASP4491 strain into which an ScePYC gene and a DacMDH gene were introduced, a deletion strain (ASP4933 strain) was prepared. fum1 is an enzyme which produces fumaric acid by using malic acid as a matrix. A method for preparing an fum1 deletion fragment is the same as the method for preparing the deletion fragment from which pdc2 was deleted.

The ASP4491 strain of S. pombe was transformed according to the Latour method, thereby preparing an ASP4933 strain from which an fum1 gene (strain name: SPCC18. 18c/SPCC290. 01c) was deleted.

For preparing a deletion fragment, total genomic DNA prepared from an ARC032 strain of S. pombe by using DNeasy (manufactured by QUIAGEN) was used as a template, and 8 kinds of synthetic oligo DNA (manufactured by Operon Biotechnologies) having the base sequences shown in Table 26 were used.

**[Table 26]**

| Oligo DNA for preparing fum1 deletion fragment | | |
|---|---|---|
| Oligo DNA | Base sequence | SEQ ID NO: |
| UF | 5' -CTCAAGTAATGGGCAATCATGCCA-3' | 86 |
| UR | 5'-TGGGATTTGTAGCTAAGCTTAGAGTGGTAAAAAATTATAC-3' | 87 |
| OF | 5'-TTCGTCAATATCACAAGCTTAATAAAATACACAAAACTGT-3' | 88 |
| OR | 5'-AGAGTGGTAAAAAATTATACAGCCATGTGGGTTATTTTAA-3' | 89 |
| DF | 5'-TTAAAATAACCCACATGGCTGTATAATTTTTTACCACTCT-3' | 90 |
| DR | 5'-GAGCAACCGAATATCAAGGAAATACACA-3' | 91 |
| FF | 5'-AGGCGAATTGATCCTTCCTGCTA-3' | 92 |
| FR | 5'-TGGTAAGCCTGGTATGAGTTCTATACTAT-3' | 93 |

### <Confirmation of malic acid producibility>

For a wild strain (ARC010 strain) of S. pombe, the PDC2 gene deletion strain (ASP4590 strain, ΔPDC2), the ASP4491 strain obtained by introducing an ScePYC gene and a DacMDH gene into the ASP4590 strain, the ASP4964 strain (ΔPDC2, Δmae2, +ScePYC, +DacMDH) obtained by deleting an mae2 gene from the ASP4491 strain, and the ASP4933 strain (ΔPDC2, Δfum1, +ScePYC, +DacMDH) obtained by deleting an fum1 gene from the ASP4491 strain, a malic acid production rate was investigated.

Specifically, the microbial cells were seeded into a YES plate and cultured for 96 hours at 30°C, thereby obtaining a colony. The obtained colony was subcultured in 5 mL of a YES medium (test tube) and shake-cultured for 24 hours at 30°C. The obtained microbial solution was subcultured in 100 mL of a YPD6 medium (Sakaguchi flask) and shake-cultured for 44 hours at 30°C.

The microbial cells obtained in this way were collected, added to 3 mL of a fermentation medium (100 g/L glucose, 111 g/L calcium carbonate) in the test tube so as to yield 36 g (weight of dry microbial cell)/L, and fermented at 30°C under shaking conditions. A sample was collected in time from the fermented liquid.

For the obtained sample, a glucose concentration and an ethanol concentration were measured using a biosensor BF-7 (manufactured by Oji Scientific Instruments) based on an enzyme electrode method, and a malic acid concentration was measured by HPLC. During the HPLC measurement, a high-performance liquid chromatograph Prominence (manufactured by Shimadzu Corporation) was used, Aminex HPX-87H 300 × 7.8 mm (manufactured by Bio-Rad Laboratories, Inc.) was used as a column, an injection volume was set to be 10 µL, 10 mM H₂SO₄ was used as a solvent, a flow rate was set to be 0.6 mL/min, a measurement time was set to be 35 minutes, a measurement temperature was set to be 60°C, and diode array detector (210 nm) and a differential refractometric detector were used for detection. Each concentration is a concentration per culture solution or fermented liquid.

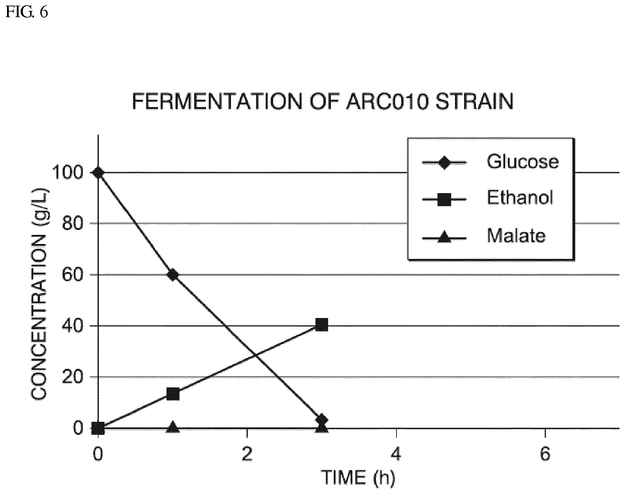

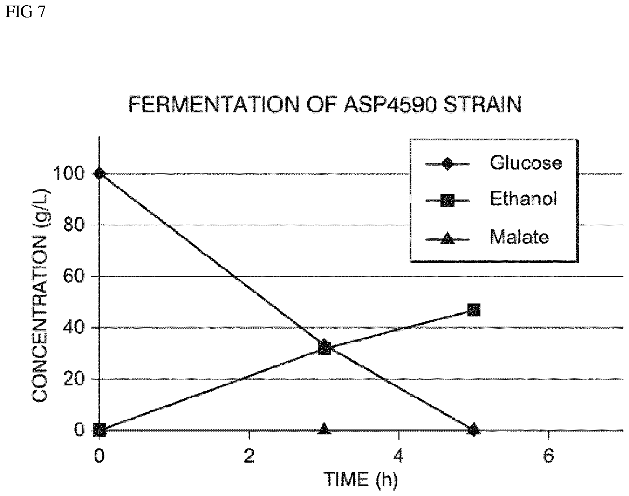

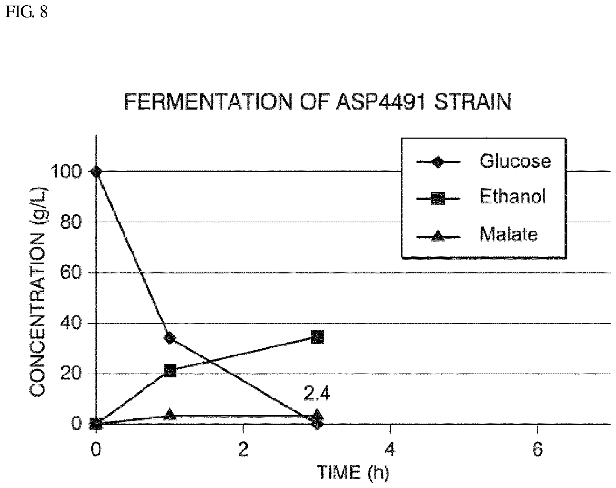

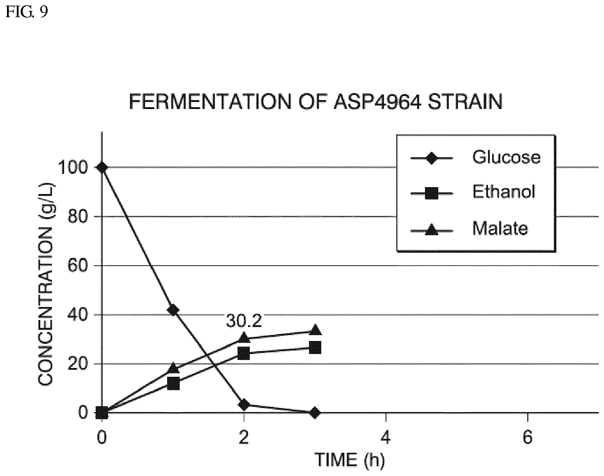

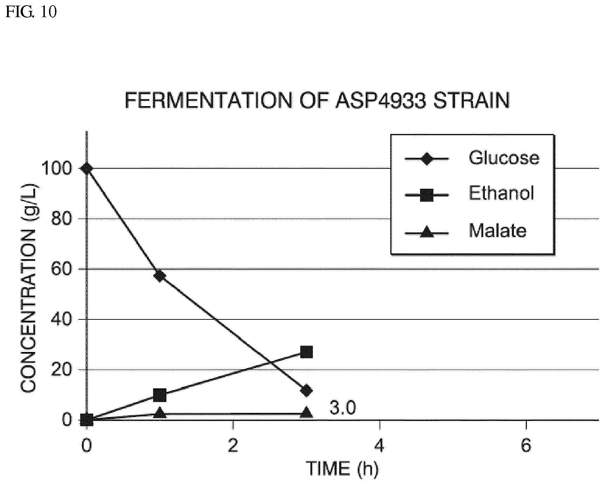

The measured results of the glucose concentration (g/L), the ethanol concentration (g/L), and a malic acid concentration (g/L) are shown in FIGS.6 to 10. The results show that the production of malic acid was confirmed only in the ASP4964 strain (ΔPDC2, Δmae2,+ScePYC, +DacMDH).

### <Measurement of malic acid production rate>

For the ASP4491 strain (ΔPDC2, +ScePYC, +DacMDH) and the ASP4892 strain (ΔPDC2, Δmae2,+ScePYC, +DacMDH), a malic acid production rate was measured.

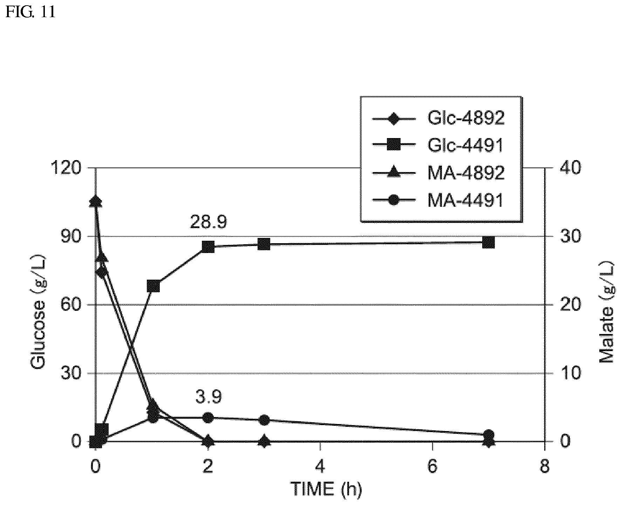

Specifically, each strain was fermented in the same manner as in <Confirmation of malic acid producibility>, a sample was collected in time from the fermented liquid, and a glucose concentration and a malic acid concentration were measured. The measured results are shown in FIG. 11. In FIG. 11, "Glu-4892" and "Glu-4491" show a temporal change of a glucose concentration of the ASP4892 strain and the ASP4491 strain respectively, and "MA-4892" and "MA-4491" show a temporal change of a malic acid concentration of the ASP4892 strain and the ASP4491 strain respectively. As a result, it was confirmed that at a point in time when 1 hour elapsed from the beginning of fermentation, a malic acid concentration of the fermented liquid of the ASP4892 strain exceeded 20 g/L, and a malic acid production rate thereof was equal to or higher than 20 g/(L·h). Furthermore, at a point in time when 2 hours elapsed, a malic acid concentration of the fermented liquid became 28.9 g/L. In contrast, in the fermented liquid of the ASP4491 strain, a malic acid concentration was 3.9 g/L at a point in time when 2 hours elapsed.

### [Example 5] Preparation of PCK introduction strain

Transformants of pombe were prepaerd (Table 47) into which PCK derived from Candida glabrata (CglPCK) (SEQ ID NO: 94), PCK derived from Citrobacter koseri (CkoPCK) (SEQ ID NO: 95), PCK derived from Cronobacter sakazakii (CsaPCK) (SEQ ID NO: 96), PCK derived from Debaryomyces hansenii (DhaPCK) (SEQ ID NO: 97), PCK derived from Escherichia fergusonii (EfePCK) (SEQ ID NO: 98), PCK derived from Edwardsiella tarda (EtaPCK) (SEQ ID NO: 99), PCK derived from Kluyveromyces lactis (KlaPCK) (SEQ ID NO: 100), PCK derived from Lodderomyces elongisporus (LelPCK) (SEQ ID NO: 101), PCK derived from Pectobacterium carotovorum (PcaPCK) (SEQ ID NO: 102), PCK derived from Photobacterium leiognathi (PlePCK) (SEQ ID NO: 103), PCK derived from Providencia rettgeri (PrePCK) (SEQ ID NO: 104), PCK derived from Saccharomyces cerevisiae (ScePCK) (SEQ ID NO: 105), PCK derived from Serratia odorifera (SodPCK) (SEQ ID NO: 106), PCK derived from Vibrio orientalis (VorPCK) (SEQ ID NO: 107), PCK derived from Vanderwaltozyma polyspora (VpoPCK) (SEQ ID NO: 108), PCK derived from Vibrio tubiashii (VtuPCK) (SEQ ID NO: 109), PCK derived from Yersinia bercovieri (YbePCK) (SEQ ID NO: 110), PCK derived from Yarrowia lipolytica (YliPCK) (SEQ ID NO: 111), PCK derived from Yersinia rohdei (YroPCK) (SEQ ID NO: 112), or PCK derived from Zygosaccharomyces rouxii (ZroPCK) (SEQ ID NO: 113) was introduced.

Specifically, the IGF836 strain (gene deletion strain of S. pombe) prepared in Example 1 was transformed according to the method of Bahler et al. (the journal of Yeast, 1998, Vol. 14, pp. 943-951) by using a digest of a restriction enzyme BsiWI of each monodentate integrative recombinant vector retaining each expression cassette of the PCK gene described in Table 47.

pSLh-CglPCK was prepared through the following process. First, by using total genomic DNA prepared by DNeasy (manufactured by QUIAGEN) from the culture of Candida glabrata as a template and using 2 kinds of synthetic oligo DNA (CglPCK-F and CglPCK-R, manufactured by Operon Biotechnologies) described in Table 27, an ORF fragment of a CglPCK gene was obtained by a PCR method using KOD-Dash (manufactured by TOYOBO CO., LTD.). The ORF fragment encoded CglPCK (SEQ ID NO: 94).

**[Table 27]**

| | Sequence | SEQ ID NO: |
|---|---|---|
| CglPCK-F | | 114 |
| CglPCK-R | 5'-GAAATCAACTTTTGTTCCAATTGTGGACCAGCAGCCAA-3' | 115 |
| CglPCK | | 94 |

By the same method as described above, a monodentate integrative recombinant vector pSLh-CkoPCK, a monodentate integrative recombinant vector pSLh-CsaPCK, a monodentate integrative recombinant vector pSLh-DhaPCK, a monodentate integrative recombinant vector pSLh-EfePCK, a monodentate integrative recombinant vector pSLh-EtaPCK, a monodentate integrative recombinant vector pSLh-KlaPCK, a monodentate integrative recombinant vector pSLh-LelPCK, a monodentate integrative recombinant vector pSLh-PcaPCK, a monodentate integrative recombinant vector pSLh-PlePCK, a monodentate integrative recombinant vector pSLh-PrePCK, a monodentate integrative recombinant vector pSLh-ScePCK, a monodentate integrative recombinant vector pSLh-SodPCK, a monodentate integrative recombinant vector pSLh-VorPCK, a monodentate integrative recombinant vector pSLh-VpoPCK, a monodentate integrative recombinant vector pSLh-VtuPCK, a monodentate integrative recombinant vector pSLh-YbePCK, a monodentate integrative recombinant vector pSMh-YliPCK, a monodentate integrative recombinant vector pSMh-YroPCK, and a monodentate integrative recombinant vector pSMh-ZroPCK were prepared. Primer sets using the respective vectors will be listed below.

**[Table 28]**

| | Sequence | SEQ ID NO: |
|---|---|---|
| CkoPCK-F | 5'-GACACTTTTTCAAAATGCGCGTTAACAGCTTAACCC-3' | 116 |
| CkoPCK-R | 5'-GAAATCAACTTTTGTTCCAGCTTCGGCCCGGC-3' | 117 |
| CkoPGK | | 95 |

**[Table 29]**

| | Sequence | SEQ ID NO. |
|---|---|---|
| CsaPCK-F | 5'**-**GACACTTTTTCAAAATGCGAGTTACAGGCATAACCC-3' | 118 |
| CsaPCK-R | 5'-GAAATCAACTTTTGTTCGCGCTGTGGGCCTGC-3' | 119 |
| CsaPCK | | 96 |

**[Table 30]**

| | Sequence | SEQ ID NO: |
|---|---|---|
| DhaPCK-F | 5'-GACACTTTTTCAAAATGACACCTCCTACTGCTGTTGA-3' | 120 |
| DhaPCK-R | 5'-GAAATCAACTTTTGTTCAGCATCAGGACCAGCAGC-3' | 121 |
| PhaPCK | | 97 |

**[Table 31]**

| | Sequence | SEQ ID NO: |
|---|---|---|
| EfePCK-F | 5'-GACACTTTTTCAAAATGCGCGTGAACAAAAGTTTAACC-3' | 122 |
| EfePCK-R | | 123 |
| EfePCK | | 98 |

**[Table 32]**

| | Sequence | SEQ ID NO: |
|---|---|---|
| EtaPCK-F | 5'-GACACTTTTTTCAAAATGCATGCCACTGGCTTAACT-3' | 124 |
| EtaPCK-R | 5'-GAAATCAACTTTTGTTCACGTTGTGGGCCGGCA-3' | 125 |
| EtaPCK | | 99 |

**[Table 33]**

| | Sequence | SEQ ID NO: |
|---|---|---|
| KlaPCK-F | | 126 |
| KlaPCK-R | | 127 |
| KlaPCK | | 100 |

**[Table 34]**

| | Sequence | SEQ ID NO: |
|---|---|---|
| LelPCK-F | 5'-GACACTTTTTCAAAATGGCACCCCCAGCAGTA -3' | 128 |
| LelPGK-R | 5'-GAAATCAACTTTTGTTCAACATCGGGTCCAGCAGCT -3' | 129 |
| LelPCK | | 101 |

**[Table 35]**

| | Sequence | SEQ ID NO: |
|---|---|---|
| PcaPCK-F | 5'-GACACTTTTTCAAAATGCAGATCAACGGTATTACCCC-3' | 130 |
| PcaPCK-R | 5'-GAAATCAACTTTTGTTCGCGCTTCGGCCCTGC-3' | 131 |
| PcaPCK | | 102 |

**[Table 36]**

| | Sequence | SEQ ID NO: |
|---|---|---|
| PlePCK-F | | 132 |
| PlePCK-R | | 133 |
| PlePCK | | 103 |

**[Table 37]**

| | Sequence | SEQ ID NO: |
|---|---|---|
| PrePCK-F | 5'-GACACTTTTTCAAAATGAGCGCTAAAAGCATTACCCT-3' | 134 |
| PrePCK-R | 5'-GAAATCAACTTTTGTTCCAGTTTTGGCCCTGCTTTCAC-3' | 135 |
| PrePCK | | 104 |

**[Table 38]**

| | Sequence | SEQ ID NO: |
|---|---|---|
| ScePCK-F | | 136 |
| ScePCK-R | 5'- GAAATCAACTTTTGTTCCTCGAATTGAGGACCAGCGGC -3' | 137 |
| ScePCK | | 105 |

**[Table 39]**

| | Sequence | SEQ ID NO: |
|---|---|---|
| SodPCK-F | 5'-GACACTTTTTCAAAATGCGTGCTAAAGGTATCACCC-3' | 138 |
| SodPCK-R | 5'-GAAATCAACTTTTGTTCCAGCTTCGGCCGGCG-3' | 139 |
| SodPCK | | 106 |

**[Table 40]**

| | Sequence | SEQ ID NO: |
|---|---|---|
| VorPCK-F | | 140 |
| VorPCK-R | 5'- GAAATCAACTTTTGTTCATCTAGCTGAGGACCTGCAGC -3' | 141 |
| vorPCK | | 107 |

**[Table 41]**

| | Sequence | SEQ ID NO: |
|---|---|---|
| VpoPCK-F | | 142 |
| VpoPCK-R | 5'- GAAATCAACTTTTGTTCTAGGGCTGGACCAGCTGC -3' | 143 |
| VpoPCK | | 108 |

**[Table 42]**

| | Sequence | SEQ ID NO: |
|---|---|---|
| VtuPCK-F | | 144 |
| VtuPCK-R | 5'-GAAATCAACTTTTGTTCATCTAGCTGAGGTCCAGCCG-3' | 145 |
| VtuPCK | | 109 |

**[Table 43]**

| | Sequence | SEQ ID NO: |
|---|---|---|
| YbePCK-F | | 146 |
| YbePCK-R | 5' - GAAATCAACTTTTGTTCGATCTTCGGCCCTGCGCT -3' | 147 |
| YbePCK | | 110 |

**[Table 44]**

| | Sequence | SEQ ID NO: |
|---|---|---|
| YliPCK-F | 5'-GACACTTTTTCAAAATGTCTCCCCCCGTCAACC-3' | 148 |
| YliPCK-R | 5'-GAAATCAACTTTTGTTCAGCCTTAGGGCCGGCA-3' | 149 |
| YliPCK | | 111 |

**[Table 45]**

| | Sequence | SEQ ID NO: |
|---|---|---|
| YroPCK-F | | 150 |
| YroPCK-R | 5'-GAAATCAACTTTTGTTCGACTTTTGGCCCAGCACTGA-3' | 151 |
| YroPCK | | 112 |

**[Table 46]**

| | Sequence | SEQ ID NO: |
|---|---|---|
| ZroPCK-F | 5'-GACACTTTTTCAAAATGTCGCCCTCCAAAGTTCC-3' | 152 |
| ZroPCK-R | | 153 |
| ZroPCK | | 113 |

**[Table 47]**

| Name of transformant strain | Name of host strain | Name of introduced foreign genes |
|---|---|---|
| 22 | IGF836 | CgIPCK |
| 23 | IGF836 | CkoPCK |
| 24 | IGF836 | CsaPCK |
| 25 | IGF836 | DhaPCK |
| 26 | IGF836 | EfePC K |
| 27 | IGF836 | EtaPCK |
| 28 | IGF836 | KlePCK |
| 29 | IGF836 | LeIPCK |
| 30 | IGF836 | PoaPCK |
| 31 | IGF836 | PlePCK |
| 32 | IGF836 | PrePCK |
| 33 | IGF836 | ScePCK |
| 34 | IGF836 | SodPCK |
| 35 | IGF836 | VorPCK |
| 36 | IGF836 | VpoPCK |
| 37 | IGF836 | VtuPCK |
| 38 | IGF836 | YbePCK |
| 39 | IGF836 | YIIPCK |
| 40 | IQF836 | YroPCK |
| 41 | IGF836 | ZroPCK |

### <Confirmation of PCK expression>

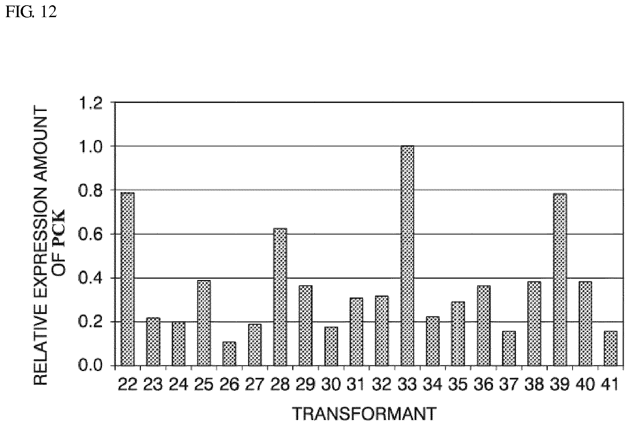

For 20 kinds of transformant into which only the PCK gene was introduced, an expression amount of PCK was confirmed. Specifically, each of the transformants was shake-cultured for 44 hours in 20 mL of a YPD6 medium, and the grown microbial cells were then collected and pulverized using a multibead shocker. The obtained microbial cell lysate was purified according to a protocol by using ANTI-FLAG (registered trademark) M1 Agarose Affinity Gel (manufactured by Sigma-Aldrich Co. LLC.). SDS-PAGE was performed using a purified enzyme liquid, and the detected band was digitized using an image analysis device LAS 4000, thereby calculating a relative expression amount. The results of the PCK expression amount (relative value) calculated for each of the transformants are shown in FIG. 12. The results show that the expression of PCK was confirmed in all of the transformants.

### [Example 6] Preparation of EcoPCK introduction strain, GglPYC introduction strain, and EcoMDH introduction strain

Transformants of pombe were prepared into which PCK derived from Escherichia coli (EcoPCK) (SEQ ID NO: 154), PYC derived from Gallus gallus (GglPYC) (SEQ ID NO: 155), and MDH derived from Escherichia coli (EcoMDH) (SEQ ID NO: 156) were introduced.

Specifically, the IGF836 strain (gene deletion strain of S. pombe) prepared in Example 1 was transformed according to the method of Bahler et al. (the journal of Yeast, 1998, Vol. 14, pp. 943-951) by using a digest of a restriction enzyme BsiWI of a monodentate integrative recombinant vector pSNh-EcoPCK retaining an EcoPCK gene expression cassette, a monodentate integrative recombinant vector pSLh-GglPYC retaining a GglPYC gene expression cassette, or a monodentate integrative recombinant vector pSMh-EcoMDH retaining an EcoMDH gene expression cassette.

pSNh-EcoPCK was prepared through the following process. First, by using total genomic DNA prepared by DNeasy (manufactured by QUIAGEN) from the culture of Escherichia coli as a template and using 2 kinds of synthetic oligo DNA (EcoPCK-F and EcoPCK-R, manufactured by Operon Biotechnologies) described in Table 48, an ORF fragment of an EcoPCK gene was obtained by a PCR method using KOD-Dash (manufactured by TOYOBO CO., LTD.). The ORF fragment encoded EcoPCK (SEQ ID NO: 154).

**[Table 48]**

| | Sequence | SEQ ID NO: |
|---|---|---|
| EcoPCK-F | | 157 |
| EcoPCK-R | 5'-GAAATCAACTTTTGTTCCAGCTTCGGACCAGC-3' | 158 |
| EcoPCK | | 154 |

By using an In-Fusion (registered trademark) HD Cloning Kit (manufactured by Clontech Laboratories, Inc.), the obtained amplified fragment was incorporated into pSNh, thereby preparing pSNh-EcoPCK. The In-Fusion method was performed according to the manual included in the kit. That is, the obtained PCR product was purified using a spin column, added to an In-Fusion reaction solution together with pSNh, and reacted for 15 minutes at 50°C.

By the same method as described above, a monodentate integrative recombinant vector pSLh-GalPYC and a monodentate integrative recombinant vector pSMh-EcoMDH were prepared. Primer sets using the respective vectors will be listed below.

**[Table 49]**

| | Sequence | SEQ ID NO: |
|---|---|---|
| GglPYC F | 5'-CATCTTTTTCAAACATGTCGCAGCTGTGTGTCCC-3' | 159 |
| GglPYC-R | | 160 |
| GglPYC | | 155 |

**[Table 50]**

| | Sequence | SEQ ID NO: |
|---|---|---|
| EcoMDH-F | 5'-ACTTTTTCAAACATGAAAGTCGCAGTCCTCGG-3' | 161 |
| EcoMDH-R | | 162 |
| EcoMDH | | 156 |

The name and host of each of the manufactured transformants and the name of the introduced foreign genes are shown in Table 51.

**[Table 51]**

| Name of transformant strain | Name of host strain | Introduced foreign genes |
|---|---|---|
| 42 | IGF836 | EcoPCK |
| 43 | IGF836 | GglPYC |
| 44 | IGF836 | EcoMDH |

### <Confirmation of expression of PCK, PYC, or MDH>

For 3 kinds of transformant into which only the PCK gene, the PYC gene, or the MDH gene was introduced, an expression amount of PCK, PYC, or MDH was confirmed. Specifically, each of the transformants was shake-cultured for 44 hours in 20 mL of a YPD6 medium, and the grown microbial cells were then collected and pulverized using a multibead shocker. The obtained microbial cell lysate was purified according to a protocol by using ANTI-FLAG (registered trademark) M1 Agarose Affinity Gel (manufactured by Sigma-Aldrich Co. LLC.). SDS-PAGE was performed using a purified enzyme liquid. As a result, no band was detected in any of the enzyme liquids. This result shows that even if the EcoPCK gene, the GglPYC gene, or the EcoMDH gene is introduced into S. pombe, these enzymes are not expressed, and hence effective activity is not obtained.

### [Example 7] Preparation of malic acid production strain

According the method described in Examples 2, 3, and 5, transformants shown in Table 52 were prepared.

**[Table 52]**

| Name of transformant strain | Name of host strain | Name of introduced foreign genes or name of deleted genes |
|---|---|---|
| ASP5127 | IGF836 | Δ mae2 |
| ASP5125 | IGF836 | SoePYC, Δ mae2 |
| ASP5126 | IGF836 | DacMDH, Δ mae2 |
| ASP5087 | IGF836 | SoePCK, DacMDH, Δ mae2 |
| AS P5088 | IGF836 | PlePCK, DacMDH, Δ mae2 |
| ASP5089 | IG F836 | YbePCK, DacMDH, Δ mae2 |
| ASP5132 | IGF836 | KlaPYC, DacMDH, Δ mae2 |
| ASP5135 | IGF836 | SpoPYC, DacMDH, Δ mae2 |
| ASP5215 | IGF836 | CliMDH, SoePYC, Δ mae2 |
| ASP5216 | IGF836 | HelMDH, ScePYC, Δ mae2 |
| ASP5217 | IGF836 | SpuMDH, ScePYC, Δ mae2 |
| ASP5129 | IGF836 | CliMDH, ScePYC, Δ mae2 |
| ASP5131 | IGF836 | SpuMDH, ScePYC, Δ mae2 |
| ASP5235 | IGF836 | ScePYC, LelPYC, DacMDH, HelMDH, Δ mae2 |

### [Example 8] Confirmation of effect resulting from deletion of mae2

In order to confirm the effect resulting from the deletion of mae2, fermentative production of malic acid was performed using the ASP4491 strain, the ASP4964 strain, the ASP4933 strain, and the ASP5127 strain.

Specifically, the microbial cells were seeded into a YES plate and cultured for 96 hours at 30°C, thereby obtaining a colony. The obtained colony was subcultured in 5 mL of a YES medium (test tube) and shake-cultured for 24 hours at 30°C. The obtained microbial solution was subcultured in 100 mL of a YPD6 medium (Sakaguchi flask) and shake-cultured for 44 hours at 30°C.

The microbial cells obtained in this way were collected, added to 3 mL of a fermentation medium (100 g/L glucose, 111 g/L calcium carbonate) in the test tube so as to yield 36 g (weight of dry microbial cell)/L, and fermented at 30°C under shaking conditions. A sample was collected in time from the fermented liquid.

For the obtained sample, a glucose concentration and an ethanol concentration were measured using a biosensor BF-7 (manufactured by Oji Scientific Instruments) based on an enzyme electrode method, and a malic acid concentration was measured by HPLC. During the HPLC measurement, a high-performance liquid chromatograph Prominence (manufactured by Shimadzu Corporation) was used, Aminex HPX-87H 300 × 7.8 mm (manufactured by Bio-Rad Laboratories, Inc.) was used as a column, an injection volume was set to be 10 µL, 10 mM H₂SO₄ was used as a solvent, a flow rate was set to be 0.6 mL/min, a measurement time was set to be 35 minutes, a measurement temperature was set to be 60°C, and diode array detector (210 nm) and a differential refractometric detector were used for detection. Each concentration is a concentration per culture solution or fermented liquid. The results are shown in Table 53.

**[Table 53]**

| Strain name | Fermentation time [hour] | Glucose concentration [g/L] | Ethanol concentration [g/L] | Malie acid concentration [g/L] |
|---|---|---|---|---|
| ASP4491 | 0 | 100.0 | 0.0 | 0.0 |
| | 1 | 33,2 | 21.1 | 2.9 |
| | 3 | 0.0 | 34.3 | 0.0 |
| | 20 | 0.0 | 24.7 | 0.0 |
| ASP4964 | 0 | 1 00.0 | 0.0 | 0.0 |
| | 1 | 26.6 | 13.8 | 19.0 |
| | 3 | 0.4 | 19.2 | 26.7 |
| | 20 | 0.3 | 8.0 | 28.1 |
| ASP4933 | 0 | 100.0 | 0.0 | 0.0 |
| | 1 | 57.9 | 10.6 | 0.0 |
| | 3 | 11.8 | 27.2 | 3.0 |
| | 20 | 0.0 | 23.4 | 0.0 |
| ASP5127 | 0 | 100.0 | 0.0 | 0.0 |
| | 1 | 27.0 | 23.3 | 2.0 |
| | 2 | 0.1 | 32.9 | 3.0 |
| | 31 | 0.1 | 0.0 | 5.3 |

As a result, in the ASP4964 strain from which mae2 was deleted, the amount of malic acid produced further increased by about 10 fold than in the ASP4491 strain. In contrast, in the ASP4933 strain obtained by deleting fum1, which uses malic acid as a matrix just like mae2, from the ASP4491 strain, the amount of malic acid produced practically did not increase. Furthermore, in the ASP5127 strain from which only mae2 and pdc2 were deleted and into which PYC and MDH were not introduced, the production of malic acid in an amount of about 5 g/L was confirmed.

### [Example 9] Confirmation of effect resulting from introduction of PCK

In order to confirm the effect resulting from the introduction of PCK, fermentative production of malic acid was performed using the ASP5126 strain, the ASP5087 strain, the ASP5088 strain, and the ASP5089 strain.

The fermentative production of malic acid was performed in the same manner as in Example 7. The results are shown in Table 54.

**[Table 54]**

| Strain name | Fermentation time [hour] | Glucose concentration [g/L] | Ethanol concentration [g/L] | Malic acid concentration [g/L] |
|---|---|---|---|---|
| ASP5126 | 0 | 100.0 | 0.0 | 0.0 |
| | 1 | 36.8 | 19.2 | 12.5 |
| | 2 | 3.5 | 30.8 | 19.5 |
| | 31 | 0.0 | 0.0 | 22.2 |
| ASP5087 | 0 | 100.0 | 0.0 | 0.0 |
| | 1 | 78.6 | 5.8 | 8.7 |
| | 2 | 45.9 | 12.9 | 18.5 |
| | 4 | 2.7 | 22.8 | 31.1 |
| ASP5088 | 0 | 100.0 | 0.0 | 0.0 |
| | 1 | 44.8 | 9.7 | 13.3 |
| | 3 | 0.5 | 20.4 | 22.2 |
| | 20 | 0.3 | 2.0 | 23.6 |
| ASP5089 | 0 | 100.0 | 0.0 | 0.0 |
| | 1 | 38.3 | 11.7 | 13.6 |
| | 3 | 0.3 | 20.6 | 20.4 |
| | 20 | 0.3 | 4.3 | 21.3 |

It was confirmed that in the ASP5087 strain into which PCK derived from Saccharomyces cerevisiae (ScePCK) was introduced, the ASP5088 strain into which PCK derived from Photobacterium leiognathi (PlePCK) was introduced, and the ASP5089 strain into which PCK derived from Yersinia bercovieri (YbePCK) was introduced, a malic acid production rate and a final concentration of malic acid were further improved than in the ASP5126 strain into which none of PCK and PYC were introduced.

### [Example 10] Confirmation of effect resulting from introduction of PYC

In order to confirm the effect resulting from the introduction of PYC, fermentative production of malic acid was performed using the ASP5126 strain, the ASP4964 strain, the ASP5132 strain, and the ASP5135 strain.

The fermentative production of malic acid was performed in the same manner as in Example 7. The results are shown in Table 55.

**[Table 55]**

| Strain name | Fermentation time [hour] | Glucose concentration [g/L] | Ethanol concentration [g/L] | Malic acid concentration [g/L] |
|---|---|---|---|---|
| ASP5126 | 0 | 100.0 | 0.0 | 0.0 |
| | 1 | 36.8 | 19.2 | 12.5 |
| | 2 | 3.5 | 30.8 | 19.5 |
| | 31 | 0.0 | 0.0 | 22.2 |
| ASP4964 | 0 | 100.0 | 0.0 | |
| | 1 | 20.6 | 14.1 | 26.9 |
| | 2 | 0.1 | 18.5 | 35.3 |
| | 4 | 0.0 | 14.6 | 35.1 |
| ASP5132 | 0 | 100.0 | 0.0 | 0.0 |
| | 1 | 25.9 | 8.6 | 29.7 |
| | 2 | 0.0 | 15.9 | 42.8 |
| | 4 | 0.0 | 12.0 | 42.8 |
| ASP5135 | 0 | 100.0 | 0.0 | 0.0 |
| | 1 | 41.6 | 6.5 | 27.8 |
| | 2 | 9.6 | 13.0 | 50.4 |
| | 4 | 0.4 | 14.0 | 54.5 |

It was confirmed that in the ASP4964 strain into which PYC derived from Saccharomyces cerevisiae was introduced, the ASP5132 strain into which PYC derived from Kluyveromyces lactis was introduced (KlaPYC), and the ASP5135 strain into which PYC derived from S. pombe (SpoPYC) was introduced, a malic acid production rate and a final concentration of malic acid were further improved than in the ASP5126 strain into which none of PCK and PYC were introduced.

### [Example 11] Confirmation of effect resulting from introduction of MDH

In order to confirm the effect resulting from the introduction of MDH, fermentative production of malic acid was performed using the ASP5125 strain, the ASP5215 strain, the ASP5216 strain, the ASP5127 strain, the ASP4964 strain, the ASP5129 strain, and the ASP5131 strain.

The fermentative production of malic acid was performed in the same manner as in Example 7. The results are shown in Table 56.

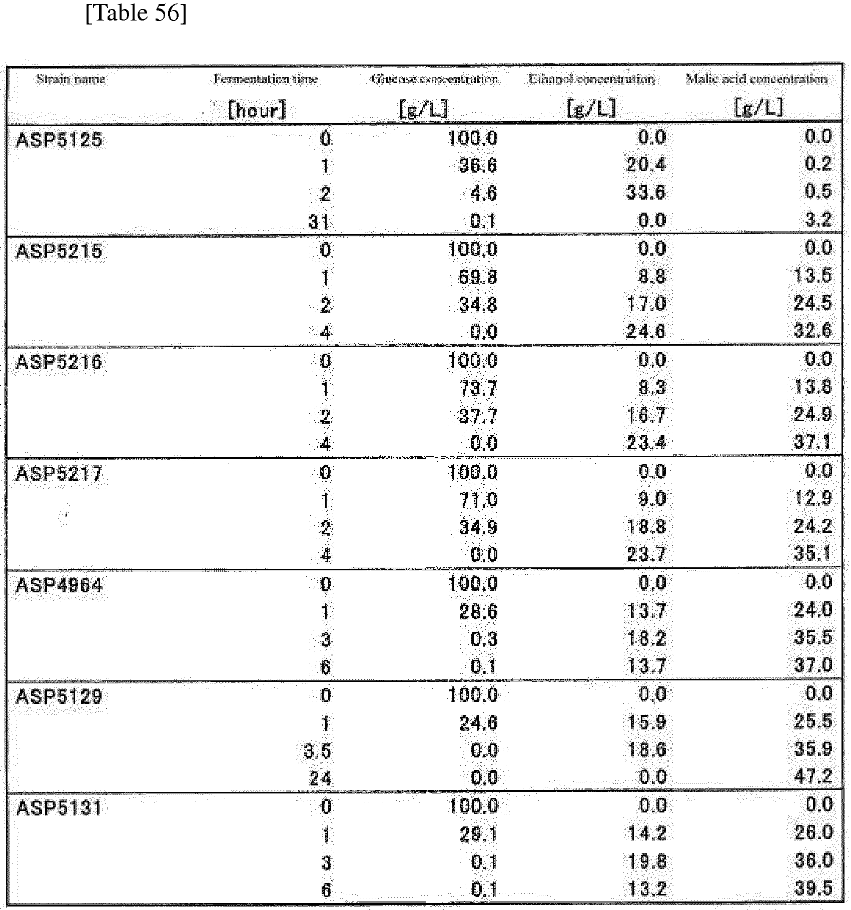

It was confirmed that in the ASP5215 strain into which CliMDH and PCK were introduced, the ASP5216 strain into which HelMDH and PCK were introduced, the ASP5217 strain into which SpuMDH and PCK were introduced, the ASP4964 strain into which HelMDH and PYC were introduced, the ASP5129 strain into which CliMDH and PYC were introduced, and the ASP5131 strain into which SpuMDH and PYC were introduced, a malic acid production rate and a final concentration of malic acid were further improved than in the ASP5125 strain into which MDH was not introduced but PYC was introduced.

### [Example 12] Confirmation of effect resulting from increasing number of copies of introduced genes

In order to confirm the effect resulting from increasing the number of copies of the introduced genes, fermentative production of malic acid was performed using the ASP4964 strain and the ASP5235 strain.

The fermentative production of malic acid was performed in the same manner as in Example 7. The results are shown in Table 57.

**[Table 57]**

| Strain name | Fermentation time [hour] | Glucose concentration [g/L] | Ethanol concentration [g/L] | Malic acid concentration [g/L] |
|---|---|---|---|---|
| ASP4964 | 0 | 100.0 | 0.0 | 0.0 |
| | 1 | 35.5 | 15.0 | 22.2 |
| | 2 | 0.0 | 22.6 | 36.9 |
| | 4 | 0.0 | 19.1 | 38.4 |
| ASP5235 | 0 | 100.0 | 0.0 | 0.0 |
| | 1 | 49.7 | | 28.4 |
| | 2 | 3.7 | 19.4 | 53.6 |
| | 4 | 0.0 | 17.7 | 56.9 |

It was confirmed that in the ASP5235 strain into which 2 copies of PYC and 2 copies of MDH were introduced, a malic acid production rate and a final concentration of malic acid were further improved than in the ASP4964 strain into which one copy of PYC and one copy of MDH were introduced.

### [Example 13] Fermentative production of malic acid under non-neutralization condition

In order to confirm the production of malic acid under non-neutralization conditions, fermentative production of malic acid was performed using the ASP4964 strain and the ASP5235 strain.

Specifically, microbial cells grown by the same method as in Example 7 were added to 3 mL of a fermentation medium (100 g/L glucose) in a test tube so as to yield 36 g (weight of dry microbial cells)/L, and fermented at 30°C under shaking conditions. A sample was collected in time from the fermented liquid.

The obtained sample was measured in the same manner as in Example 7. The results are shown in Table 58. As a result, it was confirmed that all of the strains produced malic acid under non-neutralization conditions. The pH of the ASP4964 strain sample that was collected after fermentation was performed for 1 hour was 3.5, and the pH of the sample that was collected after fermentation was performed for 2 hours was 3.0. Furthermore, the pH of the ASP5235 strain sample that was collected after fermentation was performed for 1 hour was 3.0, and the pH of the sample that was collected after fermentation was performed for 2 hours was 2.8.

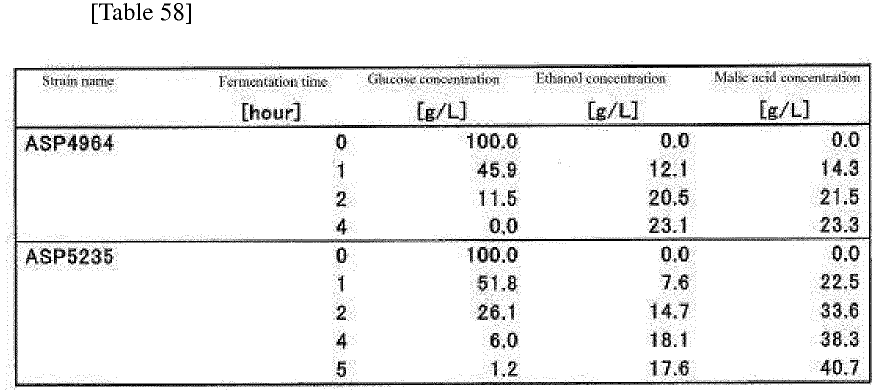

Priority is claimed on Japanese Patent Application No. 2014-135043, filed on June 30, 2014.

## Claims

1. A transformant comprising one or more foreign genes of one or more kinds selected from the group consisting of a phosphoenolpyruvate carboxykinase gene and a pyruvate carboxylase gene which are incorporated into a chromosome of *Schizosaccharomyces pombe* as a host,
wherein a gene encoding pyruvate decarboxylase 2 in the host has been deleted or deactivated,
the phosphoenolpyruvate carboxykinase gene encodes a polypeptide which includes an amino acid sequence represented by any of SEQ ID NOS: 94 to 113, or a polypeptide which includes an amino acid sequence sharing a sequence identity of equal to or higher than 80% with an amino acid sequence represented by any of SEQ ID NOS: 94 to 113 and has phosphoenolpyruvate carboxykinase activity,
the pyruvate carboxylase gene encodes a polypeptide which includes an amino acid sequence represented by any of SEQ ID NOS: 1 to 16, or a polypeptide which includes an amino acid sequence sharing a sequence identity of equal to or higher than 80% with an amino acid sequence represented by any of SEQ ID NOS: 1 to 16 and has pyruvate carboxylase activity, and
a gene encoding malic enzyme 2 in the host has further been deleted or deactivated.

2. The transformant according to claim 1, wherein one or more foreign malate dehydrogenase genes are further incorporated therein.

3. The transformant according to claim 2, wherein the malate dehydrogenase genes encode a polypeptide which includes an amino acid sequence represented by any of SEQ ID NOS: 17 to 21, or a polypeptide which includes an amino acid sequence sharing a sequence identity of equal to or higher than 80% with an amino acid sequence represented by any of SEQ ID NOS: 17 to 21 and has malate dehydrogenase activity.

4. The transformant according to claim 1, wherein one or more foreign phosphoenolpyruvate carboxykinase genes or one or more foreign pyruvate carboxylase genes and one or more foreign malate dehydrogenase genes are incorporated into the chromosome of the host.

5. A method for manufacturing a dicarboxylic acid having 4 carbon atoms, comprising:
culturing the transformant according to any one of claims 1 to 4 in a culture solution; and
obtaining a dicarboxylic acid having 4 carbon atoms from the cultured transformant or a culture supernatant.

6. The method for manufacturing a dicarboxylic acid having 4 carbon atoms according to claim 5, wherein the dicarboxylic acid having 4 carbon atoms is malic acid or oxaloacetic acid.

7. The method for manufacturing a dicarboxylic acid having 4 carbon atoms according to claim 5 or 6, wherein the culturing is continued even after a pH of the culture solution becomes equal to or less than 3.5.

## Patentansprüche

1. Transformante, umfassend ein oder mehrere Fremdgene einer oder mehrerer Arten, die aus der Gruppe bestehend aus einem Phosphoenolpyruvatcarboxykinase-Gen und einem Pyruvatcarboxylase-Gen ausgewählt sind, die in ein Chromosom von *Schizosaccharomyces pombe* als Wirt eingebaut sind,
wobei ein Gen, das für Pyruvatdecarboxylase 2 codiert, im Wirt deletiert oder deaktiviert wurde,
das Phosphoenolpyruvatcarboxykinase-Gen für ein Polypeptid codiert, das eine Aminosäuresequenz umfasst, die durch eine beliebige von SEQ ID NOS: 94 bis 113 dargestellt wird, oder ein Polypeptid, das eine Aminosäuresequenz umfasst, die sich eine Sequenzidentität gleich oder über 80 % mit einer Aminosäuresequenz teilt, die durch eine beliebige von SEQ ID NOS: 94 bis 113 dargestellt wird, und Phosphoenolpyruvatcarboxykinase-Aktivität aufweist,
das Pyruvatcarboxylase-Gen für ein Polypeptid codiert, das eine Aminosäuresequenz umfasst, die durch eine beliebige von SEQ ID NOS: 1 bis 16 dargestellt wird, oder ein Polypeptid, das eine Aminosäuresequenz umfasst, die sich eine Sequenzidentität gleich oder über 80 % mit einer Aminosäuresequenz teilt, die durch eine beliebige von SEQ ID NOS: 1 bis 16 dargestellt wird, und Pyruvatcarboxylase-Aktivität aufweist, und
ferner ein Gen, das für Malatenzym 2 codiert, im Wirt deletiert oder deaktiviert wurde.

2. Transformante nach Anspruch 1, wobei ferner ein oder mehrere fremde Malatdehydrogenase-Gene darin eingebaut sind.

3. Transformante nach Anspruch 2, wobei die Malatdehydrogenase-Gene für ein Polypeptid codieren, das eine Aminosäuresequenz umfasst, die durch eine beliebige von SEQ ID NOS: 17 bis 21 dargestellt wird, oder ein Polypeptid, das eine Aminosäuresequenz umfasst, die sich eine Sequenzidentität gleich oder über 80 % mit einer Aminosäuresequenz teilt, die durch eine beliebige von SEQ ID NOS: 17 bis 21 dargestellt wird, und Malatdehydrogenase-Aktivität aufweist.

4. Transformante nach Anspruch 1, wobei ein oder mehrere fremde Phosphoenolpyruvatcarboxykinase-Gene oder ein oder mehrere fremde Pyruvatcarboxylase-Gene und ein oder mehrere fremde Malatdehydrogenase-Gene in das Chromosom des Wirts eingebaut sind.

5. Verfahren zur Herstellung einer Dicarbonsäure mit 4 Kohlenstoffatomen, umfassend:
Kultivieren der Transformanten nach einem der Ansprüche 1 bis 4 in einer Kulturlösung; und
Erhalten einer Dicarbonsäure mit 4 Kohlenstoffatomen aus der kultivierten Transformanten oder einem Kulturüberstand.

6. Verfahren zur Herstellung einer Dicarbonsäure mit 4 Kohlenstoffatomen nach Anspruch 4, wobei es sich bei der Dicarbonsäure mit 4 Kohlenstoffatomen um Apfelsäure oder Oxalessigsäure handelt.

7. Verfahren zur Herstellung einer Dicarbonsäure mit 4 Kohlenstoffatomen nach Anspruch 5 oder 6, wobei das Kultivieren auch fortgesetzt wird, nachdem ein pH der Kulturlösung gleich wie oder weniger als 3,5 wird.

## Revendications

1. Transformant comprenant un ou plusieurs gènes étrangers d'un ou plusieurs types sélectionnés dans le groupe consistant en un gène de la phosphoénolpyruvate carboxykinase et un gène de la pyruvate carboxylase qui sont incorporés dans un chromosome de *Schizosaccharomyces pombe* en tant qu'hôte,
dans lequel un gène codant pour la pyruvate décarboxylase 2 chez l'hôte a été supprimé ou désactivé,
le gène de la phosphoénolpyruvate carboxykinase code pour un polypeptide qui comprend une séquence d'acides aminés représentée par l'une quelconque de SEQ ID NO: 94 à 113, ou un polypeptide qui comprend une séquence d'acides aminés partageant une identité de séquence supérieure ou égale à 80 % avec une séquence d'acides aminés représentée par l'une quelconque de SEQ ID NO: 94 à 113 et qui possède une activité phosphoénolpyruvate carboxykinase,
le gène de la pyruvate carboxylase code pour un polypeptide qui comprend une séquence d'acides aminés représentée par l'une quelconque de SEQ ID NO: 1 à 16, ou un polypeptide qui comprend une séquence d'acides aminés partageant une identité de séquence supérieure ou égale à 80 % avec une séquence d'acides aminés représentée par l'une quelconque de SEQ ID NO: 1 à 16 et qui possède une activité pyruvate carboxylase, et
un gène codant pour l'enzyme malique 2 chez l'hôte a en outre été supprimé ou désactivé.

2. Transformant selon la revendication 1, dans lequel un ou plusieurs gènes de la malate déshydrogénase étrangers sont en outre incorporés dans celui-ci.

3. Transformant selon la revendication 2, dans les gènes de la malate déshydrogénase codent pour un polypeptide qui comprend une séquence d'acides aminés représentée par l'une quelconque de SEQ ID NO: 17 à 21, ou un polypeptide qui comprend une séquence d'acides aminés partageant une identité de séquence supérieure ou égale à 80 % avec une séquence d'acides aminés représentée par l'une quelconque de SEQ ID NO: 17 à 21 et qui possède une activité malate déshydrogénase.

4. Transformant selon la revendication 1, dans lequel un ou plusieurs gènes de la phosphoénolpyruvate carboxykinase étrangers ou un ou plusieurs gènes de la pyruvate carboxylase étrangers et un ou plusieurs gènes de la malate déshydrogénase étrangers sont incorporés dans le chromosome de l'hôte.

5. Procédé de fabrication d'un acide dicarboxylique comportant 4 atomes de carbone, comprenant :
la mise en culture du transformant selon l'une quelconque des revendications 1 à 4 dans une solution de culture ; et
l'obtention d'un acide dicarboxylique comportant 4 atomes de carbone à partir du transformant en culture ou d'un surnageant de culture.

6. Procédé de fabrication d'un acide dicarboxylique comportant 4 atomes de carbone selon la revendication 5, dans lequel l'acide dicarboxylique comportant 4 atomes de carbone est l'acide malique ou l'acide oxaloacétique.

7. Procédé de fabrication d'un acide dicarboxylique comportant 4 atomes de carbone selon la revendication 5 ou 6, dans lequel la mise en culture est poursuivie même après que le pH de la solution de culture devienne inférieur ou égal à 3,5.
